# EUROPEAN PATENT APPLICATION

(11) **EP 4 851 170 A1**
(43) Date of publication of application: **09.12.2026**
(21) Application number: 24921813.2
(22) Date of filing: 27.12.2024
(51) Int. Cl.: C07D 519/00, C07D 487/04, A61K 31/519, A61K 31/5383, A61P 35/00

(54) **SPIRO DERIVATIVE SERVING AS WRN INHIBITOR AND USE OF SPIRO DERIVATIVE**

(30) Priority: 01.02.2024 CN 202410144913; 21.05.2024 CN 202410630927
(71) Applicant: Foresight Therapeutics (Hefei) Co., Ltd., Hefei, Anhui 230601 (CN)
(72) Inventor: LI, Dongbo, Hefei, Anhui 230601 (CN); LI, Bin, Hefei, Anhui 230601 (CN); HU, Xianhe, Hefei, Anhui 230601 (CN); LU, Yuting, Hefei, Anhui 230601 (CN); PAN, Wei, Hefei, Anhui 230601 (CN); JIANG, Bing, Hefei, Anhui 230601 (CN); ZHOU, Yu, Hefei, Anhui 230601 (CN); LIU, Yaqian, Hefei, Anhui 230601 (CN)
(74) Representative: HGF
(86) International application number: PCT/CN2024/143115
(87) International publication number: WO 2025/161791

(57) **Abstract**

Disclosed in the present invention are a spiro derivative serving as a WRN helicase inhibitor and a use of the spiro derivative. The spiro derivative has good WRN helicase inhibitory activity, can be used for inhibiting the growth of microsatellite-instable (MSI) tumor cells by inhibiting the occurrence of a high level of DNA double-strand breaks (DSBs) in MSI cells caused by WRN and is used for preventing and/or treating related cancers, especially colorectal cancer, gastric cancer, ovarian cancer, endometrial tumors, and the like, can compensate for the lack of specific MSI subtype cancer targeting drugs, and has extremely good prospects for application and values in the field of medicine.

## Description

### TECHNICAL FIELD

The present disclosure relates to the technical field of chemical medicine, and specifically to a spiro derivative as a WRN helicase inhibitor and use thereof, particularly use in the treatment of a cancer (especially a cancer with microsatellite instability (MSI), such as colorectal cancer, stomach/gastric cancer, ovarian cancer, and an endometrial tumor).

### BACKGROUND

Microsatellite instability (MSI) is a common feature associated with various cancers, most frequently observed in colon cancer, stomach/gastric cancer, ovarian cancer, and endometrial tumors. It is characterized by minor expansions and contractions at short repetitive DNA elements (microsatellites) interspersed throughout the genome. MSI results from mutations in one or more core components of the mismatch repair (MMR) mechanism, and MMR deficiency may lead to the occurrence of MSI (Nat Rev Clin Oncol, 7 (2010), pp. 153-162).

Generally, MSI tumors exhibit a better prognosis and a lower probability of metastasis compared with microsatellite stable (MSS) tumors derived from the same tissue. However, there is some evidence indicating a difference in chemotherapy efficacy between MSS and MSI cancers, with MSI cancers showing a poorer response to current chemotherapy regimens (J Clin Oncol, 28 (2010), pp. 3219-3226).

WRN is one of the five human RecQ-like helicases, and WRN plays an important role in HR-mediated replication fork restart and in preventing replication fork collapse. In 2019, multiple research groups demonstrated that the survival of MSI cancer cells selectively depends on WRN helicase (Nature, 568 (2019), pp. 551-556; Nature, 568 (2019), pp. 511-516). Depletion of WRN leads to high levels of DNA double-strand breaks (DSBs) in MSI cells, thereby resulting in cell death. Conversely, microsatellite stable (MSS) cells are insensitive to the depletion of WRN. These studies clearly identify WRN as a therapeutic target for MSI cancers.

By developing novel WRN inhibitors, the growth of MSI cancer cells can be selectively inhibited, while the inhibitors exhibit a favorable safety profile toward MSS normal paracancerous tissues. Therefore, in the field of MSI cancer treatment, there is a need to further develop inhibitor drugs targeting WRN, so as to accelerate the development of precision therapy for cancer patients characterized by MSI.

### SUMMARY

In a first aspect, the present disclosure provides a compound or a pharmaceutically acceptable salt, stereoisomer, ester, prodrug, solvate, or deuterated compound thereof, and the compound has the following structure: 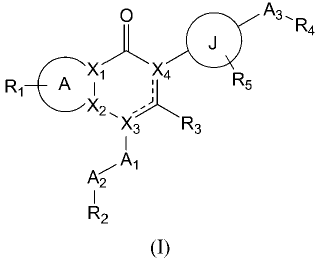 wherein
-̅-̅-̅-̅-̅-̅ represents a single bond or a double bond, and two -̅-̅-̅-̅-̅-̅ are not both double bonds;
ring A is a 4- to 6-membered heterocyclic ring;
ring J is a 6- to 18-membered spiro ring;
X₁, X₂, X₃, and X₄ are independently selected from: C(R₅) and N; R₅ is selected from: H, D, C₁-C₁₀ alkyl, -(C₀-C₆ alkylene)-(C₃-C₁₀ cycloalkyl), -(C₀-C₆ alkylene)-(C₆-C₁₀ aryl), -(C₀-C₆ alkylene)-(4- to 10-membered heterocyclyl), halogen, cyano, nitro, azido, C₁-C₁₀ haloalkyl, C₁-C₁₀ haloalkoxy, -N(C₀₋₁₀ alkyl)(C₀₋₁₀ alkyl), -N(C₀₋₁₀ alkyl)(C₃-C₁₀ cycloalkyl), -N(C₀₋₁₀ alkyl)CO(C₀₋₁₀ alkyl), -N(C₀₋₁₀ alkyl)CON(C₀₋₁₀ alkyl), -N(C₀₋₁₀ alkyl)SO₂(C₀₋₁₀ alkyl), -O(C₀₋₁₀ alkyl), -O(C₃-C₁₀ cycloalkyl), -S(C₀₋₁₀ alkyl), -S(C₃-C₁₀ cycloalkyl), -SO(C₀₋₁₀ alkyl), -SO₂(C₀₋₁₀ alkyl), -SO₂(C₃-C₁₀ cycloalkyl), -SO₂N(C₀₋₁₀ alkyl)(C₀₋₁₀ alkyl), -SO₂N(C₀₋₁₀ alkyl)(C₃-C₁₀ cycloalkyl), -COO(C₀₋₁₀ alkyl), -OCO(C₀₋₁₀ alkyl), -CON(C₀₋₁₀ alkyl)(C₀₋₁₀ alkyl), -CON(C₀₋₁₀ alkyl)(C₃-C₁₀ cycloalkyl), and -CO(C₀₋₁₀ alkyl);
A₁, A₂, and A₃ are independently selected from: a single bond and C₁-C₁₀ alkylene, wherein 0-6 methylene units in the C₁-C₁₀ alkylene are independently substituted with the following groups: -Cy-, -O-, -S-, -C(O)-, -C(S)-, -C(O)O-, -OC(O)-, -OC(O)O-, -O-S(O)₂-, -OC(O)N(R₄)-, -C(O)N(R₄)-, -N(Rₐ)C(O)-, -N(Rₐ)C(O)O-, -N(Rₐ)C(O)N(R_{b})-, - N(Rₐ)-, -S(O)₂-, -S(O)₂N(Rₐ)-, -N(R₂)S(O)₂-, -S(O)-, -S(O)N(Rₐ)-, -N(Rₐ)S(O)-, -Si-, 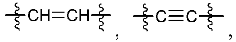 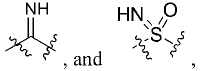 wherein Rₐ and R_{b} are independently selected from: H, C₁-C₁₀ alkyl, C₂-C₁₀ alkenyl, C₂-C₁₀ alkynyl, -(C₀-C₆ alkylene)-(C₃-C₁₀ cycloalkyl), -(C₀-C₆ alkylene)-(C₆-C₁₀ aryl), and -(C₀-C₆ alkylene)-(4- to 10-membered heterocyclyl); each -Cy- is independently an optionally substituted divalent ring selected from: arylene, cycloalkylene, and heterocyclylene; H in the C₁-C₁₀ alkylene is optionally substituted with one or more R₀;
R₁ is one or more independent substituents on ring A and is selected from: H, D, C₁-C₁₀ alkyl, -(C₀-C₆ alkylene)-(C₃-C₁₀ cycloalkyl), -(C₀-C₆ alkylene)-(C₆-C₁₀ aryl), -(C₀-C₆ alkylene)-(4- to 10-membered heterocyclyl), halogen, cyano, nitro, azido, C₁-C₁₀ haloalkyl, C₁-C₁₀ haloalkoxy, -N(C₀₋₁₀ alkyl)(C₀₋₁₀ alkyl), -N(C₀₋₁₀ alkyl)(C₃-C₁₀ cycloalkyl), - N(C₀₋₁₀ alkyl)CO(C₀₋₁₀ alkyl), -N(C₀₋₁₀ alkyl)CON(C₀₋₁₀ alkyl), -N(C₀₋₁₀ alkyl)SO₂(C₀₋₁₀ alkyl), -O(C₀₋₁₀ alkyl), - O(C₃-C₁₀ cycloalkyl), -S(C_{0.10} alkyl), -S(C₃-C₁₀ cycloalkyl), -SO(C₀₋₁₀ alkyl), -SO₂(C₀₋₁₀ alkyl), -SO₂(C₃-C₁₀ cycloalkyl), -SO₂N(C₀₋₁₀ alkyl)(C₀₋₁₀ alkyl), -SO₂N(C₀₋₁₀ alkyl)(C₃-C₁₀ cycloalkyl), -COO(C₀₋₁₀ alkyl), -OCO(C₀₋₁₀ alkyl), -CON(C₀₋₁₀ alkyl)(C₀₋₁₀ alkyl), -CON(C₀₋₁₀ alkyl)(C₃-C₁₀ cycloalkyl), -CO(C₀₋₁₀ alkyl), and -Si(C₀₋₁₀ alkyl)(C₀₋₁₀ alkyl)(C₀₋₁₀ alkyl), wherein H in the C₀-C₆ alkylene, C₁-C₁₀ alkyl, C₂-C₁₀ alkenyl, C₂-C₁₀ alkynyl, C₀₋₁₀ alkyl, C₃-C₁₀ cycloalkyl, C₆-C₁₀ aryl, or 4- to 10-membered heterocyclyl may be optionally substituted with one or more R₀;
R₂, R₃, and R₄ are independently selected from: H, D, C₁-C₁₀ alkyl, -(C₀-C₆ alkylene)-(C₃-C₁₀ cycloalkyl), -(C₀-C₆ alkylene)-(C₆-C₁₀ aryl), -(C₀-C₆ alkylene)-(4- to 10-membered heterocyclyl), halogen, cyano, nitro, azido, C₁-C₁₀ haloalkyl, C₁-C₁₀ haloalkoxy, -N(C₀₋₁₀ alkyl)(C₀₋₁₀ alkyl), -N(C₀₋₁₀ alkyl)(C₃-C₁₀ cycloalkyl), -N(C₀₋₁₀ alkyl)CO(C₀₋₁₀ alkyl), -N(C₀₋₁₀ alkyl)CON(C₀₋₁₀ alkyl), -N(C₀₋₁₀ alkyl)SO₂(C₀₋₁₀ alkyl), -O(C₀₋₁₀ alkyl), -O(C₃-C₁₀ cycloalkyl), -S(C₀₋₁₀ alkyl), -S(C₃-C₁₀ cycloalkyl), -SO(C₀₋₁₀ alkyl), -SO₂(C₀₋₁₀ alkyl), -SO₂(C₃-C₁₀ cycloalkyl), -SO₂N(C₀₋₁₀ alkyl)(C₀₋₁₀ alkyl), -SO₂N(C₀₋₁₀ alkyl)(C₃-C₁₀ cycloalkyl), -COO(C₁₀₋₁₀ alkyl), -OCO(C₀₋₁₀ alkyl), -CON(C₀₋₁₀ alkyl)(C₀₋₁₀ alkyl), -CON(C₀₋₁₀ alkyl)(C₃-C₁₀ cycloalkyl), -CO(C₀₋₁₀ alkyl), and -Si(C₀₋₁₀ alkyl)(C₀₋₁₀ alkyl)(C₀₋₁₀ alkyl), wherein H in the C₀-C₆ alkylene, C₁-C₁₀ alkyl, C₂-C₁₀ alkenyl, C₂-C₁₀ alkynyl, C₀₋₁₀ alkyl, C₃-C₁₀ cycloalkyl, C₆-C₁₀ aryl, or 4- to 10-membered heterocyclyl may be optionally substituted with one or more R₀; or, R₃ and A₁, together with the atoms to which they are attached, form a carbocyclic or heterocyclic ring, wherein H in the carbocyclic or heterocyclic ring may be optionally substituted with one or more R₀;
R₅ is one or more independent substituents on ring J and is selected from: H, D, C₁-C₁₀ alkyl, -(C₀-C₆ alkylene)-(C₃-C₁₀ cycloalkyl), -(C₀-C₆ alkylene)-(C₆-C₁₀ aryl), -(C₀-C₆ alkylene)-(4- to 10-membered heterocyclyl), halogen, cyano, nitro, azido, C₁-C₁₀ haloalkyl, C₁-C₁₀ haloalkoxy, -N(C₀₋₁₀ alkyl)(C₀₋₁₀ alkyl), -N(C₀₋₁₀ alkyl)(C₃-C₁₀ cycloalkyl), - N(C₀₋₁₀ alkyl)CO(C₀₋₁₀ alkyl), -N(C₀₋₁₀ alkyl)CON(C₀₋₁₀ alkyl), -N(C₀₋₁₀ alkyl)SO₂(C₀₋₁₀ alkyl), -O(C₀₋₁₀ alkyl), - O(C₃-C₁₀ cycloalkyl), -S(C₀₋₁₀ alkyl), -S(C₃-C₁₀ cycloalkyl), -SO(C₀₋₁₀ alkyl), -SO₂(C₀₋₁₀ alkyl), -SO₂(C₃-C₁₀ cycloalkyl), -SO₂N(C₀₋₁₀ alkyl)(C₀₋₁₀ alkyl), -SO₂N(C₀₋₁₀ alkyl)(C₃-C₁₀ cycloalkyl), -COO(C₁₀₋₁₀ alkyl), -OCO(C₀₋₁₀ alkyl), -CON(C₀₋₁₀ alkyl)(C₀₋₁₀ alkyl), -CON(C₀₋₁₀ alkyl)(C₃-C₁₀ cycloalkyl), -CO(C₀₋₁₀ alkyl), and -Si(C₀₋₁₀ alkyl)(C₀₋₁₀ alkyl)(C₀₋₁₀ alkyl), wherein H in the C₀-C₆ alkylene, C₁-C₁₀ alkyl, C₂-C₁₀ alkenyl, C₂-C₁₀ alkynyl, C₀₋₁₀ alkyl, C₃-C₁₀ cycloalkyl, C₆-C₁₀ aryl, or 4- to 10-membered heterocyclyl may be optionally substituted with one or more R₀;
R₀ is selected from: D, =O, halogen, cyano, nitro, azido, -SF₅, -OR', -C(O)R', -C(S)R', -C(O)OR', -C(S)SR', -OC(O)R', -OC(S)R', -OC(S)SR', -C(O)NR'R", -OC(O)NR'R", -NR'C(O)OR", -NR'SO₂R", -SO₂NR'R", -OSO₂NR'R", - NR'C(O)R", -NR'R", -SR', -SOR', -SO₂R', -OSO₂R', -SO₃H, -SiR'R'R", C₁-C₁₀ alkyl, C₂-C₁₀ alkenyl, C₂-C₁₀ alkynyl, C₁-C₁₀ haloalkyl, C₁-C₁₀ haloalkoxy, -(C₀-C₆ alkylene)-(C₃-C₁₀ cycloalkyl), -(C₀-C₆ alkylene)-(C₆-C₁₀ aryl), and - (C₀-C₆ alkylene)-(4- to 10-membered heterocyclyl);
each R' and R" is independently selected from: H, D, C₁-C₁₀ alkyl, C₂-C₁₀ alkenyl, C₂-C₁₀ alkynyl, -(C₀-C₆ alkylene)-(C₃-C₁₀ cycloalkyl), -(C₀-C₆ alkylene)-(C₆-C₁₀ aryl), and -(C₀-C₆ alkylene)-(4- to 10-membered heterocyclyl), wherein H in the C₀-C₆ alkylene, C₁-C₁₀ alkyl, C₂-C₁₀ alkenyl, C₂-C₁₀ alkynyl, C₃-C₁₀ cycloalkyl, C₆-C₁₀ aryl, or 4-to 10-membered heterocyclyl may be optionally substituted with one or more groups selected from: halogen, cyano, nitro, azido, C₁-C₁₀ alkyl, -(C₀-C₆ alkylene)-(C₃-C₁₀ cycloalkyl), -(C₀-C₆ alkylene)-(C₆-C₁₀ aryl), -(C₀-C₆ alkylene)-(4- to 10-membered heterocyclyl), C₁-C₁₀ haloalkyl, C₁-C₁₀ haloalkoxy, -N(C₀₋₁₀ alkyl)(C₀₋₁₀ alkyl), -N(C₀₋₁₀ alkyl)(C₃-C₁₀ cycloalkyl), -N(C₀₋₁₀ alkyl)CO(C₀₋₁₀ alkyl), -N(C₀₋₁₀ alkyl)CON(C₀₋₁₀ alkyl), -N(C₀₋₁₀ alkyl)SO₂(C₀₋₁₀ alkyl), -O(C₀₋₁₀ alkyl), -O(C₃-C₁₀ cycloalkyl), -S(C₀₋₁₀ alkyl), -S(C₃-C₁₀ cycloalkyl), -SO(C₀₋₁₀ alkyl), -SO₂(C₀₋₁₀ alkyl), -SO₂(C₃-C₁₀ cycloalkyl), -SO₂N(C₀₋₁₀ alkyl)(C₀₋₁₀ alkyl), -SO₂N(C₀₋₁₀ alkyl)(C₃-C₁₀ cycloalkyl), -COO(C₀₋₁₀ alkyl), -OCO(C₀₋₁₀ alkyl), -CON(C₀₋₁₀ alkyl)(C₀₋₁₀ alkyl), -CON(C₀₋₁₀ alkyl)(C₃-C₁₀ cycloalkyl), -CO(C₀₋₁₀ alkyl), and -Si(C₀₋₁₀ alkyl)(C₀₋₁₀ alkyl)(C₀₋₁₀ alkyl).

Specifically, each -CY- is independently selected from the following optionally substituted divalent rings: phenylene, bicyclic arylene, tricyclic arylene, monocyclic cycloalkylene, bicyclic cycloalkylene, tricyclic cycloalkylene, monocyclic heteroarylene, bicyclic heteroarylene, tricyclic heteroarylene, monocyclic heterocycloalkylene, bicyclic heterocycloalkylene, and tricyclic heterocycloalkylene.

In some embodiments of the present disclosure, each -CY- is independently selected from the following optionally substituted divalent rings: monocyclic cycloalkylene, bicyclic cycloalkylene, monocyclic saturated heterocyclylene, and bicyclic saturated heterocyclylene.

Specifically, each -CY - is optionally substituted with the following groups: halogen, cyano, nitro, azido, C₁-C₁₀ alkyl, -N(C₀-C₁₀ alkyl)(C₀-C₁₀ alkyl), -O(C₀-C₁₀ alkyl), -CON(C₀-C₁₀ alkyl)(C₀-C₁₀ alkyl), -N(C₀-C₁₀ alkyl)CO(C₀-C₁₀ alkyl), -SO₂N(C₀-C₁₀ alkyl)(C₀-C₁₀ alkyl), -N(C₀-C₁₀ alkyl)SO₂(C₀-C₁₀ alkyl), -OCH₂F, -OCHF₂, -OCF₃, C₃-C₁₀ cycloalkyl, C₆-C₁₀ aryl, and 4- to 10-membered heterocyclyl.

In some embodiments of the present disclosure, ring A is a 4- to 6-membered nitrogen-containing heterocyclic ring, particularly a 5-membered nitrogen-containing heteroaromatic ring, e.g., 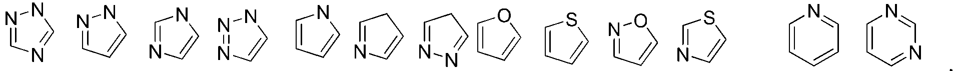 particularly

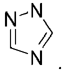

In some embodiments of the present disclosure, ring 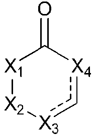 is 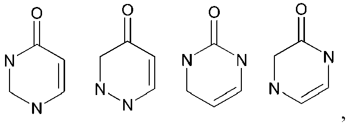 particularly

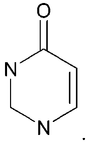

In some embodiments of the present disclosure, ring independently selected from: C(H) and N, e.g., 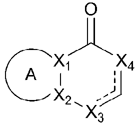 is 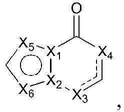 wherein X₅ and X₆ are 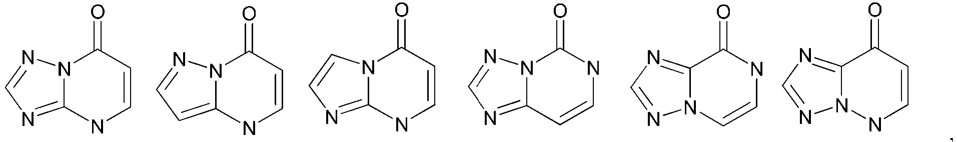 particularly

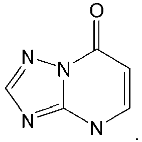

Specifically, A₁ is C₁-C₁₀ alkylene, wherein 1-3 methylene units are independently substituted with the following groups: -O-, -S-, -C(O)-, -C(S)-, -C(O)O-, -OC(O)-, -N(Rₐ)-, -C(O)N(R₄)-, -N(Rₐ)C(O)-, -S(O)-, -S(O)₂-, 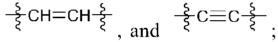 in some embodiments of the present disclosure, A₁ is -(C₀-C₃ alkylene)-N(C₀₋₆ alkyl)-(C₀-C₃ alkylene)-, -(C₀-C₃ alkylene)-C(O)N(C₀₋₆ alkyl)-(C₀-C₃ alkylene)-, -(C₀-C₃ alkylene)-N(C₀₋₆ alkyl)C(O)-(C₀-C₃ alkylene)-, -(C₀-C₃ alkylene)-O-(C₀-C₃ alkylene)-, or -(C₀-C₃ alkylene)-S-(C₀-C₃ alkylene)-.

In some embodiments of the present disclosure, A₁ is C₁-C₆ alkylene, wherein H is optionally substituted with one or more groups selected from: H, D, halogen, -(C₀-C₃ alkylene)-(C₃-C₆ cycloalkyl), -(C₀-C₃ alkylene)-(phenyl), and -(C₀-C₃ alkylene)-(4- to 6-membered saturated heterocyclyl).

In some embodiments of the present disclosure, A₁ is 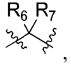 wherein R₆ and R₇ are independently selected from: H, D, C₁-C₁₀ alkyl, C₁-C₁₀ haloalkyl, -O(C₀₋₁₀ alkyl), -N(C₀₋₁₀ alkyl)(C₀₋₁₀ alkyl), and -COO(C₁₀₋₁₀ alkyl).

Specifically, R₆ and R₇ are independently selected from: H, D, C₁-C₆ alkyl, C₁-C₆ haloalkyl, C₁-C₆ alkoxy, -OH, - NH₂, and -COOH; more specifically, R₆ and R₇ are independently selected from: H, D, and C₁-C₃ alkyl.

In some embodiments of the present disclosure, R₃ and R₇, together with the atoms to which they are attached, form a 4- to 8-membered (e.g., 4-, 5-, 6-, 7-, or 8-membered) carbocyclic or heterocyclic ring, wherein H in the carbocyclic or heterocyclic ring may be optionally substituted with one or more R₀; specifically, the carbocyclic or heterocyclic ring is optionally substituted with one or more groups selected from: H, D, halogen, cyano, hydroxy, sulfydryl, amino, C₁-C₆ alkyl, -(C₀-C₆ alkylene)-(C₃-C₆ cycloalkyl), -(C₀-C₆ alkylene)-(phenyl), -O(C₁-C₆ alkyl), -S(C₁-C₆ alkyl), - N(H)(C₁-C₆ alkyl), -N(C₁-C₆ alkyl)(C₁-C₆ alkyl), -CON(C₀-C₆ alkyl)(C₀-C₆ alkyl), -N(C₀-C₆ alkyl)CO(C₀-C₆ alkyl), -SO₂N(C₀-C₆ alkyl)(C₀-C₆ alkyl), and -N(C₀-C₆ alkyl)SO₂(C₀-C₆ alkyl), wherein H in the C₀-C₆ alkylene, C₀-C₆ alkyl, C₁-C₆ alkyl, C₃-C₆ cycloalkyl, or phenyl may be optionally substituted with a group selected from: D, halogen, cyano, hydroxy, sulfydryl, amino, C₁-C₃ alkyl, and C₁-C₃ alkoxy; more specifically, the carbocyclic or heterocyclic ring is optionally substituted with one or more groups selected from: H, D, halogen, cyano, C₁-C₆ alkyl, C₁-C₆ alkoxy, -OH, -NH₂, and -COOH, wherein the C₁-C₆ alkyl is optionally substituted with one or more groups selected from: H, D, halogen, cyano, -OH, -NH₂, and -COOH.

In some embodiments of the present disclosure, R₃ and R₇, together with the atoms to which they are attached, form a 4- to 8-membered heterocyclic ring, wherein H in the heterocyclic ring may be optionally substituted with one or more R₀; the heterocyclic ring may contain one or more heteroatoms selected from: N, O, and S.

In some embodiments of the present disclosure, R₇ is H.

In some embodiments of the present disclosure, A₁ is 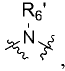 wherein R₆' is selected from: H, D, C₁-C₁₀ alkyl, C₁-C₁₀ haloalkyl, and -(C₀-C₆ alkylene)-(C₃-C₁₀ cycloalkyl).

Specifically, R6' is selected from: H, D, C₁-C₆ alkyl, and -(C₀-C₆ alkylene)-(C₃-C₆ cycloalkyl); more specifically, R₆' is H.

Specifically, A₂ is C₁-C₁₀ alkylene, wherein 1-3 methylene units are independently substituted with the following groups: -C(O)-, -C(S)-, -C(O)O-, -N(Rₐ)-, -C(O)N(R₄)-, -N(Rₐ)C(O)-, -S(O)N(Rₐ)-, and -S(O)₂N(Rₐ)-; in some embodiments of the present disclosure, A₂ is -(C₀-C₃ alkylene)-C(O)N(C₀₋₆ alkyl)-(C₀-C₃ alkylene)-, -(C₀-C₃ alkylene)-N(C₀₋₆ alkyl)C(O)-(C₀-C₃ alkylene)-, -(C₀-C₃ alkylene)-S(O)₂N(C₀₋₆ alkyl)-(C₀-C₃ alkylene)-, or -(C₀-C₃ alkylene)-S(O)N(C₀₋₆ alkyl)-(C₀-C₃ alkylene)-.

In some embodiments of the present disclosure, A₂ is -C(O)N(R₈)- or -S(O)₂N(R₈)-, and R₈ is selected from: H, C₁-C₆ alkyl, and -(C₀-C₃ alkylene)-(C₃-C₆ cycloalkyl).

Specifically, R₈ is selected from: H, C₁-C₃ alkyl, and -(C₀-C₃ alkylene)-(C₃-C₄ cycloalkyl); in some embodiments of the present disclosure, R₈ is H.

In some embodiments of the present disclosure, the compound has the following structure:

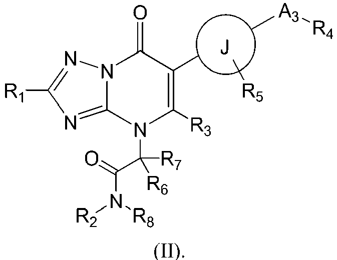

In some embodiments of the present disclosure, the compound has the following structure: 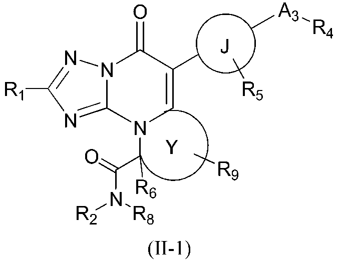 wherein
ring Y is a 5- to 7-membered heterocyclic ring;
R₉ is one or more independent substituents on ring Y and has the definition of R₀ described above.

Specifically, ring Y is a 5- to 7-membered saturated heterocyclic ring, which, in addition to the nitrogen atom shown in the above formula, may optionally further contain one or more heteroatoms selected from: N, O, and S, such as

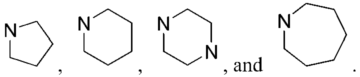

Specifically, each R₉ is independently selected from: H, D, halogen, cyano, hydroxy, sulfydryl, amino, C₁-C₆ alkyl, -(C₀-C₆ alkylene)-(C₃-C₆ cycloalkyl), -(C₀-C₆ alkylene)-(phenyl), -O(C₁-C₆ alkyl), -S(C₁-C₆ alkyl), -N(H)(C₁-C₆ alkyl), -N(C₁-C₆ alkyl)(C₁-C₆ alkyl), -CON(C₀-C₆ alkyl)(C₀-C₆ alkyl), -N(C₀-C₆ alkyl)CO(C₀-C₆ alkyl), -SO₂N(C₀-C₆ alkyl)(C₀-C₆ alkyl), and -N(C₀-C₆ alkyl)SO₂(C₀-C₆ alkyl), wherein H in the C₀-C₆ alkylene, C₀-C₆ alkyl, C₁-C₆ alkyl, C₃-C₆ cycloalkyl, or phenyl may be optionally substituted with a group selected from: D, halogen, cyano, hydroxy, sulfydryl, amino, C₁-C₃ alkyl, and C₁-C₃ alkoxy; more specifically, each R₉ is independently selected from: H, D, halogen, cyano, hydroxy, sulfydryl, amino, and C₁-C₆ alkyl, wherein the C₁-C₆ alkyl is optionally substituted with one or more groups selected from: D, halogen, cyano, hydroxy, amino, and C₁-C₃ alkoxy.

In some embodiments of the present disclosure, ring J is a 6- to 12-membered (e.g., 6-, 7-, 8-, 9-, 10-, or 11 -membered) saturated or partially unsaturated spiro heterocyclic ring; more specifically, at least one ring atom of ring J is N; in some embodiments of the present disclosure, two ring atoms of ring J are N.

Specifically, R₅ is selected from: H, halogen, hydroxy, sulfydryl, amino, C₁-C₆ alkyl, -(C₀-C₆ alkylene)-(C₃-C₆ cycloalkyl), -O(C₁₋₆ alkyl), -S(C₁₋₆ alkyl), -N(H)(C₁₋₆ alkyl), and -N(C₁₋₆ alkyl)(C₁₋₆ alkyl), wherein H in the C₀-C₆ alkylene, C₁-C₆ alkyl, or C₃-C₆ cycloalkyl may be optionally substituted with a group selected from: halogen, hydroxy, and C₁-C₃ alkoxy; more specifically, R₅ is selected from: H, halogen (e.g., F or Cl), hydroxy, sulfydryl, amino, C₁-C₄ alkyl, C₁-C₄ haloalkyl, C₁-C₄ hydroxy-substituted alkyl, -(C₀-C₃ alkylene)-(C₃-C₅ cycloalkyl), -O(C₁₋₃ alkyl), - S(C₁₋₃ alkyl), -N(H)(C₁₋₃ alkyl), and -N(C₁₋₃ alkyl)(C₁₋₃ alkyl).

In some embodiments of the present disclosure, R₅ is selected from: H, F, Cl, -OH, -NH₂, methyl, and ethyl.

Specifically, moiety 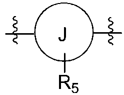 has the following structure: 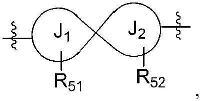 wherein
ring J₁ is a 3- to 8-membered (e.g., 3-, 4-, 5-, 6-, 7-, or 8-membered) monocyclic carbocyclic or heterocyclic ring;
ring J₂ is a 3- to 8-membered (e.g., 3-, 4-, 5-, 6-, 7-, or 8-membered) monocyclic carbocyclic or heterocyclic ring;
R₃₁ and R₃₂ are one or more independent substituents on rings J₁ and J₂, respectively, and have the definition of R₅. Specifically, ring J₁ is a 3- to 6-membered saturated or partially unsaturated carbocyclic or heterocyclic ring, such as 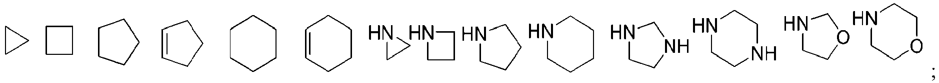
in some embodiments of the present disclosure, ring J₁ is a 3- to 6-membered partially unsaturated carbocyclic ring; in some embodiments of the present disclosure, ring J₁ is a 3- to 6-membered saturated heterocyclic ring in which at least one ring atom is N.

Specifically, ring J₂ is a 3- to 6-membered saturated or partially unsaturated carbocyclic or heterocyclic ring, such as 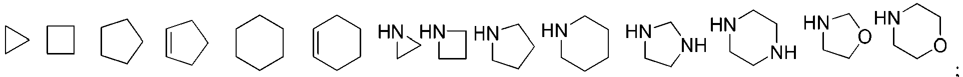 in some embodiments of the present disclosure, ring J₂ is a 3- to 6-membered saturated heterocyclic ring in which at least one ring atom is N, such as

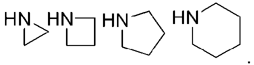

Specifically, moiety 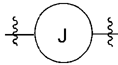 has the following structures:

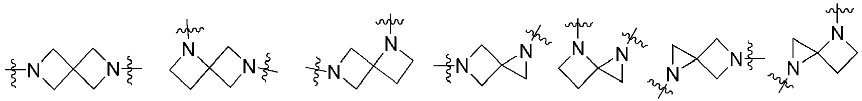

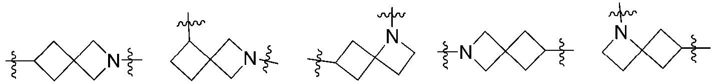

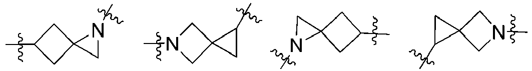

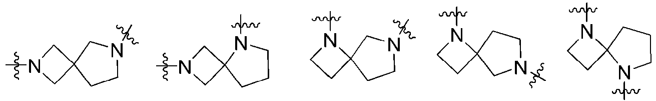

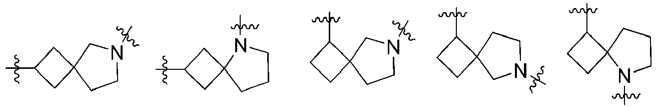

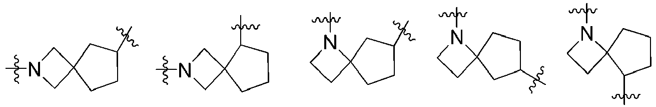

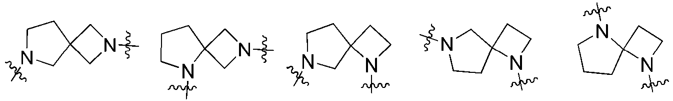

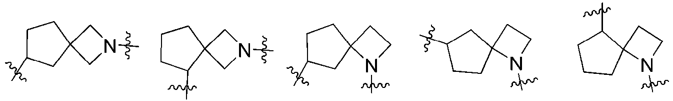

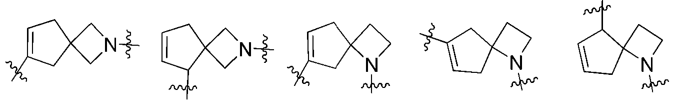

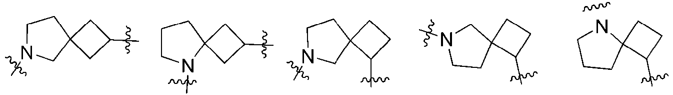

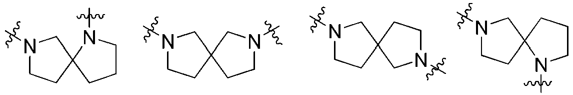

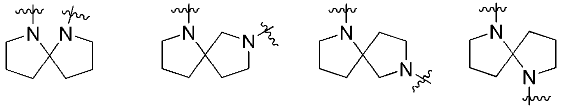

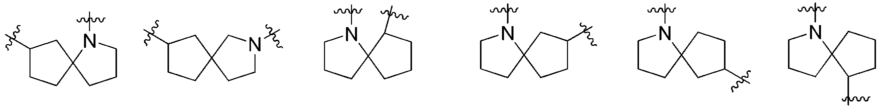

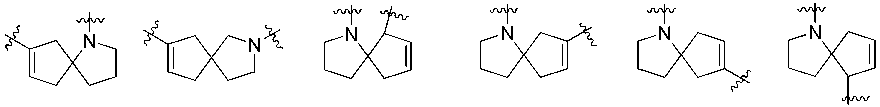

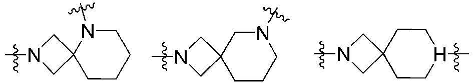

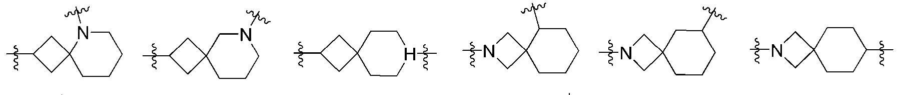

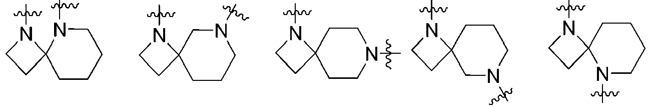

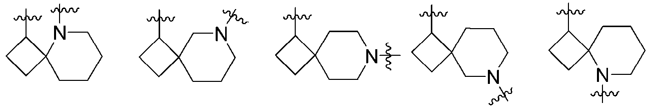

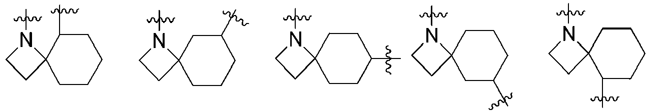

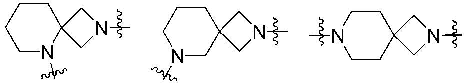

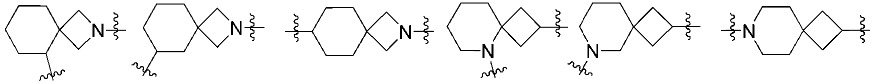

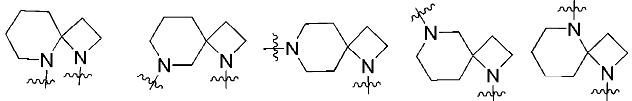

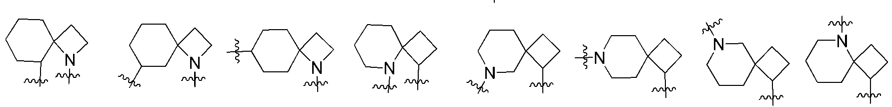

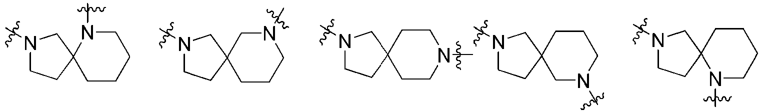

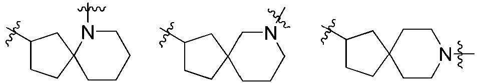

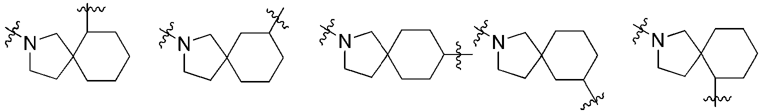

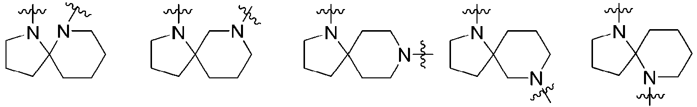

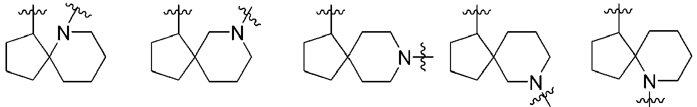

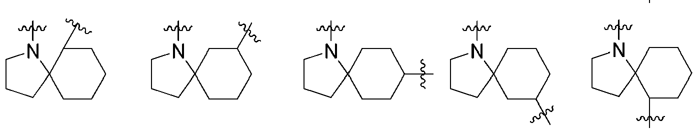

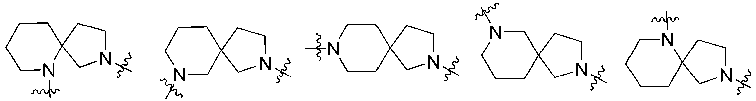

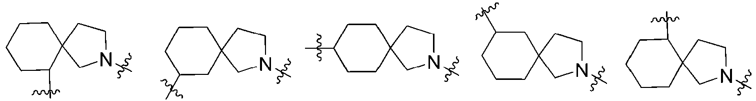

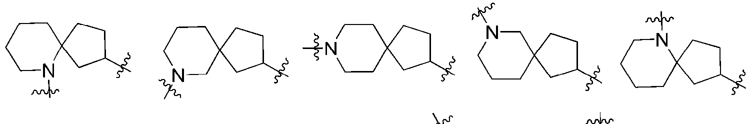

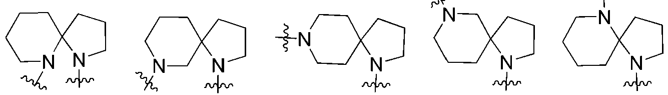

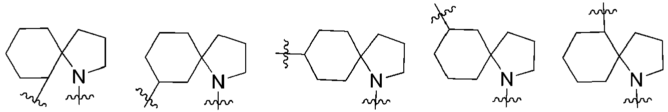

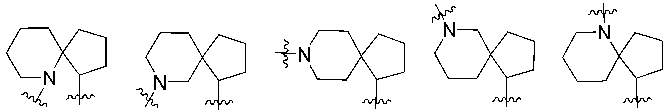

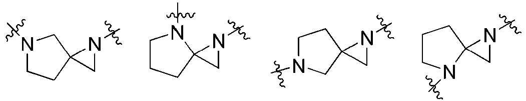

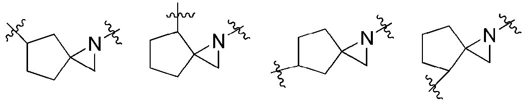

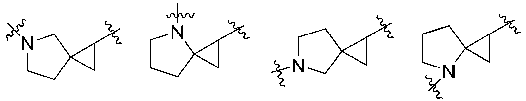

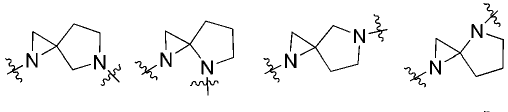

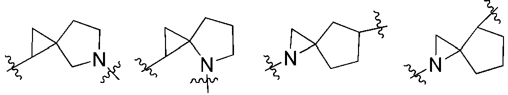

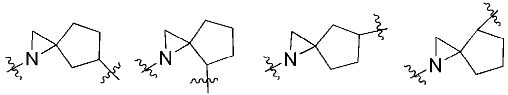

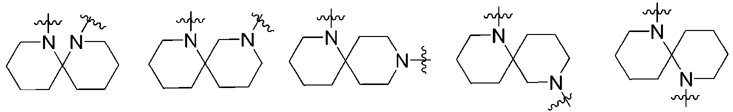

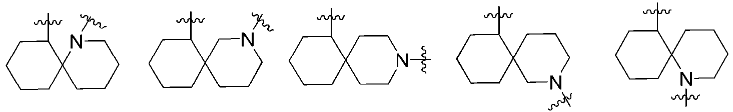

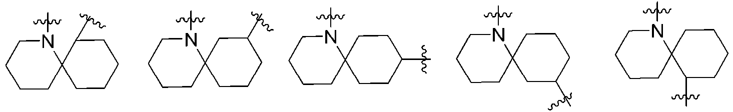

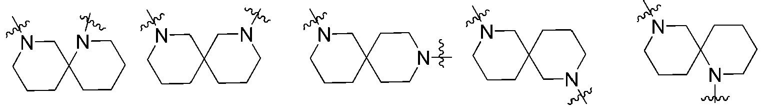

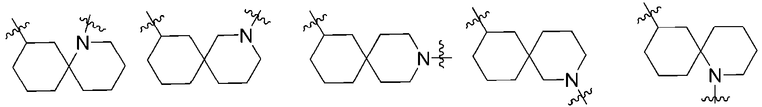

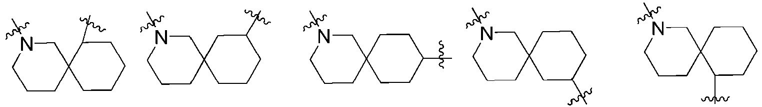

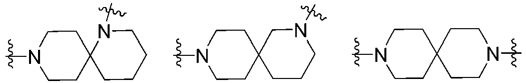

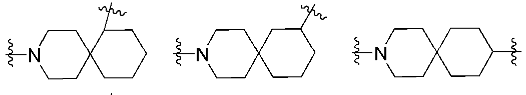

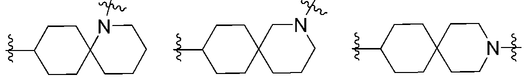

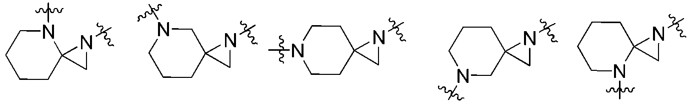

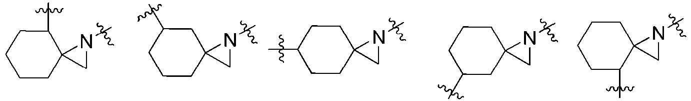

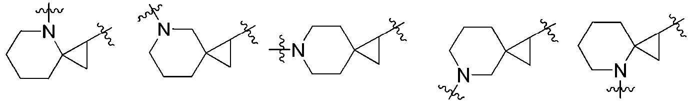

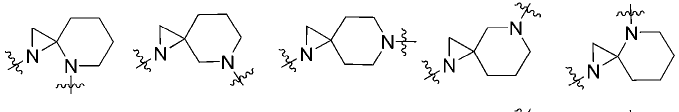

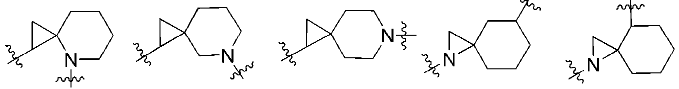

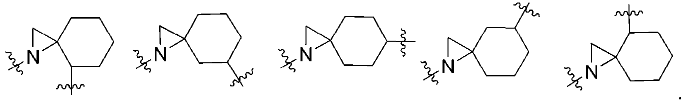

In some embodiments of the present disclosure, moiety 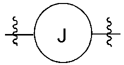 has the following structure:

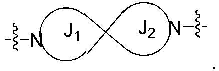

Specifically, R₅₁ is selected from: H, halogen, hydroxy, sulfydryl, amino, C₁-C₆ alkyl, -(C₀-C₆ alkylene)-(C₃-C₆ cycloalkyl), -O(C₁₋₆ alkyl), -S(C₁₋₆ alkyl), -N(H)(C₁₋₆ alkyl), and -N(C₁₋₆ alkyl)(C₁₋₆ alkyl), wherein H in the C₀-C₆ alkylene, C₁-C₆ alkyl, or C₃-C₆ cycloalkyl may be optionally substituted with a group selected from: halogen, hydroxy, and C₁-C₃ alkoxy; more specifically, R₅₁ is selected from: H, halogen (e.g., F or Cl), hydroxy, sulfydryl, amino, C₁-C₄ alkyl, C₁-C₄ haloalkyl, C₁-C₄ hydroxy-substituted alkyl, -(C₀-C₃ alkylene)-(C₃-C₅ cycloalkyl), -O(C₁₋₃ alkyl), - S(C₁₋₃ alkyl), -N(H)(C₁₋₃ alkyl), and -N(C₁₋₃ alkyl)(C₁₋₃ alkyl); in some embodiments of the present disclosure, R₅₁ is H.

Specifically, R₅₂ is selected from: H, halogen, hydroxy, sulfydryl, amino, C₁-C₆ alkyl, -(C₀-C₆ alkylene)-(C₃-C₆ cycloalkyl), -O(C₁₋₆ alkyl), -S(C₁₋₆ alkyl), -N(H)(C₁₋₆ alkyl), and -N(C₁₋₆ alkyl)(C₁₋₆ alkyl), wherein H in the C₀-C₆ alkylene, C₁-C₆ alkyl, or C₃-C₆ cycloalkyl may be optionally substituted with a group selected from: halogen, hydroxy, and C₁-C₃ alkoxy; more specifically, R₅₂ is selected from: H, halogen (e.g., F or Cl), hydroxy, sulfydryl, amino, C₁-C₄ alkyl, C₁-C₄ haloalkyl, C₁-C₄ hydroxy-substituted alkyl, -(C₀-C₃ alkylene)-(C₃-C₅ cycloalkyl), -O(C₁₋₃ alkyl), - S(C₁₋₃ alkyl), -N(H)(C₁₋₃ alkyl), and -N(C₁₋₃ alkyl)(C₁₋₃ alkyl); in some embodiments of the present disclosure, R₅₂ is selected from: H, F, Cl, -OH, -NH₂, methyl, and ethyl.

In some embodiments of the present disclosure, moiety 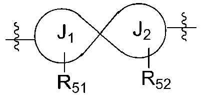 has the following structure: 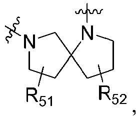 particularly 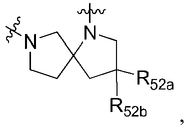 wherein R₅₂ₐ and R_{52b} have the definition of R₅₂.

In some embodiments of the present disclosure, moiety 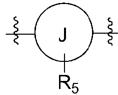 has the following structures:

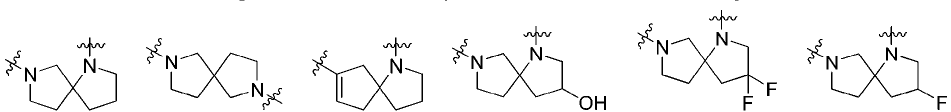

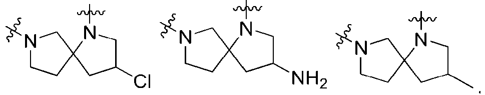

In other embodiments of the present disclosure, moiety 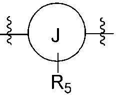 has the following structures:

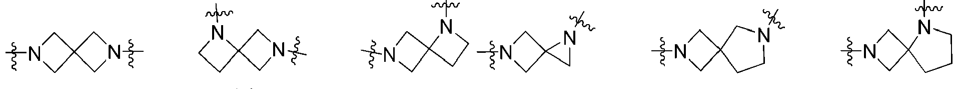

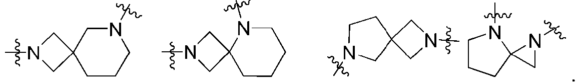

In some embodiments of the present disclosure, R₁ has the following structure: 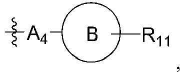 wherein:
A₄ is selected from: a single bond and C₁-C₆ alkylene, wherein 0-3 methylene units in the C₁-C₆ alkylene are independently substituted with the following groups: -O-, -S-, -C(O)-, -C(S)-, -C(O)O-, -OC(O)-, -C(O)N(H)-, - N(H)C(O)-, -N(H)-, and -S(O)₂-;
ring B is a 4- to 10-membered carbocyclic or heterocyclic ring;
R₁₁ is one or more independent substituents on ring B and is selected from: H, D, C₁-C₁₀ alkyl, C₂-C₁₀ alkenyl, C₂-C₁₀ alkynyl, -(C₀-C₆ alkylene)-(C₃-C₁₀ cycloalkyl), -(C₀-C₆ alkylene)-(C₆-C₁₀ aryl), -(C₀-C₆ alkylene)-(4- to **10-**membered heterocyclyl), halogen, cyano, nitro, azido, C₁-C₁₀ haloalkyl, C₁-C₁₀ haloalkoxy, -N(C₀₋₁₀ alkyl)(C₀₋₁₀ alkyl), -N(C₀₋₁₀ alkyl)CO(C₀₋₁₀ alkyl), -N(C₀₋₁₀ alkyl)CON(C₀₋₁₀ alkyl), -N(C₀₋₁₀ alkyl)SO₂(C₀₋₁₀ alkyl), -O(C₀₋₁₀ alkyl), -S(C₀₋₁₀ alkyl), -SO(C₀₋₁₀ alkyl), -SO₂(C₀₋₁₀ alkyl), -SO₂N(C₀₋₁₀ alkyl)(C₀₋₁₀ alkyl), -COO(C₀₋₁₀ alkyl), - OCO(C₀₋₁₀ alkyl), -CON(C₀₋₁₀ alkyl)(C₀₋₁₀ alkyl), and -CO(C₀₋₁₀ alkyl), wherein H in the C₀-C₆ alkylene, C₁-C₁₀ alkyl, C₂-C₁₀ alkenyl, C₂-C₁₀ alkynyl, C₀₋₁₀ alkyl, C₃-C₁₀ cycloalkyl, C₆-C₁₀ aryl, or 4- to 10-membered heterocyclyl may be optionally substituted with one or more R₀.

Specifically, ring B is a 5- to 8-membered (saturated, partially saturated, or aromatic) carbocyclic or heterocyclic ring, for example:

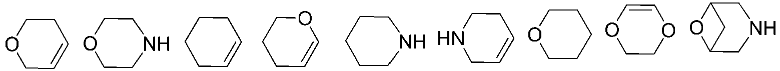

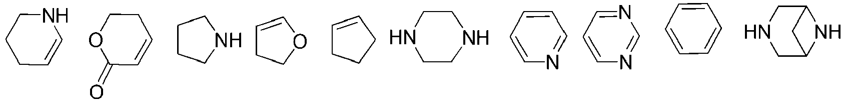

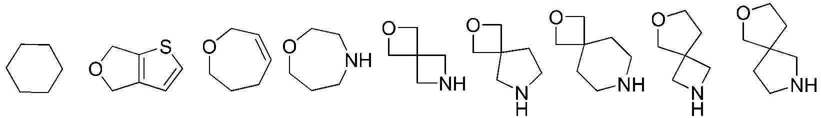

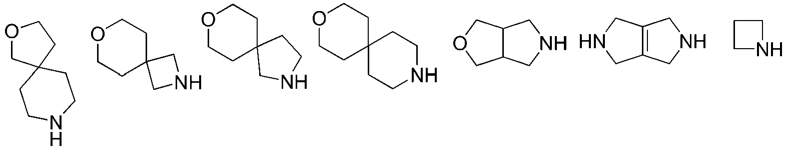

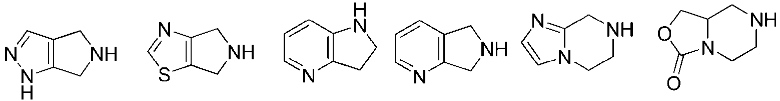

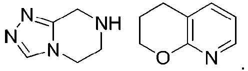

Specifically, moiety 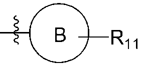 has the following structures: 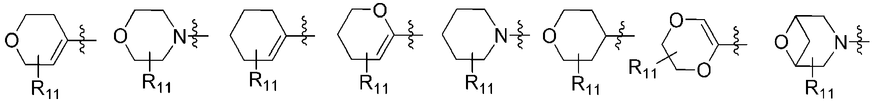 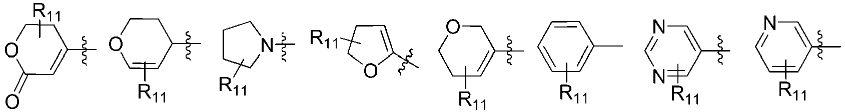 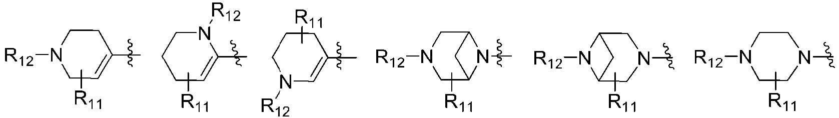 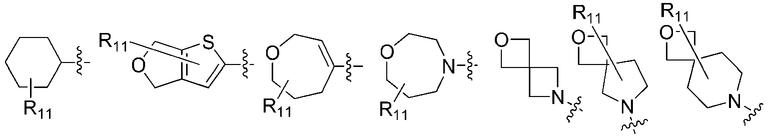 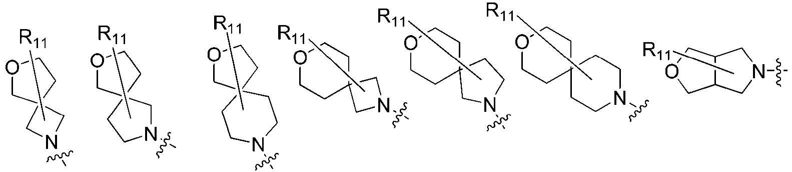 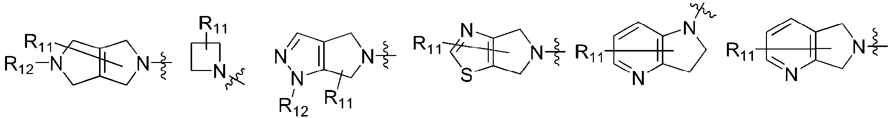 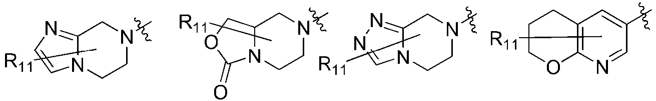 wherein R₁₂ is selected from: H, D, C₁-C₁₀ alkyl, C₂-C₁₀ alkenyl, C₂-C₁₀ alkynyl, -(C₀-C₆ alkylene)-(C₃-C₁₀ cycloalkyl), -(C₀-C₆ alkylene)-(C₆-C₁₀ aryl), -(C₀-C₆ alkylene)-(4- to 10-membered heterocyclyl), C₁-C₁₀ haloalkyl, C₁-C₁₀ haloalkoxy, -SO₂(C₀₋₁₀ alkyl), -SO₂N(C₀₋₁₀ alkyl)(C₀₋₁₀ alkyl), -COO(C₀₋₁₀ alkyl), -CON(C₀₋₁₀ alkyl)(C₀₋₁₀ alkyl), and -CO(C₀₋₁₀ alkyl), wherein H in the C₀-C₆ alkylene, C₁-C₁₀ alkyl, C₂-C₁₀ alkenyl, C₂-C₁₀ alkynyl, C₀₋₁₀ alkyl, C₃-C₁₀ cycloalkyl, C₆-C₁₀ aryl, or 4- to 10-membered heterocyclyl may be optionally substituted with one or more R₀.

Specifically, R₁₂ is selected from: H, C₁-C₆ alkyl, -C(O)OH, -C(O)O(C₁₋₆ alkyl), -C(O)H, and -C(O)(C₁₋₆ alkyl), wherein H in the C₁-C₆ alkyl may be optionally substituted with a group selected from: halogen, -O(C₀₋₆ alkyl), - COO(C₀₋₆ alkyl), -OCO(C₀₋₆ alkyl), -CON(C₀₋₆ alkyl)(C₀₋₆ alkyl), and -CO(C₀₋₆ alkyl); more specifically, R₁₂ is selected from: H, C₁-C₄ alkyl, C₁-C₄ haloalkyl, -(C₁-C₄ alkylene)-C(O)OH, -(C₁-C₄ alkylene)-C(O)-(C₀₋₄ alkyl), - (C₁-C₄ alkylene)-C(O)O-(C₀₋₄ alkyl), and -CO(C₁₋₄ alkyl). In some embodiments of the present disclosure, R₁₂ is H,

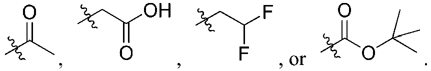

Specifically, each R₁₁ is independently selected from: H, halogen, hydroxy, sulfydryl, amino, C₁-C₆ alkyl, -N(H)(C₁₋₆ alkyl), -N(C₁₋₆ alkyl)(C₁₋₆ alkyl), -O(C₁₋₆ alkyl), -S(C₁₋₆ alkyl), -C(O)OH, and -C(O)O(C₁₋₆ alkyl), wherein H in the C₁-C₆ alkyl may be optionally substituted with a group selected from: halogen, -O(C₀₋₆ alkyl), -COO(C₀₋₆ alkyl), - OCO(C₀₋₆ alkyl), -CON(C₀₋₆ alkyl)(C₀₋₆ alkyl), and -CO(C₀₋₆ alkyl); specifically, each R₁₁ is independently selected from: H, halogen (e.g., F), C₁-C₄ alkyl, C₁-C₄ haloalkyl, -OH, C₁-C₄ alkoxy, C₁-C₄ haloalkoxy, -NH₂, -N(H)(C₁₋₄ alkyl), -N(H)(C₁₋₄ haloalkyl), -N(C₁₋₄ alkyl)(C₁₋₄ haloalkyl), -(C₁-C₄ alkylene)-C(O)OH, -(C₁-C₄ alkylene)-C(O)-(C₀₋₄ alkyl), -(C₁-C₄ alkylene)-C(O)O-(C₀₋₄ alkyl), and -CO(C₁₋₄ alkyl). In some embodiments of the present disclosure, R₁₁ is H, F, methyl, ethyl,

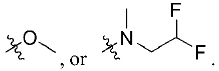

In some embodiments of the present disclosure, moiety 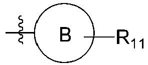 has the following structures: 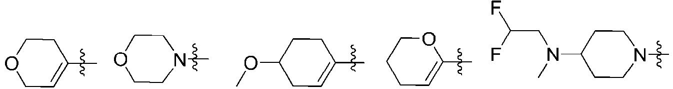 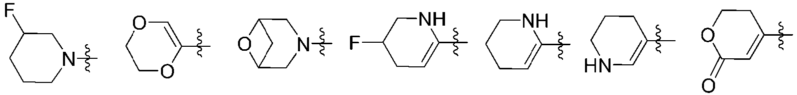 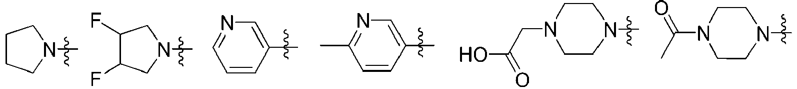 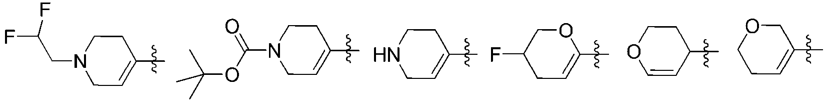 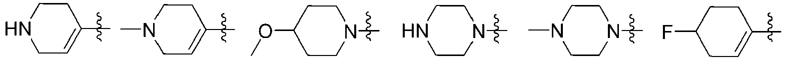 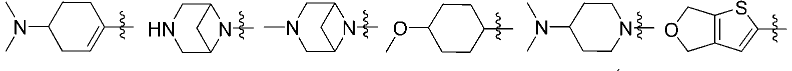 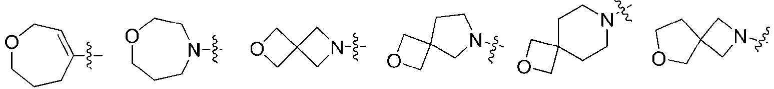 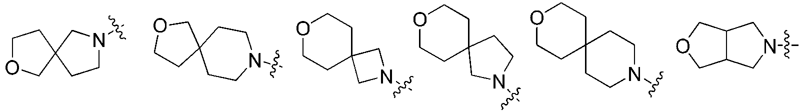 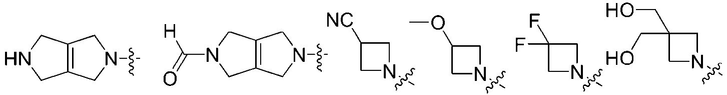 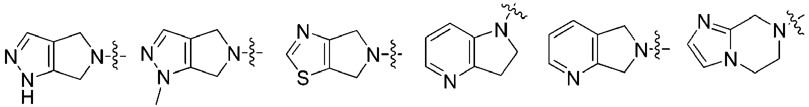 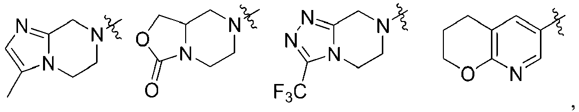 particularly

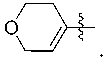

In some embodiments of the present disclosure, A₄ is a single bond, i.e., R₁ is

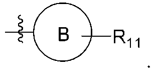

In some embodiments of the present disclosure, R₂ has the following structure: 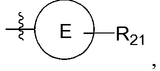 wherein:
ring E is a 4- to 12-membered carbocyclic or heterocyclic ring;
R₂₁ is one or more independent substituents on ring E and is selected from: H, D, C₁-C₁₀ alkyl, C₂-C₁₀ alkenyl, C₂-C₁₀ alkynyl, -(C₀-C₆ alkylene)-(C₃-C₁₀ cycloalkyl), -(C₀-C₆ alkylene)-(C₆-C₁₀ aryl), -(C₀-C₆ alkylene)-(4- to 10-membered heterocyclyl), halogen, cyano, nitro, azido, -SF₅, C₁-C₁₀ haloalkyl, C₁-C₁₀ haloalkoxy, -N(C₀₋₁₀ alkyl)(C₀₋₁₀ alkyl), -N(C₀₋₁₀ alkyl)CO(C₀₋₁₀ alkyl), -N(C₀₋₁₀ alkyl)CON(C₀₋₁₀ alkyl), -N(C₀₋₁₀ alkyl)SO₂(C₀₋₁₀ alkyl), -O(C₀₋₁₀ alkyl), -S(C₀₋₁₀ alkyl), -SO(C₀₋₁₀ alkyl), -SO₂(C₀₋₁₀ alkyl), -SO₂N(C₀₋₁₀ alkyl)(C₀₋₁₀ alkyl), -COO(C₀₋₁₀ alkyl), - OCO(C₀₋₁₀ alkyl), -CON(C₀₋₁₀ alkyl)(C₀₋₁₀ alkyl), and -CO(C₀₋₁₀ alkyl), wherein H in the C₀-C₆ alkylene, C₁-C₁₀ alkyl, C₂-C₁₀ alkenyl, C₂-C₁₀ alkynyl, C₀₋₁₀ alkyl, C₃-C₁₀ cycloalkyl, C₆-C₁₀ aryl, or 4- to 10-membered heterocyclyl may be optionally substituted with one or more R₀; or, two R₂₁, together with the ring atoms to which they are attached, form a carbocyclic or heterocyclic ring, wherein H in the carbocyclic or heterocyclic ring is optionally substituted with one or more R₀.

Specifically, ring E is a 5- to 10-membered aromatic or heteroaromatic ring, for example: 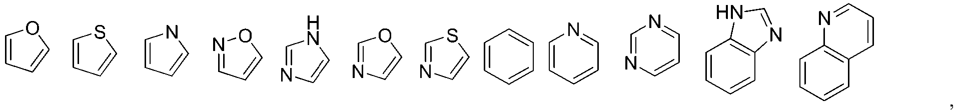 particularly

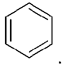

In some embodiments of the present disclosure, moiety 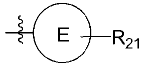 has the following structures: 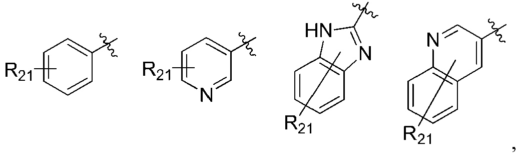 particularly

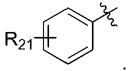

Specifically, R₂₁ is selected from: H, halogen, cyano, -SF₅, hydroxy, sulfydryl, -C(O)H, C₁-C₆ alkyl, C₂-C₆ alkenyl, C₂-C₆ alkynyl, -(C₀-C₆ alkylene)-(C₃-C₆ cycloalkyl), -O(C₁₋₆ alkyl), -S(C₁₋₆ alkyl), and -CO(C₁₋₆ alkyl), wherein H in the C₁-C₆ alkyl, C₂-C₆ alkenyl, C₂-C₆ alkynyl, C₀-C₆ alkylene, or C₃-C₆ cycloalkyl may be optionally substituted with a group selected from: halogen, hydroxy, and C₁-C₃ alkoxy; or, two R₂₁, together with the ring atoms to which they are attached, form a carbocyclic or heterocyclic ring, wherein H in the carbocyclic or heterocyclic ring may be optionally substituted with a group selected from: halogen, hydroxy, and C₁-C₃ alkoxy.

In some embodiments of the present disclosure, R₂ has the following structure: 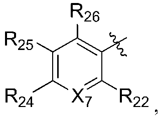 wherein X₇ is selected from: C(R₂₃) and N, and R₂₂ to R₂₆ have the definition of R₂₁; or, R₂₃ and R₂₄ or R₂₂, together with the atoms to which they are attached, form a 4- to 6-membered saturated carbocyclic ring, wherein H in the carbocyclic ring may be optionally substituted with a group selected from: halogen, hydroxy, and C₁-C₃ alkoxy.

Specifically, R₂₂ is selected from: H, halogen, cyano, hydroxy, C₁-C₆ alkyl, -(C₀-C₆ alkylene)-(C₃-C₆ cycloalkyl), and -O(C₁₋₆ alkyl), wherein H in the C₁-C₆ alkyl or C₃-C₆ cycloalkyl may be optionally substituted with a group selected from: halogen, hydroxy, and C₁-C₃ alkoxy; more specifically, R₂₂ is selected from: H, halogen (e.g., F, Cl, or Br), cyano, hydroxy, C₁-C₄ alkyl (e.g., methyl or ethyl), C₁-C₄ haloalkyl (e.g., -CF₃, -CHF₂, or -CH₂F), C₁-C₄ alkoxy (e.g., methoxy), and C₁-C₄ haloalkoxy (e.g., -OCF₃, -OCHF₂, or -OCH₂F).

Specifically, R₂₃ is selected from: H and halogen (e.g., F, Cl, or Br); more specifically, R₂₃ is H or F.

Specifically, R₂₄ is selected from: H, halogen, cyano, -SF₅, hydroxy, -C(O)H, C₁-C₆ alkyl, C₂-C₆ alkenyl, C₂-C₆ alkynyl, -(C₀-C₆ alkylene)-(C₃-C₆ cycloalkyl), -CO(C₁₋₆ alkyl), and -O(C₁₋₆ alkyl), wherein H in the C₁-C₆ alkyl or C₃-C₆ cycloalkyl may be optionally substituted with a group selected from: halogen, hydroxy, and C₁-C₃ alkoxy; more specifically, R₂₄ is selected from: H, halogen (e.g., F, Cl, or Br), cyano, -SF₅, C₁-C₄ alkyl (e.g., methyl or ethyl), C₁-C₄ haloalkyl (e.g., -CF₃, -CHF₂, or -CH₂F), C₁-C₄ haloalkyl (e.g., -CF₃, -CHF₂, or -CH₂F), C₁-C₄ alkoxy (e.g., methoxy), C₁-C₄ haloalkoxy (e.g., -OCF₃, -OCHF₂, or -OCH₂F), and -C(O)H.

Specifically, R₂₅ is selected from: H, halogen, C₁-C₆ alkyl, and C₁-C₆ haloalkyl; more specifically, R₂₅ is selected from: H, halogen (e.g., F, Cl, or Br), C₁-C₄ alkyl (e.g., methyl or ethyl), and C₁-C₄ haloalkyl (e.g., -CF₃, -CHF₂, or - CH₂F).

Specifically, R₂₆ is selected from: H, halogen, cyano, hydroxy, C₁-C₆ alkyl, and -O(C₁₋₆ alkyl); more specifically, R₂₆ is selected from: H, halogen (e.g., F, Cl, or Br), cyano, hydroxy, and C₁-C₄ alkoxy (e.g., methoxy).

In some embodiments of the present disclosure, moiety 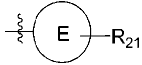 has the following structures: 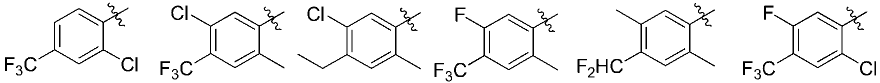 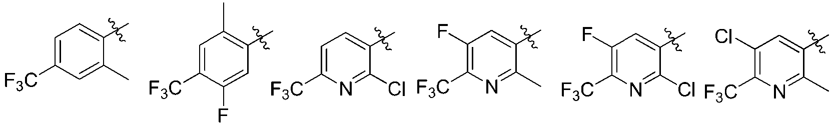 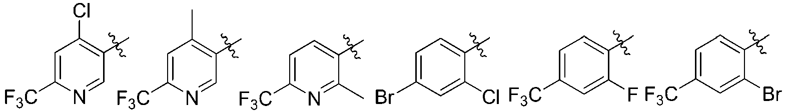 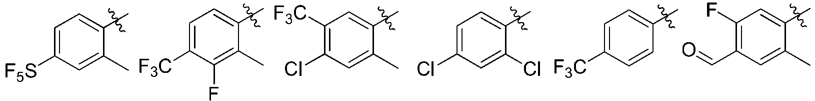 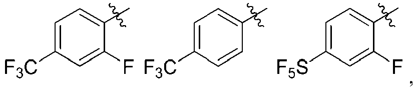 particularly

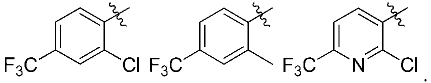

In some embodiments of the present disclosure, moiety 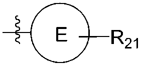 has the following structures:

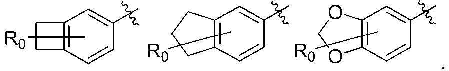

Specifically, R₀ is selected from: H, halogen, hydroxy, and C₁-C₃ alkoxy.

More specifically, moiety 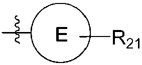 has the following structures:

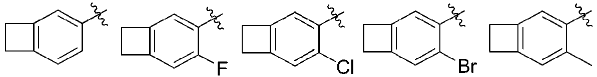

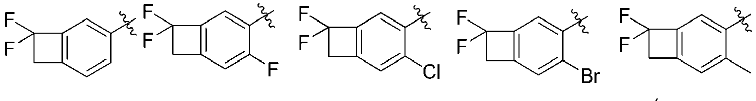

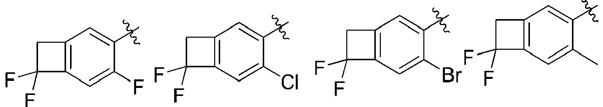

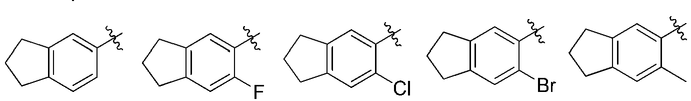

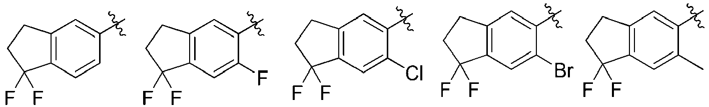

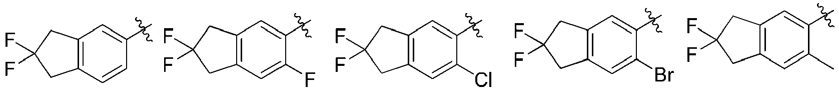

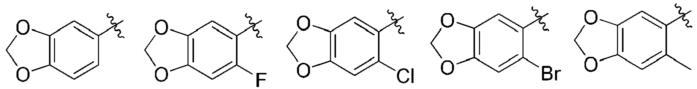

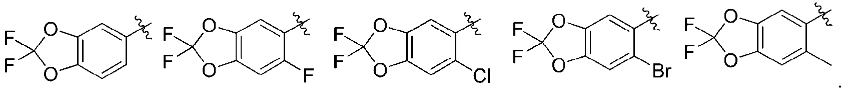

In some embodiments of the present disclosure, A₃ is selected from: -C(O)-, -S(O)-, -S(O)₂-, and 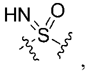 particularly -C(O)-.

In some embodiments of the present disclosure, R₄ has the following structure: 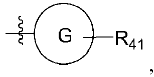 wherein: ring G is a 4- to 10-membered carbocyclic or heterocyclic ring;

R₄₁ is one or more independent substituents on ring G and is selected from: H, D, C₁-C₁₀ alkyl, C₂-C₁₀ alkenyl, C₂-C₁₀ alkynyl, -(C₀-C₆ alkylene)-(C₃-C₁₀ cycloalkyl), -(C₀-C₆ alkylene)-(C₆-C₁₀ aryl), -(C₀-C₆ alkylene)-(4- to 10-membered heterocyclyl), halogen, cyano, nitro, azido, C₁-C₁₀ haloalkyl, C₁-C₁₀ haloalkoxy, -N(C₀₋₁₀ alkyl)(C₀₋₁₀ alkyl), -N(C₀₋₁₀ alkyl)CO(C₀₋₁₀ alkyl), -N(C₀₋₁₀ alkyl)CON(C₀₋₁₀ alkyl), -N(C₀₋₁₀ alkyl)SO₂(C₀₋₁₀ alkyl), -O(C₀₋₁₀ alkyl), -S(C₀₋₁₀ alkyl), -SO(C₀₋₁₀ alkyl), -SO₂(C₀₋₁₀ alkyl), -SO₂N(C₀₋₁₀ alkyl)(C₀₋₁₀ alkyl), -COO(C₀₋₁₀ alkyl), - OCO(C₀₋₁₀ alkyl), -CON(C₀₋₁₀ alkyl)(C₀₋₁₀ alkyl), and -CO(C₀₋₁₀ alkyl), wherein H in the C₀-C₆ alkylene, C₁-C₁₀ alkyl, C₂-C₁₀ alkenyl, C₂-C₁₀ alkynyl, C₀₋₁₀ alkyl, C₃-C₁₀ cycloalkyl, C₆-C₁₀ aryl, or 4- to 10-membered heterocyclyl may be optionally substituted with one or more R₀.

Specifically, ring G is a 5- to 10-membered aromatic or heteroaromatic ring, for example: 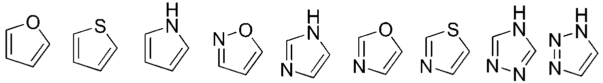 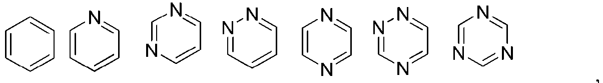 particularly

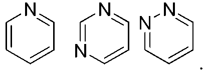

In some embodiments of the present disclosure, moiety 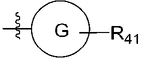 has the following structures: 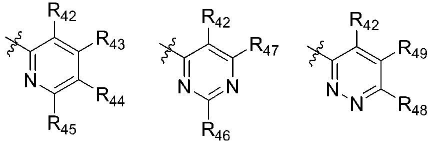 wherein R₄₃ to R₄₉ have the definition of R₄₁, and R₄₂ is selected from: H, hydroxy, and protected hydroxy.

Specifically, R₄₂ is selected from: H, -OH, -O(C₁-C₆ alkyl), -O(benzyl), -O(p-methoxybenzyl), and -O(C₁-C₆ silyl); in some embodiments of the present disclosure, R₄₂ is -OH; in some embodiments of the present disclosure, R₄₂ is H. Specifically, R₄₃ to R₄₉ are independently selected from: H, halogen, cyano, hydroxy, sulfydryl, C₁-C₆ alkyl, C₂-C₆ alkenyl, C₂-C₆ alkynyl, -(C₀-C₆ alkylene)-(C₃-C₆ cycloalkyl), -O(C₁₋₆ alkyl), -O(C₃-C₆ cycloalkyl), -S(C₁₋₆ alkyl), - C(O)H, -CO(C₁₋₆ alkyl), and -NR₄₀₁R₄₀₂, wherein H in the C₀-C₆ alkylene, C₁-C₆ alkyl, or C₃-C₆ cycloalkyl may be optionally substituted with a group selected from: halogen, hydroxy, and C₁-C₃ alkoxy; R₄₀₁ and R₄₀₂ are independently selected from: H, C₁-C₆ alkyl, C₁-C₆ hydroxyalkyl, and C₁-C₆ alkoxyalkyl, or R₄₀₁ and R₄₀₂, together with the nitrogen atom to which they are attached, form a 4- to 8-membered heterocyclic ring (particularly 4- to 6-membered saturated heterocyclic ring, e.g., 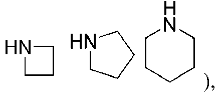 the heterocyclic ring being optionally substituted with a group selected from: halogen, hydroxy, and C₁-C₃ alkoxy.

More specifically, R₄₃ is selected from: H, halogen (e.g., F, Cl, or Br), C₁-C₄ alkyl (e.g., methyl or ethyl), C₁-C₄ haloalkyl (e.g., -CF₃, -CHF₂, or -CH₂F), C₁-C₄ alkoxy (e.g., methoxy), and C₁-C₄ haloalkoxy (e.g., -OCF₃, -OCHF₂, or -OCH₂F); in some embodiments of the present disclosure, R₄₃ is selected from: H, F, Cl, methyl, and -OCF₃. More specifically, R₄₄ is selected from: H, halogen (e.g., F, Cl, or Br), C₁-C₄ alkyl (e.g., methyl or ethyl), and C₁-C₄ haloalkyl (e.g., -CF₃, -CHF₂, or -CH₂F); in some embodiments of the present disclosure, R₄₄ is selected from: H, F, Cl, and methyl.

More specifically, R₄₅ is selected from: H, halogen (e.g., F, Cl, or Br), C₁-C₄ alkyl (e.g., methyl or ethyl), and C₁-C₄ haloalkyl (e.g., -CF₃, -CHF₂, or -CH₂F); in some embodiments of the present disclosure, R₄₅ is selected from: H, Cl, and methyl.

More specifically, R₄₆ is selected from: H, halogen (e.g., F, Cl, or Br), C₁-C₄ alkyl (e.g., methyl or ethyl), C₁-C₄ haloalkyl (e.g., -CF₃, -CHF₂, or -CH₂F), -S(C₁-C₄ alkyl) (e.g., -S-CH₃), -NH₂, -N(H)(C₁₋₆ alkyl), and -N(C₁₋₆ alkyl)(C₁₋₆ alkyl); in some embodiments of the present disclosure, R₄₆ is selected from: H, -S-CH₃, methyl, and -NH₂. More specifically, R₄₇ is selected from: H, halogen (e.g., F, Cl, or Br), C₁-C₄ alkyl (e.g., methyl or ethyl), C₁-C₄ haloalkyl (e.g., -CF₃, -CHF₂, or -CH₂F), C₁-C₄ alkoxy (e.g., methoxy), C₁-C₄ haloalkoxy (e.g., -OCF₃, -OCHF₂, or - OCH₂F), and C₃-C₄ cycloalkyl (e.g., cyclopropyl); in some embodiments of the present disclosure, R₄₇ is selected from: H, Cl, methyl, ethyl, cyclopropyl, -OCF₃, and -OCHF₂.

Specifically, R₄₈ and R₄₉ are independently selected from: H, halogen (e.g., F, Cl, or Br), C₁-C₄ alkyl (e.g., methyl or ethyl), C₁-C₄ haloalkyl (e.g., -CF₃, -CHF₂, or -CH₂F), C₁-C₄ alkoxy (e.g., methoxy), and C₁-C₄ haloalkoxy (e.g., - OCF₃, -OCHF₂, or -OCH₂F).

In some embodiments of the present disclosure, moiety 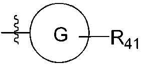 has the following structures: 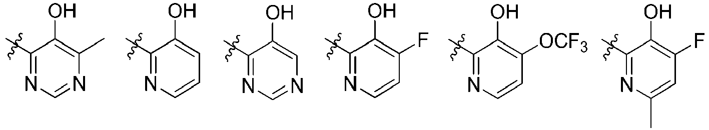 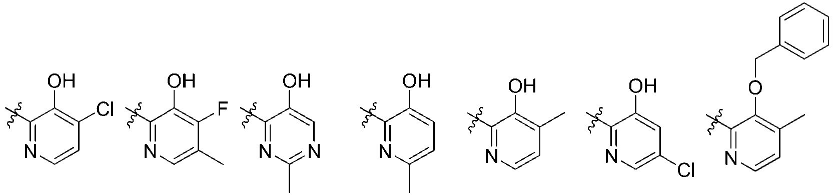 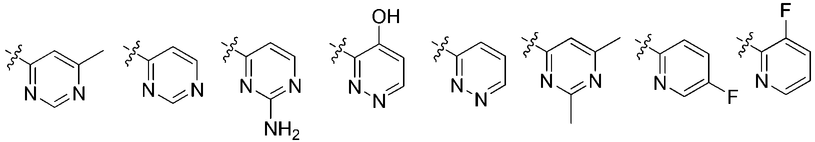 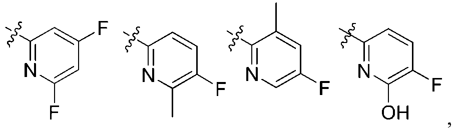 particularly

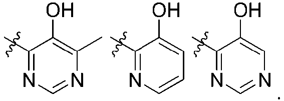

Specifically, R₃ is selected from: H, halogen, hydroxy, sulfydryl, amino, C₁-C₆ alkyl, -(C₀-C₆ alkylene)-(C₃-C₆ cycloalkyl), -O(C₁₋₆ alkyl), -S(C₁₋₆ alkyl), -N(H)(C₁₋₆ alkyl), and -N(C₁₋₆ alkyl)(C₁₋₆ alkyl), wherein H in the C₀-C₆ alkylene, C₁-C₆ alkyl, or C₃-C₆ cycloalkyl may be optionally substituted with a group selected from: D, halogen, hydroxy, and C₁-C₃ alkoxy; more specifically, R₃ is selected from: H, halogen, hydroxy, sulfydryl, amino, C₁-C₄ alkyl, C₁-C₄ haloalkyl, C₁-C₄ deuterated alkyl, C₁-C₄ hydroxy-substituted alkyl, -(C₀-C₃ alkylene)-(C₃-C₄ cycloalkyl), -O(C₁₋₃ alkyl), -S(C₁₋₃ alkyl), -N(H)(C₁₋₃ alkyl), and -N(C₁₋₃ alkyl)(C₁₋₃ alkyl).

In some embodiments of the present disclosure, R₃ is selected from: H and

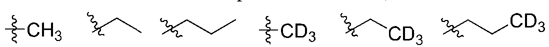

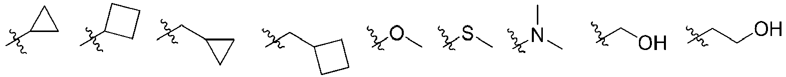

In some embodiments of the present disclosure, the compound has the following structure:

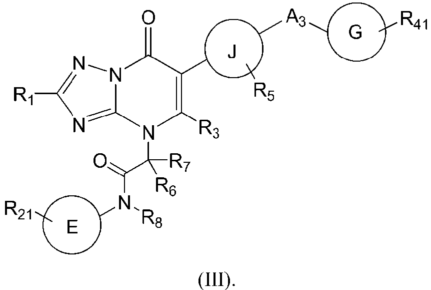

In some embodiments of the present disclosure, the compound has the following structure:

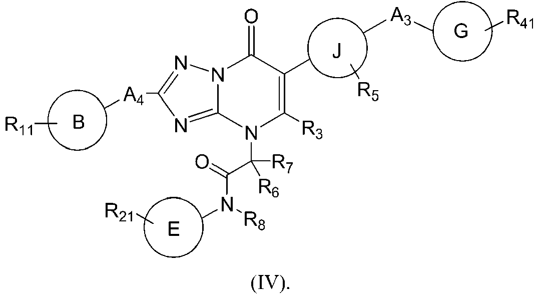

In some embodiments of the present disclosure, the compound has the following structure:

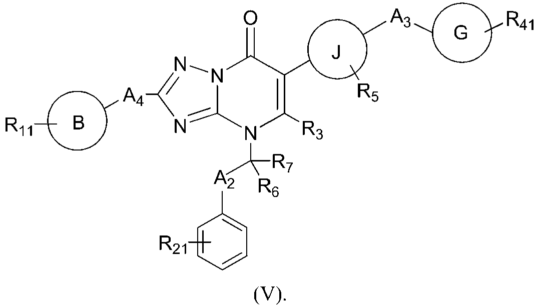

In some embodiments of the present disclosure, the compound has the following structure:

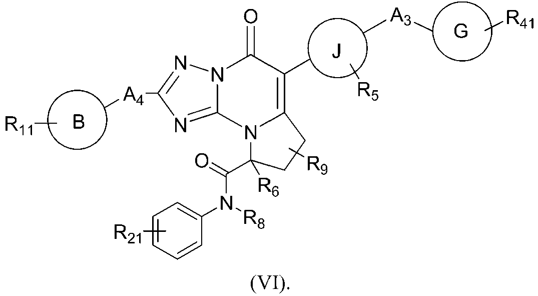

In some embodiments of the present disclosure, the compound has the following structure:

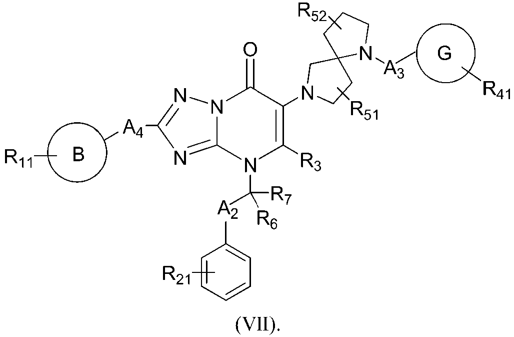

In some embodiments of the present disclosure, the compound has the following structure:

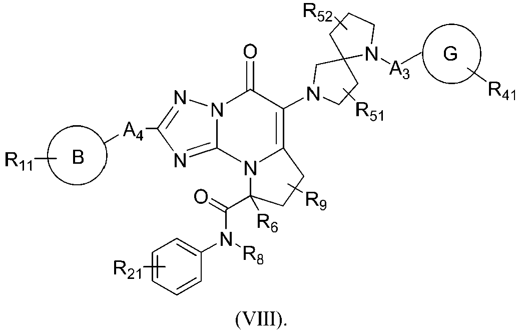

In some embodiments of the present disclosure, a stereoisomer of the compound has the following structures:

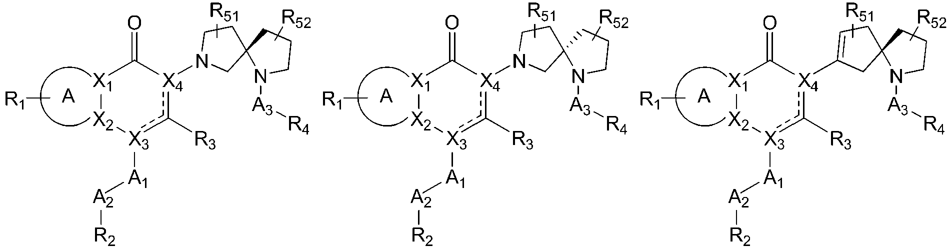

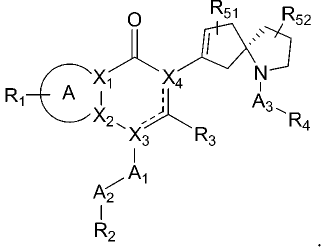

In some embodiments of the present disclosure, a stereoisomer of the compound has the following structures:

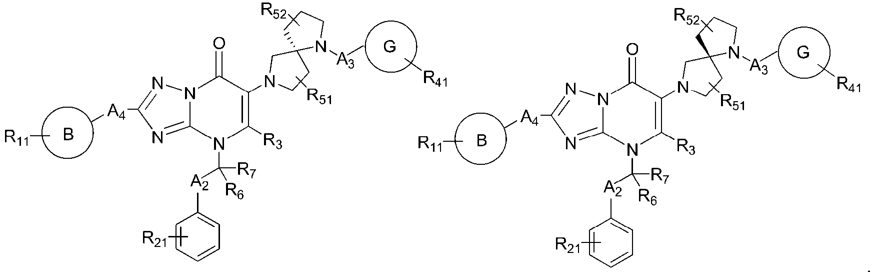

In some embodiments of the present disclosure, a stereoisomer of the compound has the following structures:

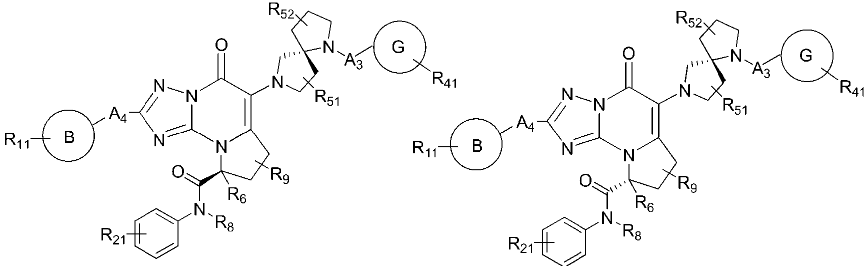

In some embodiments of the present disclosure, the compound has the following structures:

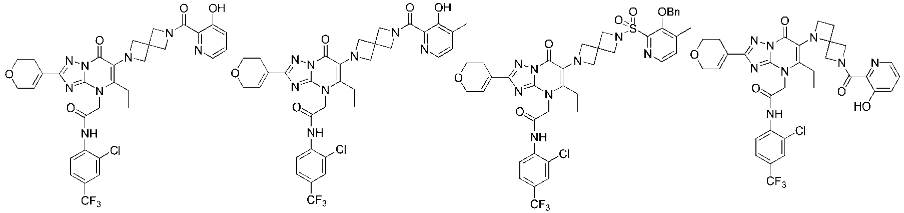

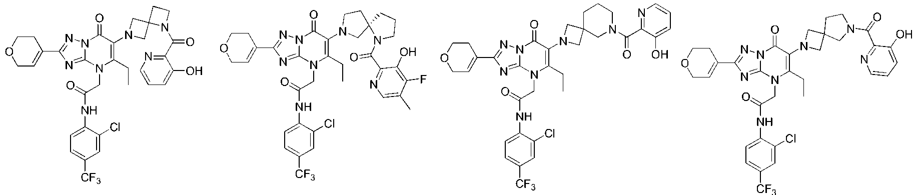

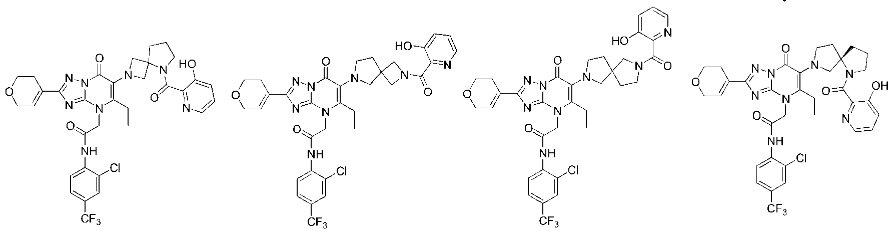

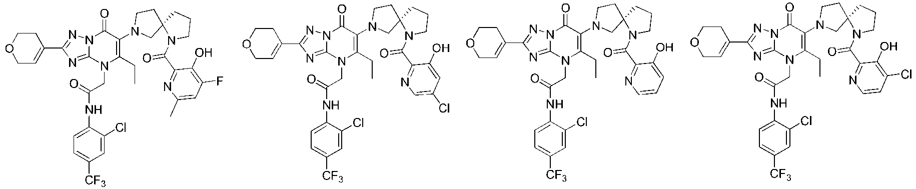

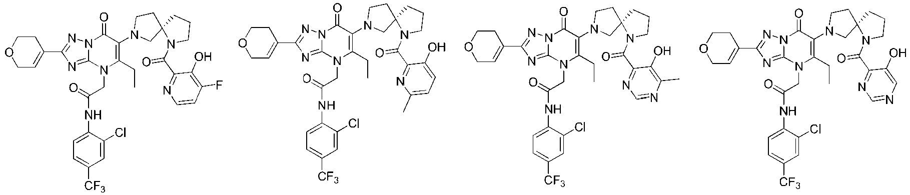

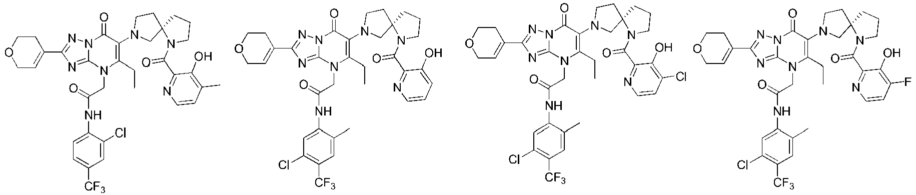

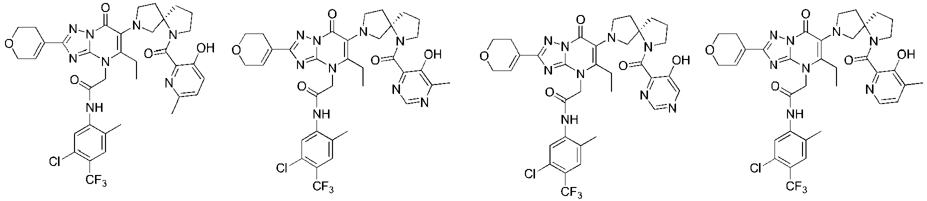

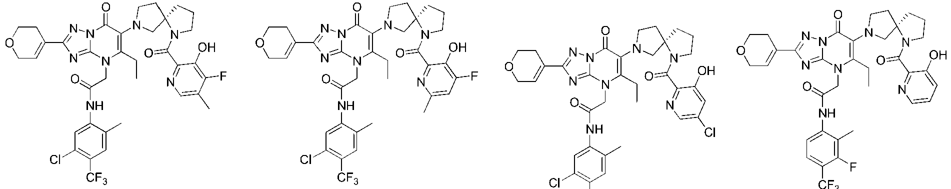

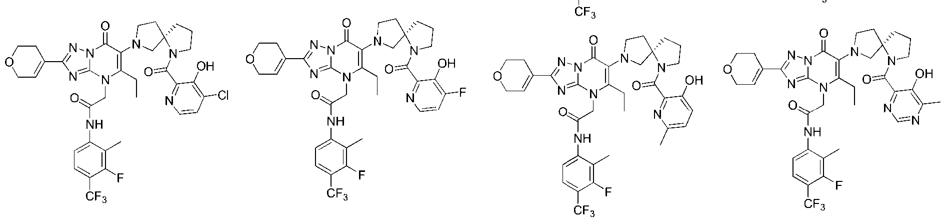

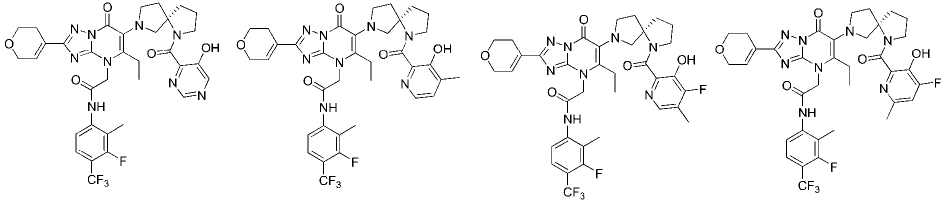

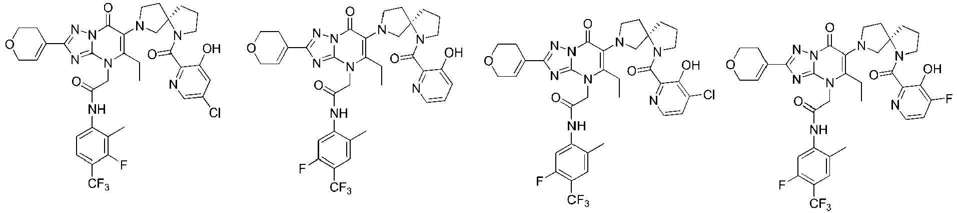

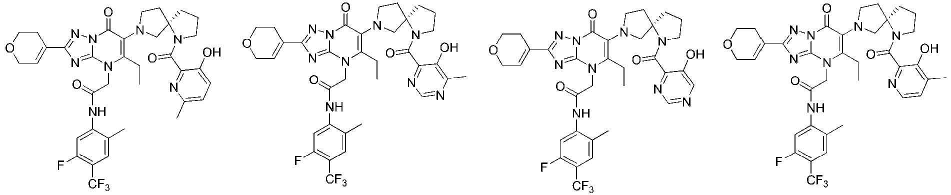

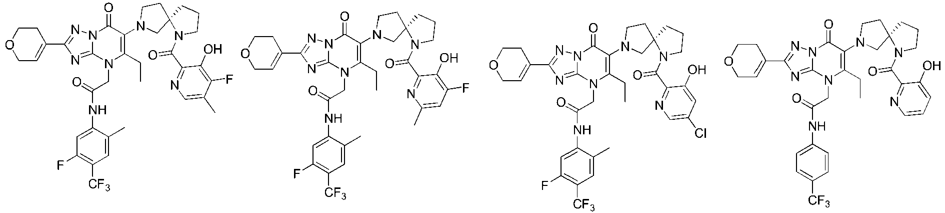

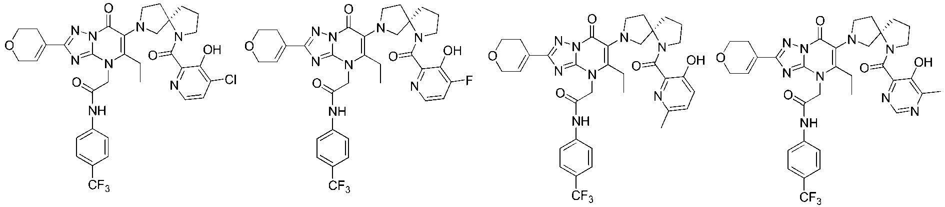

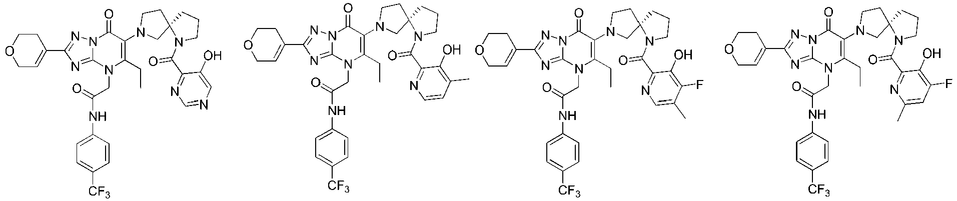

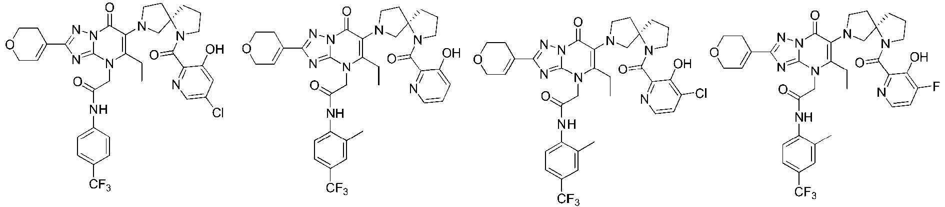

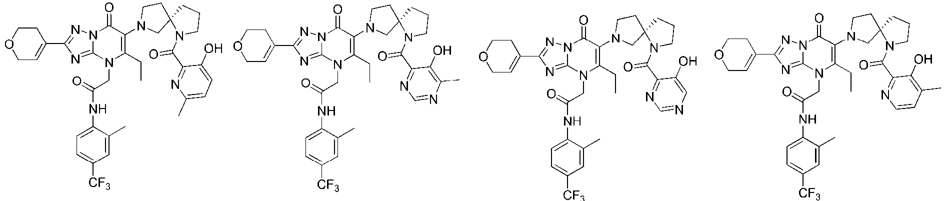

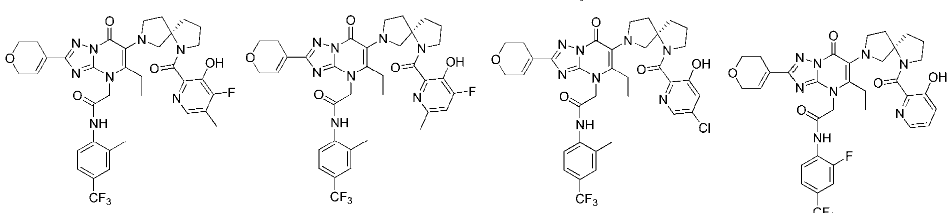

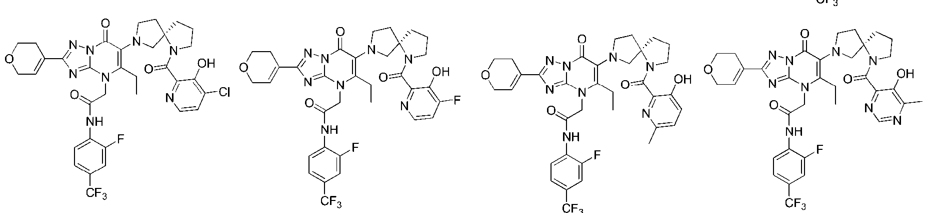

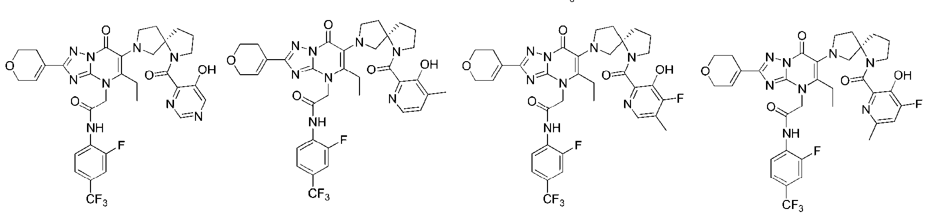

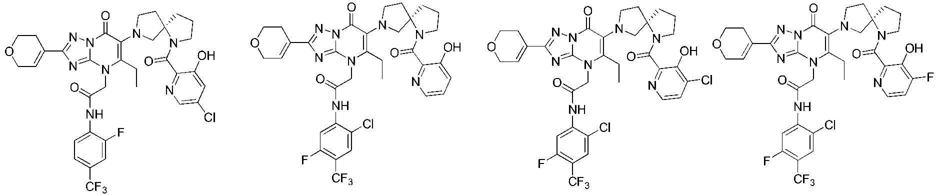

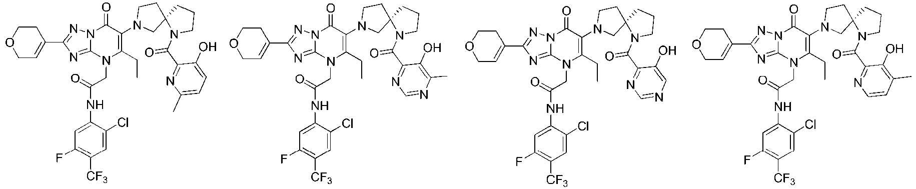

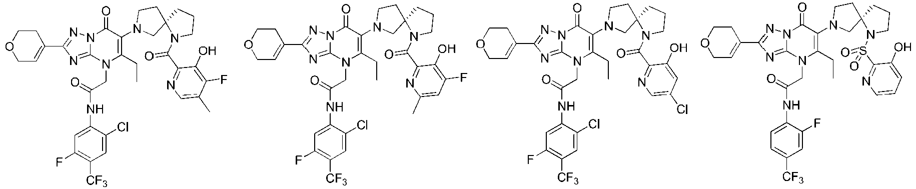

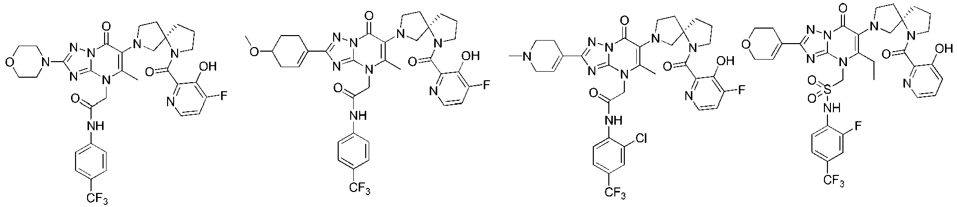

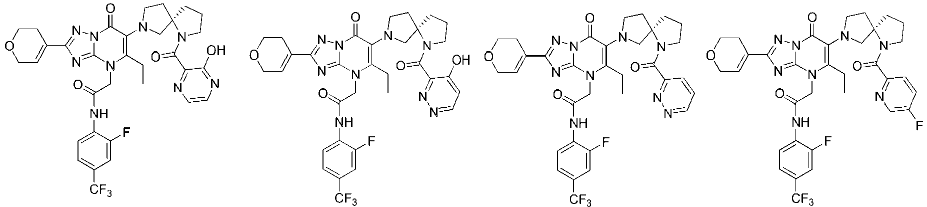

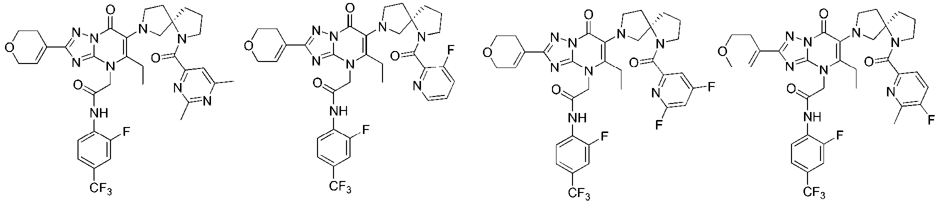

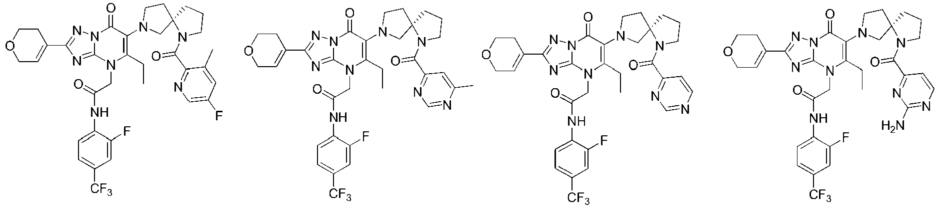

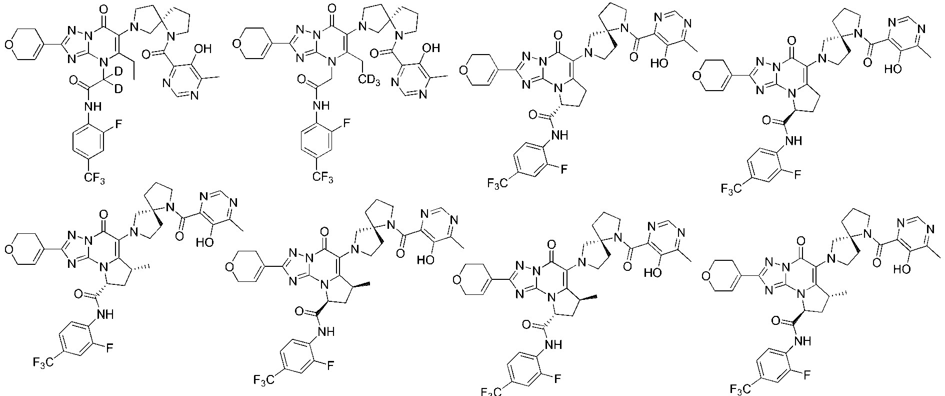

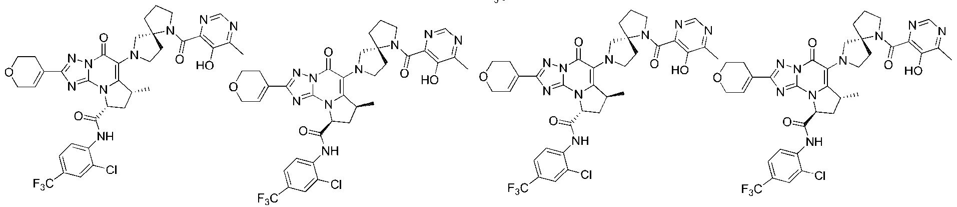

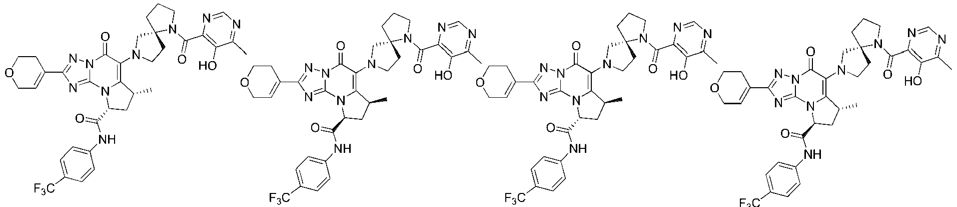

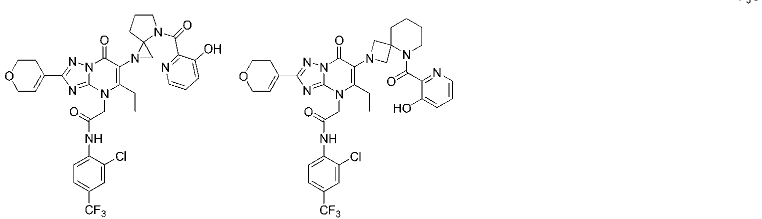

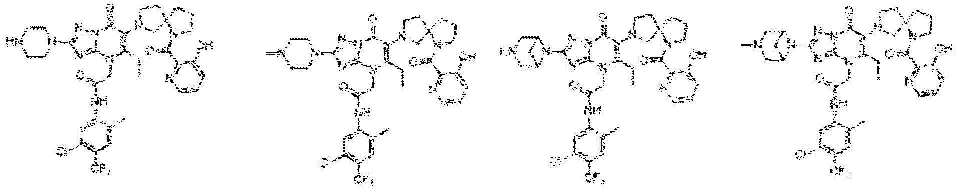

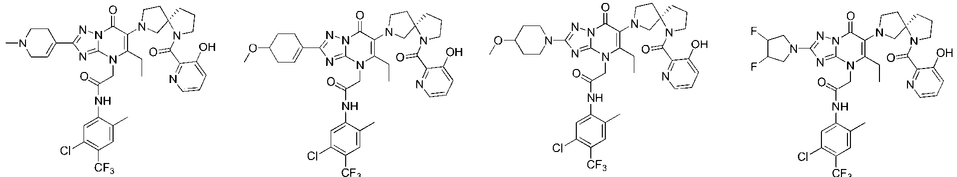

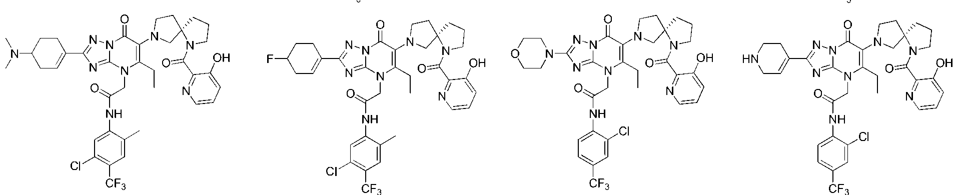

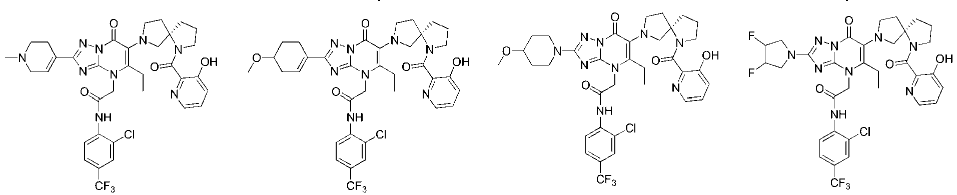

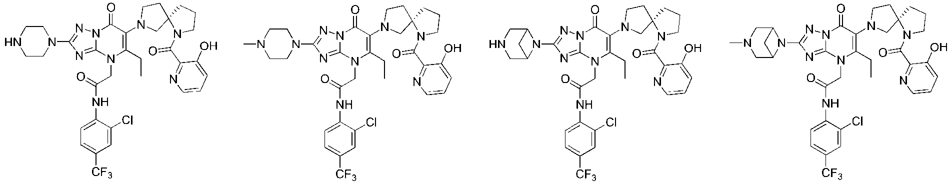

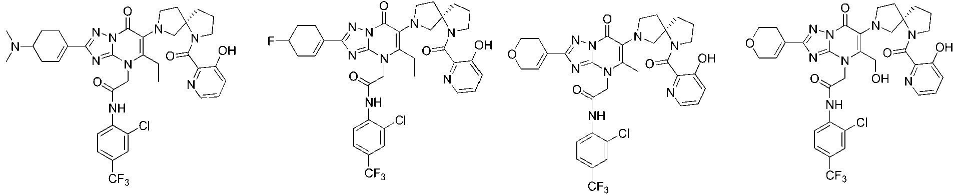

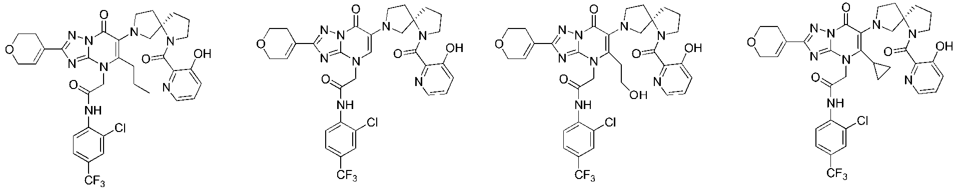

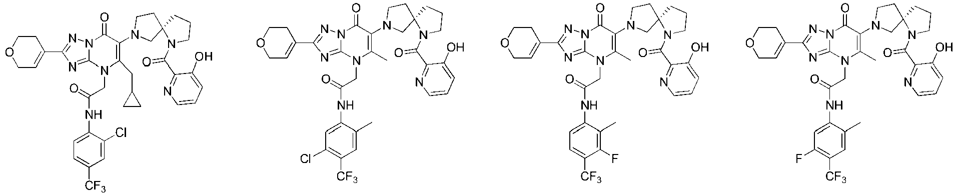

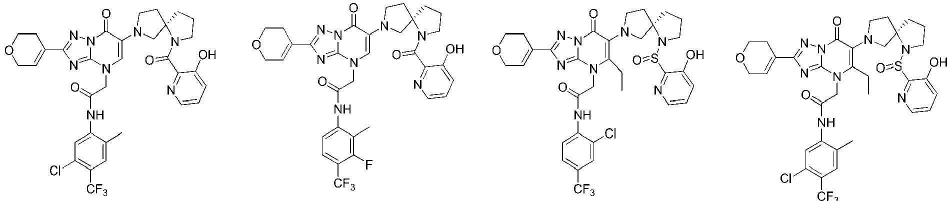

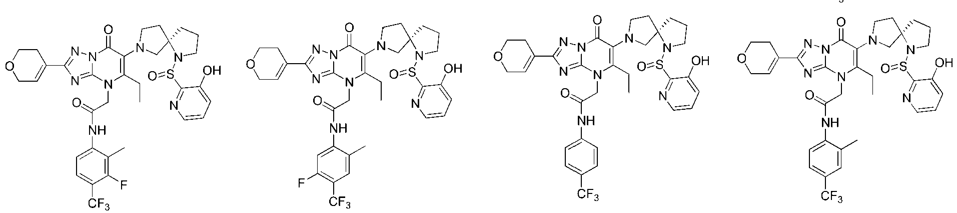

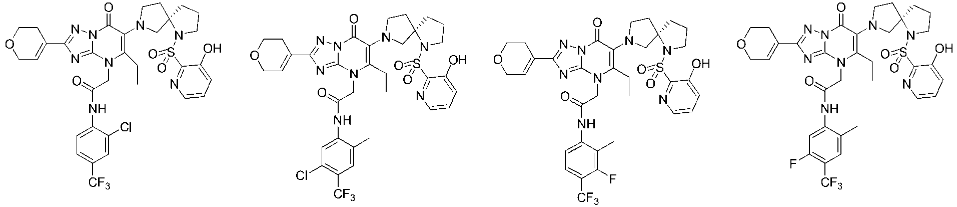

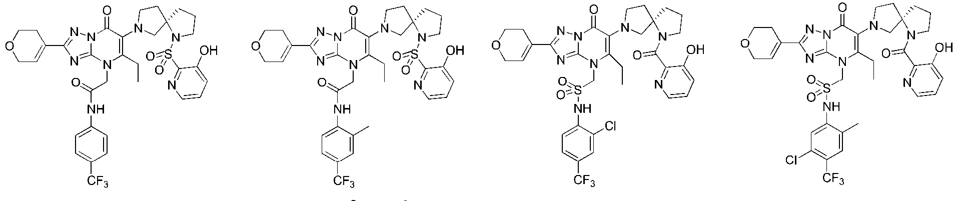

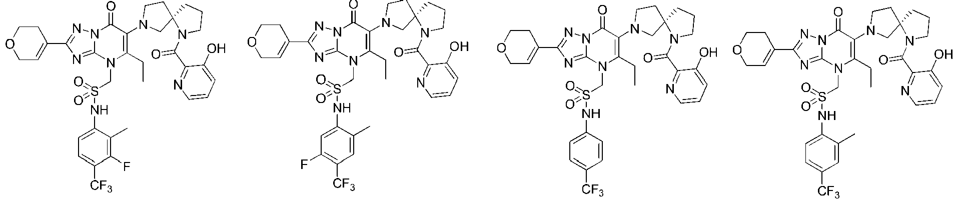

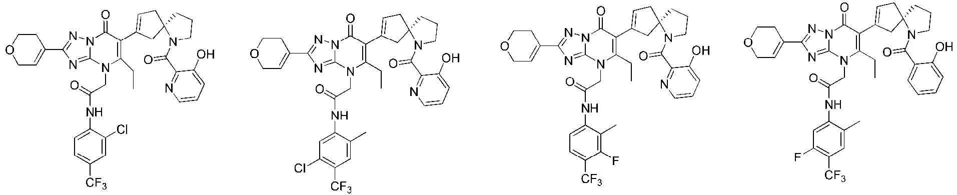

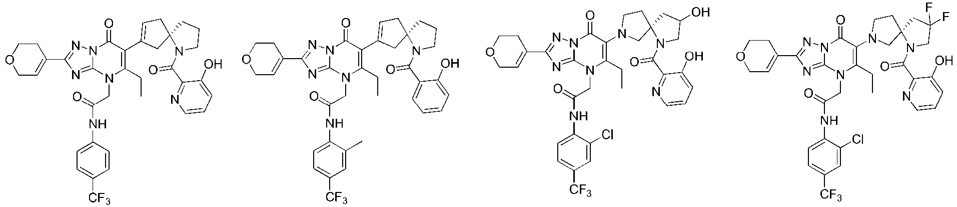

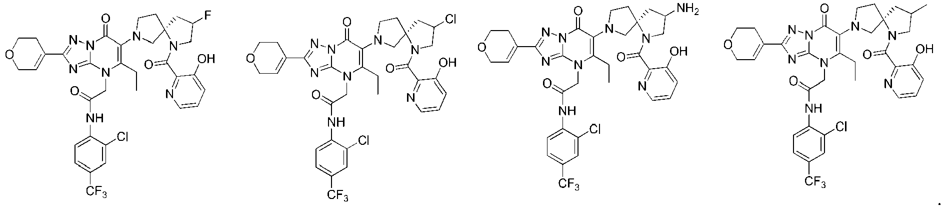

In some embodiments of the present disclosure, the compound has the following structures:

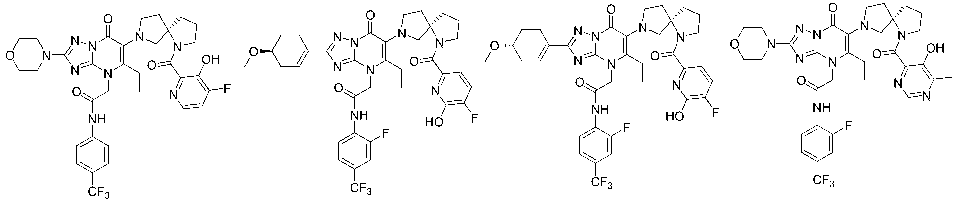

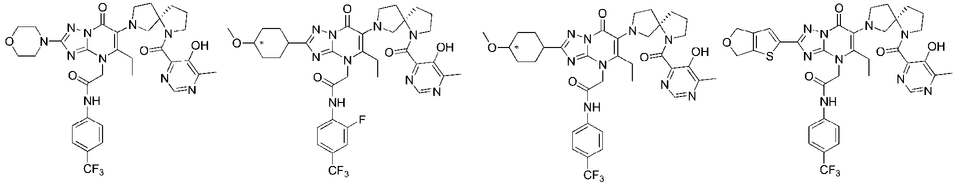

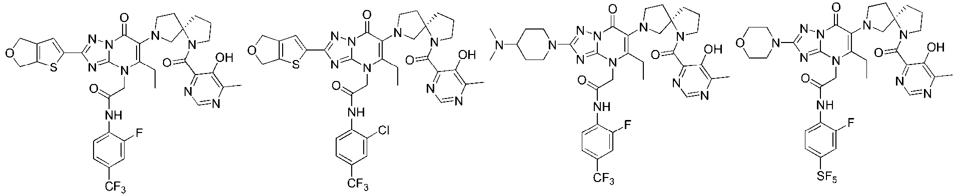

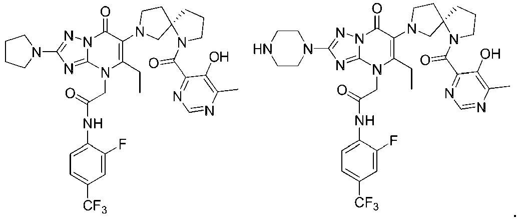

In a second aspect, the present disclosure provides an intermediate compound and a stereoisomer thereof, which has the following structure: 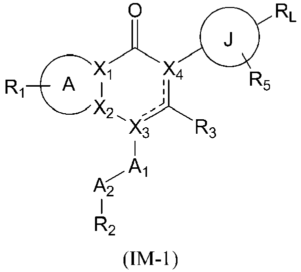
wherein ring A, ring J, X₁, X₂, X₃, X₄, A₁, A₂, R₁, R₂, R₃, and R₅ each have the definition according to the first aspect of the present disclosure;
R_{L} is a reactive group.

In some embodiments of the present disclosure, moiety 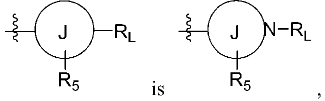 and R_{L} is H or an amino-protecting group (e.g., a Boc group).

In some embodiments of the present disclosure, the intermediate compound has the following structure: 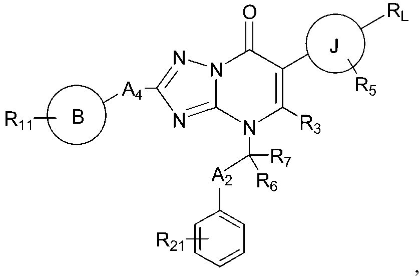 such as 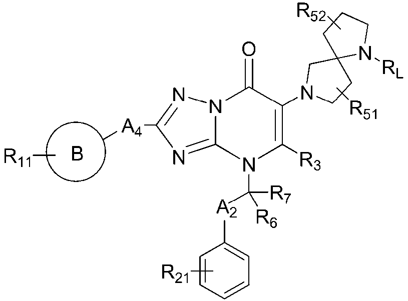 wherein R_{L} may be H or an amino-protecting group (e.g., a Boc group).

In some embodiments of the present disclosure, the intermediate compound has the following structure: 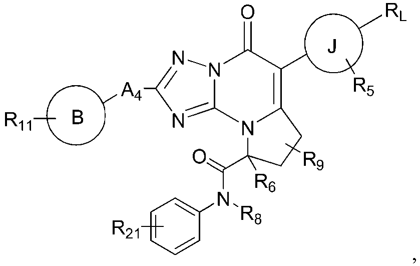 such as

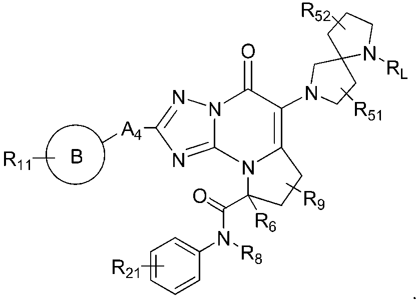

In some embodiments of the present disclosure, R_{L} is H.

In some embodiments of the present disclosure, the intermediate compound has the following structures:

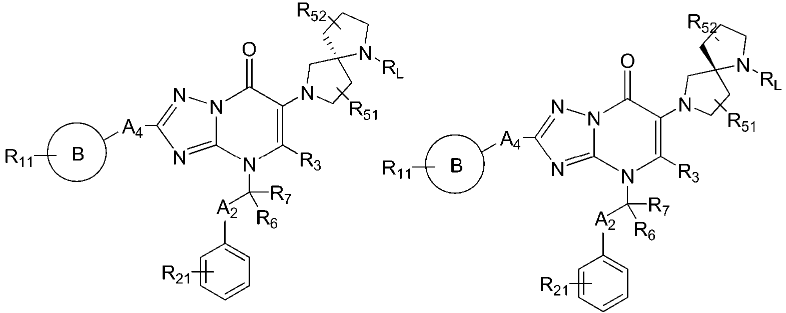

In some embodiments of the present disclosure, the intermediate compound has the following structures:

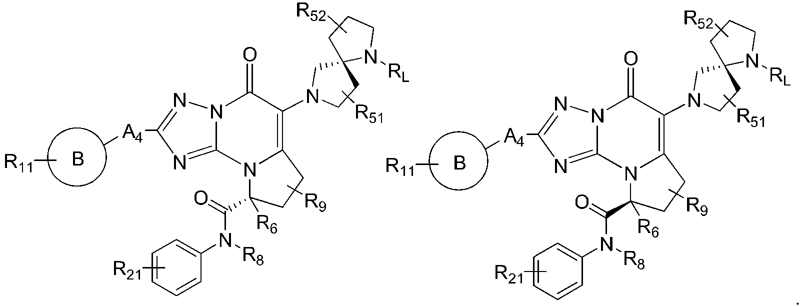

In some embodiments of the present disclosure, the intermediate compound has the following structures:

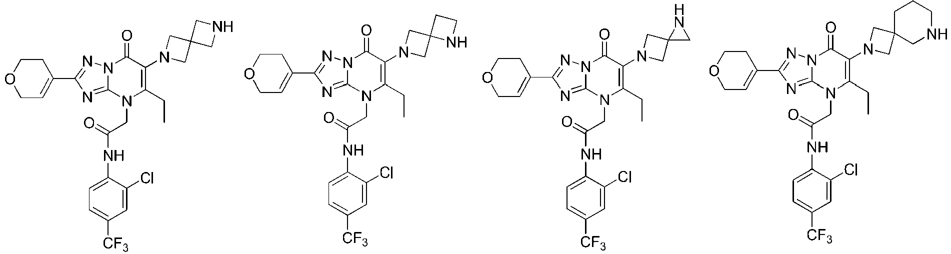

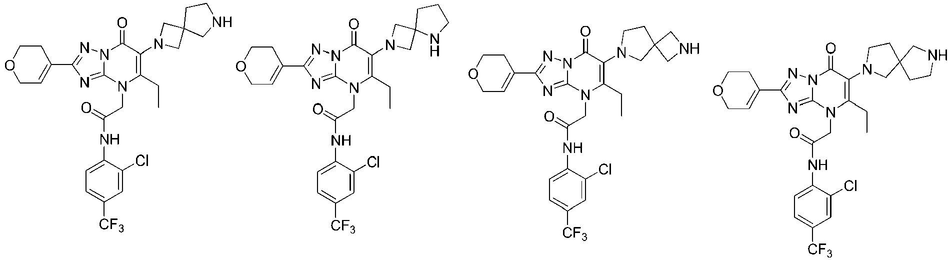

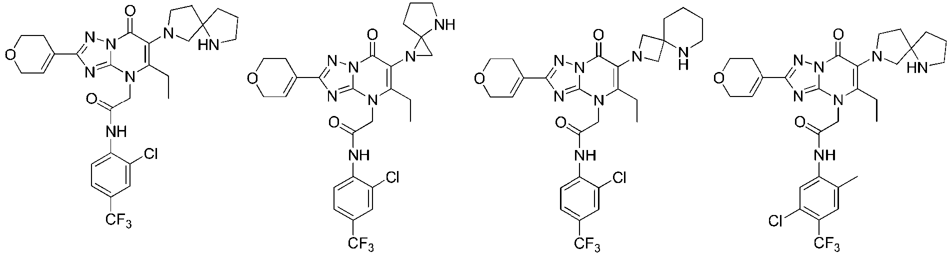

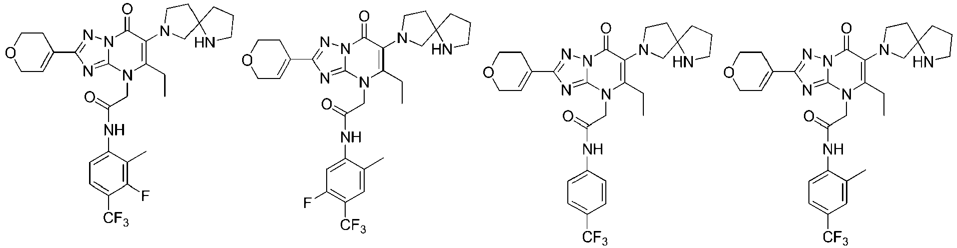

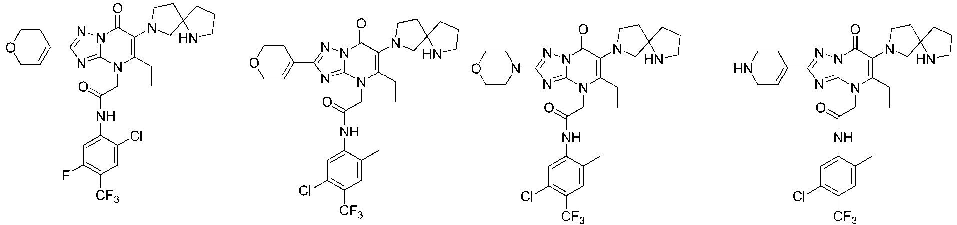

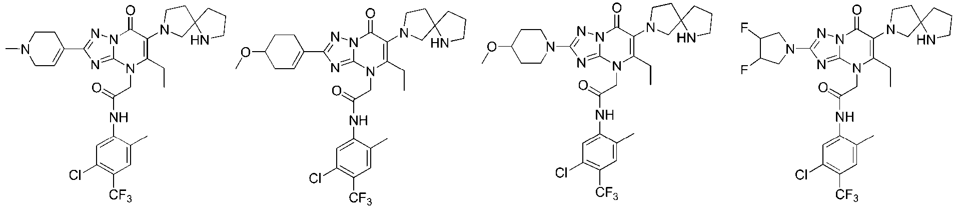

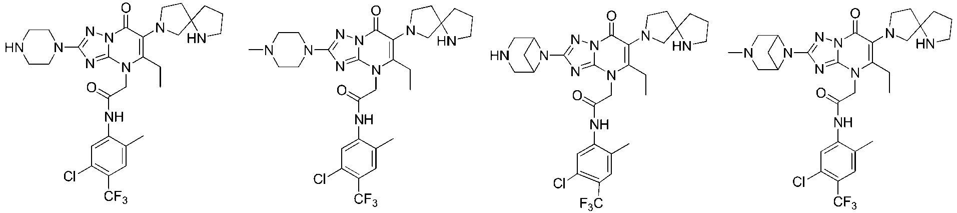

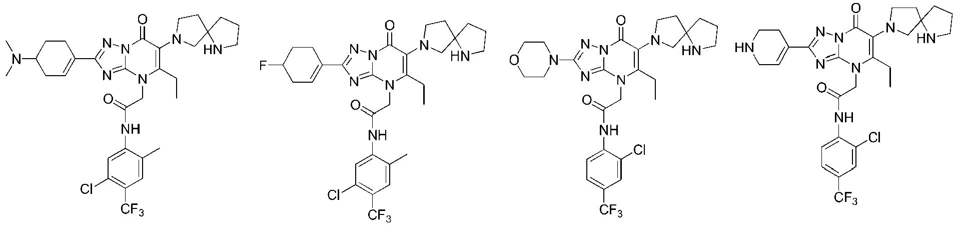

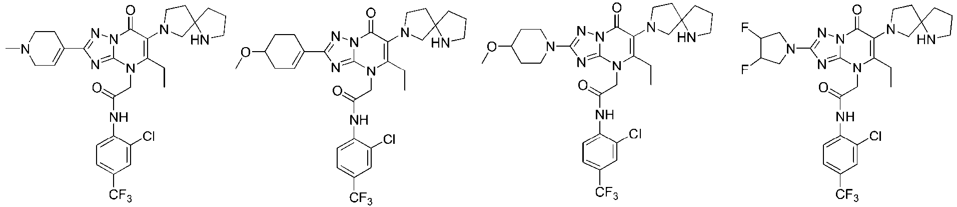

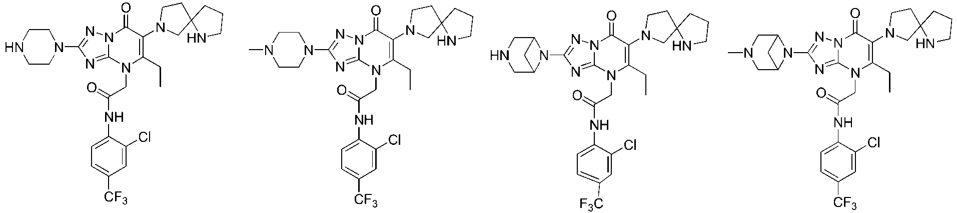

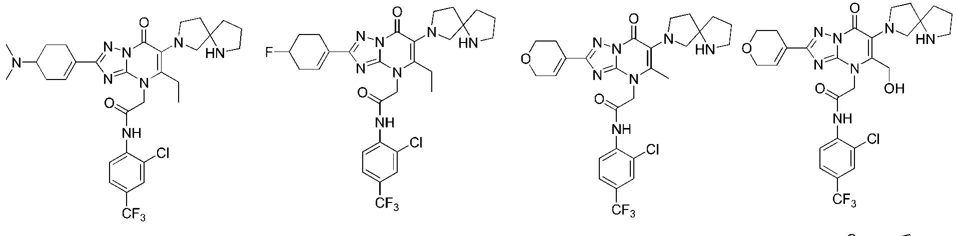

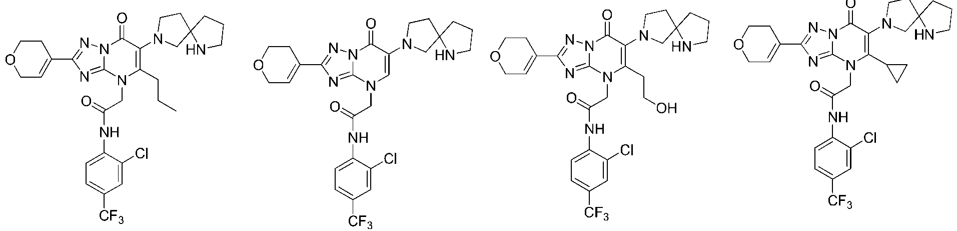

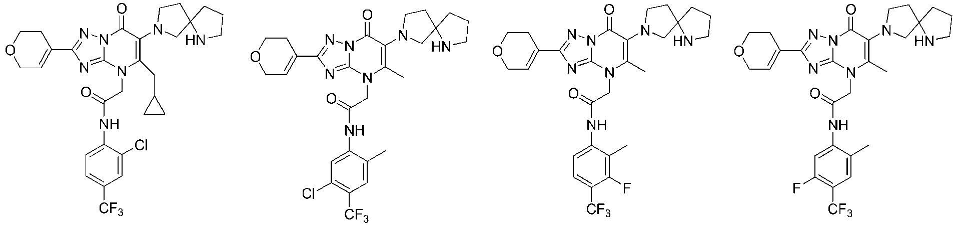

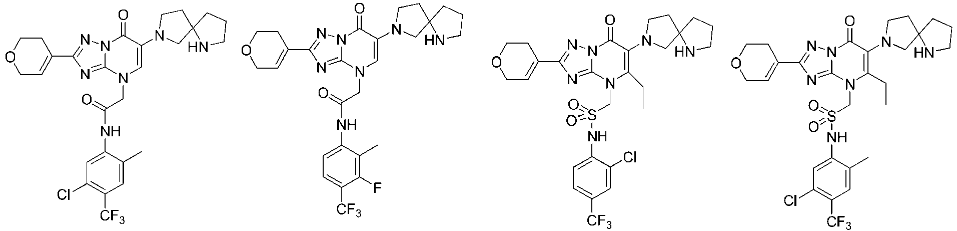

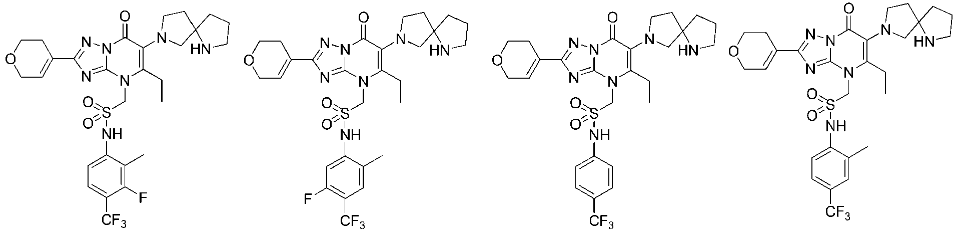

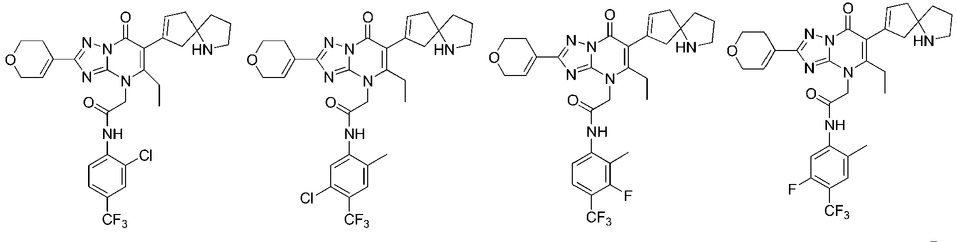

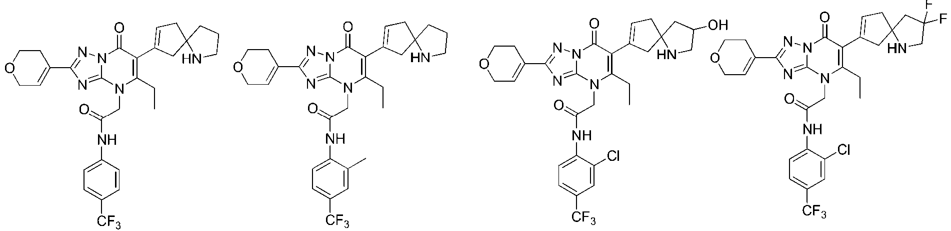

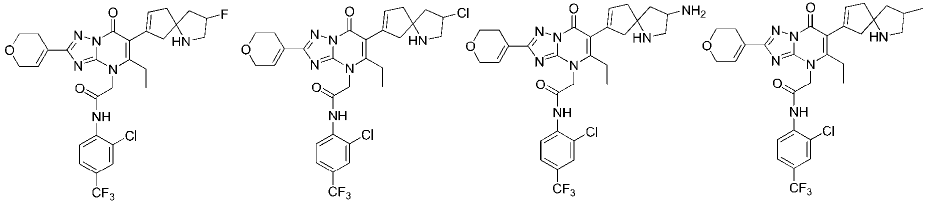

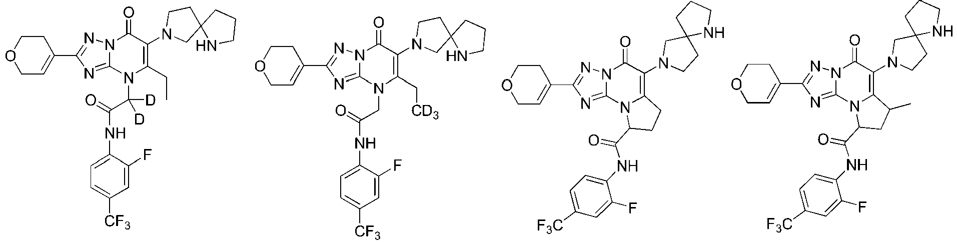

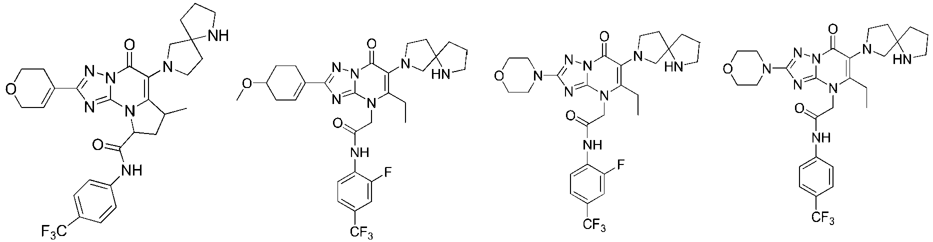

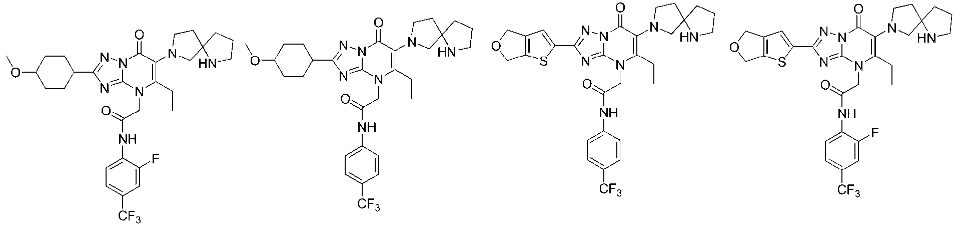

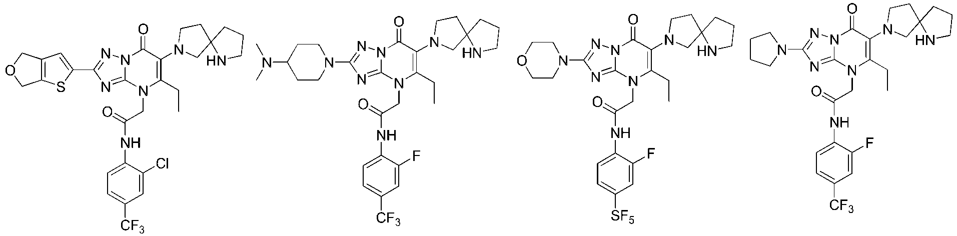

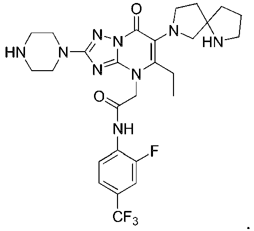

In some embodiments of the present disclosure, also provided is an intermediate compound having the following structures: 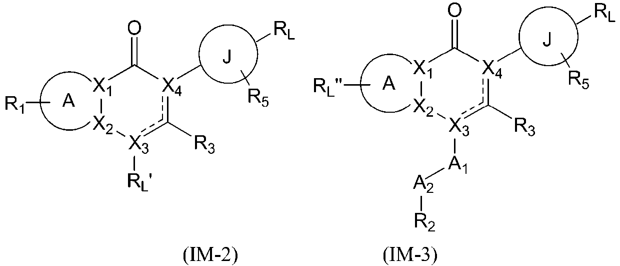
wherein ring A, ring J, X₁, X₂, X₃, X₄, A₁, A₂, R₁, R₂, R₃, and R₅ each have the definition according to the first aspect of the present disclosure;
R_{L}, R_{L}', and R_{L}" are independent reactive groups.

In some embodiments of the present disclosure, a compound represented by formula IM-2 has the following structure: 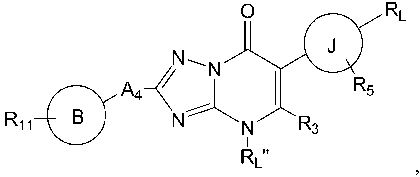 such as 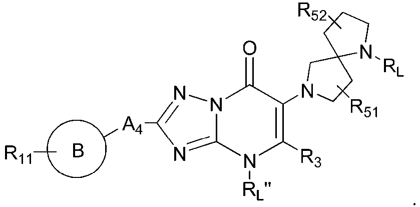 In some embodiments of the present disclosure, a compound represented by formula IM-3 has the following structure: 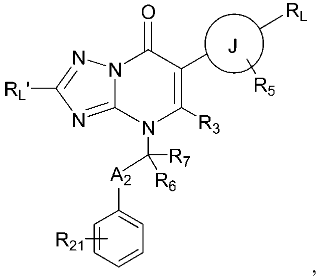 such as

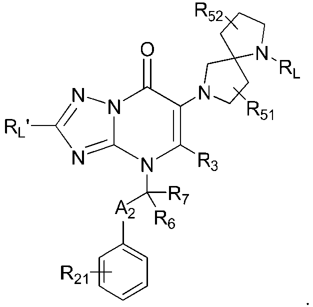

In some embodiments of the present disclosure, a compound represented by formula IM-3 has the following structure: 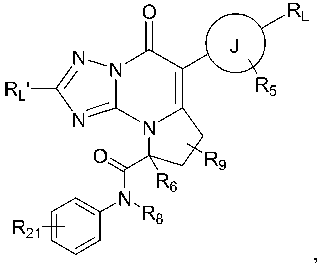 such as

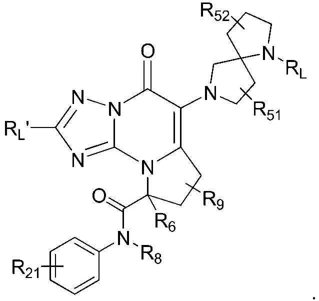

In some embodiments of the present disclosure, R_{L}' is a leaving group, such as halogen (e.g., F, Cl, Br, or I), alkanesulfonyloxy, and arylsulfonyloxy, particularly halogen.

In some embodiments of the present disclosure, R_{L}" is H or an amino-protecting group (e.g., a Boc group).

In a third aspect, the present disclosure provides a preparation method for the compound according to the first aspect of the present disclosure, which comprises a step of reacting a compound represented by formula IM-1 with 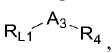 wherein A₃ and R₄ each have the definition according to the first aspect of the present disclosure, and R_{L1} is a leaving group.

In some embodiments of the present disclosure, R_{L1} is halogen (e.g., Cl or Br) or

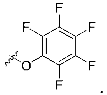

In some embodiments of the present disclosure, the method further comprises a step of preparing a compound represented by formula IM-1, for example, by reacting a compound represented by formula IM-2 with R_{L2}-R₁ or by reacting a compound represented by formula IM-3 with 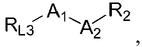 wherein R_{L2} and R_{L3} are leaving groups.

In some embodiments of the present disclosure, the method further comprises a step of preparing a compound represented by formula IM-2, for example, by reacting 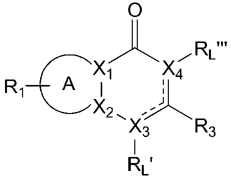 with 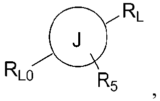 wherein R_{L}‴ and R_{L0} are reactive groups.

In some embodiments of the present disclosure, the method further comprises a step of preparing a compound represented by formula IM-3, for example, by reacting 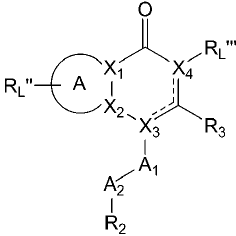 with 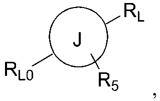 wherein R_{L}‴ and R_{L0} are reactive groups.

In other embodiments of the present disclosure, the method further comprises a step of preparing a compound represented by formula IM-1, for example, by reacting compound 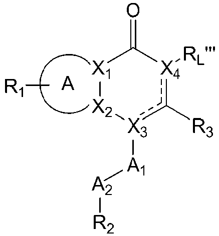 with 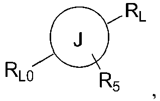 wherein R_{L}‴ and R_{L0} are reactive groups. In some embodiments of the present disclosure, R_{L}‴ is a leaving group, such as halogen (e.g., F, Cl, Br, or I), alkanesulfonyloxy, and arylsulfonyloxy, e.g., Br.

In some embodiments of the present disclosure, 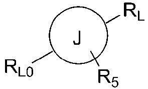 is 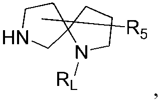 such as

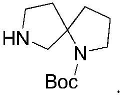

In a fourth aspect, the present disclosure provides a combination, which comprises the compound according to the first aspect or a pharmaceutically acceptable salt, stereoisomer, ester, prodrug, solvate, or deuterated compound thereof, and one or more additional therapeutically active agents.

Specifically, the additional therapeutically active agent may be an anti-cancer agent or a chemotherapeutic agent.

Specifically, the chemotherapeutic agent includes, for example, anastrozole, bicalutamide, bleomycin sulfate, busulfan, capecitabine, N4-pentoxycarbonyl-5-deoxy-5-fluorocytidine, carboplatin, carmustine, chlorambucil, cisplatin, cladribine, cyclophosphamide, cytarabine, cytosine arabinoside, cytarabine liposome injection, dacarbazine, dactinomycin (actinomycin D or Cosmegan), daunorubicin hydrochloride, daunorubicin citrate liposome injection, dexamethasone, docetaxel, doxorubicin hydrochloride, etoposide, fludarabine phosphate 5-fluorouracil, flutamide tezacitibine, gemcitabine (2'-fluoro-2'-deoxycytidine), hydroxyurea, idarubicin, ifosfamide, irinotecan, L-asparaginase, calcium folinate, melphalan, 6-mercaptopurine, methotrexate, mitoxantrone, gemtuzumab (Mylotarg), paclitaxel, phoenix (Yttrium90/MX-DTPA), pentostatin, polifeprosan 20 with carmustine implant, tamoxifen citrate, teniposide, 6-thioguanine, thiotepa, tirapazamine, topotecan hydrochloride for injection, vinblastine, vincristine, and vinorelbine, particularly irinotecan.

In some embodiments of the present disclosure, the additional therapeutically active agent is a PD-1 (e.g., human PD-1) inhibitor. In some embodiments of the present disclosure, the additional therapeutically active agent is a PD-L1 (e.g., human PD-L1) inhibitor. Specifically, the PD-1 or PD-L1 inhibitor may be a PD-1 or PD-L1 antibody molecule.

Specifically, PD-1 inhibitors include, for example, PDR001 (Novartis AG), nivolumab (Bristol-Myers Squibb), pembrolizumab (Merck & Co), pidilizumab (CureTech), MEDI0680 (Medimmune), cemiplimab (REGN2810, Regeneron), dostarlimab (TSR-042, Tesaro), PF-06801591 (Pfizer), tislelizumab (BGB-A317, Beigene), BGB-108 (Beigene), INCSHR1210 (Incyte), balstilimab (AGEN2035, Agenus), sintilimab (InnoVent), toripalimab (Shanghai Junshi Bioscience), camrelizumab (Jiangsu Hengrui Medicine Co.), AMP-224 (Amplimmune), penpulimab (Akeso Biopharma Inc), zimberelimab (Arcus Biosciences), and prolgolimab (Biocad Ltd.), particularly PDR001, and more particularly tislelizumab (BGB-A317, Beigene).

In a fifth aspect, the present disclosure provides a pharmaceutical composition, which comprises the compound according to the first aspect or a pharmaceutically acceptable salt, stereoisomer, ester, prodrug, solvate, or deuterated compound thereof, and one or more pharmaceutically acceptable excipients.

Specifically, the pharmaceutically acceptable excipients may be selected from: one or more of fillers, binders, lubricants, disintegrants, antioxidants, buffers, bacteriostats, suspending agents, solubilizers, thickeners, stabilizers, preservatives, and the like.

Specifically, the pharmaceutical composition may be administered by any suitable administration route, such as a gastrointestinal administration route (e.g., oral, sublingual, or rectal administration) or a parenteral administration route (e.g., intravenous, intramuscular, intranasal, intraocular, intracerebral, intravaginal, intraperitoneal, transdermal, subcutaneous, intradermal, or respiratory administration).

In some embodiments of the present disclosure, the pharmaceutical composition is an oral formulation, including, but not limited to, a tablet (including a sugar-coated tablet, a film-coated tablet, a sublingual tablet, an orally disintegrating tablet, a buccal tablet, etc.), a pill, a powder, a granule, a capsule (including a soft capsule and a microcapsule), a lozenge, a syrup, a liquid, an emulsion, a suspension, and a controlled release formulation (e.g., an instantaneous release formulation, a sustained release formulation, or a sustained release microcapsule).

In some embodiments of the present disclosure, the pharmaceutical composition is an injection (e.g., a subcutaneous injection, an intravenous injection, an intramuscular injection, or an intraperitoneal injection).

In other embodiments of the present disclosure, the pharmaceutical composition is an intravenous infusion formulation, a transdermal formulation, a lotion, a suppository (e.g., a rectal suppository or a vaginal suppository), a nasal formulation, a pulmonary formulation (inhalant), an eye drop formulation, or the like.

Specifically, the pharmaceutical composition is preferably in a unit dosage form. In this form, the formulation is subdivided into unit dosages containing an appropriate amount of active ingredients. The unit dosage form may be capsules, tablets, or any other dosage forms; in addition, the unit dosage form may be packaged formulations such as tablets, capsules, and powders packaged in vials or ampoules.

Specifically, in the pharmaceutical composition, the compound or the pharmaceutically acceptable salt, stereoisomer, ester, prodrug, solvate, or deuterated compound thereof may be used alone or in combination with other therapeutically active agents (as described in the fourth aspect of the present disclosure).

Specifically, various dosage forms of the pharmaceutical composition can be prepared according to conventional production methods in the pharmaceutical field. For example, the active ingredient may be mixed with one or more carriers and then prepared into the desired dosage form.

Specifically, the pharmaceutical composition comprises the active ingredient (the compound according to the first aspect or a pharmaceutically acceptable salt, stereoisomer, ester, prodrug, solvate, or deuterated compound thereof, or a combination thereof with other types of active ingredients) in a weight ratio of 0.1-99.5% (e.g., 0.1%, 0.5%, 1%, 5%, 10%, 15%, 20%, 25%, 30%, 35%, 40%, 45%, 50%, 55%, 60%, 65%, 70%, 75%, 80%, 85%, 90%, 95%, 99%, or 99.5%).

Specifically, the pharmaceutical composition comprises a pharmaceutically acceptable auxiliary material in a weight ratio of 0.5%-99.9% (e.g., 0.5%, 1%, 5%, 10%, 15%, 20%, 25%, 30%, 35%, 40%, 45%, 50%, 55%, 60%, 65%, 70%, 75%, 80%, 85%, 90%, 95%, 99%, 99.5%, or 99.9%).

In a sixth aspect, the present disclosure provides use of the compound according to the first aspect or a pharmaceutically acceptable salt, stereoisomer, ester, prodrug, solvate, or deuterated compound thereof as a WRN helicase inhibitor, for example, in preparing a medicament for preventing and/or treating a WRN-mediated disease. Specifically, the disease is a disease whose prevention and/or treatment can benefit from inhibition of WRN.

In some preferred embodiments of the present disclosure, the disease is a tumor, e.g., acute myeloid leukemia, adolescent cancer, adrenocortical carcinoma of childhood, AIDS-related cancer (e.g., lymphoma and Kaposi's sarcoma), anal cancer, appendix cancer, astrocytoma, atypical teratoid tumor, basal cell carcinoma, bile duct cancer, bladder cancer, bone cancer, brain stem cell glioma, brain tumor, breast cancer, bronchial tumor, Burkitt lymphoma, carcinoid tumor, atypical teratoid tumor, embryonal tumor, blastoma, primary lymphoma, cervical cancer, childhood cancer, chordoma, cardiac tumor, chronic lymphocytic leukemia (CLL), chronic myeloid leukemia (CML), chronic myeloproliferative disorder, colon cancer, colorectal cancer, craniopharyngioma, cutaneous T-cell lymphoma, extrahepatic ductal carcinoma in situ (DCIS), embryonal tumor, CNS cancer, endometrial cancer, ependymoma, esophageal cancer, nasal glioma, Ewing sarcoma, extracranial blastoma, extragonadal blastoma, eye cancer, fibrous histiocytoma of bone, gallbladder cancer, stomach/gastric cancer, gastrointestinal carcinoid tumor, gastrointestinal stromal tumor (GIST), blastoma, gestational trophoblastic tumor, hairy cell leukemia, head and neck cancer, cardiac cancer, liver cancer, Hodgkin lymphoma, hypopharyngeal cancer, intraocular melanoma, islet cell tumor, pancreatic neuroendocrine tumor, renal cancer, laryngeal cancer, lip and oral cancer, liver cancer, lobular carcinoma in situ (LCIS), lung cancer, lymphoma, metastatic squamous neck cancer with occult primary, nuclear protein in testis carcinoma, oral cancer, multiple endocrine neoplasia syndrome, multiple myeloma/plasmacytoma, mycosis fungoides, myelodysplastic syndrome, myelodysplastic/myeloproliferative tumor, multiple myeloma, Merkel cell carcinoma, malignant mesothelioma, malignant fibrous histiocytoma of bone, and osteosarcoma, nasal and paranasal sinus cancer, nasopharyngeal cancer, neuroblastoma, non-Hodgkin lymphoma, non-small cell lung cancer (NSCLC), oral cancer, lip and oral cancer, oropharyngeal cancer, ovarian cancer, pancreatic cancer, papillomatosis, paraganglioma, paranasal sinus and nasal cancer, parathyroid cancer, penile cancer, pharyngeal cancer, pleuropulmonary blastoma, primary central nervous system (CNS) lymphoma, prostate cancer, rectal cancer, transitional cell carcinoma, retinoblastoma, rhabdomyosarcoma, salivary gland cancer, skin cancer, stomach/gastric cancer, small cell lung cancer, small intestine cancer, soft tissue sarcoma, T-cell lymphoma, testicular cancer, throat cancer, thymoma and thymic carcinoma, thyroid cancer, transitional cell carcinoma of the renal pelvis and ureter, trophoblastic tumor, childhood rare cancer, urethral cancer, uterine sarcoma, vaginal cancer, vulvar cancer, or virusinduced cancer.

Particularly, the tumor is a cancer with microsatellite instability (MSI), such as colorectal cancer, stomach/gastric cancer, ovarian cancer, endometrial tumor, and ovarian cancer.

In some embodiments of the present disclosure, the disease is a non-cancerous hyperproliferative disorder, such as benign hyperplasia of skin (e.g., psoriasis), restenosis, or prostatic hypertrophy (e.g., benign prostatic hypertrophy (BPH)).

In a seventh aspect, the present disclosure provides a method for inhibiting WRN helicase, which comprises a step of using the compound according to the first aspect or a pharmaceutically acceptable salt, stereoisomer, ester, prodrug, solvate, or deuterated compound thereof.

In some embodiments of the present disclosure, the method is performed *in vivo.*

In some embodiments of the present disclosure, the method is performed *in vitro.*

In an eighth aspect, the present disclosure provides a method for preventing and/or treating a WRN-mediated disease, which comprises a step of administering to a subject in need thereof an effective amount of the compound according to the first aspect or a pharmaceutically acceptable salt, stereoisomer, ester, prodrug, solvate, or deuterated compound thereof, or the combination according to the fourth aspect, or the pharmaceutical composition according to the fifth aspect.

Specifically, the disease is as described in the sixth aspect of the present disclosure.

Specifically, the subject is a mammal, such as a human.

The present disclosure provides a novel WRN helicase inhibitor, which has good inhibitory activity and can be used to induce high levels of DNA double-strand breaks (DSBs) in cells with microsatellite instability (MSI) by inhibiting WRN, thereby inhibiting the growth of tumor cells with microsatellite instability (MSI). The inhibitor can be used for the prevention and/or treatment of related cancers, particularly colorectal cancer, stomach/gastric cancer, ovarian cancer, endometrial tumors, and the like. It fills the gap of targeted drugs specific to MSI-subtype cancers and possesses promising application prospects and great practical value in the pharmaceutical field.

### BRIEF DESCRIPTION OF THE DRAWINGS

FIG. 1 shows the IC50 curve of compound T074 for inhibiting the WRN helicase activity.
FIG. 2 shows the IC50 curve of compound T074 for inhibiting the ATPase activity.
FIG. 3 shows the EC50 curve of compound T074 for inhibiting the growth of SW48 tumor cells.

### DETAILED DESCRIPTION

Unless otherwise defined, all scientific and technical terms used in the present disclosure have the same meaning as commonly understood by those skilled in the art to which the present disclosure relates.

In the present disclosure, the term "aliphatic group" refers to a linear or branched hydrocarbon chain that is fully saturated or contains one or more unsaturated units, or cyclohydrocarbyl that is fully saturated or contains one or more unsaturated units (also referred to herein as an "aliphatic ring"), which is attached to the rest of the molecule via a single bond. Suitable aliphatic groups include, but are not limited to, linear or branched, substituted or unsubstituted alkyl, alkenyl, alkynyl, and mixtures thereof, such as (cycloalkyl)alkyl, (cycloalkenyl)alkyl, and (cycloalkyl)alkenyl. A typical aliphatic group contains 1 to 10 (e.g., 1, 2, 3, 4, 5, 6, 7, 8, 9, or 10) carbon atoms, preferably 1 to 6 carbon atoms.

The term "carbocyclic ring" consists entirely of carbon atoms and may be classified into aliphatic and aromatic rings. The term "alkyl" refers to a hydrocarbon chain radical that is linear or branched and that does not contain unsaturated bonds, which is attached to the rest of the molecule via a single bond. A typical alkyl group contains 1 to 10 (e.g., 1, 2, 3, 4, 5, 6, 7, 8, 9, or 10) carbon atoms, preferably 1 to 6 carbon atoms; examples include methyl, ethyl, n-propyl, isopropyl, n-butyl, isobutyl, tert-butyl, n-pentyl, isopentyl, neopentyl, tert-pentyl, n-hexyl, isohexyl, and the like. If alkyl is substituted with cycloalkyl, it is correspondingly "cycloalkylalkyl", such as cyclopropylmethyl, cyclopropylethyl, cyclobutylmethyl, cyclopentylmethyl, or cyclohexylmethyl. If alkyl is substituted with aryl, it is correspondingly "aralkyl", such as benzyl, benzhydryl, or phenethyl. If alkyl is substituted with heterocyclyl, it is correspondingly "heterocyclylalkyl". In the present disclosure, C₀ alkyl refers to H, i.e., C₀₋₁₀ alkyl (or C₀-C₁₀ alkyl), which includes H and C₁₋₁₀ alkyl (or C₁-C₁₀ alkyl).

The term "alkylene" refers to hydrocarbyl (divalent alkyl) formed from an alkane molecule by losing two hydrogen atoms, which may be linear or branched and is attached to the rest of the molecule via a single bond. A typical alkylene group herein contains 1 to 10 (e.g., 1, 2, 3, 4, 5, 6, 7, 8, 9, or 10) carbon atoms, preferably 1 to 6 carbon atoms; examples include methylene (-CH₂-), ethylidene, propylidene, butylidene, and the like. In the present disclosure, C₀ alkylene refers to a single bond, i.e., C₀₋₁₀ alkylene (or C₀-C₁₀ alkylene) includes a single bond and C₁₋₁₀ alkylene (or C₁-C₁₀ alkylene).

The term "cycloalkyl" refers to an alicyclic hydrocarbon, for example, containing 1 to 4 monocyclic and/or fused rings and 3-18 carbon atoms, preferably 3-10 (e.g., 3, 4, 5, 6, 7, 8, 9, or 10) carbon atoms; examples include cyclopropyl, cyclobutyl, cyclopentyl, cyclohexyl, adamantyl, or the like.

The term "alkoxy" refers to a substituent formed from a hydroxy group by substituting the hydrogen atom with alkyl, such as an alkoxy group containing 1-10 carbon atoms, e.g., methoxy, ethoxy, propoxy, butoxy, and the like.

The term "aryl" refers to a monocyclic or polycyclic radical, including polycyclic radicals containing monoaryl and/or fused aryl groups, e.g., containing 1-3 monocyclic or fused rings and 6-18 (e.g., 6, 8, 10, 12, 14, 16, or 18) carbon ring atoms. Typical aryl is an aryl group containing 6-12 carbon ring atoms, such as phenyl, naphthyl, biphenyl, or indenyl. "Arylene" refers to a divalent group derived from an aromatic hydrocarbon by the removal of two hydrogen atoms.

The term "heterocyclyl" includes heteroaromatic and heteroalicyclic groups containing 1 to 3 monocyclic and/or fused rings and 3 to about 18 (specifically 3, 4, 5, 6, 7, 8, 9, 10, 11, 12, 14, 16, or 18) ring atoms. Preferred heteroaromatic and heteroalicyclic groups contain 4 to about 10 (e.g., 4, 5, 6, 7, 8, 9, or 10) ring atoms. Suitable heteroaryl in the compound of the present disclosure contains 1, 2, or 3 heteroatoms selected from N, O, and S atoms. Examples of heteroaryl are, for example, but not limited to, coumarin, including 8-coumarin, quinolyl, including 8-quinolyl, isoquinolyl, pyridinyl, pyrazinyl, pyrazolyl, pyrimidinyl, furyl, pyrrolyl, thienyl, thiazolyl, isothiazolyl, triazolyl, tetrazolyl, isoxazolyl, oxazolyl, imidazolyl, indolyl, isoindolyl, indazolyl, indolizinyl, phthalazinyl, pteridinyl, purinyl, oxadiazolyl, thiadiazolyl, furazanyl, pyridazinyl, triazinyl, cinnolinyl, benzimidazolyl, benzofuryl, benzofurazanyl, benzothienyl, benzothiazolyl, benzoxazolyl, quinazolinyl, quinoxalinyl, naphthyridinyl, furopyridinyl, and the like. Suitable heteroalicyclic groups in the compound of the present disclosure contain 1, 2, or 3 heteroatoms selected from N, O, and S atoms. Examples of heteroalicyclic groups are, for example, but not limited to, pyrrolidinyl, tetrahydrofuryl, dihydrofuran, tetrahydrothienyl, tetrahydrothiopyranyl, piperidinyl, morpholinyl, thiomorpholinyl, oxathianyl, piperazinyl, azetidinyl, oxetanyl, thietanyl, homopiperidinyl, oxiranyl, thiiranyl, azepinyl, oxazepanyl, diazepinyl, triazepinyl, 1,2,3,6-tetrahydropyridinyl, 2-pyrrolinyl, 3-pyrrolinyl, indolinyl, 2H-pyranyl, 4H-pyranyl, dioxanyl, 1,3-dioxolanyl, pyrazolinyl, dithianyl, dithiolanyl, dihydropyranyl, dihydrothienyl, pyrazolidinyl, imidazolinyl, imidazolidinyl, 3-azabicyclo[3.1.0]hexyl, 3-azabicyclo[4.1.0]heptyl, 3H-indolyl, quinolizinyl, and the like.

The groups described above may be substituted at one or more available positions with one or more suitable groups such as, OR', =O, SR', SOR', SO₂R', OSO₂R', OSO₃R', NO₂, NHR', N(R')₂, =N-R', N(R')COR', N(COR')₂, N(R')SO₂R', N(R')C(=NR')N(R')R', N₃, CN, halogen, COR', COOR', OCOR', OCOOR', OCONHR', OCON(R')₂, CONHR', CON(R')₂, CON(R')OR', CON(R')SO₂R', PO(OR')₂, PO(OR')R', PO(OR')(N(R')R'), C₁-C₁₂ alkyl, C₃-C₁₀ cycloalkyl, C₂-C₁₂ alkenyl, C₂-C₁₂ alkynyl, aryl, and heterocyclyl, wherein each R' group is independently selected from hydrogen, OH, NO₂, NH₂, SH, CN, halogen, COH, CO alkyl, COOH, C₁-C₁₂ alkyl, C₃-C₁₀ cycloalkyl, C₂-C₁₂ alkenyl, C₂-C₁₂ alkynyl, aryl, and heterocyclyl. These groups themselves are substituted, and the substituents may be selected from the foregoing list.

"Halogen" refers to bromine, chlorine, iodine, or fluorine. Haloalkyl refers to a group in which a hydrogen atom on an alkyl group is substituted with a halogen atom (F, Cl, Br, and I), such as -CH₂Rh, -CHRh₂, and -CRh₃, wherein Rh is F, Cl, Br, or I; such as -CF₃.

The term "pharmaceutically acceptable salt" refers to an acidic or basic salt that is theoretically non-toxic, nonirritating, and non-allergic, and capable of achieving or providing clinically acceptable pharmacokinetic, absorption, distribution, and metabolic properties of pharmaceutical molecules to be capable of achieving intended purposes. The salt described in the present disclosure includes a pharmaceutically acceptable acid or basic salt of an acidic, basic, or amphoteric group in the compound. A list of suitable salts can be found in S.M. Birge et al., J. Pharm. Sci., 66, 1-19 (1977).

The pharmaceutically acceptable salts described in the present disclosure include acid addition salts and base addition salts.

The acid addition salts include, but are not limited to, salts derived from inorganic acids, such as hydrochloric acid, nitric acid, phosphoric acid, sulfuric acid, hydrobromic acid, hydroiodic acid, and phosphonic acid, and salts derived from organic acids, such as aliphatic mono-carboxylic acid and aliphatic dicarboxylic acid, phenyl-substituted alkanoic acid, hydroxyalkanoic acid, alkanedioic acids, aromatic acid, aliphatic sulfonic acid, and aromatic sulfonic acid. Thus, these salts include, but are not limited to, sulfate, pyrosulfate, bisulfate, sulfite, bisulfite, nitrate, phosphate, monohydrogenphosphate, dihydrogenphosphate, metaphosphate, pyrophosphate, hydrochloride, hydrobromide, iodate, acetate, propionate, caprylate, isobutyrate, oxalate, malonate, succinate, suberate, sebacate, fumarate, maleate, mandelate, benzoate, chlorobenzoate, methylbenzoate, dinitrobenzoate, phthalate, benzenesulfonate, tosylate, phenylacetate, citrate, lactate, maleate, tartrate, and methanesulfonate, and salts comprising amino acids such as arginate, gluconate, and galacturonate. Acid addition salts can be prepared by contacting the free base form with a sufficient amount of the desired acid to form the salt in a conventional manner. The free base form can be regenerated by contacting the salt form with a base and isolating the free base in a conventional manner.

The base addition salts according to the present disclosure are salts with metals or amines, such as hydroxides of alkali metals and alkaline earth metals, or with organic amines.Examples of metals used as cations include, but are not limited to, sodium, potassium, magnesium, and calcium. Examples of suitable amines include, but are not limited to, N,N'-dibenzylethylenediamine, chloroprocaine, choline, diethanolamine, ethylenediamine(ethane-1,2-diamine), N-methylglucamine, and procaine. Base addition salts can be prepared by contacting the free acid form with a sufficient amount of the desired base to form the salt in a conventional manner. The free acid form can be regenerated by contacting the salt form with an acid and isolating the free acid in a conventional manner.

The term "solvate" is to be understood as any form of the compound of the present disclosure, wherein the compound is linked to another molecule (usually a polar solvent) via a non-covalent bond, particularly including hydrate and alcoholate such as methanolate. A preferred solvate is a hydrate.

The term "prodrug" is used in its broad sense and encompasses derivatives that can be converted into the compound of the present disclosure *in vivo.* Examples of prodrugs include, but are not limited to, derivatives and metabolites of the compound, including biohydrolyzable moieties such as biohydrolyzable amides, biohydrolyzable esters, biohydrolyzable carbamates, biohydrolyzable carbonates, biohydrolyzable ureides, and biohydrolyzable phosphate analogs. Preferably, the prodrug having a carboxyl functional group is a lower alkyl ester of a carboxylic acid. The carboxylic acid ester is readily esterified from any carboxylic acid moiety present in the molecule. Prodrugs can generally be prepared by known methods, such as those described in "Burger's Medicinal Chemistry and Drug Discovery", 6th Ed., (Donald J. Abraham ed., 2001, Wiley) and "Design and Applications of Prodrugs" (H. Bundgaard ed., 1985, Harwood Academic Publishers).

The term "absent" means that the linking group is a linking bond.

Any compound referred to herein is intended to represent such particular compound and certain variations or certain forms thereof. Particularly, the compounds referred to herein may have asymmetric centers and thus have different enantiomeric or diastereomeric forms. Thus, any given compound referred to herein represents any one of racemates thereof, one or more enantiomeric forms, one or more diastereomeric forms, and mixtures thereof. Likewise, molecules with a double bond may also have stereoisomers or geometric isomers. Thus, in some cases, an (E)-isomer or (Z)-isomer (trans and cis isomers) may be present. If a molecule contains multiple double bonds, each double bond will have its own stereoisomerism, which may be the same as or different from the stereoisomerisms of other double bonds of the molecule. In addition, the compounds referred to herein may have atropisomers. All stereoisomers of the compounds referred to herein, including enantiomers, diastereomers, geometric isomers, atropisomers, and mixtures thereof, are within the scope of the present disclosure.

The term "leaving group" is given its ordinary meaning in the art of synthetic organic chemistry and refers to an atom or a group capable of being displaced by a nucleophile. See, for example, Smith, March's Advanced Organic Chemistry, 6th Ed., (501-502). Examples of suitable leaving groups include, but are not limited to, halogen (e.g., F, Cl, Br, or I), alkanesulfonyloxy, arylsulfonyloxy, and the like.

The disclosures of the various publications, patents, and published patent specifications cited herein are hereby incorporated by reference in their entirety.

The technical solutions of the present disclosure will be clearly and completely described below with reference to the examples of the present disclosure, and it is obvious that the described examples are only a part of the examples of the present disclosure, but not all of them. Based on the examples of the present disclosure, all other examples obtained by those of ordinary skill in the art without creative work shall fall within the protection scope of the present disclosure.

### Compound Synthesis Examples

### Example 1

The synthetic route was as follows:

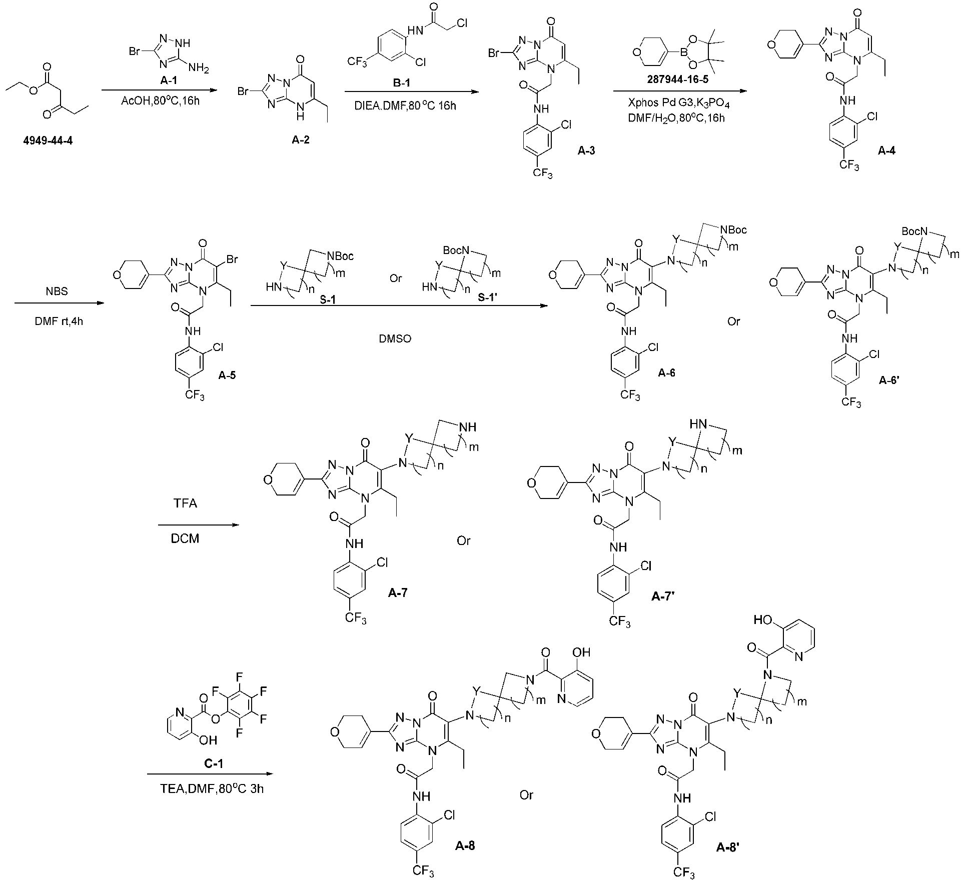

The specific procedures were as follows:

### 1.1 Synthesis of intermediate A-1

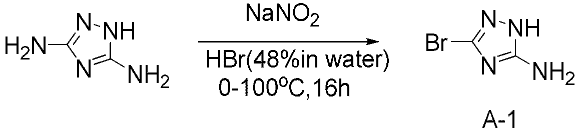

3,5-Diamino-1,2,4-triazole (1.0 e.q.) was added to a 48% aqueous hydrobromic acid solution (1 mmol:0.4 mL) under a nitrogen atmosphere, and an aqueous sodium nitrite solution (1.1 e.q.) was added dropwise at 0 °C. Subsequently, the mixture was reacted at 100 °C for 16 h. After the reaction was completed, as monitored by LC-MS, the reaction solution was cooled to 0 °C, and the pH was adjusted to 4 with a 10% sodium hydroxide solution. The mixture was extracted 4 times with ethyl acetate, and the organic phase was washed with saturated brine, dried over anhydrous sodium sulfate, and concentrated under reduced pressure to give intermediate A-1.

### 1.2 Synthesis of intermediate B-1

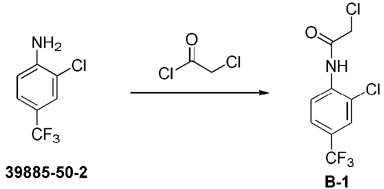

3-Chloro-4-aminobenzotrifluoride (1.0 e.q.) was added to dichloromethane (1 mmol:2 mL), and chloroacetyl chloride (1.05 e.q.) was added dropwise at 0 °C. Subsequently, the mixture was reacted at room temperature for 16 h. After the reaction was completed, as monitored by TLC, the reaction solution was concentrated under reduced pressure to give a crude product, which was slurried with petroleum ether to give intermediate B-1.

### 1.3 Synthesis of intermediate C-1

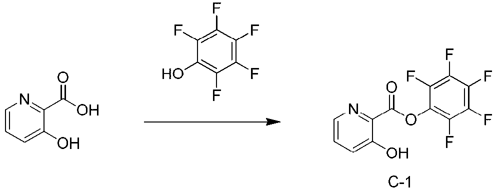

3-Hydroxypyridine-2-carboxylic acid (1.0 e.q.) and pentafluorophenol (1.05 e.q.) were added to dichloromethane (1 mmol:2 mL) under a nitrogen atmosphere, and N,N'-diisopropylcarbodiimide (1.05 e.q.) was added dropwise at 0 °C. Subsequently, the mixture was reacted at room temperature for 16 h. After the reaction was completed, as monitored by LC-MS, the reaction solution was concentrated under reduced pressure and subjected to column chromatography to give intermediate C-1.

### 1.4 Synthesis of intermediate A-2

Ethyl propionylacetate (1.1 e.q.) and intermediate A-1 (1.0 e.q.) were added to acetic acid (1 mmol:0.5 mL) at room temperature, and subsequently, the mixture was heated to 80 °C and reacted for 16 h. After the reaction was completed, as monitored by LC-MS, the reaction solution was cooled to 0 °C, stirred for 1 h, and filtered, and the resulting solid was washed with a small amount of ethanol and dried to give intermediate A-2.

### 1.5 Synthesis of intermediate A-3

Intermediate A-2 (1.0 e.q.), intermediate B-1 (1.2 e.q.), and N,N-diisopropylethylamine (2.5 e.q.) were added to N,N-dimethylformamide (1 mmol:2 mL) at room temperature, and subsequently, the mixture was heated to 80 °C and reacted for 16 h. After the reaction was completed, as monitored by LC-MS, the reaction solution was diluted with ethyl acetate and washed twice with water and once with brine. The resulting organic phase was dried over anhydrous sodium sulfate, concentrated under reduced pressure, and subsequently subjected to column chromatography to give intermediate A-3.

### 1.6 Synthesis of intermediate A-4

Intermediate A-3 (1.0 e.q.), 3,6-dihydro-2H-pyran-4-boronic acid pinacol ester (1.2 e.q.), XPhos Pd G3 (0.05 e.q.), and potassium phosphate (2.5 e.q.) were added to N,N-dimethylformamide and water (7:1, 1 mmol:5 mL). The mixture was purged three times with nitrogen and subsequently heated to 80 °C and reacted for 16 h. After the reaction was completed, as monitored by LC-MS, the reaction solution was diluted with ethyl acetate and washed twice with water and once with brine. The resulting organic phase was dried over anhydrous sodium sulfate, concentrated under reduced pressure, and subsequently subjected to column chromatography to give intermediate A-4.

### 1.7 Synthesis of intermediate A-5

Intermediate A-4 (1.0 e.q.) was dissolved in N,N-dimethylformamide (1 mmol:10 mL) at room temperature, followed by the addition of N-bromosuccinimide (1.5 e.q.) in batches, and the mixture was reacted for 4 h. After the reaction was completed, as monitored by LC-MS, the reaction solution was diluted with ethyl acetate and washed once with saturated sodium bicarbonate, twice with water, and once with saturated brine. The resulting organic phase was dried over anhydrous sodium sulfate, concentrated under reduced pressure, and subsequently subjected to column chromatography to give intermediate A-5.

### 1.8 Synthesis of intermediate A-6 or A-6'

A-5 (1.0 e.q.), N,N-diisopropylethylamine (3.0 e.q.), and commercially available tert-butoxycarbonyl-protected spiro amine (S-1 or S-1') (2.5 e.q.) were dissolved in dimethyl sulfoxide (1 mmol:5 mL), and subsequently, the mixture was reacted at 120 °C for 16 h under a nitrogen atmosphere. After the reaction was completed, as monitored by LC-MS, the reaction solution was diluted with ethyl acetate and washed twice with water and once with saturated brine. The resulting organic phase was dried over anhydrous sodium sulfate, concentrated under reduced pressure, and subsequently subjected to column chromatography to give intermediate A-6 or A-6'.

### 1.9 Synthesis of intermediate A-7 or A-7'

Intermediates A-6 and A-6' (1.0 e.q.) were dissolved in dichloromethane (1 mmol:5 mL) at room temperature, followed by the addition of trifluoroacetic acid (1 mmol:1 mL) at 0 °C, and subsequently, the mixture was warmed to room temperature and reacted for 2 h. After the reaction was completed, as monitored by LC-MS, the reaction solution was concentrated to near dryness under reduced pressure and diluted with dichloromethane, and the aqueous phase was adjusted to pH = 8-9 with saturated sodium bicarbonate. Extraction was performed twice with dichloromethane. The organic phase was washed with saturated brine, and the resulting organic phase was dried over anhydrous sodium sulfate and concentrated under reduced pressure to give intermediate A-7 or A-7'.

### 1.10 Synthesis of compound A-8 or A-8'

Intermediate A-7 or A-7' (1.0 e.q.), triethylamine (3.0 e.q.), and intermediate C-1 (1.2 e.q.) were dissolved in N,N-dimethylformamide (1 mmol:10 mL), and subsequently, the mixture was reacted at 80 °C for 2 h under a nitrogen atmosphere. After the reaction was completed, as monitored by LC-MS, the reaction solution was diluted with ethyl acetate and washed twice with water and once with saturated brine. The resulting organic phase was dried over anhydrous sodium sulfate, concentrated under reduced pressure, and subsequently subjected to preparative high-pressure reverse-phase chromatography to give compound A-8 or A-8'.

When Y is CH₂, n = 1, and m = 1, A-8 obtained according to the above synthesis procedures is compound T001. When Y is absent, n = 2, and m = 1, A-8 obtained according to the above synthesis procedures is compound T004. When Y is CH₂, n = 1, and m = 1, A-8' obtained according to the above synthesis procedures is compound T005. When Y is CH₂, n = 1, and m = 0, A-8' obtained according to the above synthesis procedures is compound T006. When Y is CH₂, n = 1, and m = 3, A-8 obtained according to the above synthesis procedures is compound T007. When Y is CH₂, n = 1, and m = 2, A-8 obtained according to the above synthesis procedures is compound T008. When Y is CH₂, n = 1, and m = 2, A-8' obtained according to the above synthesis procedures is compound T009. When Y is CH₂, n = 2, and m = 1, A-8 obtained according to the above synthesis procedures is compound T010. When Y is CH₂, n = 2, and m = 2, A-8 obtained according to the above synthesis procedures is compound T011. When Y is CH₂, n = 2, and m = 2, A-8' obtained according to the above synthesis procedures, particularly when the configuration of the spiro atom is the S-configuration, is compound T12.

When Y is CH₂, n = 0, and m = 2, A-8' obtained according to the above synthesis procedures is compound T013. When Y is CH₂, n = 1, and m = 3, A-8' obtained according to the above synthesis procedures is compound T014. When Y is CH₂, n = 2, and m = 2, A-8' obtained according to the above synthesis procedures, particularly when the configuration of the spiro atom is the R-configuration, is compound T015.

The structures and general analytical data of the target compounds are shown in Table 1.

### Example 2

The specific procedures were as follows:

### 2.1 Synthesis of intermediate C-2

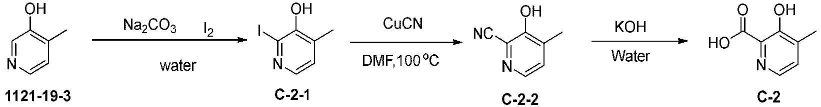

### 2.1.1 Synthesis of intermediate C-2

3-Hydroxy-4-methylpyridine (1.0 e.q.) and sodium carbonate (2.3 e.q.) were dissolved in water (1 mmol:5 mL), followed by the addition of iodine (1.0 e.q.), and the mixture was reacted at room temperature for 4 h. After the reaction was completed, as monitored by LC-MS, the reaction solution was adjusted to pH 4-5 with 2 mol/L hydrochloric acid at 0 °C and extracted three times with ethyl acetate. The organic phase was washed with saturated brine, and the resulting organic phase was dried over anhydrous sodium sulfate, concentrated under reduced pressure, and subsequently subjected to column chromatography to give intermediate C-2-1.

### 2.1.2 Synthesis of intermediate C-2-2

Intermediate C-2-1 (1.0 e.q.) and cuprous cyanide (4.0 e.q.) were added to N,N-dimethylformamide (1 mmol:2 mL) at room temperature, and the mixture was purged three times with nitrogen and subsequently heated to 100 °C and reacted for 2 h. After the reaction was completed, as monitored by LC-MS, the reaction solution was cooled to room temperature, diluted with ethyl acetate and water, and filtered to separate the organic phase. The organic phase was washed once with water and once with brine, and the resulting organic phase was dried over anhydrous sodium sulfate, concentrated under reduced pressure, and subsequently subjected to column chromatography to give intermediate C-2-2.

### 2.1.3 Synthesis of intermediate C-2

Potassium hydroxide (6.0 e.q.) was dissolved in water (1 mmol:2 mL), followed by the addition of intermediate C-2-2 (1.0 e.q.), and the mixture was heated to 90 °C and reacted for 16 h. After the reaction was completed, as monitored by LC-MS, the reaction solution was cooled to 0 °C and adjusted to about pH 3 with 2 mol/L hydrochloric acid. The solid obtained by filtration was washed twice with a small amount of water, collected, and dried to give intermediate C-2.

### 2.2 Synthesis of compound T002

Compound T002 was synthesized by condensation of intermediate A-7 (Y is CH₂, n = 1, and m = 1) obtained according to the synthesis procedures of Example 1 with C-2.

Intermediate A-7 (1.0 e.q.), C-2 (2.0 e.q.), and N,N-diisopropylethylamine (5 e.q.) were dissolved in N,N-dimethylformamide (1 mmol:5 mL) at room temperature, followed by the addition of (benzotriazol-1-yloxy)tripyrrolidinophosphonium hexafluorophosphate (2.0 e.q.) at 0 °C, and subsequently, the mixture was warmed to room temperature and reacted for 2 h. After the reaction was completed, as monitored by LC-MS, the reaction solution was diluted with ethyl acetate and washed twice with water and once with saturated brine. The resulting organic phase was dried over anhydrous sodium sulfate, concentrated under reduced pressure, and subsequently subjected to preparative high-pressure reverse-phase chromatography to give compound T002, the structure and general analytical data of which are shown in Table 1.

### Example 3

### 3.1 Synthesis of intermediate C-3

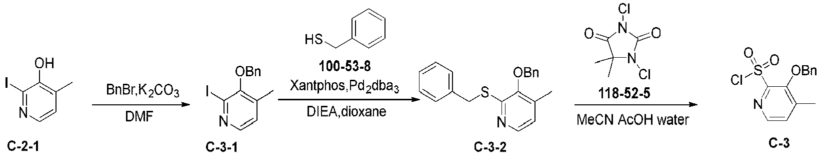

### 3.1.1 Synthesis of intermediate C-3-1

Intermediate C2-1-1 (1.0 e.q.) and potassium carbonate (1.5 e.q.) were added to N,N-dimethylformamide (1 mmol:3 mL) at room temperature, followed by the addition of benzyl bromide (1.05 e.q.), and subsequently, the mixture was heated to 40 °C and reacted for 2 h. After the reaction was completed, as monitored by LC-MS, the reaction solution was diluted with a large amount of water and extracted twice with ethyl acetate, and the resulting organic phase was washed once with brine, dried over anhydrous sodium sulfate, concentrated under reduced pressure, and subsequently purified by column chromatography to give intermediate C-3-1.

### 3.1.2 Synthesis of intermediate C-3-2

Intermediate C-3-1 (1.0 e.q.), benzyl mercaptan (2.0 e.q.), XanPhos (0.15 e.q.), Pd₂(dba)₃ (0.075 e.q.), and N,N-diisopropylethylamine (3.0 e.q.) were added to 1,4-dioxane (1 mmol:4 mL) at room temperature, and the mixture was purged three times with nitrogen and subsequently heated to 100 °C and reacted for 2 h. After the reaction was completed, as monitored by LC-MS, the reaction solution was concentrated under reduced pressure and purified by column chromatography to give intermediate C-3-2.

### 3.1.3 Synthesis of intermediate C-3

Intermediate C-3-2 (1.0 e.q.) was dissolved in acetonitrile (1 mmol:4 mL) and water (1 mmol:0.5 mL). The mixture was cooled to 0 °C, and acetic acid (6.0 e.q.) and 1,3-dichloro-5,5-dimethylhydantoin (1.0 e.q.) were added. Subsequently, the mixture was warmed to room temperature and reacted for 2 h. After the reaction was completed, as monitored by LC-MS, the reaction solution was adjusted to pH 7 with saturated sodium bicarbonate and extracted three times with ethyl acetate. The organic phases were combined, washed with brine, dried over anhydrous sodium sulfate, concentrated under reduced pressure, and subsequently subjected to column chromatography to give intermediate C-3.

### 3.2 Synthesis of compound T003

Compound T003 was synthesized by condensation of intermediate A-7 (Y is CH₂, n = 1, and m = 1) obtained according to the synthesis procedures of Example 1 with C-3.

Intermediate A-7 (1.0 e.q.) and triethylamine (5 e.q.) were dissolved in dichloromethane (1 mmol:10 mL), and subsequently, the reaction solution was cooled to 0 °C. Intermediate C-3 (2.5 e.q.) was added, and subsequently, the mixture was warmed to room temperature and reacted for 2 h. After the reaction was completed, as monitored by LC-MS, the reaction solution was diluted with water and extracted twice with dichloromethane. The organic phases were combined, washed once with saturated brine, dried over anhydrous sodium sulfate, concentrated under reduced pressure, and subsequently subjected to preparative high-pressure reverse-phase chromatography to give compound T003, the structure and general analytical data of which are shown in Table 1.

### Example 4

The synthetic route was as follows:

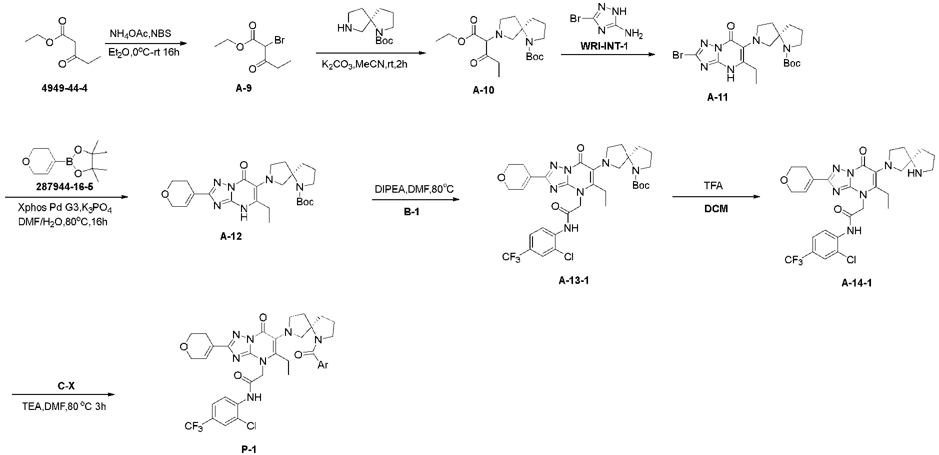

The specific procedures were as follows:

### 4.1 Synthesis of intermediate A-9

Ethyl acetoacetate (1.0 e.q.) and ammonium acetate (0.3 e.q.) were added to anhydrous diethyl ether (1 mmol:5 mL), followed by the addition of NBS (1.08 e.q.) in batches, and subsequently, the solution was stirred at room temperature for 4 h. After the reaction was completed, as monitored by TLC, the mixture was concentrated by rotary evaporation to remove diethyl ether, diluted with ethyl acetate, and washed three times with brine. The organic phase was collected and dried over anhydrous sodium sulfate to give crude A-9, which could be directly used in the next step without purification.

### 4.2 Synthesis of intermediate A-10

Intermediate A-9 (1.0 e.q.), (R)-1-BOC-1,7-diazaspiro[4.4]nonane (0.8 e.q.), and potassium carbonate (3.0 e.q.) were weighed out and dissolved in acetonitrile (1 mmol:2 mL). Subsequently, the mixture was stirred at room temperature for 2-3 h. After the reaction was completed, as monitored by LC-MS, the mixture was concentrated by rotary evaporation to remove acetonitrile, diluted with ethyl acetate, washed three times with brine, and dried over anhydrous sodium sulfate. The organic phase was retained and concentrated to dryness by rotary evaporation to give a crude product. Finally, the crude product was subjected to column chromatography to give A-10.

### 4.3 Synthesis of intermediate A-11

WRI-INT-1 (1.5 e.q.) and an 85% aqueous phosphoric acid solution (1.0 e.q.) were added to a solution of intermediate A-10 (1.0 e.q.) in ethanol (1 mmol:0.5 mL) under a nitrogen atmosphere. Subsequently, the mixture was stirred at 90 °C for 16 h. After the reaction was completed, as monitored by LC-MS, the solution was cooled to room temperature, concentrated by rotary evaporation to remove ethanol, diluted with ethyl acetate, and washed with an aqueous ammonium chloride solution. The organic phase was collected, dried over anhydrous sodium sulfate, and concentrated to dryness by rotary evaporation to give a crude product. Finally, the crude product was subjected to column chromatography to give the product A-11.

### 4.4 Synthesis of intermediate A-12

Intermediate A-11 (1.0 e.q.), 3,6-dihydro-2H-pyran-4-boronic acid pinacol ester (1.2 e.q.), XPhos Pd G3 (0.05 e.q.), and potassium phosphate (2.5 e.q.) were added to 1,4-dioxane and water (5:1, 1 mmol:5 mL). The mixture was purged three times with nitrogen and subsequently heated to 80 °C and reacted for 4 h. After the reaction was completed, as monitored by LC-MS, the reaction solution was diluted with ethyl acetate and washed with brine. The organic phase was collected, dried over anhydrous sodium sulfate, concentrated to dryness by rotary evaporation, and subsequently subjected to column chromatography to give intermediate A-12.

### 4.5 Synthesis of intermediate A-13-1

Intermediate A-12 (1.0 e.q.), intermediate B-1 (1.2 e.q.), and N,N-diisopropylethylamine (2.5 e.q.) were added to N,N-dimethylformamide (1 mmol:2 mL) at room temperature, and subsequently, the mixture was heated to 80 °C and reacted for 16 h. After the reaction was completed, as monitored by LC-MS, the reaction solution was diluted with ethyl acetate and washed twice with water and once with brine. The resulting organic phase was dried over anhydrous sodium sulfate, concentrated under reduced pressure, and subsequently subjected to column chromatography to give intermediate A-13-1.

### 4.6 Synthesis of intermediate A-14-1

Intermediate A-13-1 (1.0 e.q.) was dissolved in dichloromethane (1 mmol:5 mL) at room temperature, followed by the addition of trifluoroacetic acid (1 mmol:3 mL) at 0 °C, and subsequently, the mixture was heated to 40 °C and reacted for 16 h. After the reaction was completed, as monitored by LC-MS, the reaction solution was concentrated to near dryness under reduced pressure and diluted with dichloromethane, and the aqueous phase was adjusted to pH = 8-9 with saturated sodium bicarbonate. Extraction was performed twice with dichloromethane. The organic phase was washed with saturated brine, and the resulting organic phase was dried over anhydrous sodium sulfate and concentrated under reduced pressure to give intermediate A-14-1.

### 4.7 Synthesis of target products

### 4.7.1 Synthesis of target product T016

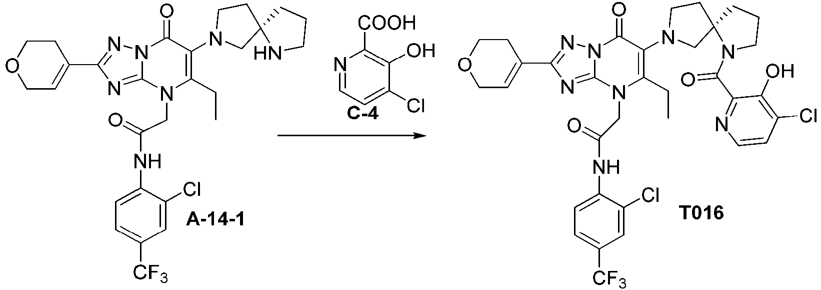

Intermediate A-14-1 (1.0 e.q.), C-4 (2.0 e.q.), and N,N-diisopropylethylamine (5 e.q.) were dissolved in N,N-dimethylformamide (1 mmol:5 mL) at room temperature, followed by the addition of (benzotriazol-1-yloxy)tripyrrolidinophosphonium hexafluorophosphate (2.0 e.q.) at 0 °C, and subsequently, the mixture was heated to 40 °C and reacted for 2 h. After the reaction was completed, as monitored by LC-MS, the reaction solution was diluted with ethyl acetate and washed twice with water and once with saturated brine. The resulting organic phase was dried over anhydrous sodium sulfate, concentrated under reduced pressure, and subsequently subjected to preparative high-pressure reverse-phase chromatography to give compound T016, the structure and general analytical data of which are shown in Table 1.

### 4.7.2 Synthesis of target product T017

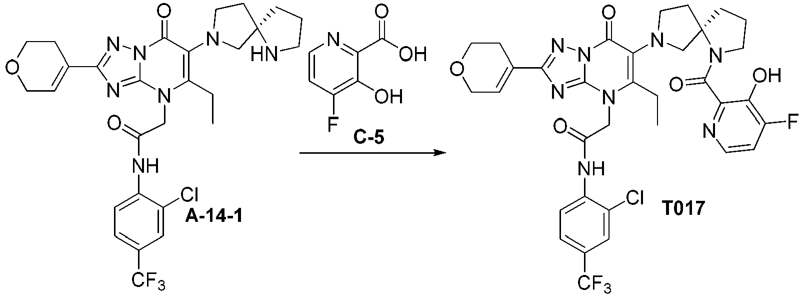

Intermediate A-14-1 (1.0 e.q.), C-5 (2.0 e.q.), and N,N-diisopropylethylamine (5 e.q.) were dissolved in N,N-dimethylformamide (1 mmol:5 mL) at room temperature, followed by the addition of (benzotriazol-1-yloxy)tripyrrolidinophosphonium hexafluorophosphate (2.0 e.q.) at 0 °C, and subsequently, the mixture was heated to 40 °C and reacted for 2 h. After the reaction was completed, as monitored by LC-MS, the reaction solution was diluted with ethyl acetate and washed twice with water and once with saturated brine. The resulting organic phase was dried over anhydrous sodium sulfate, concentrated under reduced pressure, and subsequently subjected to preparative high-pressure reverse-phase chromatography to give compound T017, the structure and general analytical data of which are shown in Table 1.

### 4.7.3 Synthesis of target product T018

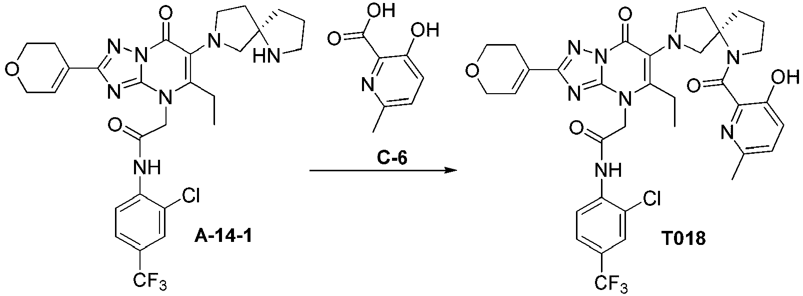

Intermediate A-14-1 (1.0 e.q.), C-6 (2.0 e.q.), and N,N-diisopropylethylamine (5 e.q.) were dissolved in N,N-dimethylformamide (1 mmol:5 mL) at room temperature, followed by the addition of O-(7-azabenzotriazol-1-yl)-N,N,N',N'-tetramethyluronium hexafluorophosphate (1.5 e.q.) at 0 °C, and subsequently, the mixture was heated to 40 °C and reacted for 2 h. After the reaction was completed, as monitored by LC-MS, the reaction solution was diluted with ethyl acetate and washed twice with water and once with saturated brine. The resulting organic phase was dried over anhydrous sodium sulfate, concentrated under reduced pressure, and subsequently subjected to preparative high-pressure reverse-phase chromatography to give compound T018, the structure and general analytical data of which are shown in Table 1.

### 4.7.4 Synthesis of target product T019

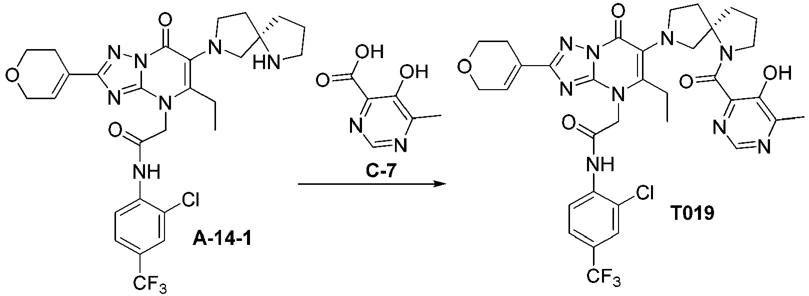

Intermediate A-14-1 (1.0 e.q.), C-7 (2.0 e.q.), and N,N-diisopropylethylamine (5 e.q.) were dissolved in N,N-dimethylformamide (1 mmol:5 mL) at room temperature, followed by the addition of (benzotriazol-1-yloxy)tripyrrolidinophosphonium hexafluorophosphate (2.0 e.q.) at 0 °C, and subsequently, the mixture was heated to 40 °C and reacted for 2 h. After the reaction was completed, as monitored by LC-MS, the reaction solution was diluted with ethyl acetate and washed twice with water and once with saturated brine. The resulting organic phase was dried over anhydrous sodium sulfate, concentrated under reduced pressure, and subsequently subjected to preparative high-pressure reverse-phase chromatography to give compound T019, the structure and general analytical data of which are shown in Table 1.

### 4.7.5 Synthesis of target product T020

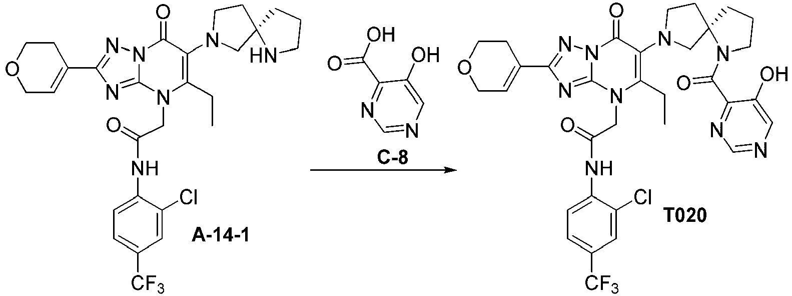

Intermediate A-14-1 (1.0 e.q.), C-8 (2.0 e.q.), and N,N-diisopropylethylamine (5 e.q.) were dissolved in N,N-dimethylformamide (1 mmol:5 mL) at room temperature, followed by the addition of (benzotriazol-1-yloxy)tripyrrolidinophosphonium hexafluorophosphate (2.0 e.q.) at 0 °C, and subsequently, the mixture was heated to 40 °C and reacted for 2 h. After the reaction was completed, as monitored by LC-MS, the reaction solution was diluted with ethyl acetate and washed twice with water and once with saturated brine. The resulting organic phase was dried over anhydrous sodium sulfate, concentrated under reduced pressure, and subsequently subjected to preparative high-pressure reverse-phase chromatography to give compound T020, the structure and general analytical data of which are shown in Table 1.

### 4.7.6 Synthesis of target product T021

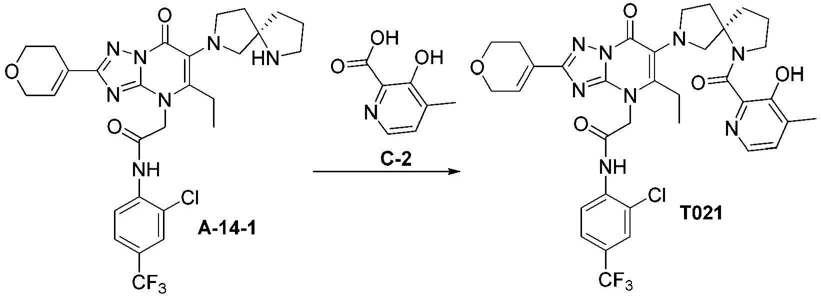

Intermediate A-14-1 (1.0 e.q.), C-2 (2.0 e.q.), and N,N-diisopropylethylamine (5 e.q.) were dissolved in N,N-dimethylformamide (1 mmol:5 mL) at room temperature, followed by the addition of (benzotriazol-1-yloxy)tripyrrolidinophosphonium hexafluorophosphate (2.0 e.q.) at 0 °C, and subsequently, the mixture was heated to 40 °C and reacted for 2 h. After the reaction was completed, as monitored by LC-MS, the reaction solution was diluted with ethyl acetate and washed twice with water and once with saturated brine. The resulting organic phase was dried over anhydrous sodium sulfate, concentrated under reduced pressure, and subsequently subjected to preparative high-pressure reverse-phase chromatography to give compound T021, the structure and general analytical data of which are shown in Table 1.

### 4.7.7 Synthesis of target product T006

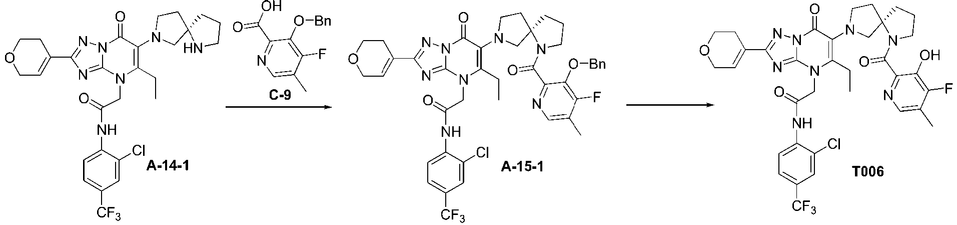

### 4.7.7.1 Synthesis of intermediate A-15-1

Intermediate A-14-1 (1.0 e.q.), C-9 (2.0 e.q.), and N,N-diisopropylethylamine (5 e.q.) were dissolved in N,N-dimethylformamide (1 mmol:5 mL) at room temperature, followed by the addition of (benzotriazol-1-yloxy)tripyrrolidinophosphonium hexafluorophosphate (2.0 e.q.) at 0 °C, and subsequently, the mixture was heated to 40 °C and reacted for 2 h. After the reaction was completed, as monitored by LC-MS, the reaction solution was diluted with ethyl acetate and washed twice with water and once with saturated brine. The resulting organic phase was dried over anhydrous sodium sulfate, concentrated under reduced pressure, and subsequently purified by column chromatography to give intermediate A-15-1.

### 4.7.7.2 Synthesis of target product T006

Intermediate A-15-1 (1.0 e.q.) was dissolved in a trifluoroacetic acid solution (1 mmol:1 mL) at room temperature, and the reaction solution was heated to 40 °C and reacted for 12 h. After the reaction was completed, as monitored by LC-MS, the reaction solution was concentrated by rotary evaporation to remove trifluoroacetic acid. The concentrated solution was diluted with ethyl acetate and washed once with a saturated sodium bicarbonate solution and once with saturated brine. The organic phase was concentrated to dryness by rotary evaporation and subsequently subjected to preparative high-pressure reverse-phase chromatography to give compound T006, the structure and general analytical data of which are shown in Table 1.

### 4.7.8 Synthesis of target product T013

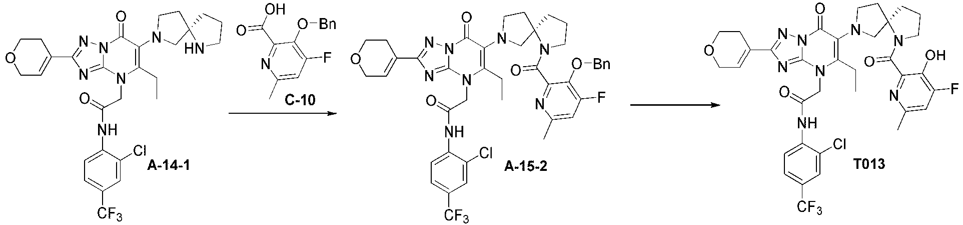

### 4.7.8.1 Synthesis of intermediate A-15-2

Intermediate A-14-1 (1.0 e.q.), C-10 (2.0 e.q.), and N,N-diisopropylethylamine (5 e.q.) were dissolved in N,N-dimethylformamide (1 mmol:5 mL) at room temperature, followed by the addition of (benzotriazol-1-yloxy)tripyrrolidinophosphonium hexafluorophosphate (2.0 e.q.) at 0 °C, and subsequently, the mixture was heated to 40 °C and reacted for 2 h. After the reaction was completed, as monitored by LC-MS, the reaction solution was diluted with ethyl acetate and washed twice with water and once with saturated brine. The resulting organic phase was dried over anhydrous sodium sulfate, concentrated under reduced pressure, and subsequently purified by column chromatography to give intermediate A-15-1.

### 4.7.8.2 Synthesis of target product T013

Intermediate A-15-2 (1.0 e.q.) was dissolved in a trifluoroacetic acid solution (1 mmol:1 mL) at room temperature, and the reaction solution was heated to 40 °C and reacted for 12 h. After the reaction was completed, as monitored by LC-MS, the reaction solution was concentrated by rotary evaporation to remove trifluoroacetic acid. The concentrated solution was diluted with ethyl acetate and washed once with a saturated sodium bicarbonate solution and once with saturated brine. The organic phase was concentrated to dryness by rotary evaporation and subsequently subjected to preparative high-pressure reverse-phase chromatography to give compound T013, the structure and general analytical data of which are shown in Table 1.

### 4.7.9 Synthesis of target product T014

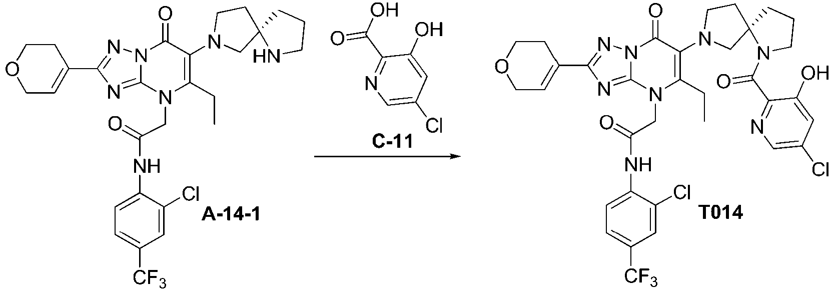

Intermediate A-14-1 (1.0 e.q.), C-11 (2.0 e.q.), and N,N-diisopropylethylamine (5 e.q.) were dissolved in N,N-dimethylformamide (1 mmol:5 mL) at room temperature, followed by the addition of (benzotriazol-1-yloxy)tripyrrolidinophosphonium hexafluorophosphate (2.0 e.q.) at 0 °C, and subsequently, the mixture was heated to 40 °C and reacted for 2 h. After the reaction was completed, as monitored by LC-MS, the reaction solution was diluted with ethyl acetate and washed twice with water and once with saturated brine. The resulting organic phase was dried over anhydrous sodium sulfate, concentrated under reduced pressure, and subsequently subjected to preparative high-pressure reverse-phase chromatography to give compound T014, the structure and general analytical data of which are shown in Table 1.

### Example 5

The synthetic route was as follows:

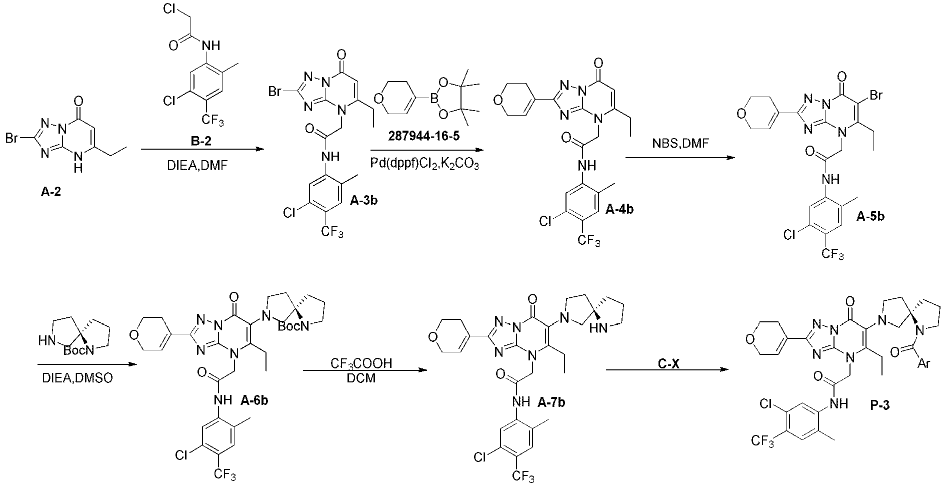

The specific procedures were as follows:
5.1 The synthetic route of intermediate B-2 was as follows:

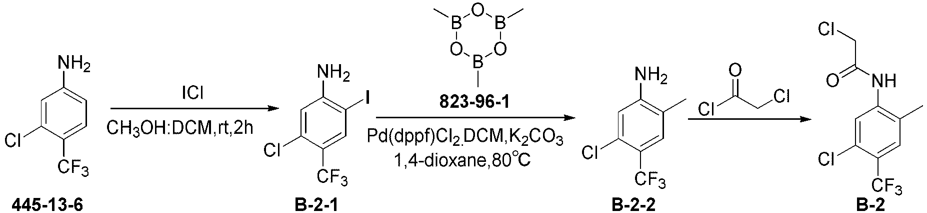

### 5.1.1 Synthesis of intermediate B-2-1

4-Amino-2-chlorobenzotrifluoride (1.0 e.q.) was dissolved in CH₃OH:DCM = 3:2 mL (1.0 mmol:5 mL) at room temperature, and iodine monochloride (1.5 e.q.) was added at 0 °C. Subsequently, the mixture was warmed to room temperature and reacted for 2 h. After the reaction was completed, as monitored by LC-MS, the reaction solution was concentrated to dryness under reduced pressure, diluted with ethyl acetate, and slowly added to a saturated sodium thiosulfate solution, and the mixture was stirred for 5-10 min. Finally, the mixture was washed twice with water, and the organic phase was washed with saturated brine. The resulting organic phase was dried over anhydrous sodium sulfate and concentrated under reduced pressure to give crude intermediate B-2-1. The crude product was purified by column chromatography to give pure intermediate B-2-1.

### 5.1.2 Synthesis of intermediate B-2-2

Intermediate B-2-1 (1.0 e.q.), trimethylboroxine (2.5 e.q.), DPPF-dichloropalladium(II) methane complex (0.05 e.q.), and potassium carbonate (2.5 e.q.) were added to 1,4-dioxane (1 mmol:2 mL). The mixture was purged three times with nitrogen and subsequently heated to 100 °C and reacted for 16 h. After the reaction was completed, as monitored by LC-MS, the reaction solution was diluted with ethyl acetate and washed with brine. The organic phase was collected, dried over anhydrous sodium sulfate, concentrated to dryness by rotary evaporation, and subsequently subjected to column chromatography to give intermediate B-2-2.

### 5.1.3 Synthesis of intermediate B-2

Intermediate B-2-2 (1.0 e.q.) was added to dichloromethane (1 mmol:2 mL), and chloroacetyl chloride (1.05 e.q.) was added dropwise at 0 °C. Subsequently, the mixture was reacted at room temperature for 16 h. After the reaction was completed, as monitored by TLC, the reaction solution was concentrated under reduced pressure to give a crude product, which was slurried with petroleum ether to give intermediate B-2.

### 5.2 Synthesis of intermediate A-3b

Intermediate A-2 (1.0 e.q.), intermediate B-2 (1.2 e.q.), and N,N-diisopropylethylamine (2.5 e.q.) were added to N,N-dimethylformamide (1 mmol:2 mL) at room temperature, and subsequently, the mixture was heated to 80 °C and reacted for 16 h. After the reaction was completed, as monitored by LC-MS, the reaction solution was diluted with ethyl acetate and washed twice with water and once with brine. The resulting organic phase was dried over anhydrous sodium sulfate, concentrated under reduced pressure, and subsequently subjected to column chromatography to give intermediate A-3b.

### 5.3 Synthesis of intermediate A-4b

Intermediate A-3b (1.0 e.q.), 3,6-dihydro-2H-pyran-4-boronic acid pinacol ester (1.2 e.q.), XPhos Pd G3 (0.05 e.q.), and potassium phosphate (2.5 e.q.) were added to N,N-dimethylformamide and water (7:1, 1 mmol:5 mL). The mixture was purged three times with nitrogen and subsequently heated to 80 °C and reacted for 16 h. After the reaction was completed, as monitored by LC-MS, the reaction solution was diluted with ethyl acetate and washed twice with water and once with brine. The resulting organic phase was dried over anhydrous sodium sulfate, concentrated under reduced pressure, and subsequently subjected to column chromatography to give intermediate A-4b.

### 5.4 Synthesis of intermediate A-5b

Intermediate A-4b (1.0 e.q.) was dissolved in N,N-dimethylformamide (1 mmol:10 mL) at room temperature, followed by the addition of N-bromosuccinimide (1.5 e.q.) in batches, and the mixture was reacted for 4 h. After the reaction was completed, as monitored by LC-MS, the reaction solution was diluted with ethyl acetate and washed once with saturated sodium bicarbonate, twice with water, and once with saturated brine. The resulting organic phase was dried over anhydrous sodium sulfate, concentrated under reduced pressure, and subsequently subjected to column chromatography to give intermediate A-5b.

### 5.5 Synthesis of intermediate A-6b

Intermediate A-5b (1.0 e.q.), N,N-diisopropylethylamine (3.0 e.q.), and commercially available (R)-1-BOC-1,7-diazaspiro[4.4]nonane (2.5 e.q.) were dissolved in dimethyl sulfoxide (1 mmol:5 mL). Subsequently, the mixture was reacted at 120 °C for 16 h under a nitrogen atmosphere. After the reaction was completed, as monitored by LC-MS, the reaction solution was diluted with ethyl acetate and washed twice with water and once with saturated brine. The resulting organic phase was dried over anhydrous sodium sulfate, concentrated under reduced pressure, and subsequently subjected to column chromatography to give intermediate A-6b.

### 5.6 Synthesis of intermediate A-7b

Intermediate A-6b (1.0 e.q.) was dissolved in dichloromethane (1 mmol:5 mL) at room temperature, followed by the addition of trifluoroacetic acid (1 mmol:1 mL) at 0 °C, and subsequently, the mixture was warmed to room temperature and reacted for 2 h. After the reaction was completed, as monitored by LC-MS, the reaction solution was concentrated to near dryness under reduced pressure and diluted with dichloromethane, and the aqueous phase was adjusted to pH = 8-9 with saturated sodium bicarbonate. Extraction was performed twice with dichloromethane. The organic phase was washed with saturated brine, and the resulting organic phase was dried over anhydrous sodium sulfate and concentrated under reduced pressure to give intermediate A-7b.

### 5.7 Synthesis of target products

5.7.1 The synthesis procedures of the target product T022 were based on the synthesis of A-8, and its structure and general analytical data are shown in Table 1.

5.7.2 The synthesis procedures of the target product T023 were based on the synthesis of T016, and its structure and general analytical data are shown in Table 1.

5.7.3 The synthesis procedures of the target product T024 were based on the synthesis of T017, and its structure and general analytical data are shown in Table 1.

5.7.4 The synthesis procedures of the target product T025 were based on the synthesis of T018, and its structure and general analytical data are shown in Table 1.

5.7.5 The synthesis procedures of the target product T026 were based on the synthesis of T019, and its structure and general analytical data are shown in Table 1.

5.7.6 The synthesis procedures of the target product T027 were based on the synthesis of T020, and its structure and general analytical data are shown in Table 1.

5.7.7 The synthesis procedures of the target product T028 were based on the synthesis of T021, and its structure and general analytical data are shown in Table 1.

5.7.8 The synthesis procedures of the target product T029 were based on the synthesis of T006, and its structure and general analytical data are shown in Table 1.

5.7.9 The synthesis procedures of the target product T030 were based on the synthesis of T013, and its structure and general analytical data are shown in Table 1.

5.7.10 The synthesis procedures of the target product T031 were based on the synthesis of T014, and its structure and general analytical data are shown in Table 1.

### Example 6

The synthetic route was as follows:

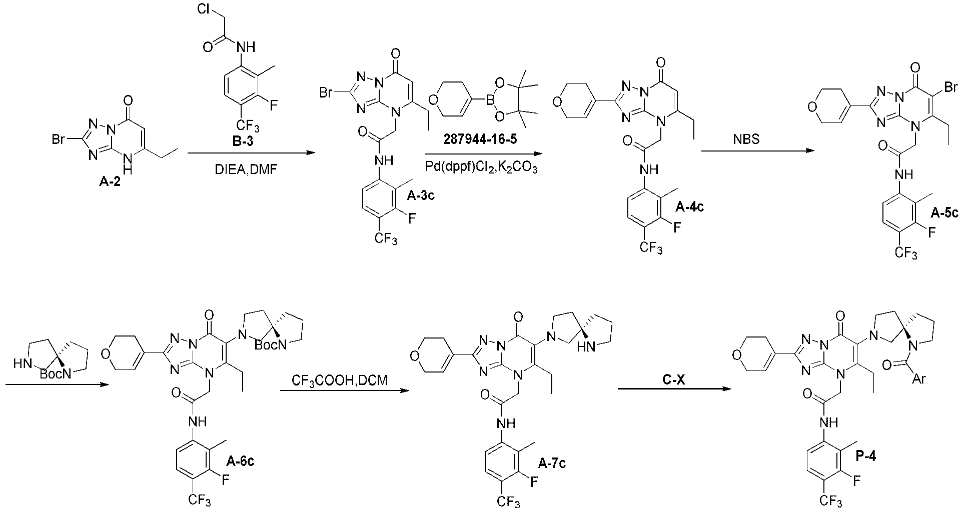

### 6.1 Synthesis of intermediate B-3

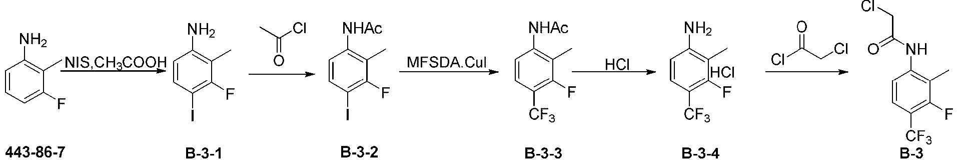

### 6.1.1 Synthesis of intermediate B-3-1

3-Fluoro-2-methylaniline was dissolved in acetic acid (1 mmol:1 mL) at room temperature, followed by the addition of N-iodosuccinimide (1.0 e.q.). Subsequently, the reaction solution was heated to 95 °C and reacted for 2.5 h. After the reaction was completed, as monitored by LC-MS, the reaction solution was concentrated to near dryness, diluted with ethyl acetate, and poured into a saturated sodium thiosulfate solution, and the mixture was stirred for 5-10 min. Subsequently, the mixture was washed twice with water and once with saturated brine, and the resulting organic phase was dried over anhydrous sodium sulfate, concentrated under reduced pressure, and subsequently subjected to column chromatography to give intermediate B-3-1.

### 6.1.2 Synthesis of intermediate B-3-2

Intermediate B-3-1 (1.0 e.q.) and triethylamine (2.0 e.q.) were dissolved in DCM (1 mmol:3 mL) at room temperature, and subsequently, the reaction solution was cooled to 0 °C. Acetyl chloride (1.5 e.q.) was added, and subsequently, the reaction solution was slowly warmed to room temperature and reacted for 4 h. After the reaction was completed, as monitored by TLC, the reaction solution was diluted with dichloromethane and washed twice with water and once with saturated brine. The resulting organic phase was dried over anhydrous sodium sulfate, concentrated under reduced pressure, and subsequently subjected to column chromatography to give intermediate B-3-2.

### 6.1.3 Synthesis of intermediate B-3-3

Intermediate B-3-2 (1.0 e.q.), hexamethylphosphoramide (5.0 e.q.), methyl 2,2-difluoro-2-(fluorosulfonyl)acetate (5.0 e.q.), and cuprous iodide (2.0 e.q.) were dissolved in DMF (1 mmol:2 mL). After being purged three times with nitrogen, the reaction solution was heated to 80 °C and reacted for 16 h. After the reaction was completed, as monitored by LCMS, the reaction solution was diluted with ethyl acetate and washed 3 times with water and once with saturated brine. The resulting organic phase was dried over anhydrous sodium sulfate, concentrated under reduced pressure, and subsequently subjected to column chromatography to give intermediate B-3-3.

### 6.1.4 Synthesis of intermediate B-3-4

Intermediate B-3-3 (1.0 e.q.) was dissolved in ethanol (1 mmol:2 mL) at room temperature, and 2 N HCl in dioxane (5.0 e.q.) was added. Subsequently, the reaction solution was heated and refluxed, and reacted for 2 h. After the reaction was completed, as monitored by LCMS, the reaction solution was concentrated to near dryness, and the concentrated solution was slurried with ethyl acetate and petroleum ether (1:2) to give pure intermediate B-3-4.

### 6.1.5 Synthesis of intermediate B-3

Intermediate B-3-4 (1.0 e.q.) and sodium bicarbonate (3.0 e.q.) were added to dichloromethane (1 mmol:2 mL), and chloroacetyl chloride (1.05 e.q.) was added dropwise at 0 °C. Subsequently, the mixture was reacted at room temperature for 16 h. After the reaction was completed, as monitored by TLC, the reaction solution was concentrated under reduced pressure to give a crude product, which was slurried with petroleum ether to give intermediate B-3.

6.2 The synthesis of intermediate A-3c was based on the synthesis procedures of Reaction Example A-3b.

6.3 The synthesis of intermediate A-4c was based on the synthesis procedures of Reaction Example A-4b.

6.4 The synthesis of intermediate A-5c was based on the synthesis procedures of Reaction Example A-5b.

6.5 The synthesis of intermediate A-6c was based on the synthesis procedures of Reaction Example A-6b.

6.6 The synthesis of intermediate A-7c was based on the synthesis procedures of Reaction Example A-7b.

### 6.7 Synthesis of target products

6.7.1 The synthesis procedures of the target product T032 were based on the synthesis of A-8, and its structure and general analytical data are shown in Table 1.

6.7.2 The synthesis procedures of the target product T033 were based on the synthesis of T016, and its structure and general analytical data are shown in Table 1.

6.7.3 The synthesis procedures of the target product T034 were based on the synthesis of T017, and its structure and general analytical data are shown in Table 1.

6.7.4 The synthesis procedures of the target product T035 were based on the synthesis of T018, and its structure and general analytical data are shown in Table 1.

6.7.5 The synthesis procedures of the target product T036 were based on the synthesis of T019, and its structure and general analytical data are shown in Table 1.

6.7.6 The synthesis procedures of the target product T037 were based on the synthesis of T020, and its structure and general analytical data are shown in Table 1.

6.7.7 The synthesis procedures of the target product T038 were based on the synthesis of T021, and its structure and general analytical data are shown in Table 1.

6.7.8 The synthesis procedures of the target product T039 were based on the synthesis of T006, and its structure and general analytical data are shown in Table 1.

6.7.9 The synthesis procedures of the target product T040 were based on the synthesis of T013, and its structure and general analytical data are shown in Table 1.

6.7.10 The synthesis procedures of the target product T041 were based on the synthesis of T014, and its structure and general analytical data are shown in Table 1.

### Example 7

The synthetic route was as follows:

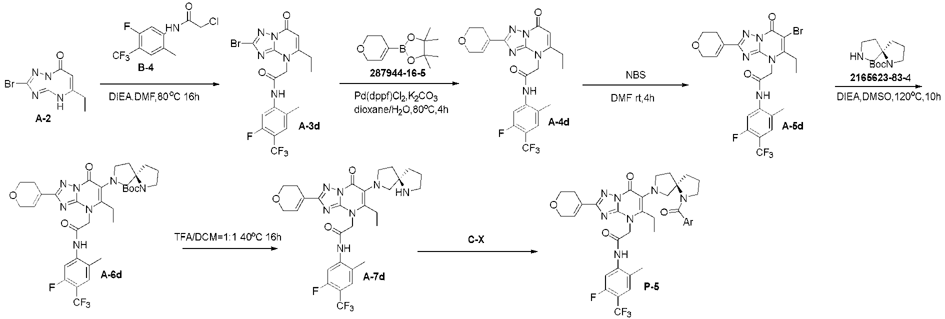

### 7.1 Synthesis of intermediate B-4

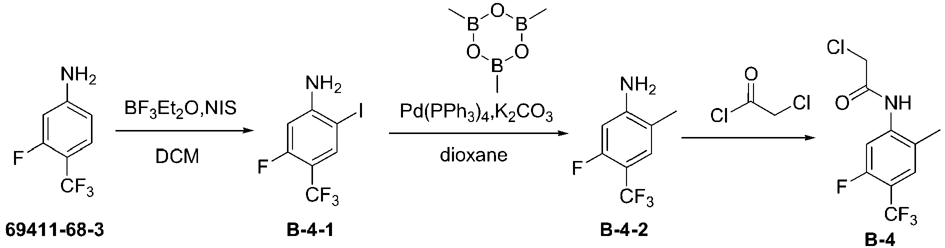

### 7.1.1 Synthesis of intermediate B-4-1

The intermediate 4-amino-2-fluorobenzotrifluoride (1.0 e.q.) and boron trifluoride diethyl etherate (1.0 e.q.) were dissolved in dichloromethane (1 mmol:3 mL) at room temperature. Subsequently, the reaction solution was cooled to 0 °C, and NIS (1.0 e.q.) was added dropwise. The reaction solution was warmed to room temperature and reacted for 2 h. After the reaction was completed, as monitored by LCMS, the reaction solution was concentrated to dryness by rotary evaporation, diluted with ethyl acetate, and poured into a saturated sodium thiosulfate solution. The mixture was stirred at room temperature for 5-10 min, and subsequently, the organic phase was retained and washed twice with water and once with saturated brine. The organic phase was concentrated to dryness by rotary evaporation and subsequently purified by column chromatography to give intermediate B-4-1.

### 7.1.2 Synthesis of intermediate B-4-2

Intermediate B-4-1 (1.0 e.q.), trimethylboroxine (3.2 e.q.), Pd(PPh₃)₄ (0.05 e.q.), and potassium carbonate (4.6 e.q.) were dissolved in 1,4-dioxane (1 mmol:3 mL). The reaction solution was heated to 80 °C and reacted for 16 h. After the reaction was completed, as monitored by LCMS, the reaction solution was cooled to room temperature, diluted with ethyl acetate, and washed twice with water and once with saturated brine. The organic phase was concentrated to dryness by rotary evaporation and subsequently purified by column chromatography to give intermediate B-4-2.

### 7.1.3 Synthesis of intermediate B-4

Intermediate B-4-2 (1.0 e.q.) and sodium bicarbonate (3.0 e.q.) were added to dichloromethane (1 mmol:2 mL), and chloroacetyl chloride (1.05 e.q.) was added dropwise at 0 °C. Subsequently, the mixture was reacted at room temperature for 16 h. After the reaction was completed, as monitored by TLC, the reaction solution was concentrated under reduced pressure to give a crude product, which was slurried with petroleum ether to give intermediate B-4. 7.2 The synthesis of intermediate A-3d was based on the synthesis procedures of Reaction Example A-3b.

7.3 The synthesis of intermediate A-4d was based on the synthesis procedures of Reaction Example A-4b.

7.4 The synthesis of intermediate A-5d was based on the synthesis procedures of Reaction Example A-5b.

7.5 The synthesis of intermediate A-6d was based on the synthesis procedures of Reaction Example A-6b.

7.6 The synthesis of intermediate A-7d was based on the synthesis procedures of Reaction Example A-7b.

7.7 Synthesis of target products

7.7.1 The synthesis procedures of the target product T042 were based on the synthesis of A-8, and its structure and general analytical data are shown in Table 1.

7.7.2 The synthesis procedures of the target product T043 were based on the synthesis of T016, and its structure and general analytical data are shown in Table 1.

7.7.3 The synthesis procedures of the target product T044 were based on the synthesis of T017, and its structure and general analytical data are shown in Table 1.

7.7.4 The synthesis procedures of the target product T045 were based on the synthesis of T018, and its structure and general analytical data are shown in Table 1.

7.7.5 The synthesis procedures of the target product T046 were based on the synthesis of T019, and its structure and general analytical data are shown in Table 1.

7.7.6 The synthesis procedures of the target product T047 were based on the synthesis of T020, and its structure and general analytical data are shown in Table 1.

7.7.7 The synthesis procedures of the target product T048 were based on the synthesis of T021, and its structure and general analytical data are shown in Table 1.

7.7.8 The synthesis procedures of the target product T049 were based on the synthesis of T006, and its structure and general analytical data are shown in Table 1.

7.7.9 The synthesis procedures of the target product T050 were based on the synthesis of T013, and its structure and general analytical data are shown in Table 1.

7.7.10 The synthesis procedures of the target product T051 were based on the synthesis of T014, and its structure and general analytical data are shown in Table 1.

### Example 8

The synthetic route was as follows:

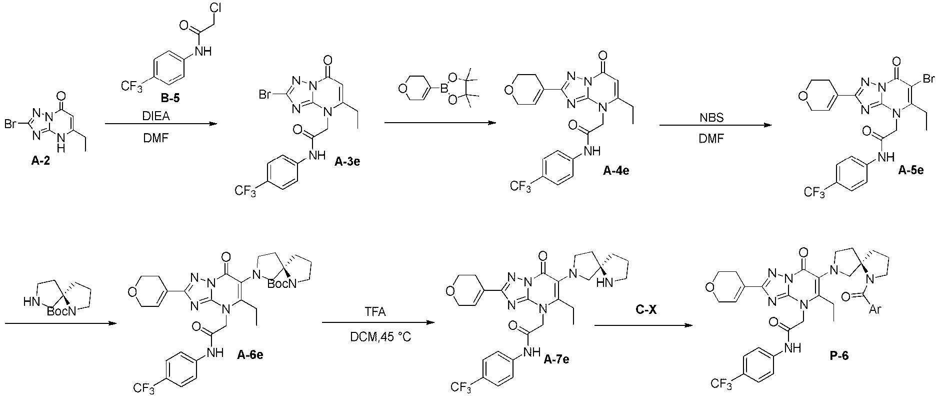

### 8.1 Synthesis of intermediate B-5

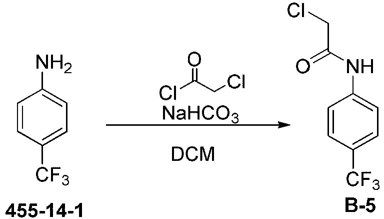

4-(Trifluoromethyl)aniline (1.0 e.q.) was added to dichloromethane (1 mmol:2 mL), and chloroacetyl chloride (1.05 e.q.) was added dropwise at 0 °C. Subsequently, the mixture was reacted at room temperature for 16 h. After the reaction was completed, as monitored by TLC, the reaction solution was concentrated under reduced pressure to give a crude product, which was slurried with petroleum ether to give intermediate B-5.

8.2 The synthesis of intermediate A-3e was based on the synthesis procedures of Reaction Example A-3b.

8.3 The synthesis of intermediate A-4e was based on the synthesis procedures of Reaction Example A-4b.

8.4 The synthesis of intermediate A-5e was based on the synthesis procedures of Reaction Example A-5b.

8.5 The synthesis of intermediate A-6e was based on the synthesis procedures of Reaction Example A-6b.

8.6 The synthesis of intermediate A-7e was based on the synthesis procedures of Reaction Example A-7b.

### 8.7 Synthesis of target products

8.7.1 The synthesis procedures of the target product T052 were based on the synthesis of A-8, and its structure and general analytical data are shown in Table 1.

8.7.2 The synthesis procedures of the target product T053 were based on the synthesis of T016, and its structure and general analytical data are shown in Table 1.

8.7.3 The synthesis procedures of the target product T054 were based on the synthesis of T017, and its structure and general analytical data are shown in Table 1.

8.7.4 The synthesis procedures of the target product T055 were based on the synthesis of T018, and its structure and general analytical data are shown in Table 1.

8.7.5 The synthesis procedures of the target product T056 were based on the synthesis of T019, and its structure and general analytical data are shown in Table 1.

8.7.6 The synthesis procedures of the target product T057 were based on the synthesis of T020, and its structure and general analytical data are shown in Table 1.

8.7.7 The synthesis procedures of the target product T058 were based on the synthesis of T021, and its structure and general analytical data are shown in Table 1.

8.7.8 The synthesis procedures of the target product T059 were based on the synthesis of T006, and its structure and general analytical data are shown in Table 1.

8.7.9 The synthesis procedures of the target product T060 were based on the synthesis of T013, and its structure and general analytical data are shown in Table 1.

8.7.10 The synthesis procedures of the target product T061 were based on the synthesis of T014, and its structure and general analytical data are shown in Table 1.

### Example 9

The synthetic route was as follows:

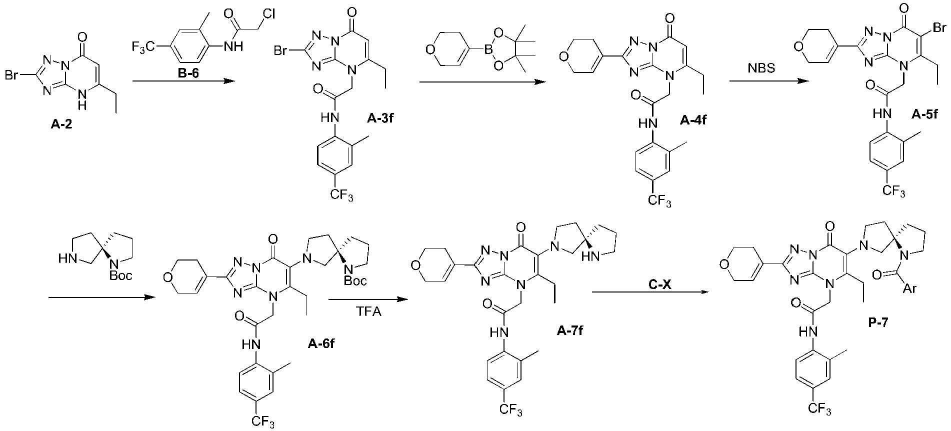

### 9.1 Synthesis of intermediate B-6

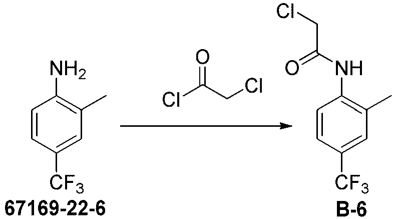

2-Methyl-4-(trifluoromethyl)aniline (1.0 e.q.) was added to dichloromethane (1 mmol:2 mL), and chloroacetyl chloride (1.05 e.q.) was added dropwise at 0 °C. Subsequently, the mixture was reacted at room temperature for 16 h. After the reaction was completed, as monitored by TLC, the reaction solution was concentrated under reduced pressure to give a crude product, which was slurried with petroleum ether to give intermediate B-6.

9.2 The synthesis of intermediate A-3f was based on the synthesis procedures of Reaction Example A-3b.

9.3 The synthesis of intermediate A-4f was based on the synthesis procedures of Reaction Example A-4b.

9.4 The synthesis of intermediate A-5f was based on the synthesis procedures of Reaction Example A-5b.

9.5 The synthesis of intermediate A-6f was based on the synthesis procedures of Reaction Example A-6b.

9.6 The synthesis of intermediate A-7f was based on the synthesis procedures of Reaction Example A-7b.

### 9.7 Synthesis of target products

9.7.1 The synthesis procedures of the target product T062 were based on the synthesis of A-8, and its structure and general analytical data are shown in Table 1.

9.7.2 The synthesis procedures of the target product T063 were based on the synthesis of T016, and its structure and general analytical data are shown in Table 1.

9.7.3 The synthesis procedures of the target product T064 were based on the synthesis of T017, and its structure and general analytical data are shown in Table 1.

9.7.4 The synthesis procedures of the target product T065 were based on the synthesis of T018, and its structure and general analytical data are shown in Table 1.

9.7.5 The synthesis procedures of the target product T066 were based on the synthesis of T019, and its structure and general analytical data are shown in Table 1.

9.7.6 The synthesis procedures of the target product T067 were based on the synthesis of T020, and its structure and general analytical data are shown in Table 1.

9.7.7 The synthesis procedures of the target product T068 were based on the synthesis of T021, and its structure and general analytical data are shown in Table 1.

9.7.8 The synthesis procedures of the target product T069 were based on the synthesis of T006, and its structure and general analytical data are shown in Table 1.

9.7.9 The synthesis procedures of the target product T070 were based on the synthesis of T013, and its structure and general analytical data are shown in Table 1.

9.7.10 The synthesis procedures of the target product T071 were based on the synthesis of T014, and its structure and general analytical data are shown in Table 1.

### Example 10

The synthetic route was as follows:

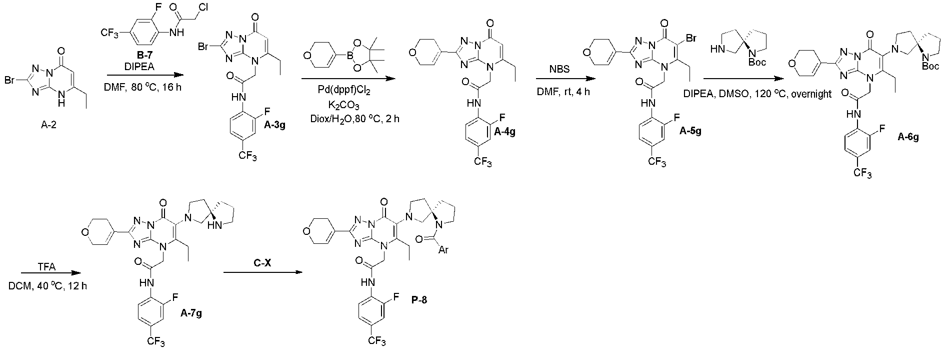

### 10.1 Synthesis of intermediate B-7

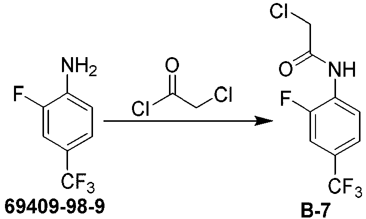

2-Fluoro-4-(trifluoromethyl)aniline (1.0 e.q.) was added to dichloromethane (1 mmol:2 mL), and chloroacetyl chloride (1.05 e.q.) was added dropwise at 0 °C. Subsequently, the mixture was reacted at room temperature for 16 h. After the reaction was completed, as monitored by TLC, the reaction solution was concentrated under reduced pressure to give a crude product, which was slurried with petroleum ether to give intermediate B-7.

10.2 The synthesis of intermediate A-3g was based on the synthesis procedures of A-3b.

10.3 The synthesis of intermediate A-4g was based on the synthesis procedures of A-4b.

10.4 The synthesis of intermediate A-5g was based on the synthesis procedures of A-5b.

10.5 The synthesis of intermediate A-6g was based on the synthesis procedures of A-6b.

10.6 The synthesis of intermediate A-7g was based on the synthesis procedures of A-7b.

10.7 Synthesis of target products

10.7.1 The synthesis procedures of the target product T072 were based on the synthesis of A-8, and its structure and general analytical data are shown in Table 1.

10.7.2 The synthesis procedures of the target product T073 were based on the synthesis of T016, and its structure and general analytical data are shown in Table 1.

10.7.3 The synthesis procedures of the target product T074 were based on the synthesis of T017, and its structure and general analytical data are shown in Table 1.

10.7.4 The synthesis procedures of the target product T075 were based on the synthesis of T018, and its structure and general analytical data are shown in Table 1.

10.7.5 The synthesis procedures of the target product T076 were based on the synthesis of T019, and its structure and general analytical data are shown in Table 1.

10.7.6 The synthesis procedures of the target product T077 were based on the synthesis of T020, and its structure and general analytical data are shown in Table 1.

10.7.7 The synthesis procedures of the target product T078 were based on the synthesis of T021, and its structure and general analytical data are shown in Table 1.

10.7.8 The synthesis procedures of the target product T079 were based on the synthesis of T006, and its structure and general analytical data are shown in Table 1.

10.7.9 The synthesis procedures of the target product T080 were based on the synthesis of T013, and its structure and general analytical data are shown in Table 1.

10.7.10 The synthesis procedures of the target product T081 were based on the synthesis of T014, and its structure and general analytical data are shown in Table 1.

### 10.7.11 Synthesis of target product T094

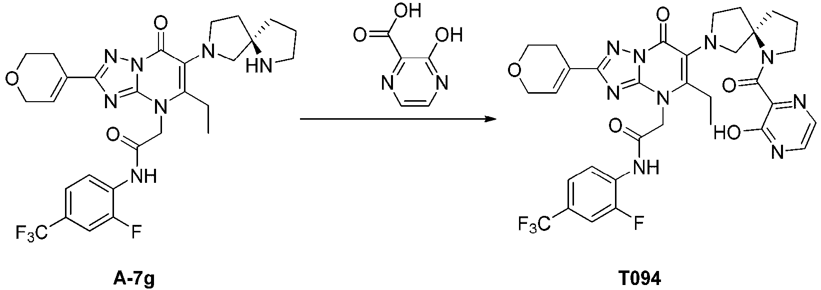

Intermediate A-7g (1.0 e.q.), PyBOP (2.5 e.q.), and DIEA (3 e.q.) were dissolved in DMF (1 mmol:10 mL), and 3-hydroxypyrazine-2-carboxylic acid (2.5 e.q.) was finally added. Subsequently, the mixture was heated to 50 °C and reacted for 2 h. After the reaction was completed, as monitored by LC-MS, the reaction solution was diluted with water and extracted twice with dichloromethane. The organic phases were combined, washed once with saturated brine, dried over anhydrous sodium sulfate, concentrated under reduced pressure, and subsequently subjected to preparative high-pressure reverse-phase chromatography to give compound T094, the structure and general analytical data of which are shown in Table 1.

### 10.7.12 Synthesis of target product T095

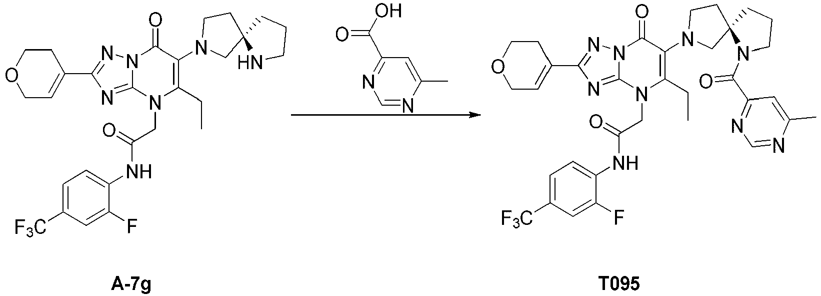

Intermediate A-7g (1.0 e.q.), PyBOP (2.5 e.q.), and DIEA (3 e.q.) were dissolved in DMF (1 mmol:10 mL), followed by the addition of 6-methylpyrimidine-4-carboxylic acid (2.5 e.q.), and subsequently, the mixture was heated to 50 °C and reacted for 2 h. After the reaction was completed, as monitored by LC-MS, the reaction solution was diluted with water and extracted twice with dichloromethane. The organic phases were combined, washed once with saturated brine, dried over anhydrous sodium sulfate, concentrated under reduced pressure, and subsequently subjected to preparative high-pressure reverse-phase chromatography to give compound T095, the structure and general analytical data of which are shown in Table 1.

### 10.7.13 Synthesis of target product T096

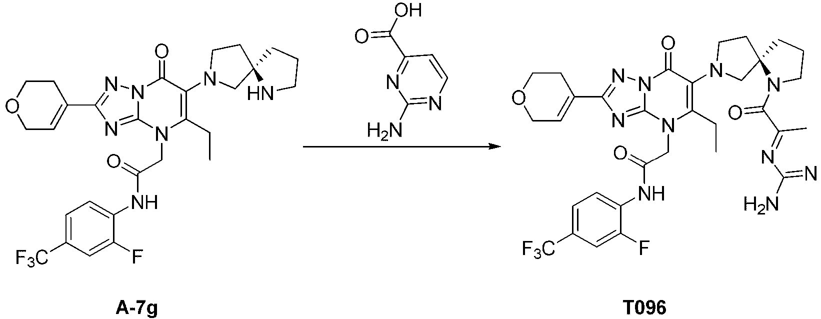

Intermediate A-7g (1.0 e.q.), PyBOP (2.5 e.q.), and DIEA (3 e.q.) were dissolved in DMF (1 mmol:10 mL), and 2-aminopyrimidine-4-carboxylic acid (2.5 e.q.) was finally added. Subsequently, the mixture was heated to 50 °C and reacted for 2 h. After the reaction was completed, as monitored by LC-MS, the reaction solution was diluted with water and extracted twice with dichloromethane. The organic phases were combined, washed once with saturated brine, dried over anhydrous sodium sulfate, concentrated under reduced pressure, and subsequently subjected to preparative high-pressure reverse-phase chromatography to give compound T096, the structure and general analytical data of which are shown in Table 1.

### 10.7.14 Synthesis of target product T099

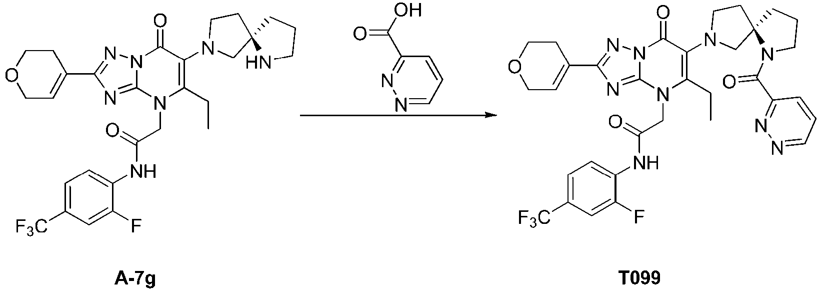

Intermediate A-7g (1.0 e.q.), PyBOP (2.5 e.q.), and DIEA (3 e.q.) were dissolved in DMF (1 mmol:10 mL), and 3-carboxypyridazine (2.5 e.q.) was added. Subsequently, the mixture was heated to 50 °C and reacted for 2 h. After the reaction was completed, as monitored by LC-MS, the reaction solution was diluted with water and extracted twice with dichloromethane. The organic phases were combined, washed once with saturated brine, dried over anhydrous sodium sulfate, concentrated under reduced pressure, and subsequently subjected to preparative high-pressure reverse-phase chromatography to give compound T099, the structure and general analytical data of which are shown in Table 1.

### 10.7.15 Synthesis of target product T100

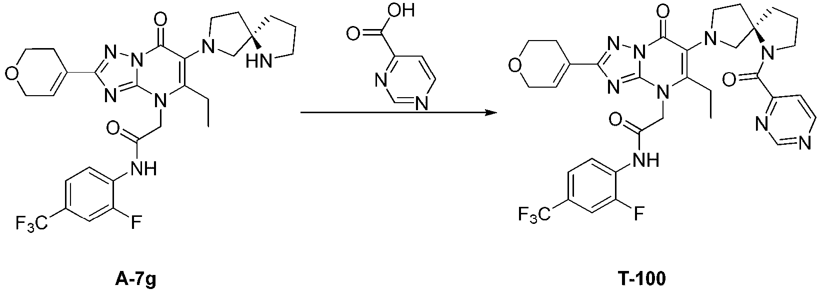

Intermediate A-7g (1.0 e.q.), PyBOP (2.5 e.q.), and DIEA (3 e.q.) were dissolved in DMF (1 mmol:10 mL), followed by the addition of 4-pyrimidinecarboxylic acid (2.5 e.q.), and subsequently, the mixture was heated to 50 °C and reacted for 2 h. After the reaction was completed, as monitored by LC-MS, the reaction solution was diluted with water and extracted twice with dichloromethane. The organic phases were combined, washed once with saturated brine, dried over anhydrous sodium sulfate, concentrated under reduced pressure, and subsequently subjected to preparative high-pressure reverse-phase chromatography to give compound T100, the structure and general analytical data of which are shown in Table 1.

### 10.7.16 Synthesis of target product T101

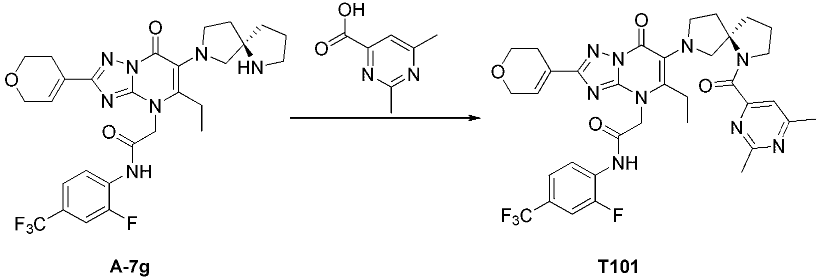

Intermediate A-7g (1.0 e.q.), PyBOP (2.5 e.q.), and DIEA (3 e.q.) were dissolved in DMF (1 mmol:10 mL), and 2,6-dimethylpyrimidine-4-carboxylic acid (2.5 e.q.) was added. Subsequently, the mixture was heated to 50 °C and reacted for 2 h. After the reaction was completed, as monitored by LC-MS, the reaction solution was diluted with water and extracted twice with dichloromethane. The organic phases were combined, washed once with saturated brine, dried over anhydrous sodium sulfate, concentrated under reduced pressure, and subsequently subjected to preparative high-pressure reverse-phase chromatography to give compound T101, the structure and general analytical data of which are shown in Table 1.

### 10.7.17 Synthesis of target product T102

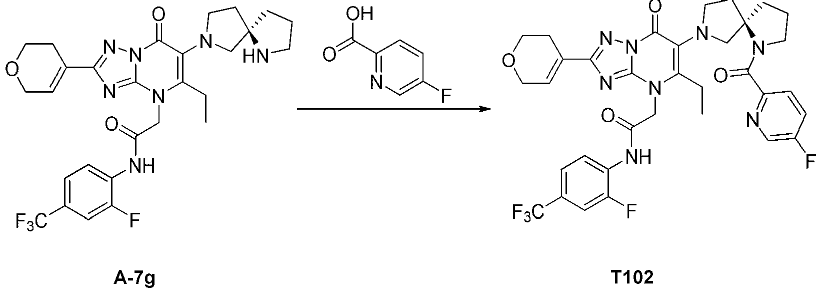

Intermediate A-7g (1.0 e.q.), PyBOP (2.5 e.q.), and DIEA (3 e.q.) were dissolved in DMF (1 mmol:10 mL), and 5-fluoro-2-pyridinecarboxylic acid (2.5 e.q.) was finally added. Subsequently, the mixture was heated to 50 °C and reacted for 2 h. After the reaction was completed, as monitored by LC-MS, the reaction solution was diluted with water and extracted twice with dichloromethane. The organic phases were combined, washed once with saturated brine, dried over anhydrous sodium sulfate, concentrated under reduced pressure, and subsequently subjected to preparative high-pressure reverse-phase chromatography to give compound T102, the structure and general analytical data of which are shown in Table 1.

### 10.7.18 Synthesis of target product T103

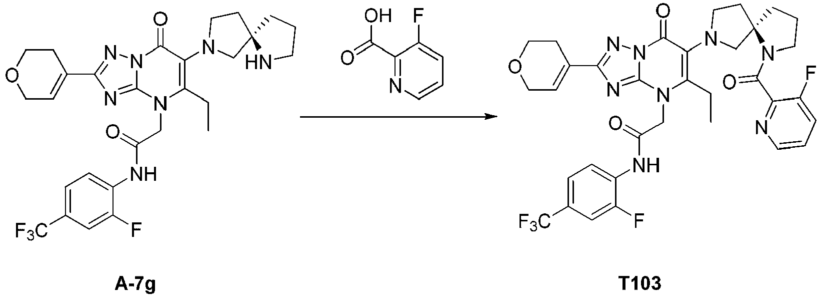

Intermediate A-7g (1.0 e.q.), PyBOP (2.5 e.q.), and DIEA (3 e.q.) were dissolved in DMF (1 mmol:10 mL), and 3-fluoropyridine-2-carboxylic acid (2.5 e.q.) was finally added. Subsequently, the mixture was heated to 50 °C and reacted for 2 h. After the reaction was completed, as monitored by LC-MS, the reaction solution was diluted with water and extracted twice with dichloromethane. The organic phases were combined, washed once with saturated brine, dried over anhydrous sodium sulfate, concentrated under reduced pressure, and subsequently subjected to preparative high-pressure reverse-phase chromatography to give compound T103, the structure and general analytical data of which are shown in Table 1.

### 10.7.19 Synthesis of target product T104

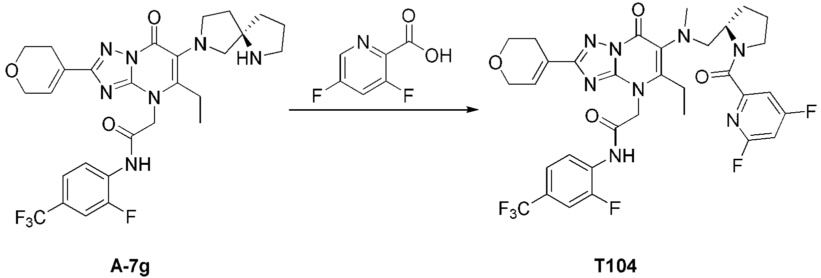

Intermediate A-7g (1.0 e.q.), PyBOP (2.5 e.q.), and DIEA (3 e.q.) were dissolved in DMF (1 mmol: 10 mL), and 3,5-difluoro-2-pyridinecarboxylic acid (2.5 e.q.) was finally added. Subsequently, the mixture was heated to 50 °C and reacted for 2 h. After the reaction was completed, as monitored by LC-MS, the reaction solution was diluted with water and extracted twice with dichloromethane. The organic phases were combined, washed once with saturated brine, dried over anhydrous sodium sulfate, concentrated under reduced pressure, and subsequently subjected to preparative high-pressure reverse-phase chromatography to give compound T104, the structure and general analytical data of which are shown in Table 1.

### 10.7.20 Synthesis of target product T105

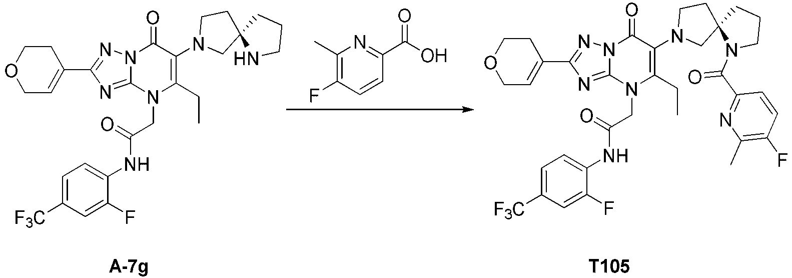

Intermediate A-7g (1.0 e.q.), PyBOP (2.5 e.q.), and DIEA (3 e.q.) were dissolved in DMF (1 mmol:10 mL), and 5-fluoro-6-methylpyridine-2-carboxylic acid (2.5 e.q.) was finally added. Subsequently, the mixture was heated to 50 °C and reacted for 2 h. After the reaction was completed, as monitored by LC-MS, the reaction solution was diluted with water and extracted twice with dichloromethane. The organic phases were combined, washed once with saturated brine, dried over anhydrous sodium sulfate, concentrated under reduced pressure, and subsequently subjected to preparative high-pressure reverse-phase chromatography to give compound T105, the structure and general analytical data of which are shown in Table 1.

### 10.7.21 Synthesis of target product T106

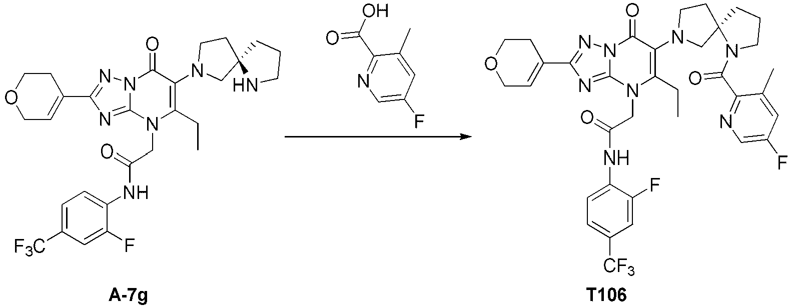

Intermediate A-7g (1.0 e.q.), PyBOP (2.5 e.q.), and DIEA (3 e.q.) were dissolved in DMF (1 mmol:10 mL), and 5-fluoro-3-methylpicolinic acid (2.5 e.q.) was finally added. Subsequently, the mixture was heated to 50 °C and reacted for 2 h. After the reaction was completed, as monitored by LC-MS, the reaction solution was diluted with water and extracted twice with dichloromethane. The organic phases were combined, washed once with saturated brine, dried over anhydrous sodium sulfate, concentrated under reduced pressure, and subsequently subjected to preparative high-pressure reverse-phase chromatography to give compound T106, the structure and general analytical data of which are shown in Table 1.

### Example 11

The synthetic route was as follows:

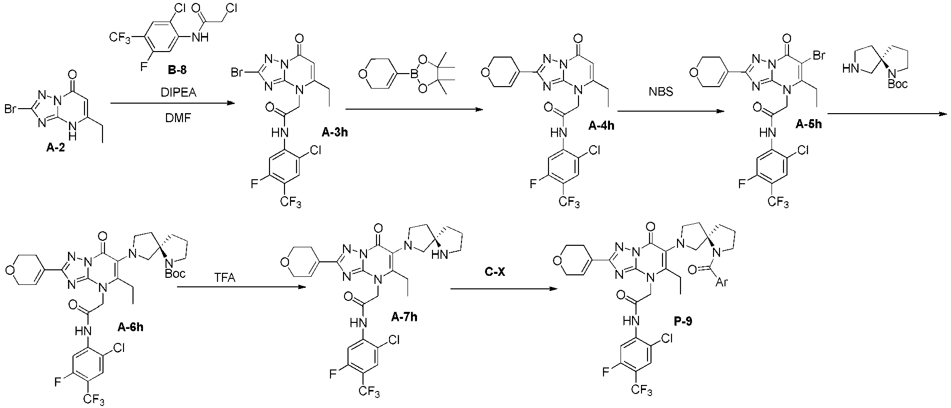

### 11.1 Synthesis of intermediate B-8

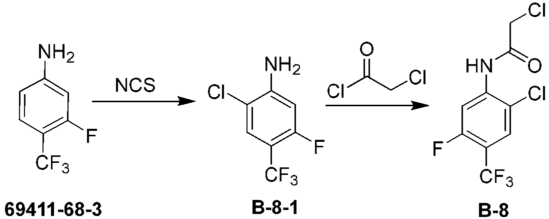

### 11.1.1 Synthesis of intermediate B-8-1

The intermediate 4-amino-2-fluorobenzotrifluoride (1.0 e.q.) was dissolved in acetonitrile (1 mmol:2 mL) at room temperature, and N-chlorosuccinimide (1.05 e.q.) was added in batches. Subsequently, the reaction solution was heated to 50 °C and reacted for 6 h. After the reaction was completed, as monitored by LCMS, acetonitrile was removed by rotary evaporation, and the concentrated solution was diluted with ethyl acetate. The organic phase was washed twice with water and once with saturated brine. The organic phase was concentrated to dryness by rotary evaporation and subsequently purified by column chromatography to give pure intermediate B-8-1.

### 11.1.2 Synthesis of intermediate B-8

Intermediate B-8-1 (1.0 e.q.) was added to dichloromethane (1 mmol:2 mL), and chloroacetyl chloride (1.05 e.q.) was added dropwise at 0 °C. Subsequently, the mixture was reacted at room temperature for 16 h. After the reaction was completed, as monitored by TLC, the reaction solution was concentrated under reduced pressure to give a crude product, which was slurried with petroleum ether to give intermediate B-8.

11.2 The synthesis of intermediate A-3h was based on the synthesis procedures of A-3b.

11.3 The synthesis of intermediate A-4h was based on the synthesis procedures of A-4b.

11.4 The synthesis of intermediate A-5h was based on the synthesis procedures of A-5b.

11.5 The synthesis of intermediate A-6h was based on the synthesis procedures of A-6b.

11.6 The synthesis of intermediate A-7h was based on the synthesis procedures of A-7b.

### 11.7 Synthesis of target products

11.7.1 The synthesis procedures of the target product T082 were based on the synthesis of A-8, and its structure and general analytical data are shown in Table 1.

11.7.2 The synthesis procedures of the target product T083 were based on the synthesis of T016, and its structure and general analytical data are shown in Table 1.

11.7.3 The synthesis procedures of the target product T084 were based on the synthesis of T017, and its structure and general analytical data are shown in Table 1.

11.7.4 The synthesis procedures of the target product T085 were based on the synthesis of T018, and its structure and general analytical data are shown in Table 1.

11.7.5 The synthesis procedures of the target product T086 were based on the synthesis of T019, and its structure and general analytical data are shown in Table 1.

11.7.6 The synthesis procedures of the target product T087 were based on the synthesis of T020, and its structure and general analytical data are shown in Table 1.

11.7.7 The synthesis procedures of the target product T088 were based on the synthesis of T021, and its structure and general analytical data are shown in Table 1.

11.7.8 The synthesis procedures of the target product T089 were based on the synthesis of T006, and its structure and general analytical data are shown in Table 1.

11.7.9 The synthesis procedures of the target product T090 were based on the synthesis of T013, and its structure and general analytical data are shown in Table 1.

11.7.10 The synthesis procedures of the target product T091 were based on the synthesis of T014, and its structure and general analytical data are shown in Table 1.

### Example 12

The synthetic route was as follows:

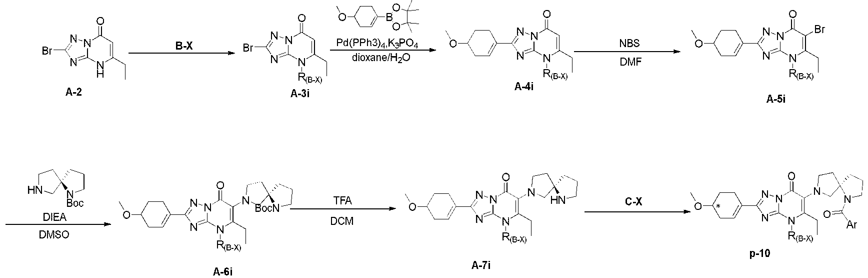

12.1 The synthesis of intermediate A-3i was based on the synthesis procedures of A-3b.

12.2 The synthesis of intermediate A-4i was based on the synthesis procedures of A-4b.

12.3 The synthesis of intermediate A-5i was based on the synthesis procedures of A-5b.

12.4 The synthesis of intermediate A-6i was based on the synthesis procedures of A-6b.

12.5 The synthesis of intermediate A-7i was based on the synthesis procedures of A-7b.

12.6 The synthesis of intermediate 8i was based on the synthesis procedures of A-8h.

### 12.7 Synthesis of target products

12.7.1 Synthesis of target products T097 and T098, wherein B-X = B-7.

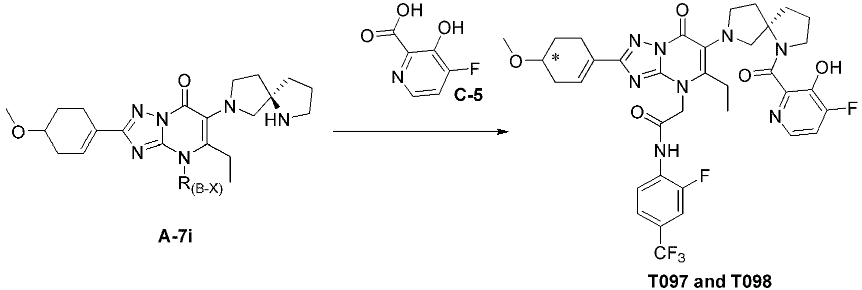

Intermediate A-7i (1.0 e.q.), C-5 (2.0 e.q.), and N,N-diisopropylethylamine (3 e.q.) were dissolved in N,N-dimethylformamide (1 mmol:5 mL) at room temperature, followed by the addition of (benzotriazol-1-yloxy)tripyrrolidinophosphonium hexafluorophosphate (2.0 e.q.) at 0 °C, and subsequently, the mixture was heated to 40 °C and reacted for 2 h. After the reaction was completed, as monitored by LC-MS, the reaction solution was diluted with ethyl acetate and washed twice with water and once with saturated brine. The resulting organic phase was dried over anhydrous sodium sulfate and concentrated under reduced pressure. The residue was subjected to column chromatography to give a pure product, which was subsequently subjected to SFC chiral resolution to sequentially give the target products T097 and T098 according to the peak elution order, the structures and general analytical data of which are shown in Table 1.

The chiral resolution conditions were as follows:

### Prep-HPLC conditions:

Chromatographic column: CHIRALPAK IG, 2 cm × 25 cm, 5 µm;
Mobile phase: A: hexane (0.1% FA); B: MeOH:DCM;
Flow rate: 20 mL/min;
Wavelength: UV 220 nm;
Column temperature: 25 °C;
Prep-HPLC chromatograph: Prep-HPLC-Gilson.

12.7.2 The synthesis of the target products T113 and T114 was based on the synthesis of T111. SFC chiral resolution was performed to sequentially give the target products T113 and T114 according to the peak elution order, the structures and general analytical data of which are shown in Table 1. The chiral resolution conditions were the same as those for T097 and T098.

12.7.3 Synthesis of target products T115 and T116, wherein B-X = B-5.

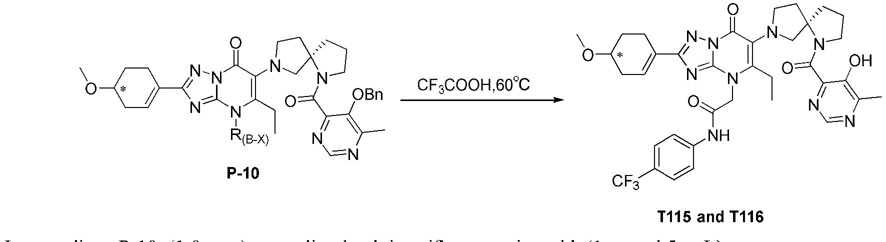

Intermediate P-10 (1.0 e.q.) was dissolved in trifluoroacetic acid (1 mmol:5 mL) at room temperature, and subsequently, the mixture was heated to 60 °C and reacted for 12 h. After the reaction was completed, as monitored by LC-MS, the reaction solution was diluted with ethyl acetate and washed twice with water and once with saturated brine. The resulting organic phase was dried over anhydrous sodium sulfate and concentrated under reduced pressure. The residue was subsequently subjected to SFC chiral resolution to sequentially give the target products T115 and T116 according to the peak elution order. The chiral resolution conditions were the same as those for T097 and T098. The structures and general analytical data of the products are shown in Table 1.

### Example 13

The synthetic route was as follows:

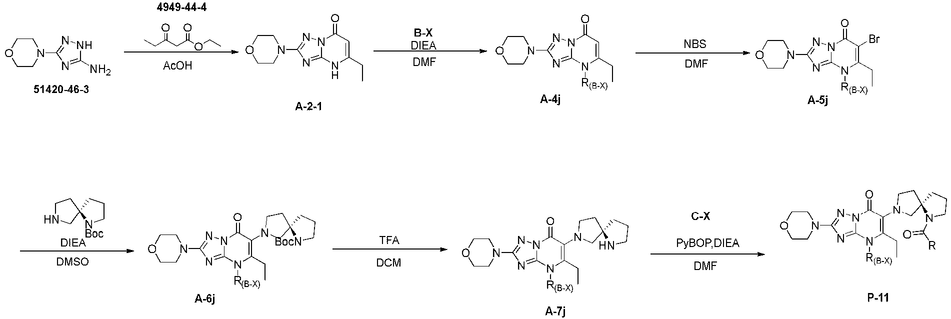

13.1 The synthesis of intermediate A-2-1 was based on the synthesis procedures of A-2.

13.2 The synthesis of intermediate A-4j was based on the synthesis procedures of A-3.

13.3 The synthesis of intermediate A-5j was based on the synthesis procedures of A-5b.

13.4 The synthesis of intermediate A-6j was based on the synthesis procedures of A-6b.

13.5 The synthesis of intermediate A-7j was based on the synthesis procedures of A-7b.

13.6 The synthesis of intermediate A-8j was based on the synthesis procedures of A-8i.

### 13.7 Synthesis of target products

13.7.1 Synthesis of target product T093, wherein B-X = B-5.

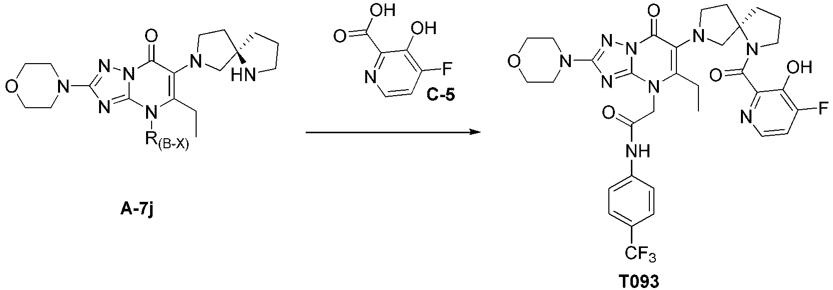

Intermediate A-71 (1.0 e.q.), C-5 (2.0 e.q.), and N,N-diisopropylethylamine (5 e.q.) were dissolved in N,N-dimethylformamide (1 mmol:5 mL) at room temperature, followed by the addition of (benzotriazol-1-yloxy)tripyrrolidinophosphonium hexafluorophosphate (2.0 e.q.) at 0 °C, and subsequently, the mixture was heated to 40 °C and reacted for 2 h. After the reaction was completed, as monitored by LC-MS, the reaction solution was diluted with ethyl acetate and washed twice with water and once with saturated brine. The resulting organic phase was dried over anhydrous sodium sulfate, concentrated under reduced pressure, and subsequently subjected to preparative high-pressure reverse-phase chromatography to give compound T093, the structure and general analytical data of which are shown in Table 1.

13.7.2 Synthesis of target product T111, wherein B-X = B-7.

The synthesis was based on the synthesis of T093, and the structure and general analytical data are shown in Table 1. 13.7.3 The synthesis of the target product T112 was based on the synthesis of T019, wherein B-X = B-5; the structure and general analytical data are shown in Table 1.

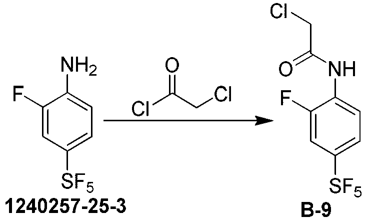

2-Fluoro-4-(pentafluorothio)aniline (1.0 e.q.) was added to dichloromethane (1 mmol:2 mL), and chloroacetyl chloride (1.05 e.q.) was added dropwise at 0 °C. Subsequently, the mixture was reacted at room temperature for 16 h. After the reaction was completed, as monitored by TLC, the reaction solution was concentrated under reduced pressure to give a crude product, which was slurried with petroleum ether to give intermediate B-9.

13.7.4 The synthesis of the target product T121 was based on the synthesis of T019, wherein B-X = B-9; the structure and general analytical data are shown in Table 1.

### Example 14

The synthetic route was as follows:

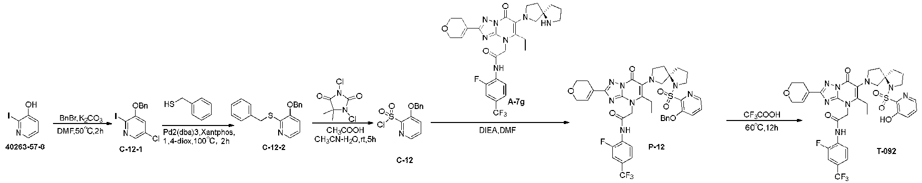

### 14.1 Synthesis of intermediate C-12-1

3-Hydroxy-2-iodopyridine (1.0 e.q.), benzyl bromide (1.05 e.q.), and potassium carbonate (1.5 e.q.) were dissolved in DMF (1 mmol:5 mL) at room temperature. The reaction solution was heated to 50 °C and stirred for 2 h. After the reaction was completed, as monitored by LC-MS, the reaction solution was diluted with ethyl acetate and washed twice with water and once with saturated brine. The resulting organic phase was dried over anhydrous sodium sulfate, concentrated under reduced pressure, and subsequently purified by column chromatography to give the product C-12-1.

### 14.2 Synthesis of intermediate C-12-2

C-12-1 (1.0 e.q.), benzyl mercaptan (1.2 e.q.), PD2DBA3 (0.02 e.q.), and Xantphos (0.06 e.q.) were dissolved in 1,4-dioxane (1 mmol:10 mL) at room temperature. The reaction solution was purged with nitrogen, heated to 100 °C, and stirred for 2 h. After the reaction was completed, as monitored by LC-MS, the reaction solution was diluted with ethyl acetate and washed twice with water and once with saturated brine. The resulting organic phase was dried over anhydrous sodium sulfate, concentrated under reduced pressure, and subsequently purified by column chromatography to give the product C-12-2.

### 14.3 Synthesis of intermediate C-12

Intermediate C-12-2 (1.0 e.q.) was dissolved in acetonitrile (1 mmol:4 mL) and water (1 mmol:0.5 mL). The mixture was cooled to 0 °C, and acetic acid (6.0 e.q.) and 1,3-dichloro-5,5-dimethylhydantoin (1.0 e.q.) were added. Subsequently, the mixture was warmed to room temperature and reacted for 5 h. After the reaction was completed, as monitored by LC-MS, the reaction solution was adjusted to pH 7 with saturated sodium bicarbonate and extracted three times with ethyl acetate. The organic phases were combined, washed with brine, dried over anhydrous sodium sulfate, concentrated under reduced pressure, and subsequently subjected to column chromatography to give intermediate C-12.

### 14.4 Synthesis of intermediate P-12

Compound P-12 was synthesized by condensation of intermediate A-7g (Y is CH₂, n = 1, and m = 1) obtained according to the synthesis procedures of Example 10 with C-12.

Intermediate A-7 (1.0 e.q.) and triethylamine (5 e.q.) were dissolved in dichloromethane (1 mmol:10 mL), and subsequently, the reaction solution was cooled to 0 °C. Intermediate C-3 (2.5 e.q.) was added, and subsequently, the mixture was warmed to room temperature and reacted for 2 h. After the reaction was completed, as monitored by LC-MS, the reaction solution was diluted with water and extracted twice with dichloromethane. The organic phases were combined, washed once with saturated brine, dried over anhydrous sodium sulfate, concentrated under reduced pressure, and subsequently purified by column chromatography to give pure intermediate P-12.

### 14.5 Synthesis of target product T092

Intermediate P-12 was dissolved in a trifluoroacetic acid solution (1 mmol:5 mL), and the reaction solution was heated to 80 °C and reacted overnight. After the reaction was completed, as monitored by LC-MS, the reaction solution was diluted with ethyl acetate and washed twice with water and once with saturated brine. The resulting organic phase was dried over anhydrous sodium sulfate, concentrated under reduced pressure, and subsequently subjected to preparative high-pressure reverse-phase chromatography to give compound T092, the structure and general analytical data of which are shown in Table 1.

### Example 15

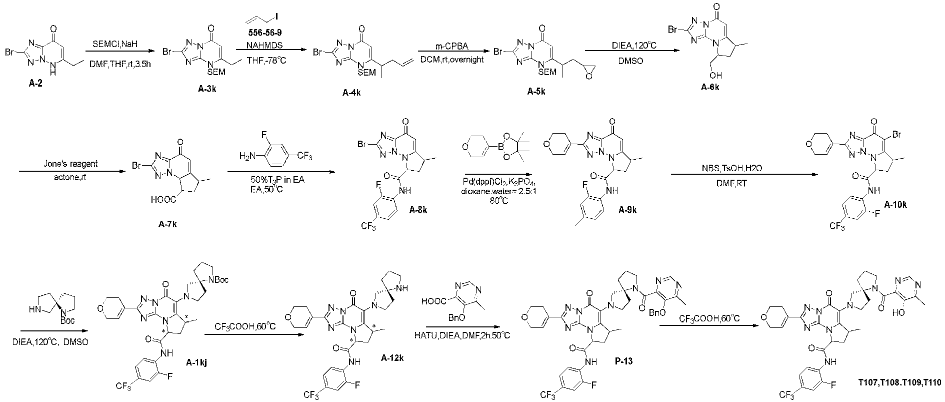

### 15.1 Synthesis of intermediate A-3k

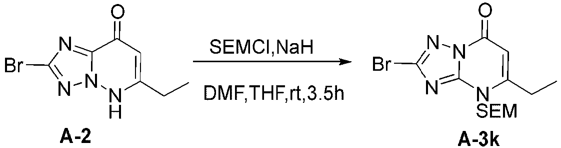

Intermediate A-3k was prepared from intermediate A-2 obtained according to the procedures of Example 1.

A-2 (1.0 e.q.) was weighed out and dissolved in DMF:THF = 1:1 (1 mmol:15 mL) at room temperature, and the reaction solution was cooled to 0 °C. NaH (1.5 e.q.) was added in batches, and the mixture was reacted in an ice bath for 2 h. After the air in the reaction flask was replaced with nitrogen, SEMCl (2.0 e.q.) was added dropwise at 0 °C, and the reaction solution was slowly warmed to room temperature and stirred overnight. After the reaction was completed, as monitored by LC-MS, the reaction solution was diluted with water and extracted three times with ethyl acetate. The organic phases were combined, washed once with saturated brine, dried over anhydrous sodium sulfate, concentrated under reduced pressure, and subsequently purified by column chromatography to give pure intermediate A-3k.

### 15.2 Synthesis of intermediate A-4k

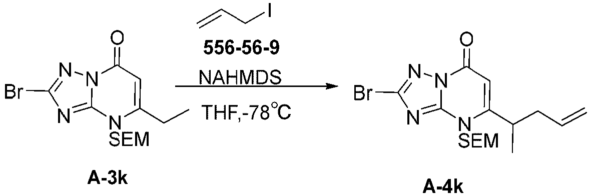

A-3k (1.0 e.q.) was weighed out and dissolved in anhydrous THF at room temperature. After the air in the reaction flask was replaced with nitrogen, the mixture was cooled to -78 °C. NaHMDS (1.5 e.q.) was added dropwise, and after the mixture was stirred at -78 °C for 2 h, allyl iodide (1.5 e.q.) was added dropwise. The reaction solution was slowly warmed to room temperature and stirred overnight. After the reaction was completed, as monitored by LC-MS, the reaction was quenched with an aqueous ammonium chloride solution, and extraction was performed three times with ethyl acetate. The organic phases were combined, washed once with a saturated aqueous ammonium chloride solution, dried over anhydrous sodium sulfate, concentrated under reduced pressure, and subsequently purified by column chromatography to give pure A-4k.

### 15.3 Synthesis of intermediate A-5k

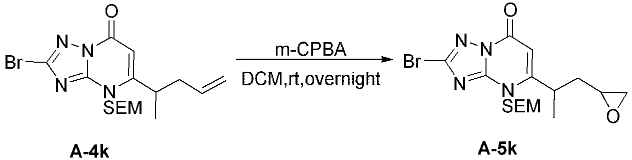

A-4k (1.0 e.q.) and m-CPBA (2.5 e.q.) were weighed out and dissolved in DCM (1 mmol:10 mL) at room temperature, and the mixture was reacted overnight at room temperature. After the reaction was completed, as monitored by LC-MS, the mixture was diluted with water and extracted twice with dichloromethane. The organic phases were combined, washed once with saturated brine, dried over anhydrous sodium sulfate, concentrated under reduced pressure, and subsequently purified by column chromatography to give pure intermediate A-5k.

### 15.4 Synthesis of intermediate A-6k

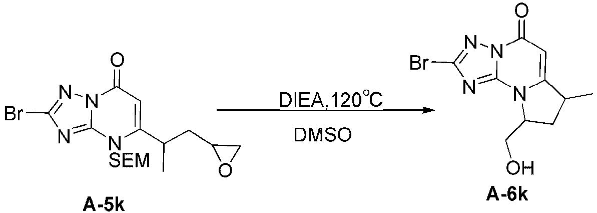

A-5k (1.0 e.q.) and DIEA (3.0 e.q.) were weighed out and dissolved in DMSO (1 mmol:10 mL) at room temperature, and the reaction solution was heated to 120 °C and stirred overnight under a nitrogen atmosphere. After the reaction was completed, as monitored by LC-MS, the reaction solution was diluted with water and extracted twice with ethyl acetate. The organic phases were combined, washed once with saturated brine, dried over anhydrous sodium sulfate, concentrated under reduced pressure, and subsequently purified by column chromatography to give intermediate A-6k.

### 15.5 Synthesis of intermediate A-7k

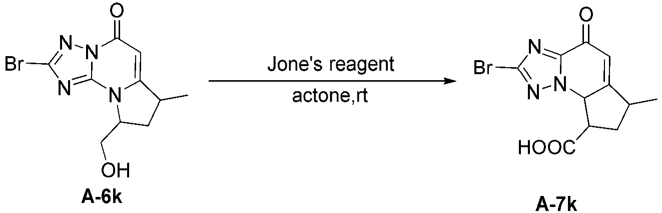

First, Jones reagent was freshly prepared: chromium trioxide (5 g, 0.25 mol) was dissolved in water (15 mL) in a 100 mL beaker, and concentrated sulfuric acid (25 mL) was slowly added dropwise with stirring in an ice bath. The temperature of the solution was maintained at 0-5 °C. The prepared reagent had a solubility of 2.5 M. A-6k (1.0 e.q.) was weighed out and dissolved in acetone (1 mmol, 10 mL). The reaction solution was cooled to 0 °C, and the freshly prepared kone reagent (2.5 e.q., 2.5 M) was added dropwise. The mixture was stirred at room temperature for 2 h. After the reaction was completed, as monitored by LC-MS, the reaction solution was diluted with water and extracted twice with ethyl acetate. The organic phases were combined, washed once with saturated brine, dried over anhydrous sodium sulfate, concentrated under reduced pressure, and subsequently purified by column chromatography to give intermediate A-7k.

### 15.5 Synthesis of intermediate A-8k

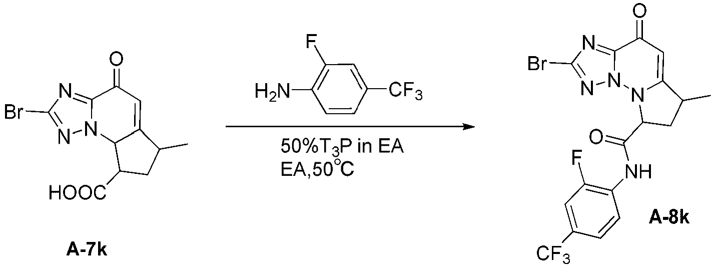

Intermediate A-7k (1.0 e.q.), 2-fluoro-4-(trifluoromethyl)aniline (1.2 e.q.), 50% T₃P in EA (4.5 e.q.), and triethylamine (15 e.q.) were weighed out and dissolved in ethyl acetate (1 mmol, 10 mL), and subsequently, the reaction solution was heated to 50 °C and stirred for 1 h. After the reaction was completed, as monitored by LC-MS, the reaction solution was diluted with water and extracted twice with ethyl acetate. The organic phases were combined, washed once with saturated brine, dried over anhydrous sodium sulfate, concentrated under reduced pressure, and subsequently purified by column chromatography to give intermediate A-8k.

### 15.6 Synthesis of intermediate A-9k

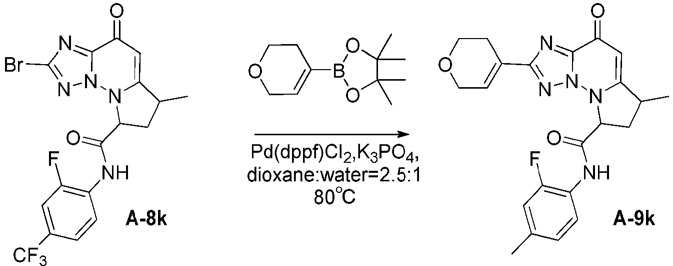

Intermediate A-8k (1.0 e.q.), 3,6-dihydro-2H-pyran-4-boronic acid pinacol ester (1.2 e.q.), Pd(dppf)Cl2 (0.05 e.q.), and potassium phosphate (2.5 e.q.) were added to 1,4-dioxane and water (2.5:1, 1 mmol:5 mL). The mixture was purged three times with nitrogen and subsequently heated to 80 °C and reacted for 4 h. After the reaction was completed, as monitored by LC-MS, the reaction solution was diluted with ethyl acetate and washed with brine. The organic phase was collected, dried over anhydrous sodium sulfate, concentrated to dryness by rotary evaporation, and subsequently subjected to column chromatography to give intermediate A-9k.

### 15.7 Synthesis of intermediate A-10k

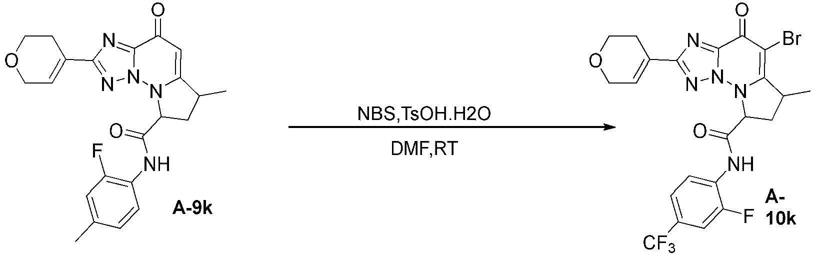

Intermediate A-9k (1.0 e.q.) was dissolved in N,N-dimethylformamide (1 mmol:10 mL) at room temperature, followed by the addition of N-bromosuccinimide (1.5 e.q.) in batches, and the mixture was reacted for 4 h. After the reaction was completed, as monitored by LC-MS, the reaction solution was diluted with ethyl acetate and washed once with saturated sodium bicarbonate, twice with water, and once with saturated brine. The resulting organic phase was dried over anhydrous sodium sulfate, concentrated under reduced pressure, and subsequently subjected to column chromatography to give intermediate A-10k.

### 15.8 Synthesis of intermediate A-11k

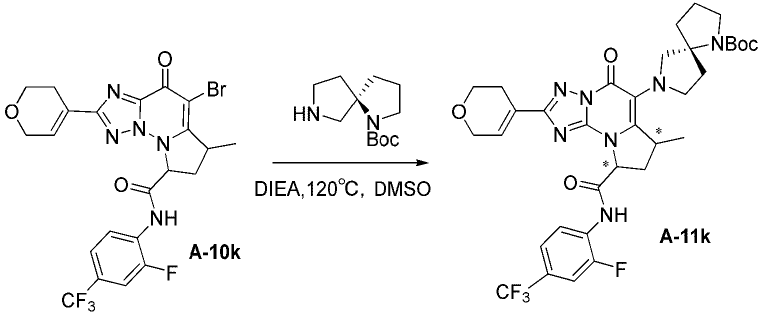

Intermediate A-9 (1.0 e.q.), (R)-1-BOC-1,7-diazaspiro[4.4]nonane (0.8 e.q.), and DIEA (3.0 e.q.) were weighed out and dissolved in DMSO (1 mmol:2 mL). Subsequently, the mixture was heated to 120 °C and stirred overnight. After the reaction was completed, as monitored by LC-MS, the reaction solution was diluted with ethyl acetate and washed once with saturated sodium bicarbonate, twice with water, and once with saturated brine. The resulting organic phase was dried over anhydrous sodium sulfate, concentrated under reduced pressure, and subsequently subjected to column chromatography to give intermediate A-11k.

### 15.9 Synthesis of intermediate A-12k

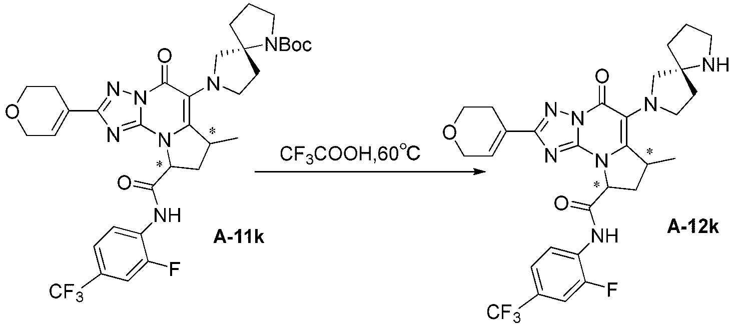

Intermediate A-11k (1.0 e.q.) was dissolved in CF3COOH:DCM = (1:1; 1 mmol:10 mL) at room temperature. The reaction solution was heated to 40 °C and reacted for 12 h. After the reaction was completed, as monitored by LC-MS, the solution was concentrated to dryness by rotary evaporation. The concentrated solution was diluted with ethyl acetate and washed once with a saturated sodium bicarbonate solution and once with saturated brine, and the organic phase was concentrated to dryness by rotary evaporation to give the product A-12k.

### 15.10 Synthesis of intermediate P-13

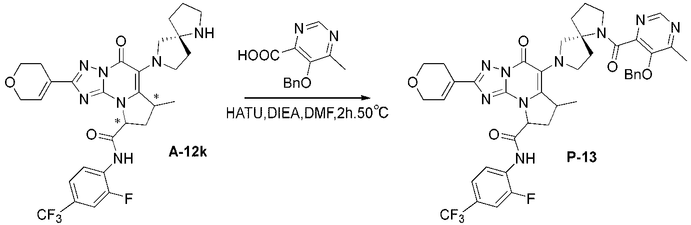

Intermediate A-12k (1.0 e.q.), 5-(benzyloxy)-6-methylpyrimidine-4-carboxylic acid (2.0 e.q.), and N,N-diisopropylethylamine (5 e.q.) were dissolved in N,N-dimethylformamide (1 mmol:5 mL) at room temperature, followed by the addition of (benzotriazol-1-yloxy)tripyrrolidinophosphonium hexafluorophosphate (2.0 e.q.) at 0 °C, and subsequently, the mixture was heated to 40 °C and reacted for 2 h. After the reaction was completed, as monitored by LC-MS, the reaction solution was diluted with ethyl acetate and washed twice with water and once with saturated brine. The resulting organic phase was dried over anhydrous sodium sulfate, concentrated under reduced pressure, and subsequently purified by column chromatography to give pure P-13.

### 15.11 Target products T107, T108, T109, and T110.

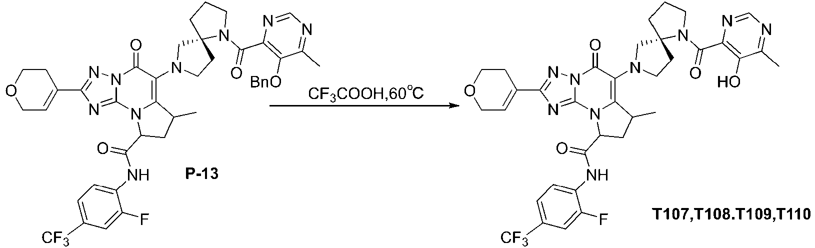

Intermediate A-11k (1.0 e.q.) was dissolved in a trifluoroacetic acid solution at room temperature, and the reaction solution was heated to 60 °C and reacted for 12 h. After the reaction was completed, as monitored by LC-MS, the reaction solution was diluted with ethyl acetate and washed twice with water and once with saturated brine. The resulting organic phase was dried over anhydrous sodium sulfate and concentrated under reduced pressure. The residue was subjected to column chromatography to give a pure product, which was subsequently subjected to SFC chiral resolution to sequentially give the target products T107, T108, T109, and T110 according to the peak elution order, the structures and general analytical data of which are shown in Table 1.

The chiral resolution conditions used were as follows:

### First Prep-HPLC conditions:

Chromatographic column: CHIRALPAK IBN, 2 cm × 25 cm, 5 µm;
Mobile phase: A: MTBE (0.1% FA); B: MeOH:DCM;
Flow rate: 20 mL/min;
Wavelength: UV 220 nm;
Column temperature: 25 °C;
Chromatograph: Prep-HPLC-Gilson.

### Second Prep-HPLC conditions:

Chromatographic column: CHIRALPAK IC, 2 cm × 25 cm, 5 µm;
Mobile phase: A: MTBE (0.1% FA); B: MEOH;
Flow rate: 20 mL/min;
Wavelength: UV 220 nm;
Column temperature: 25 °C;
Chromatograph: Prep-HPLC-Gilson.

### Example 16

The synthetic route was as follows:

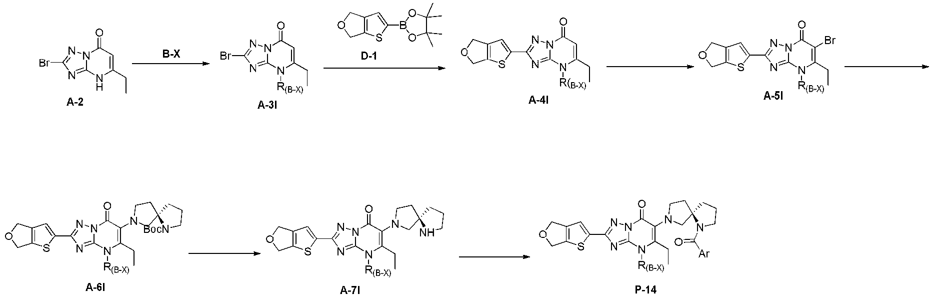

### 16.1 Synthesis of intermediate D-1

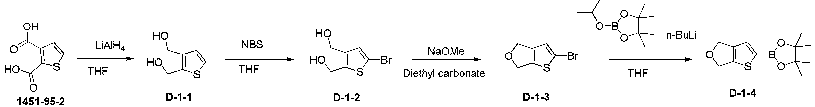

### 16.1.1 Synthesis of intermediate D-1-1

The intermediate thiophene-2,3-dicarboxylic acid (1.0 e.q.) was dissolved in tetrahydrofuran (1 mmol:2 mL) at 0 °C, and a 1 mol/L solution of lithium aluminum hydride in tetrahydrofuran (4.0 e.q.) was slowly added dropwise. Subsequently, the reaction solution was heated to 70 °C and reacted for 16 h. After the reaction was completed, as monitored by LCMS, the reaction solution was cooled to 0 °C, quenched by dropwise adding water and a 10% sodium hydroxide solution, dried over anhydrous sodium sulfate overnight, filtered, and concentrated to give pure intermediate D-1-1.

### 16.1.2 Synthesis of intermediate D-1-2

Intermediate D-1-1 (1.0 e.q.) was added to tetrahydrofuran (1 mmol:2 mL), and N-bromosuccinimide (1.05 e.q.) was added in batches at 0 °C. Subsequently, the mixture was reacted at room temperature for 4 h. After the reaction was completed, as monitored by TLC, the reaction solution was diluted with ethyl acetate, and the organic phase was washed with a saturated sodium bicarbonate solution and saturated brine and concentrated under reduced pressure at a low temperature to give intermediate D-1-2.

### 16.1.3 Synthesis of intermediate D-1-3

Intermediate D-1-2 (1.0 e.q.) was added to dimethyl carbonate (1 mmol:1 mL), and sodium methoxide (1.5 e.q.) was added in batches. Subsequently, the mixture was reacted at 120 °C for 4 h. After the reaction was completed, as monitored by LCMS, the reaction solution was cooled to room temperature, and subsequently diluted with ethyl acetate and water and filtered for liquid separation. The resulting organic phase was dried over anhydrous sodium sulfate, concentrated under reduced pressure, and subsequently subjected to column chromatography to give intermediate D-1-3.

### 16.1.4 Synthesis of intermediate D-1

Intermediate D-1-3 (1.0 e.q.) was added to tetrahydrofuran (1 mmol:2 mL), and a solution of n-butyllithium in n-hexane (1.5 e.q.) was added dropwise at -78 °C. After the mixture was reacted at -78 °C for 0.5 h, isopropyl pinacol borate (1.2 e.q.) was added dropwise, and the mixture was reacted at -78 °C for 2 h. After the reaction was completed, as monitored by LCMS, the reaction solution was cooled, quenched with saturated ammonium chloride, and extracted twice with ethyl acetate. The resulting organic phase was washed with saturated brine, dried over anhydrous sodium sulfate, concentrated under reduced pressure, and subsequently subjected to column chromatography to give intermediate D-1.

16.2 The synthesis of intermediate A-31 was based on the synthesis procedures of A-3b.

16.3 The synthesis of intermediate A-41 was based on the synthesis procedures of A-4b.

16.4 The synthesis of intermediate A-51 was based on the synthesis procedures of A-5b.

16.5 The synthesis of intermediate A-61 was based on the synthesis procedures of A-6b.

16.6 The synthesis of intermediate A-71 was based on the synthesis procedures of A-7b.

### 16.6 Synthesis of target products

16.6.1 When B-X = B-5, the synthesis procedures of the target product T117 were based on the synthesis of T019, and its structure and general analytical data are shown in Table 1.

16.6.2 When B-X = B-7, the synthesis of the target product T-118 was based on the synthesis of T019, and its structure and general analytical data are shown in Table 1.

16.6.3 When B-X = B-1, the synthesis of the target product T-119 was based on the synthesis of T019, and its structure and general analytical data are shown in Table 1.

The target product T120 was obtained by replacing intermediate D-1 in Synthesis Example 16 with 4-dimethylaminopiperidine (Cas No.: 50533-97-6), followed by the same procedures as Synthesis Examples 16.2 to 16.6, and according to the synthesis of T019, wherein B-X = B-7; its structure and general analytical data are shown in Table 1.

The target product T122 was obtained by replacing intermediate D-1 in Synthesis Example 16 with tetrahydropyrrole (Cas No.: 123-75-1), followed by the same procedures as Synthesis Examples 16.2 to 16.6, and according to the synthesis of T019, wherein B-X = B-7; its structure and general analytical data are shown in Table 1.

The corresponding intermediate A-6m was obtained by replacing intermediate D-1 in Synthesis Example 16 with N-BOC-piperazine (Cas No.: 57260-71-6), followed by the same procedures as Synthesis Examples 16.2 to 16.5, wherein B-X = B-7.

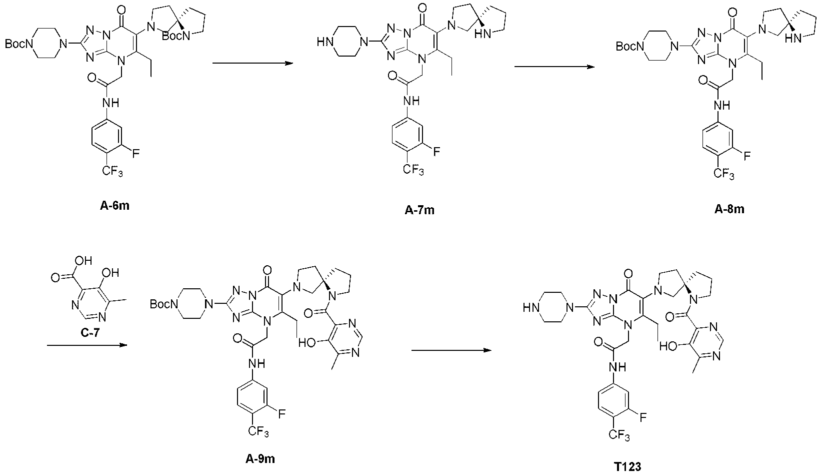

Intermediate A-6m (1.0 e.q.) was dissolved in dichloromethane (1 mmol:5 mL) at room temperature, followed by the addition of trifluoroacetic acid (1 mmol:1 mL) at 0 °C, and subsequently, the mixture was warmed to room temperature and reacted for 2 h. After the reaction was completed, as monitored by LC-MS, the reaction solution was concentrated to near dryness under reduced pressure and diluted with dichloromethane, and the aqueous phase was adjusted to pH = 8-9 with saturated sodium bicarbonate. Extraction was performed twice with dichloromethane. The organic phase was washed with saturated brine, and the resulting organic phase was dried over anhydrous sodium sulfate and concentrated under reduced pressure to give intermediate A-7m.

Intermediate A-7m (1.0 e.q.) was dissolved in tetrahydrofuran (1 mmol:5 mL) at 0 °C, and di-tert-butyl dicarbonate (1.0 e.q.) and sodium bicarbonate (1.0 e.q.) were added. The reaction solution was slowly warmed to room temperature, stirred for 1.5 h, and concentrated. The resulting organic phase was dried over anhydrous sodium sulfate, concentrated under reduced pressure, and subsequently separated by column chromatography to give compound A-8m.

Intermediate A-8m (1.0 e.q.), C-7 (2.0 e.q.), and N,N-diisopropylethylamine (5 e.q.) were dissolved in N,N-dimethylformamide (1 mmol:5 mL) at room temperature, followed by the addition of (benzotriazol-1-yloxy)tripyrrolidinophosphonium hexafluorophosphate (2.0 e.q.) at 0 °C, and subsequently, the mixture was heated to 40 °C and reacted for 2 h. After the reaction was completed, as monitored by LC-MS, the reaction solution was diluted with ethyl acetate and washed twice with water and once with saturated brine. The resulting organic phase was dried over anhydrous sodium sulfate, concentrated under reduced pressure, and subsequently separated by column chromatography to give compound A-9m.

Intermediate A-9m (1.0 e.q.) was dissolved in dichloromethane (1 mmol:5 mL) at room temperature, followed by the addition of trifluoroacetic acid (1 mmol:1 mL) at 0 °C, and subsequently, the mixture was warmed to room temperature and reacted for 2 h. After the reaction was completed, as monitored by LC-MS, the reaction solution was concentrated to near dryness under reduced pressure and diluted with dichloromethane, and the aqueous phase was adjusted to pH = 8-9 with saturated sodium bicarbonate. Extraction was performed twice with dichloromethane. The organic phase was washed with saturated brine, and the resulting organic phase was dried over anhydrous sodium sulfate, concentrated under reduced pressure, and subjected to preparative high-pressure reverse-phase chromatography to give the target product T123, the structure and general analytical data of which are shown in Table 1.

**Table 1. Example compounds and general analytical data**

| Compound No. | Chemical structure | Molecular weight | NMR |
|---|---|---|---|
| T001 | 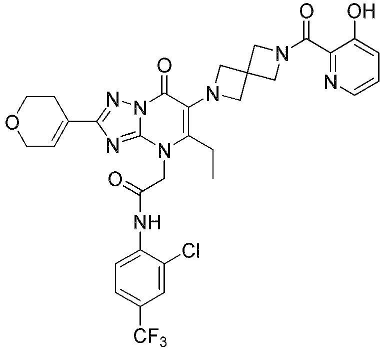 | 699.09 | ¹H NMR (400 MHz, DMSO-d6) δ 12.99 - 12.53 (m, 1H), 10.38 (s,1H), 8.19 (dd, *J* = 4.4, 1.5 Hz, 1H), 8.06 (d, *J =* 8.6 Hz, 1H), 7.96 (d,*J* = 2.1 Hz, 1H), 7.71 (dd*, J =* 8.7, 2.1 Hz, 1H), 7.47 (dd, *J* = 8.5, 4.3Hz, 1H), 7.38 (*dd, J =* 8.5, 1.5 Hz, 1H), 6.89 - 6.77 (m, 1H), 5.29 (s,2H), 4.95 (s, 2H), 4.36 (s, 2H), 4.26 (q, *J =* 2.8 Hz, 2H), 4.20 - 4.08(m, 4H), 3.82 (t, *J =* 5.4 Hz, 2H), 2.92 (q, *J =* 7.4 Hz, 2H), 2.52 (d, *J =* 3.4 Hz, 2H), 1.15 (t, *J =* 7.4 Hz, 3H). ¹⁹F NMR (376 MHz, DMSO-d6) δ -60.80. |
| T002 | 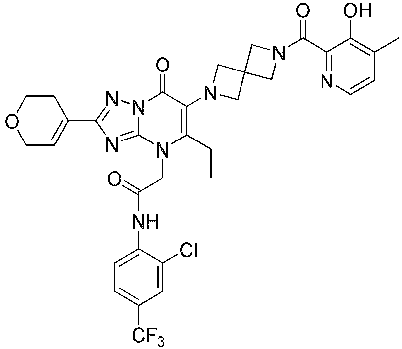 | 713.12 | ¹H NMR (400 MHz, DMSO-d6) δ 13.09 (s, 1H), 10.36 (s, 1H), 8.12 - 8.01 (m,2H), 7.96 (d, *J =* 2.2 Hz, 1H), 7.78 - 7.64 (m, 1H), 7.36 (d, *J =* 4.5 Hz, 1H), 6.84(s, 1H), 5.30 (s, 2H), 4.96 (s, 2H), 4.35 (s, 2H), 4.26 (d, *J =* 3.5 Hz, 2H), 4.15 (s,4H), 3.82 (t, *J =* 5.5 Hz, 2H), 2.92 (d, *J =* 7.6 Hz, 2H), 2.54 (s, 2H), 2.21 (s, 3H),.15 (t, *J = 7.5* Hz, 3H). ¹⁹F NMR (376 MHz, DMSO-d6) δ -60.82. |
| T003 | 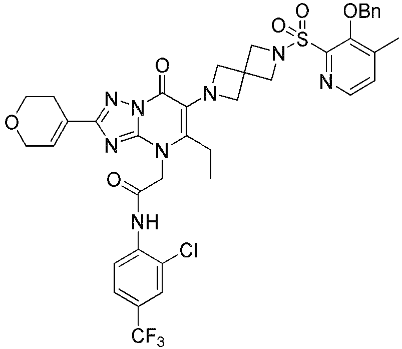 | 839.29 | ¹H NMR (400 MHz, DMSO-d6) δ 10.36 (s, 1H), 8.29 (d, *J =* 4.7 Hz, 1H), 8.05(d, *J =* 8.6 Hz, 1H), 7.96 (d*, J =* 2.1 Hz, 1H), 7.72 (dd, *J =* 8.8, 2.1 Hz, 1H),7.62 (d, *J =* 4.8 Hz, 1H), 7.59 - 7.53 (m, 2H), 7.49 - 7.35 (m, 3H), 6.88 - 6.79(m, 1H), 5.29 (s, 2H), 5.03 (s, 2H), 4.42 (s, 4H), 4.25 (q, *J =* 2.9 Hz, 2H), 4.15(s, 4H), 3.81 (t, *J = 5.4* Hz, 2H), 2.89 (q, *J =* 7.4 Hz, 2H), 2.52 (s, 2H), 2.40 (s,3H), 1.14 (t, *J* = 7.4 Hz, 3H). ¹⁹F NMR (376 MHz, DMSO-d6) δ -60.81. |
| T004 | 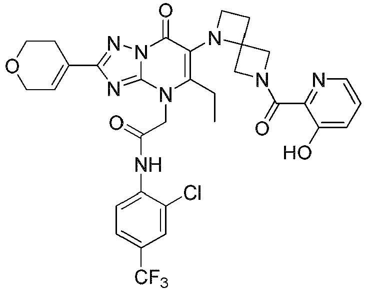 | 699.08 | ¹H NMR (400 MHz, DMSO) δ 10.36 (s, 1H), 8.18(ddd, *J =* 16.8, 4.3, 1.4 Hz, 1H), 8.06 *(t, J =* 8.5 Hz, 1H), 7.98 (d, *J =* 2.1 Hz, 1H), 7.73 (dd, *J =* 8.8, 2.3 Hz, 1H), 7.50 - 7.37 (m, 2H), 6.86 - 6.79 (m, 1H), 5.30 (s,2H), 4.73 (d, *J =* 7.6 Hz, 2H), 4.59 (t, *J =* 7.6 Hz, 2H),4.27 (dt, *J =* 5.7, 3.3 Hz, 3H), 4.16 (d, *J =* 8.0 Hz, 2H),3.94 - 3.76 (m, 3H), 3.03 - 2.95 (m, 2H), 2.76 - 2.64(m, 2H), 1.19 (t, *J =* 7.4 Hz, 3H). ¹⁹F NMR (376 MHz, DMSO) δ -60.78 |
| T005 | 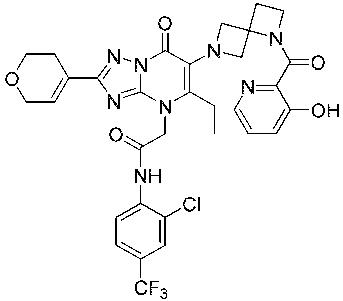 | 699.08 | ¹H NMR (400 MHz, DMSO) δ 12.97 (s, 1H), 10.38 (s,1H), 8.22 - 8.14 (m, 1H), 8.06 (t, *J =* 8.5 Hz, 1H), 7.98(d, *J* = 2.1 Hz, 1H), 7.75 - 7.68 (m, 1H), 7.47 (dd, *J =*8.5*,* 4.3 Hz, 1H), 7.40 (dd, *J =* 8.5, 1.5 Hz, 1H), 6.83 (d*,J =* 9.2 Hz, 1H), 5.31 (s, 2H), 4.76 - 4.53 (m, 3H), 4.33- 4.09 (m, 4H), 3.93 - 3.76 (m, 2H), 3.00 (d, *J =* 7.6Hz, 1H), 2.75 - 2.63 (m, 2H), 2.58 (d, *J*= 7.1 Hz, 2H),1.19 (t, *J =* 7.4 Hz, 3H). ¹⁹F NMR (376 MHz, DMSO) δ -60.79. |
| T006 | 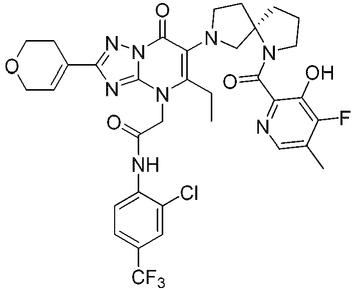 | 759.16 | ¹H NMR (400 MHz, DMSO) δ 8.07 (d, J = 8.6 Hz, 1H), 7.97 (q, J = 3.4 Hz, 2H),7.72 (dd, J = 8.7, 2.1 Hz, 1H), 6.83 (s, IH), 5.32 (s, 2H), 4.26 (d, J *=* 3.7 Hz, 2H),3.81 (t, J = 5.4 Hz, 2H), 3.56 (t, J = 6.7 Hz, 2H), 3.13 - 2.92 (m, 3H),2.5 (m,2H) 2.25 (s, 4H), 2.11 - 1.93 (m, 2H), 1.89 - 1.73 (m, 3H), 1.23 (d, J = 6.8 Hz,6H). ¹⁹F NMR (376 MHz, DMSO) δ -60.79, δ -131.14 |
| T007 | 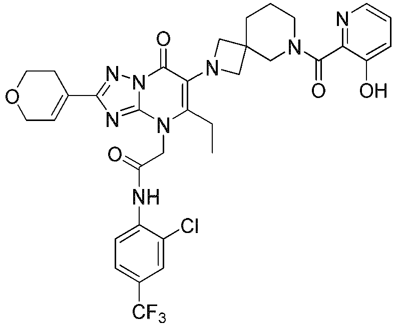 | 727.14 | ¹H NMR (400 MHz, DMSO) δ 10.34 (s, 1H), 8.09- 8.00 (m, 2H), 7.97 (d, *J =* 2.1 Hz, 1H), 7.72 (dd, *J* = 8.8, 2.2 Hz, 1H), 7.28 (dd, *J =* 16.5, 3.0 Hz, 2H),6.83 (d, *J =* 9.5 Hz, 1H), 5.38 - 5.15 (m, 2H), 4.26(dd, *J =* 5.3, 2.9 Hz, 2H), 3.94 (s, 1H), 3.81 (dq, *J* =5.7, 3.8 Hz, 4H), 3.67 - 3.55 (m, 2H), 3.49 (s, 1M),3.13 (d, *J =* 5.9 Hz, 1H), 2.98 (d, *J =* 6.5 Hz, 1H),2.63 (d, *J =* 7.7 Hz, 1H), 2.55 (s, 3H), 1.89 (dd, *J* =16.9, 10.8 Hz, 2H), 1.57 - 1.39 (m, 2H), 1.19 (t, *J =*7.4 Hz, 1H), 0.94 (t, *J =* 7.4 Hz, 2H). ¹⁹F NMR (376 MHz, DMSO) δ -60.79. |
| T008 | 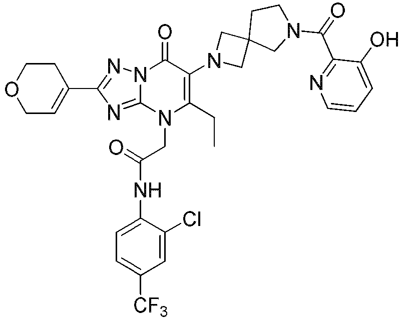 | 713.12 | ¹H NMR (400 MHz, DMSO-d6) δ 12.06 (s, 1H), 10.40 (s, 1H), 8.16(ddd, *J =* 30.6, 4.3, 1.5 Hz, 1H), 8.06 (dd, *J =* 8.6, 5.0 Hz, 1H), 7.97(d, *J* = 2.2 Hz, 1H), 7.72 (dt, *J =* 8.8, 2.4 Hz, 1H), 7.47 - 7.32 (m,2H), 6.83 (dh, *J =* 4.5*,* 1.4 Hz, 1H), 5.30 (d, *J =* 7.3 Hz, 2H), 4.26 (q,*J* = 2.9 Hz, 2H), 4.05 (s, 1H), 4.03 - 3.90 (m, 3H), 3.89 - 3.76 (m,5H), 3.62 (t, *J =* 7.0 Hz, 1H), 2.95 (dq, *J =* 15.0, 7.2 Hz, 2H), 2.56 -2.51 (m, 2H), 2.23 (dt, *J =* 10.8, 6.9 Hz, 2H), 1.17 (dt, *J* = 13.4, 7.4Hz, 3H). ¹⁹F NMR (376 MHz, DMSO-d6) δ -60.78. |
| T009 | 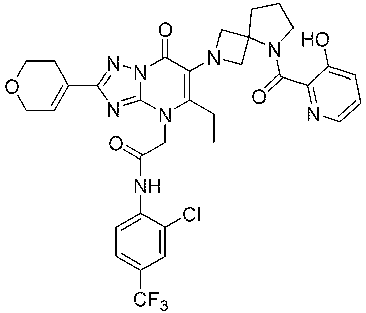 | 713.11 | ¹H NMR (400 MHz, DMSO-d6) δ 10.43 (s, 1H), 8.10 - 8.00 (m, 2H), 7.97(d, *J* = 2.1 Hz, 1H), 7.72 (d, *J* = 8.6 Hz, 1H), 7.37 - 7.28 (m, 2H), 6.83 (s,1H), 5.29 (d, *J =* 10.5 Hz, 2H), 4.80 (d, *J =* 6.1 Hz, 2H), 4.32 - 4.20 (m,2H), 4.01 (d, *J =* 6.2 Hz, 2H), 3.82 (t, *J =* 6.0 Hz, 2H), 3.41 (d, *J =* 6.9 Hz,2H), 2.99 *(d, J =* 7.7 Hz, 2H), 2.53 (s, 2H), 2.43 (t, *J =* 6.8 Hz, 2H), 1.75 (*q, J* = 6.4 Hz, 2H), 1.22 (q, *J =* 7.5*,* 6.0 Hz, 3H). ¹⁹F NMR (376 MHz, DMSO-d6) δ -60.80. |
| T010 | 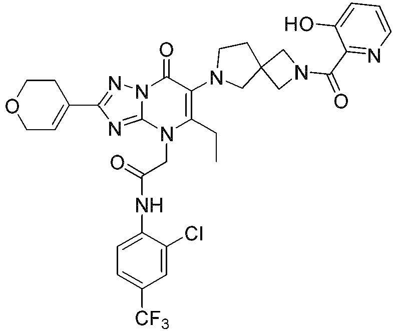 | 713.11 | ¹H NMR (400 MHz, DMSO-d6) δ 13.00 - 12.66 (m, 1H), 10.38 (s, 1H), 8.18(dd, *J* = 4.4, 1.5 Hz, 1H), 8.06 (d, *J =* 8.5 Hz, 1H), 7.97 (d, *J* = 2.1 Hz, 1H),7.72 (dd, *J =* 8.8, 2.2 Hz, 1H), 7.47 (dd, *J =* 8.5, 4.4 Hz, 1H), 7.38 (dd, *J =* 8.4*,* 1.5 Hz, 1H), 6.92 - 6.76 (m, 1H), 5.31 (s, 2H), 4.75 (q, *J* = 11.1 Hz, 2H),4.25 (q, *J=* 2.9 Hz, 2H), 4.17 (q, *J =* 10.7 Hz, 2H), 3.81 (t*, J =* 5.4 Hz, 2H),3.28 (s, 2H), 3.17 (s, 2H), 2.93 (d, *J =* 7.9 Hz, 2H), 2.51 (s, 2H), 2.24 (q, *J =*6.6 Hz, 2H), 1.30 - 1.11 (m, 4H). ¹⁹F NMR (376 MHz, DMSO-d6) δ -60.80. |
| T011 | 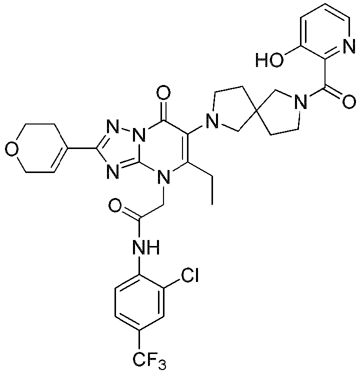 | 727.14 | ¹H NMR (400 MHz, DMSO-d6) δ 11.95 (brs, 1H), 10.38 (s, 1H), 8.13(ddd, *J =* 10.3, 4.3, 1.6 Hz, 1H), 8.07 (dd, *J =* 8.6, 5.2 Hz, 1H), 7.97 (d, *J =* 2.2 Hz, 1H), 7.72 (dt, *J* = 8.7, 1.9 Hz, 1H), 7.42 - 7.29 (m, 2H), 6.83(dh, *J* = 4.7*,* 1.5 Hz, 1H), 5.31 (d, *J =* 10.6 Hz, 2H), 4.26 (p, *J=* 2.9 Hz,2H), 3.94 - 3.60 (m, 6H), 3.30 - 2.79 (m, 6H), 2.53 (s, 2H), 2.10 - 1.92(m, 4H), 1.24 - 1.08 (m, 3H). ¹⁹F NMR (376 MHz, DMSO-d6) δ -60.80. |
| T012 | 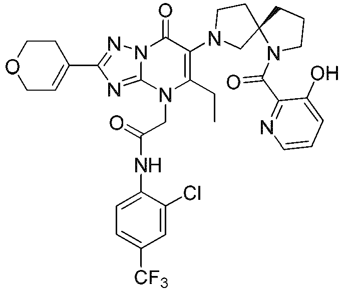 | 727.14 | ¹H NMR (400 MHz, DMSO) δ 10.93 (s, 1H),10.39 (s, 1H), 8.11 - 8.02 (m, 2H), 8.00 - 7.96 (m,1H), 7.73 (dd, *J =* 8.9, 2.1 Hz, 1H), 7.35 - 7.28 (m,2H), 6.88 - 6.80 (m, 1H), 5.32 (s, 2H), 4.26 (d, *J* =2.9 Hz, 2H), 4.06 (s, 1H), 3.82 (t, *J =* 5.4 Hz, 2H),3.47 (t, *J =* 7.1 Hz, 4H), 3.12 - 2.90 (m, 3H), 2.79(s, 1H), 2.55 (s, 2H), 2.29 (s, 1H), 2.11 - 1.99 (m,1H), 1.80 (qd*, J =* 13.0, 6.6 Hz, 3H), 1.24 - 1.19(m, 3H). ¹⁹F NMR (376 MHz, DMSO) δ -60.79. |
| T013 | 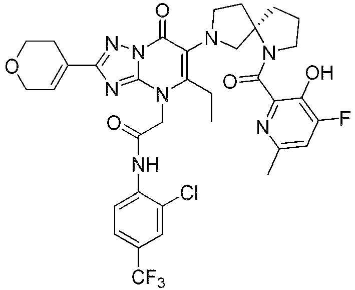 | 759.16 | ¹H NMR (400 MHz, DMSO) δ 8.07 (d, J *=* 8.6 Hz, 1H), 7.97 (d, J = 2.1Hz, 1H),7.72 (dd, J = 8.8, 2.2 Hz, 1H), 7.24 (d, J = 11.6 Hz, 1H), 6.86 - 6.80(m, 1H),5.32 (s, 2H), 4.26 (q, J = 2.9 Hz, 2H), 3.81 (t, J = 5.4 Hz, 2H), 3.49 - 3.41 (m,3H), 3.10 - 2.91 (m, 3H),2.51(m, 2H), 2.38 (s, 3H), 2.33 - 2.25 (m, 1H), 2.13 -1.94 (m, 2H), 1.88 - 1.71 (m, 3H), 1.22 (t, J = 7.3 Hz,5H). |
| T014 | 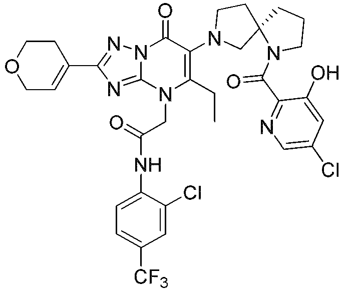 | 761.58 | ¹H NMR (400 MHz, DMSO) δ 8.49 (s, 1H), 8.06 (d, *J =* 8.6 Hz, 1H), 7.95 (s,1H), 7.76 (s, 1H), 7.70 (d*, J =* 8.6 Hz, 1H), 7.30 (s, 1H), 6.83 (s, 1H), 5.32 (s, 2H),4.29 - 4.23 (m, 2H), 3.81 (t, *J* = 5.4 Hz, 2H), 3.05 (t, *J =* 9.8 Hz, 2H), 2.95 (s,2H), 2.32 - 2.26 (m, 1H), 2.01 (p, *J =* 6.6 Hz, 2H), 1.80 - 1.67 (m, 3H), 1.22 (d, *J =* 9.2 Hz, 9H). |
| T015 | 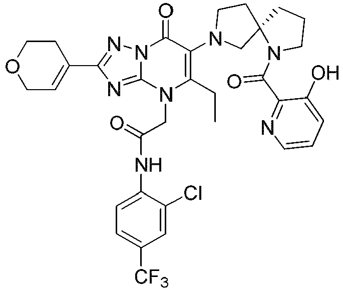 | 727.14 | ¹H NMR (400 MHz, DMSO) δ10.93 (s, 1H), 10.40 (s, 1H), 8.13 -8.02 (m, 2H), 7.98 (d, *J =* 2.1 Hz,1H), 7.73 (dd, *J =* 8.8, 2.2 Hz, 1H),7.35 - 7.28 (m, 2H), 6.87 - 6.80 (m,1H), 5.32 (s, 2H), 4.31 - 4.23 (m,2H), 3.81 (t, *J =* 5.4 Hz, 2H), 3.50 -3.42 (m, 2H), 3.18 - 2.90 (m, 4H),2.55 (s, 3H), 2.29 (s, 1H), 2.14 -1.69 (m, 5H), 1.25 - 1.20 (m, 4H). ¹⁹F NMR (376 MHz, DMSO) δ -60.79 |
| T016 | 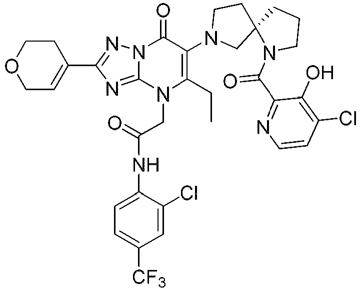 | 761.58 | ¹H NMR (400 MHz, DMSO) δ 10.40 (s, 1H), 8.07 (d, *J =* 8.4 Hz, 2H), 7.98 (d, *J =* 2.1 Hz, 1H), 7.73 (dd, *J =* 8.7, 2.2 Hz, 1H), 7.61 (d, *J* = 5.0 Hz, 1H), 6.84 (s,1H), 5.32 (s, 2H), 4.26 (q, *J* = 2.8 Hz, 2H), 3.81 (t, *J =* 5.4 Hz, 2H), 3.67 (t, *J* =6.7 Hz, 2H), 3.07 - 2.95 (m, 3H), 2.52 (s, 2H), 2.29 (s, 1H), 2.13 - 1.95 (m, 2H), 1.90 - 1.73 (m, 3H), 1.23 (dd, *J =* 9.5, 5.4 Hz, 6H). |
| T017 | 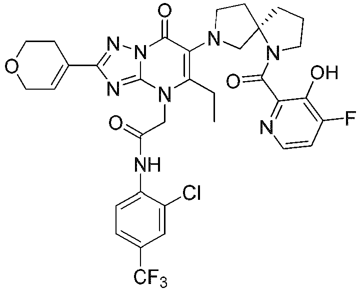 | 745.13 | ¹H NMR (400 MHz, DMSO) δ 10.40 (s, 1H), 8.06 (t, *J =* 7.7 Hz, 2H), 7.98 (d, *J* = 2.1 Hz, 1H), 7.72 (dd, *J* = 8.8, 2.2 Hz, 1H), 7.37 (dd, *J =* 10.9, 5.3 Hz, 1H), 6.86 -6.80 (m, 1H), 5.32 (s, 2H), 4.25 (t, *J =* 3.0 Hz, 2H), 3.81 (t, *J =* 5.5 Hz, 2H), 3.44(t, *J =* 6.7 Hz, 3H), 3.09 - 2.94 (m, 3H), 2.80 (s, 2H), 2.37 - 2.22 (m, 2H), 2.05 (tt,*J* = 13.1, 6.1 Hz, 2H), 1.82 (dq*, J =* 13.8, 6.8 Hz, 2H), 1.76 (s, 1H), 1.22 (t, *J* = 7.4 Hz, 4H). ¹⁹F NMR (376 MHz, DMSO) δ -60.79,-125.75. |
| T018 | 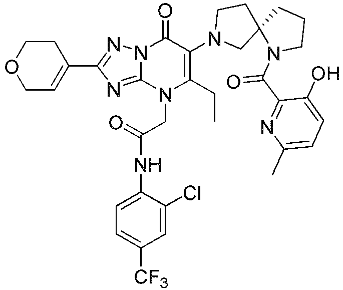 | 741.17 | ¹H NMR (400 MHz, DMSO) δ 10.73 (s, 1H), 10.40 (s, 1H),8.08 (d, J = 8.5 Hz, 1H), 7.98 (d, J = 2.1 Hz, 1H), 7.73 (dd, J =8.8, 2.2 Hz, 1H), 7.25 (d, J = 8.5 Hz, 1H), 7.18 (d, J = 8.5 Hz, 1H), 6.84 (t, J = 2.6 Hz, 1H), 5.32 (s, 2H), 4.26 (q, J = 2.8 Hz,2H), 3.81 (t, J = 5.4 Hz, 2H), 3.49 (td, J = 6.6, 2.7 Hz, 7H), 3.11 |
| | | | - 3.02 (m, 2H), 2.97 (s, 1H), 2.56 - 2.52 (m, 2H), 2.38 (s, 3H),2.05 (dt, J = 13.0, 6.9 Hz, 1H), 1.90 - 1.64 (m, 3H), 1.22 (t, J =7.4 Hz, 3H). |
| T019 | 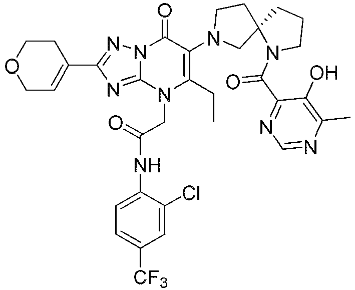 | 742.16 | ¹H NMR (400 MHz, DMSO) δ 10.85 (s, 1H), 10.33 (s, 1H), 8.51 (s, 1H), 8.00 (d, *J =*8.6 Hz, 1H), 7.91 (d, *J =* 2.1 Hz, H), 7.66 (dd, *J =* 8.9, 2.2 Hz, 1H), 6.77 (dp, *J =* 3.0,1.5 Hz, 1H), 5.25 (s, 2H), 4.19 (q, *J =* 2.7 Hz, 2H), 3.74 (t, *J =* 5.5 Hz, 2H), 3.49 (t, *J* =6.7 Hz, 2H), 3.30(s, 4H), 2.96 (s, 3H), 2.49 - 2.45 (m, 2H), 2.38 (s, 3H), 2.21 (s, 1H),2.00 (dt, *J =* 12.9, 6.9 Hz, 1H), 1.84 - 1.66 (m, 3H), 1.15 (t, *J* = 7.3 Hz, 3H). |
| T020 | 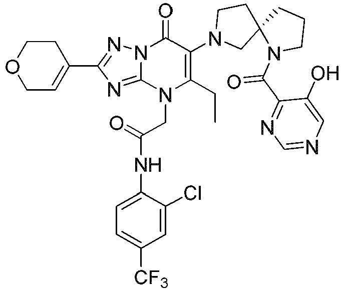 | 728.13 | ¹H NMR (400 MHz, DMSO) δ 10.91 (s, 1H), 10.39 (s, 1H), 8.69 (s, 1H), 8.48 (s,1H), 8.07 (dd, *J =* 8.7, 4.6 Hz, 1H), 7.98 (d, *J =* 2.1 Hz, 1H), 7.72 (dd, *J =* 8.8, 2.3Hz, 1H), 6.86 - 6.81 (m, 1H), 5.32 (s, 2H), 4.26 (q, *J =* 3.0 Hz, 2H), 3.81 (t, *J =* 5.4 Hz, 2H), 3.27 (dd, *J =* 6.7, 2.1 Hz, 3H), 3.09 - 2.89 (m, 4H), 2.30 (s, 1H), 2.06(dq, *J =* 16.2, 7.9 Hz, 2H), 1.88 - 1.72 (m, 4H), 1.22 (td, *J =* 7.7, 3.4 Hz, 5H). ¹⁹F NMR (376 MHz, DMSO) δ - 60.79. |
| T021 | 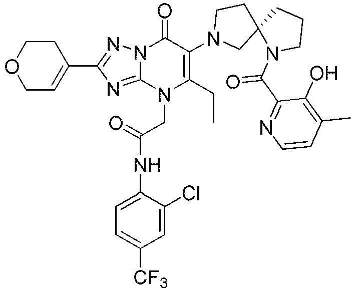 | 741.17 | ¹H NMR (400 MHz, DMSO) δ 12.03 (s, 1H), 10.40 (s, 1H), 8.07 (d, *J =* 8.5 Hz, 1H), 7.99 (dd, *J =* 9.2, 3.3 Hz, 2H), 7.73 (dd, *J =* 9.1, 2.1 Hz, 1H), 7.30 (d, *J =* 4.5Hz, 1H), 6.86 - 6.80 (m, 1H), 5.32 (s, 2H), 4.29 - 4.24 (m, 2H), 3.87 (s, 1H), 3.81(t, *J =* 5.4 Hz, 3H), 3.02 (s, 2H), 2.53 (d, *J =* 2.7 Hz, 1H), 2.52 (s, 2H), 2.23 (s,3H), 2.12 - 1.94 (m, 2H), 1.93 - 1.74 (m, 3H), 1.25 - 1.22 (m, 4H). |
| T022 | 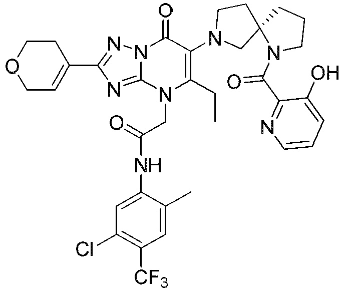 | 741.17 | ¹H NMR (400 MHz, DMSO) δ 10.94 (s,1H), 10.14 (s, 1H), 8.15 - 7.95 (m, 2H), 7.77(s, 1H), 7.32 (s, 2H), 6.83 (s, 1H), 5.28 (s,2H), 4.27 (d, *J =* 3.1 Hz, 2H),3.35(m,2H),3.82 (t, *J =* 5.5 Hz, 2H), 3.45 (d, *J =* 7.0 Hz,4H), 3.06 (s, 3H),2.51(m,2H), 2.37 (s, 3H),2.12 - 1.64 (m, 5H), 1.22 (t, *J* = 6.1 Hz, 3H). |
| | | | ¹⁹F NMR (376 MHz, DMSO) δ - 60.50. |
| T023 | 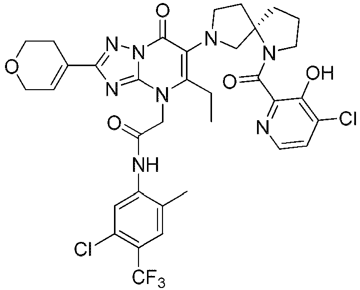 | 775.61 | ¹H NMR (400 MHz, DMSO) δ 12.04 (s, 1H), 10.12 (s, 1H), 8.06 (d, *J =* 5.0 Hz, 1H), 7.97 (s, 1H), 7.76 (s, 1H), 7.60 (d, *J =* 5.0 Hz, 1H), 6.82 (s, 1H), 5.27 (s, 2H), 4.26 (q, *J =* 2.8 Hz, 2H), 4.06 (s, 1H), 3.81 (t, *J =* 5.4 Hz, 2H), 3.67 (t, *J =* 6.7 Hz, 2H), 3,32 (m,2H), 3.03 (s, 3H),2.51(m,2H), 2.36 (s, 3H), 2.29 (s, 1H), 2.16 - 1.69 (m, 5H), 1.22 (t, *J =* 7.5 Hz, 3H). ¹⁹F NMR (376 MHz, DMSO) δ - 60.50. |
| T024 | 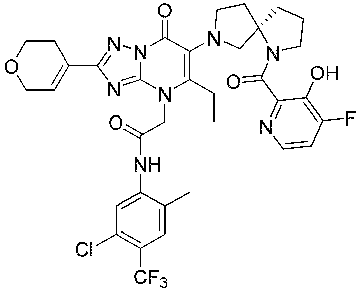 | 759.15 | ¹H NMR (400 MHz, DMSO) δ 11.24 (s, 1H), 10.13(s, 1H), 8.07 (dd, *J =* 7.0, 5.3 Hz, 1H), 7.98 (s, 1H),7.77 (s, 1H), 7.39 (dd, *J =* 10.9, 5.3 Hz, 1H), 6.86 -6.78 (m, 1H), 5.28 (s, 2H), 4.27 (d, *J =* 3.1 Hz, 2H),3.82 (t, *J =* 5.5 Hz, 2H), 3.45 (t, *J =* 6.7 Hz, 2H), 3.11-2.89 (m, 3H), 2.53 (s, 3H), 2.37 (s, 3H), 2.29 (s,1H), 2.15 - 1.65 (m, 5H), 1.21 (d, *J =* 7.5 Hz, 3H). ¹⁹F NMR (376 MHz, DMSO) δ - 60.50, -125.58. |
| T025 | 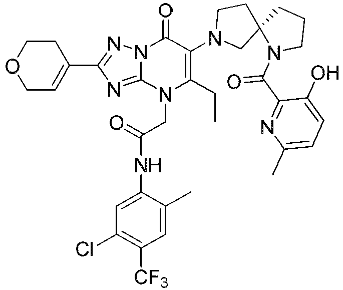 | 755.19 | ¹H NMR (400 MHz, DMSO) δ 10.68 (s, 1H),10.13 (s, 1H), 7.97 (s, 1H), 7.76 (s, 1H), 7.28 -7.09 (m, 2H), 6.86 - 6.77 (m, 1H), 5.27 (s, 2H),4.26 (q, *J =* 2.9 Hz, 2H), 3.81 (t, *J =* 5.4 Hz, 2H),3.49 (d, *J* = 7.1 Hz, 2H), 3.36 (m,3H), 3.14 - 2.93(m, 3H), 2.52 (d, *J =* 1.8 Hz, 2H), 2.37 (d, *J =* 1.7Hz, 6H), 2.29 (s, 1H), 2.10 - 1.69 (m, 5H), 1.22 (t,*J* = 7.4 Hz, 3H). ¹⁹F NMR (376 MHz, DMSO) δ -60.50. |
| T026 | 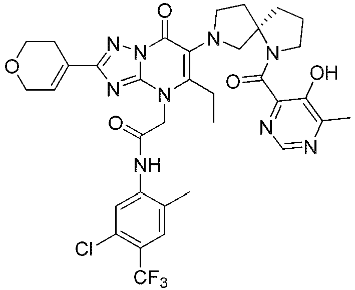 | 756.18 | ¹H NMR (400 MHz, DMSO) δ 10.91 (s,1H),10.13 (s, 1H), 8.58 (s, 1H), 7.97 (s, 1H), 7.76(s, 1H), 6.82 (dd, *J =* 3.5, 2.0 Hz, 1H), 5.27 (s,2H), 4.26 (q, *J =* 2.9 Hz, 2H), 4.05 (s, 1H),3.81 (t, *J =* 5.4 Hz, 2H), 3.56 (t, *J =* 6.7 Hz,2H),3.50 (m,3H), 3.13 - 2.92 (m, 3H), 2.52 (s,2H), 2.44 (s, 3H), 2.37 (s, 3H), 2.34 - 2.22 (m,1H), 2.13 - 1.72 (m, 4H), 1.22 (t, *J =* 7.3 Hz,3H). ¹⁹F NMR (376 MHz, DMSO) δ -60.52. |
| T027 | 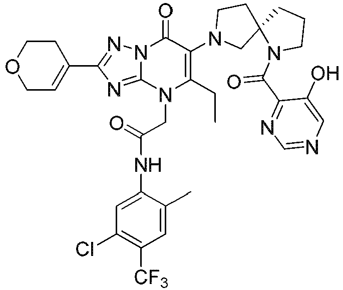 | 742.15 | ¹H NMR (400 MHz, DMSO) δ 10.91 (s, 1H), 10.13 (s, 1H), 8.68 (d, *J =* 1.1 Hz, 1H), 8.48 (s, 1H), 7.97 (s, 1H), 7.76 (s, 1H), 6.82 (d, *J* = 2.8 Hz, 1H), 5.27 (s, 2H), 4.26 (d, *J =* 3.5 Hz, 2H), 3.81 (t, *J* = 5.4 Hz, 2H), 3.31 - 3.22 (m, 3H), 3.10 - 2.89 (m, 4H), 2.52 (d, *J =* 1.9 Hz, 2H), 2.36 (s, 3H), 2.35 (s, 1H), 2.15 1.68 (m, 6H), 1.22 (t, *J =* 7.5 Hz, 3H). ¹⁹F NMR (376 MHz, DMSO) δ -60.50. |
| T028 | 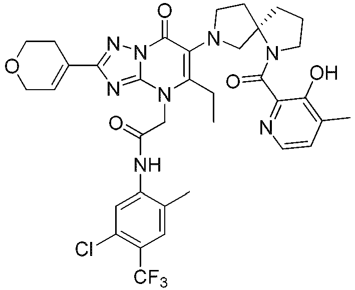 | 755.19 | ¹H NMR (400 MHz, DMSO) δ 12.04(s, 1H), 10.13 (s, 1H), 8.03 - 7.93 (m,2H), 7.76 (s, 1H), 7.30 (d, *J =* 4.5 Hz, 1H), 6.86 - 6.78 (m, 1H), 5.27 (s, 2H),4.26 (q, *J =* 2.8 Hz, 2H), 3.97 - 3.73(m, 4H), 3.02 (s, 3H), 2.52 (s, 4H),2.37 (s, 3H), 2.35 - 2.24 (m, 2H), 2.22(s, 3H), 2.14 - 1.69 (m, 5H), 1.23 (t, *J =* 7.4 Hz, 3H). ¹⁹F NMR (376 MHz, DMSO) δ -60.50. |
| T029 | 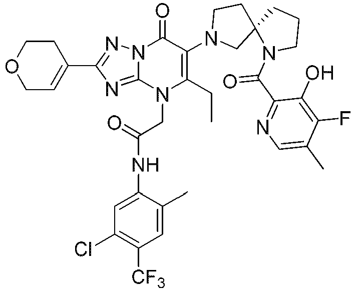 | 773.18 | ¹H NMR (400 MHz, DMSO) δ 11.47 (s, 1H),10.13 (s, 1H), 7.98 (d*, J =* 7.7 Hz, 2H), 7.76 (s,1H), 6.88 - 6.78 (m, 1H), 5.27 (s, 2H), 4.26 (q, *J =*2.8 Hz, 2H), 3.81 (t, *J* = 5.4 Hz, 2H), 3.57 (t, *J* =6.7 Hz, 2H), 3.36 (m,3H),3.04 (d, *J =* 10.8 Hz,4H), 2.53 (s, 2H), 2.36 (s, 3H), 2.25 (d, *J =* 1.5 Hz,3H), 2.14 - 1.68 (m, 5H), 1.22 (t, *J =* 7.3 Hz,3H). ¹⁹F NMR (376 MHz, DMSO) δ -60.50, -131.10. |
| T030 | 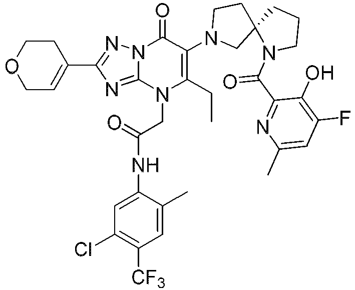 | 773.18 | ¹H NMR (400 MHz, DMSO) δ 10.76 (s,1H), 10.28 - 10.04 (m, 1H), 7.97 (s, 1H),7.76 (s, 1H), 7.24 (d, *J =* 11.6 Hz, 1H), 6.82(s, 1H), 5.27 (s, 2H), 4.26 (q, *J =* 2.9 Hz,2H), 4.04 (s, 1H), 3.81 (t, *J =* 5.4 Hz, 2H),3.46 (d, *J =* 6.8 Hz, 3H), 3.11 - 2.91 (m,3H), 2.53 (d, *J* = 6.7 Hz, 2H), 2.37 (d, *J* =6.4 Hz, 7H), 2.12 - 1.70 (m, 5H), 1.28 - 1.17(m, 4H). ¹⁹F NMR (377 MHz, DMSO) δ -60.50, -125.84. |
| T031 | 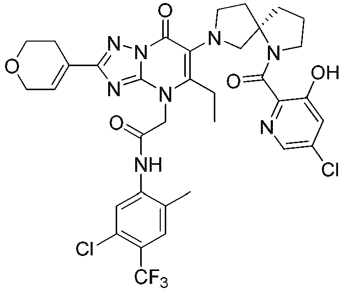 | 775.61 | ¹H NMR (400 MHz, DMSO) δ 11.38 (s,1H), 10.12 (s, 1H), 8.09 (s, 1H), 7.97 (s,1H), 7.76 (s, 1H), 7.43 (d, *J* = 2.1 Hz, 1H),6.82 (s, 1H), 5.27 (s, 2H), 4.26 (d, *J =* 3.5Hz, 2H), 3.81 (t, *J =* 5.4 Hz, 2H), 3.41 (s,4H), 3.11 - 2.91 (m, 3H), 2.53 (s, 2H),2.36 (s, 3H), 2.33 (t*, J =* 1.9 Hz, 2H), 2.12-1.69 (m, 5H), 1.27 - 1.18 (m, 3H). ¹⁹F NMR (377 MHz, DMSO) δ - 60.50. |
| T032 | 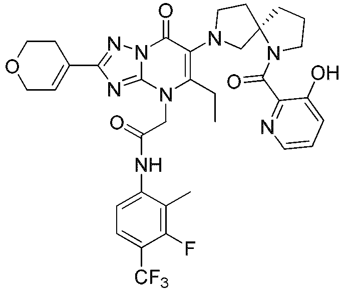 | 724.71 | ¹H NMR (400 MHz, DMSO) δ 10.94(s, 1H), 10.26 (s, 1H), 8.05 (dd, *J =*3.7*,* 2.2 Hz, 1H), 7.60 (d, *J* = 4.3 Hz,2H), 7.34 - 7.27 (m, 2H), 6.83 (s, 1H),5.26 (s, 2H), 4.27 (q, *J =* 2.9 Hz, 2H),4.14 - 3.92 (m, 1H), 3.82 (t, *J =* 5.5Hz, 2H), 3.45 (s, 4H), 3.15 - 2.91 (m,3H), 2.53 (s, 2H), 2.25 (d, *J =* 2.4 Hz,4H), 2.15 - 1.60 (m, 5H), 1.23 (t, *J* =7.4 Hz, 3H). ¹⁹F NMR (377 MHz, DMSO) δ -59.73 (d, *J =* 12.3 Hz), -117.07 (d, *J =* 12.8 Hz). |
| T033 | 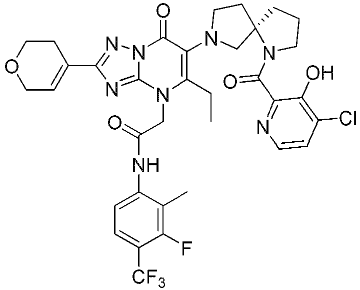 | 759.15 | ¹H NMR (400 MHz, DMSO) δ 12.05 (s, 1H),10.24 (s, 1H), 8.06 (d, *J =* 5.0 Hz, 1H), 7.69 -7.50 (m, 3H), 6.82 (s, 1H), 5.26 (s, 2H), 4.27(d, *J =* 3.0 Hz, 2H), 4.06 (s, 1H), 3.82 (t, *J =* 5.4 Hz, 2H), 3.66 (t, *J =* 6.7 Hz, 2H), 3.03 (s,3H), 2.25 (d, *J =* 2.5 Hz, 4H), 2.12 - 1.70 (m,5H), 1.22 (t, *J =* 7.4 Hz, 4H). ¹⁹F NMR (377 MHz, DMSO) δ -59.73 (d, *J =* 12.4 Hz), -117.06. |
| T034 | 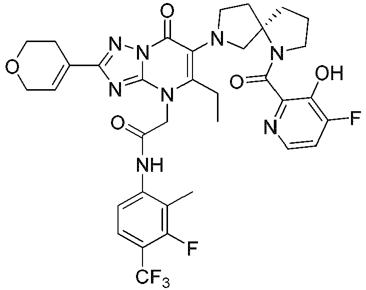 | 742.71 | ¹H NMR (400 MHz, DMSO) δ 11.27 (s, 1H),10.27 (s, 1H), 8.02 (s, 1H), 7.63 - 7.55 (m, 2H),7.35 (dd, *J =* 10.8, 5.3 Hz, 1H), 6.86 - 6.77 (m,1H), 5.26 (s, 2H), 4.27 (q, *J =* 2.9 Hz, 2H), 4.05(s, 1H), 3.81 (t, *J =* 5.4 Hz, 2H), 3.44 (t, *J =* 6.7Hz, 3H), 3.12 - 2.91 (m, 4H), 2.53 (s, 2H), 2.34- 2.28 (m, 1H), 2.25 (d, *J =* 2.4 Hz, 3H), 2.13 - 1.68 (m, 5H), 1.22 (t, *J =* 7.4 Hz, 3H). ¹⁹F NMR (377 MHz, DMSO) δ -59.73 (d*, J =* 12.4 Hz), -117.07 (d, *J =* 13.5 Hz), -125.74. |
| T035 | 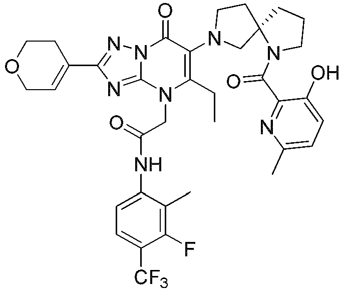 | 738.74 | ¹H NMR (400 MHz, DMSO) δ 10.68 (s,1H), 10.24 (s, 1H), 7.59 (d, *J* = 4.0 Hz, 2H),7.21 (d, *J =* 8.4 Hz, 1H), 7.15 (d, *J =* 8.4Hz, 1H), 6.82 (s, 1H), 5.26 (s, 2H), 4.27 (q,*J =* 2.8 Hz, 2H), 4.05 (s, 1H), 3.82 (t, *J =*5.4 Hz, 2H), 3.50 (td, *J* = 6.5, 2.5 Hz, 3H),3.12 - 2.91 (m, 4H), 2.53 (s, 2H), 2.37 (s,3H), 2.29 (d, *J =* 10.1 Hz, 1H), 2.25 (d, *J* =2.4 Hz, 3H), 2.12 - 1.67 (m, 5H), 1.22 (t, *J=* 7.4 Hz, 3H). ¹⁹F NMR (377 MHz, DMSO) δ -59.72 (d, *J=* 12.5 Hz), -117.06 *(d, J=* 13.4 Hz). |
| T036 | 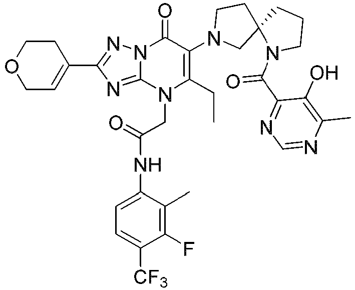 | 739.73 | ¹H NMR (400 MHz, DMSO) δ10.90 (s, 1H), 10.24 (s, 1H), 8.58 (s,1H), 7.59 (d, *J =* 5.0 Hz, 2H), 6.82(s, 1H), 5.26 (s, 2H), 4.27 (d, *J =*3.0 Hz, 2H), 4.05 (s, 1H), 3.81 (t*, J=* 5.4 Hz, 2H), 3.56 (t, *J =* 6.7 Hz,2H), 3.31 (s, 3H), 3.04 (q, *J =* 9.4 Hz, 3H), 2.44 (s, 3H),2.50(m, 1H),2.28 (s, 1H), 2.25 (d, *J =* 2.4 Hz,3H), 2.13 - 1.71 (m, 5H), 1.22 (t, *J =* 7.4 Hz, 3H). ¹⁹F NMR (377 MHz, DMSO) δ -59.73 (d, *J =* 12.4 Hz), - 117.04. |
| T037 | 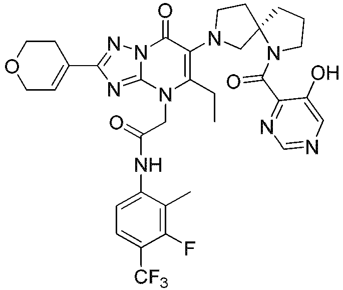 | 725.71 | ¹H NMR (400 MHz, DMSO) δ 10.25 (s,1H), 8.45 (s, 1H), 7.95 (s, 1H), 7.63 -7.54 (m, 2H), 6.87 - 6.77 (m, 1H), 5.26(s, 2H), 4.27 (q, *J* = 2.9 Hz, 2H), 4.01 (s,1H), 3.81 (t, *J =* 5.4 Hz, 2H), 3.62 (s,1H), 3.30 - 3.25 (m, 3H), 3.10 - 2.92 (m,3H), 2.50(m,3H), 2.25 (d, *J =* 2.4 Hz,4H), 2.13 - 1.70 (m, 4H), 1.22 (t, *J =* 7.4Hz, 3H). ¹⁹F NMR (377 MHz, DMSO) δ -59.73 (d, *J* = 12.4 Hz), -117.07 (d, *J =* 13.0 Hz). |
| T038 | 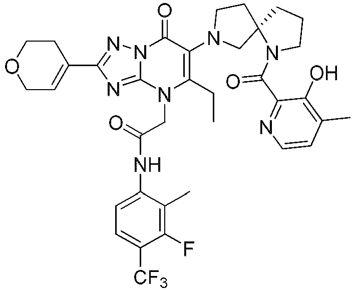 | 738.74 | ¹H NMR (400 MHz, DMSO) δ 12.03 (s, 1H), 10.24 (s, 1H), 7.99 (d, *J* = 4.5 Hz, 1H), 7.59 (d, *J =* 5.0 Hz, 2H), 7.30 (d, *J =* 4.4 Hz, 1H), 6.86 - 6.79 (m, 1H), 5.26 (s, 2H), 4.27 (d, *J =* 2.9 Hz, 2H), 4.06 (s, 1H), 3.93 - 3.76 (m, 4H), 3.02 (s, 4H),2.50(m,3H), 2.35 - 2.19 (m, 7H), 2.10 - 1.72 (m, 5H), 1.26 - 1.21 (m, 3H). ¹⁹F NMR (377 MHz, DMSO) δ -59.73 (d, *J =* 12.4 Hz), -117.06 (q, *J =* 12.4 Hz). |
| T039 | 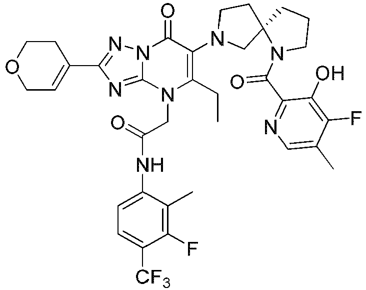 | 756.73 | ¹H NMR (400 MHz, DMSO) δ 11.41 (s, 1H), 10.24 (s, 1H), 7.98 (d, *J* = 8.5 Hz, 1H), 7.59 (d, *J =* 4.8 Hz, 2H), 6.84 - 6.78 (m, 1H), 5.26 (s, 2H), 4.27 (q, *J =* 2.9 Hz, 2H), 4.03 (q, *J =* 7.1 Hz, 1H), 3.81 (t, *J =* 5.4 Hz, 2H), 3.57 (t, *J =* 6.7 Hz, 2H), 3.12 - 2.92 (m, 4H),2.50(m,3H), 2.25 (d, *J =* 2.7 Hz, 7H), 2.09 - 1.75 (m, 5H), 1.22 (t, *J =* 7.2 Hz, 3H). ¹⁹F NMR (377 MHz, DMSO) δ -59.72 (d, *J =* 12.4 Hz), -117.06 (q, *J =* 12.5 Hz), -131.12. |
| T040 | 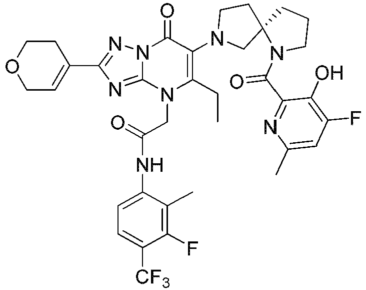 | 756.74 | ¹H NMR (400 MHz, DMSO) δ 10.75 (s, 1H), 10.29 (s, 1H), 7.59 (d*, J* = 4.1 Hz, 2H), 7.22 *(d, J =* 11.5 Hz, 1H), 6.82 (s, 1H), 5.26 (s, 2H), 4.27 (d, *J =* 3.2 Hz, 2H), 4.03 (s, 1H), 3.81 (t, *J =* 5.4 Hz, 2H), 3.45 (t, *J =* 6.8 Hz, 3H), 3.11 - 2.90 (m, 4H), 2.37 (s, 3H), 2.33 - 2.27 (m, 1H), 2.25 (d, *J =* 2.3 Hz, 3H), 2.10 - 1.68 (m, 5H), 1.22 (t, *J* = 7.3 Hz, 3H). ¹⁹F NMR (377 MHz, DMSO) δ -59.73 (d, *J=* 12.4 Hz), -117.06, -125.87. |
| T041 | 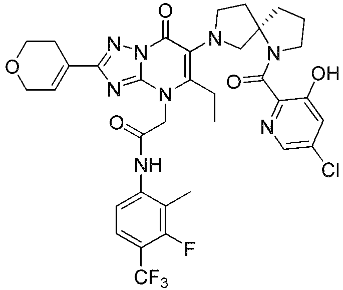 | 759.15 | ¹H NMR (400 MHz, DMSO) δ 11.53 (s,1H), 10.24 (s, 1H), 8.06 (d, *J* = 2.1 Hz, 1H),7.63 - 7.56 (m, 2H), 7.40 (d, *J =* 2.1 Hz,1H), 6.82 (s, 1H), 5.26 (s, 2H), 4.27 (q, *J* =2.8 Hz, 2H), 4.03 (s, 1H), 3.81 (t, *J =* 5.5 Hz,2H), 3.45 - 3.37 (m, 3H), 3.13 - 2.88 (m,4H), 2.52 (s, 2H), 2.28 (s, 1H), 2.25 (d, *J* =2.4 Hz, 3H), 2.10 - 1.68 (m, 5H), 1.22 (t, *J* =7.4 Hz, 3H). ¹⁹F NMR (377 MHz, DMSO) δ -59.72 (d, *J =* 12.2 Hz), - 117.04. |
| T042 | 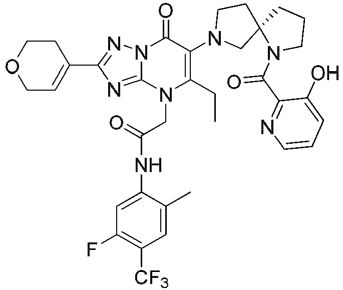 | 724.72 | ¹H NMR (400 MHz, DMSO-*d*6)δ10.92 (s, IH), 10.09 (s, 1H),8.05 (dd, *J =* 3.5, 2.4 Hz, 1H), 7.81 (d*, J =* 12.9 Hz, 1H), 7.68 (d*,J =* 8.2 Hz, 1H), 7.34 - 7.28 (m, 2H), 6.84-6.78 (m, 1H), 5.29(s, 2H), 4.26 (q, *J =* 2.9 Hz, 2H), 4.06 (s, 1H), 3.82 (t, *J =* 5.4 Hz,2H), 3.73-3.36 (m, 3H), 3.32-2.65 (m, 5H), 2.52 (s, 2H), 2.36(s, 3H), 2.34-2.24 (m, 1H), 2.06 (dt, *J =* 12.9, 7.1 Hz, 1H), 1.80(qd, *J =* 13.2, 12.7, 6.4 Hz, 3H), 1.22 (t, *J* = 7.4 Hz, 3H). ¹⁹F NMR (376 MHz, DMSO-*d*6)δ-59.31 (d, *J* = 12.3 Hz),-118.60 (d, *J =* 12.6 Hz). |
| T043 | 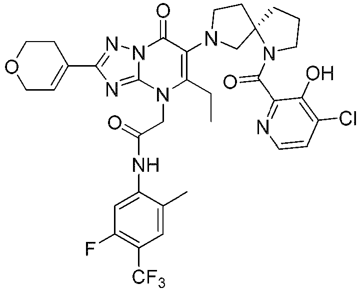 | 759.16 | ¹H NMR (400 MHz, DMSO-d6) δ 12.07 (s, 1H), 10.09 (s, 1H), 8.07(d, *J* = 5.0 Hz, 1H), 7.80 (d, *J* = 12.9 Hz, 1H), 7.68 (d, *J =* 8.2 Hz, 1H),7.61 (d*, J =* 5.0 Hz, 1H), 6.87-6.78 (m, 1H), 5.29 (s, 2H), 4.26 (q, *J* = 2.9 Hz, 2H), 4.06 (s, 1H), 3.82 (t, *J =* 5.4 Hz, 2H), 3.67 (t*, J =* 6.7 Hz,2H), 3.33-2.72 (m, 6H), 2.52 (s, 2H), 2.36 (s, 3H), 2.34-2.25 (m,1H), 2.07 (dt, *J =* 12.9, 7.1 Hz, 1H), 1.90-1.74 (m, 3H), 1.24-1.20(m, 3H). ¹⁹F NMR (376 MHz, DMSO-d6) δ -59.30 (d, *J =* 12.1 Hz),-118.60 (q*, J =* 12.2 Hz). |
| T044 | 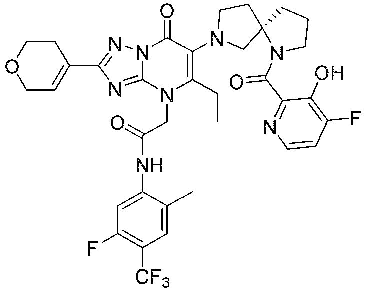 | 742.71 | ¹H NMR (400 MHz, DMSO-d6) δ 11.24 (s, 1H), 10.10 (s, 1H), 8.07 (dd, *J =* 7.0*,* 5.3 Hz, 1H), 7.81 (d, *J* = 12.9 Hz, 1H), 7.68 (d*, J =* 8.2 Hz, 1H), 7.39(dd, *J =* 10.8, 5.3 Hz, 1H), 6.86-6.77 (m, 1H), 5.29 (s, 2H), 4.26 (q, *J* =2.9 Hz, 2H), 4.05 (s, 1H), 3.82 (t, *J =* 5.4 Hz, 2H), 3.45 (t, *J =* 6.7 Hz, 2H),3.36-2.71 (m, 6H), 2.54-2.52 (m, 2H), 2.36 (s, 3H), 2.34-2.25 (m, 1H),2.06 (dt, *J =* 13.3, 7.0 Hz, 1H), 1.80 (dp, *J =* 20.2, 6.4 Hz, 3H), 1.22 (t, *J* =7.3 Hz, 3H). ¹⁹F NMR (376 MHz, DMSO-d6) δ -59.31 (d, *J =* 12.1 Hz), |
| | | | -118.60 (q, *J =* 12.1 Hz), -125.54. |
| T045 | 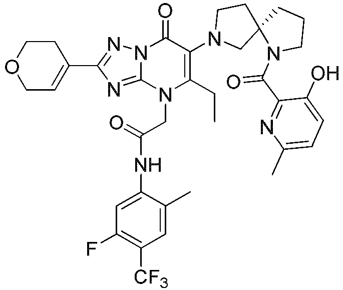 | 738.74 | ¹H NMR (400 MHz, DMSO-d6) δ 10.78 (s, 1H), 10.09 (s, 1H), 7.81 (d, *J* =12.9 Hz, 1H), 7.68 (d, *J =* 8.2 Hz, 1H), 7.30 - 7.16 (m, 2H), 6.82 (s, 1H),5.29 (s, 2H), 4.26 (q, *J =* 2.9 Hz, 2H), 4.05 (s, 1H), 3.82 (t, *J =* 5.4 Hz, 2H),3.54 - 3.47 (m, 3H), 3.24 - 2.72 (m, 5H), 2.52 (s, 2H), 2.39 (s, 3H), 2.36 (s,3H), 2.33 - 2.26 (m, 1H), 2.05 (dq, *J =* 12.2, 6.1, 5.1 Hz, 1H), 1.87 - 1.71 (m,3H), 1.24 - 1.20 (m, 3H). ¹⁹F NMR (376 MHz, DMSO-d6) δ - 59.30 (d, *J =* 12.2 Hz), -118.60 (q, *J =* 12.2 Hz). |
| T046 | 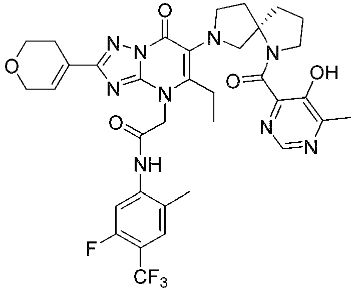 | 739.73 | ¹H NMR (400 MHz, DMSO-d6) δ 10.91 (s, 1H), 10.10 (s, 1H), 8.58 (s,1H), 7.81 (d, *J =* 12.9 Hz, 1H), 7.68 (d, *J =* 8.2 Hz, 1H), 6.87 - 6.75 (m,1H), 5.29 (s, 2H), 4.26 (q, *J =* 2.9 Hz, 2H), 4.05 (s, 1H), 3.82 (t, *J =* 5.5Hz, 2H), 3.56 (t, *J =* 6.7 Hz, 2H), 3.36 - 2.70 (m, 6H), 2.53 (s, 2H), 2.45(s, 3H), 2.36 (s, 3H), 2.29 (d, *J =* 6.9 Hz, 1H), 2.07 (dt, *J =* 13.0, 6.9 Hz,1H), 1.82 (ddd, *J* = 27.3, 12.5, 6.0 Hz, 3H), 1.22 (t, *J =* 7.3 Hz, 3H). ¹⁹F NMR (376 MHz, DMSO-d6) δ -59.31 (d, *J* = 12.0 Hz),-118.60 (d, *J =* 12.1 Hz). |
| T047 | 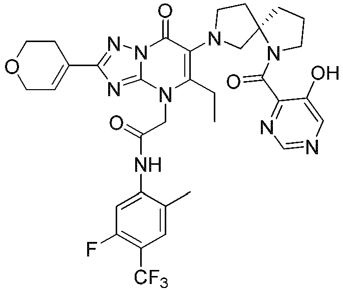 | 725.71 | ¹H NMR (400 MHz, DMSO-d6) δ 10.91 (s, 1H), 10.09 (s, 1H), 8.69(s, 1H), 8.49 (s, 1H), 7.81 (d, *J =* 12.9 Hz, 1H), 7.68 (d, *J =* 8.2 Hz, 1H), 6.86 - 6.78 (m, 1H), 5.29 (s, 2H), 4.26 (t, *J =* 3.0 Hz, 2H), 4.02(s, 1H), 3.82 (t, *J =* 5.4 Hz, 2H), 3.29 (td, *J =* 6.7, 2.0 Hz, 4H), 3.12 -2.69 (m, 4H), 2.54 - 2.52 (m, 2H), 2.36 (s, 3H), 2.34 - 2.27 (m, 1H),2.07 (dt, *J =* 13.0, 7.1 Hz, 1H), 1.80 (ddt, *J =* 18.7, 11.9, 5.9 Hz, 3H), 1.22 (t, *J =* 7.4 Hz, 3H). ¹⁹F NMR (376 MHz, DMSO-d6) δ -59.30 (d, *J =* 12.2 Hz), -118.60 (q*, J =* 12.2 Hz). |
| T048 | 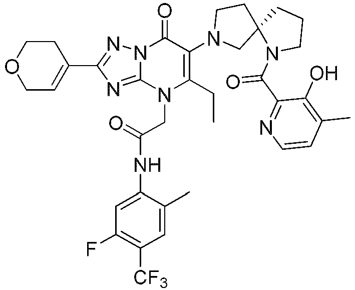 | 738.74 | ¹H NMR (400 MHz, DMSO-d6) δ 12.05 (s, 1H), 10.10 (s, 1H),8.00 (d*, J =* 4.5 Hz*,* 1H), 7.81 (d*, J =* 13.0 Hz, 1H), 7.68 (d, *J* =8.2 Hz, 1H), 7.30 (d, *J* = 4.5 Hz, 1H), 6.82 (dd, *J =* 3.4, 1.9 Hz,1H), 5.29 (s, 2H), 4.26 (q, *J =* 2.9 Hz, 2H), 4.08 (s, 1H), 3.85(dt, *J =* 22.9, 6.1 Hz, 4H), 3.33 - 2.64 (m, 6H), 2.52 (s, 2H),2.36 (s, 3H), 2.30 (d, *J =* 24.7 Hz, 1H), 2.23 (s, 3H), 2.06 (dt, *J =* 13.2, 7.1 Hz, 1H), 1.83 (ddt, *J =* 34.3, 14.0, 6.3 Hz, 3H), 1.23(t, *J =* 7.4 Hz, 3H). ¹⁹F NMR (376 MHz, DMSO-d6) δ -59.31 (d, *J =* 12.0 Hz),-118.60 (q, *J* = 12.1 Hz). |
| T049 | 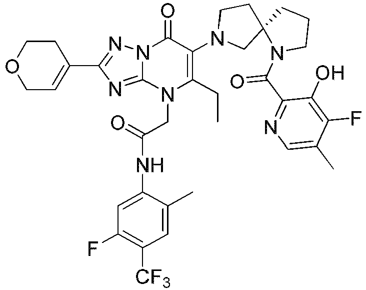 | 756.74 | ¹H NMR (400 MHz, DMSO-d6) δ 11.51 (s, 1H), 10.10 (s, 1H), 7.98 (d, *J=*8.4 Hz, 1H), 7.81 (d*, J =* 13.0 Hz, 1H), 7.68 (d, *J=* 8.2 Hz, 1H), 6.87 - 6.76(m, 1H), 5.29 (s, 2H), 4.26 (q, *J =* 2.8 Hz, 2H), 4.05 (s, 1H), 3.82 (t, *J =* 5.4Hz, 2H), 3.58 (t, *J =* 6.7 Hz, 2H), 3.02 (dd, *J =* 15.4, 7.8 Hz, 6H), 2.54 - 2.52(m, 2H), 2.37 - 2.24 (m, 7H), 2.04 (dq, *J =* 19.7, 7.6 Hz, 1H), 1.82 (ddt, *J =* 23.1, 14.8, 7.1 Hz, 3H), 1.22 (t*, J =* 7.3 Hz, 3H). ¹⁹F NMR (376 MHz, DMSO-d6) δ - 59.31 (d, *J =* 12.2Hz), -118.60 (q, *J =* 12.0 Hz), -131.13. |
| T050 | 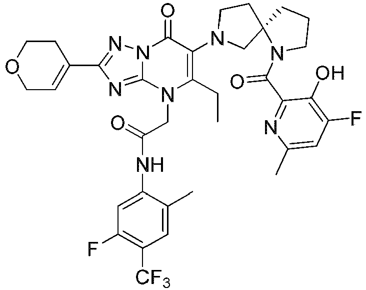 | 756.74 | ¹H NMR (400 MHz, DMSO-d6) δ 10.84 (s, 1H), 10.09 (s, 1H), 7.80 (d, *J =* 13.0 Hz, 1H), 7.67 (d, *J =* 8.2 Hz, 1H), 7.25 (d*, J =* 11.6 Hz, 1H), 6.84 - 6.78(m, 1H), 5.29 (s, 2H), 4.26 (q, *J =* 2.9, 2.5 Hz, 2H), 4.00 (d, *J =* 35.9 Hz, 1H),3.81 (t*, J* = 5.5 Hz, 2H), 3.59 - 3.38 (m, 3H), 3.23 - 2.71 (m, 5H), 2.52 (s,2H), 2.38 (s, 3H), 2.36 (s, 3H), 2.29 (s, 1H), 2.06 (dt, *J =* 13.0, 6.8 Hz, 1H),1.88 - 1.72 (m, 3H), 1.22 (t, *J =* 7.4 Hz, 3H). ¹⁹F NMR (377 MHz, DMSO-d6) δ -59.31 (d, *J =* 12.2 Hz),-118.48 - -118.71 (m), - 125.82 (d, *J =* 11.7 Hz). |
| T051 | 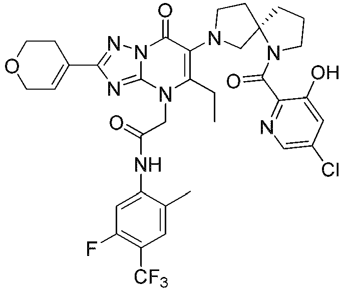 | 759.16 | ¹H NMR (400 MHz, DMSO-d6) δ11.41 (s, 1H), 10.09 (s, 1H), 8.09 (d, *J =* 2.0 Hz, 1H), 7.81 (d, *J =* 13.0 Hz, 1H), 7.68 (d, *J =* 8.2 Hz, 1H), 7.42 (d,*J* = 2.1 Hz, 1H), 6.82 (t, *J =* 2.6 Hz, 1H), 5.29 (s, 2H), 4.26 (q, *J =* 2.9 Hz,2H), 4.04 (s, 1H), 3.81 (t, *J =* 5.4 Hz, 2H), 3.41 (t, *J =* 6.7 Hz, 2H), 3.32 -2.72 (m, 6H), 2.52 (s, 2H), 2.36 (s, 3H), 2.29 (s, 1H), 2.06 (dt, *J* = 13.0,7.0 Hz, 1H), 1.85 - 1.71 (m, 3H), 1.22 (t*, J* = 7.4 Hz, 3H). ¹⁹F NMR (376 MHz, DMSO-d6) δ -59.30 (d, *J =* 11.9 Hz),-118.60 (q, *J =* 12.1 Hz). |
| T052 | 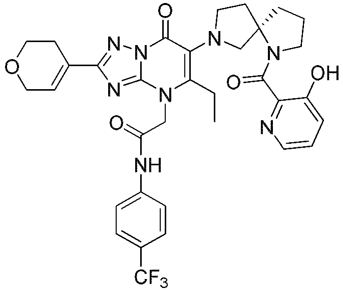 | 692.27 | ¹H NMR (400 MHz, DMSO-d6) δ 10.94 (d, *J =* 14.3 Hz, 2H), 8.04 (dd, *J =* 3.9*,* 2.0 Hz, 1H), 7.84 - 7.65 (m, 4H), 7.31 (d, *J* = 3.8 Hz, 2H), 6.79 (s, 1H),5.20 (s, 2H), 4.24 (d, *J =* 3.2 Hz, 2H), 4.06 (s, 1H), 3.80 (t, *J =* 5.4 Hz, 2H),3.44 (d, *J =* 7.0 Hz, 2H), 3.17 - 2.70 (m, 5H), 2.29 (s, 1H), 2.04 (dd, *J =* 12.8,7.1 Hz, 1H), 1.85 - 1.69 (m, 3H), 1.20 (t, *J =* 7.3 Hz, 3H) |
| T053 | 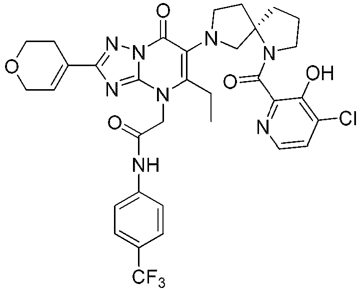 | 726.23 | ¹H NMR (400 MHz, DMSO-d6) δ 12.06 (s, 1H), 10.89 (s, 1H), 8.06 (d, *J =* 5.0 Hz, 1H), 7.84 - 7.67 (m, 4H), 7.61 (d*, J =* 5.0 Hz, 1H), 6.84 - 6.73(m, 1H), 5.20 (s, 2H), 4.24 (q, *J =* 2.8 Hz, 2H), 4.06 (s, 1H), 3.80 (t, *J =* 5.4 Hz, 2H), 3.67 (t, *J =* 6.7 Hz, 2H), 3.25 - 2.71 (m, 5H), 2.29 (s, 1H),2.07 (dt, *J =* 12.9, 7.4 Hz, 1H), 1.83 (ddt, *J =* 28.9, 13.4, 6.4 Hz, 3H), 1.21(t, *J* = 7.4 Hz, 3H). ¹⁹F NMR (377 MHz, DMSO-*d6*) δ -60.39 |
| T054 | 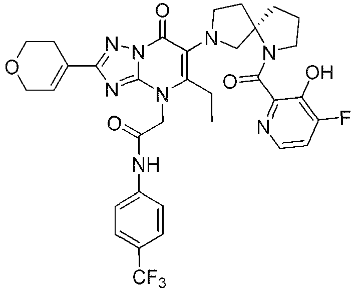 | 710.26 | ¹H NMR (400 MHz, DMSO-*d*6) δ 11.24 (s, 1H), 10.89 (s, 1H), 8.07 (dd, *J* = 7.0, 5.2 Hz, 1H), 7.82 - 7.68 (m, 4H), 7.39 (dd, *J =* 10.9, 5.3 Hz, 1H), 6.79 (td, *J* = 3.0, 1.5 Hz, 1H), 5.20 (s, 2H), 4.24 (t, *J =* 3.0Hz, 2H), 4.13 - 3.94 (m, 1H), 3.80 (t, *J* = 5.4 Hz, 2H), 3.45 (t, *J* = 7.1 Hz, 3H), 3.27 - 2.72 (m, 5H), 2.51 (s, 2H), 2.35 - 2.25 (m, 1H), 2.06 (dt, *J =* 13.0, 7.1 Hz, 1H), 1.80 (dh, *J* = 19.9, 6.3 Hz, 3H), 1.20 (t, *J* =7.4 Hz, 3H). ¹⁹F NMR (377 MHz, DMSO-d6) δ - 60.39, -125.54 (t, *J =* 9.3 Hz). |
| T055 | 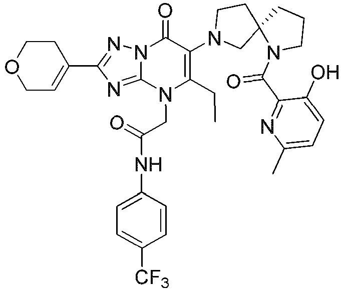 | 706.28 | ¹H NMR (400 MHz, DMSO-*d*6) δ 10.89 (s, 2H), 7.82 - 7.67 (m, 4H), 7.34 - 7.18 (m, 2H), 6.81 - 6.76(m, 1H), 5.20 (s, 2H), 4.24 (q, *J* = 2.9 Hz, 2H), 4.05 (s, 1H), 3.80 (t, *J =* 5.5 Hz, 2H), 3.62-3.57 (m, 2H)3.19 - 2.74 (m, 5H), 2.51 (s, 2H), 2.40 (d, *J* = 2.6 Hz, 3H), 2.30 (s, 1H), 2.06 (dt, *J* = 13.1, 7.0 Hz,1 H), 1.80 (ddt, *J* = 26.8, 13.2, 6.5 Hz, 3H), 1.21 (q, *J* = 7.5, 6.5 Hz, 3H).¹⁹F NMR (377 MHz, DMSO-d6) δ -60.39. |
| T056 | 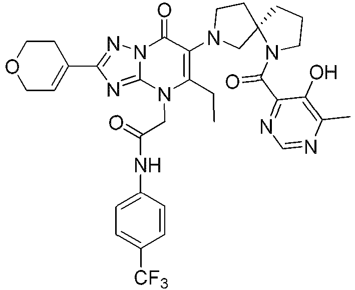 | 707.28 | ¹H NMR (400 MHz, DMSO-*d*6) δ 10.90 (d, *J =* 6.7 Hz, 2H), 8.58 (s, 1H), 7.79 (d, *J* =8.6 Hz, 2H), 7.71 (d, *J* = 8.7 Hz, 2H), 6.81 - 6.76 (m, 1H), 5.20 (s, 2H), 4.24 (q, *J =* 2.9Hz, 2H), 4.05 (s, 1H), 3.80 (t, *J* = 5.5 Hz, 2H), 3.56 (t, *J* = 6.6 Hz, 2H), 3.01 (ddt, *J* =27.7, 14.3, 7.5 Hz, 6H), 2.51 (s, 2H), 2.44 (s, 3H), 2.34 - 2.26 (m, 1H), 2.07 (dt, *J* =13.0, 6.8 Hz, 1H), 1.82 (ddt, *J* = 27.7, 13.4, 6.2 Hz, 3H), 1.20 (t, *J* = 7.4 Hz, 3H). ¹⁹F NMR (377 MHz, DMSO-d6) δ -60.39. |
| T057 | 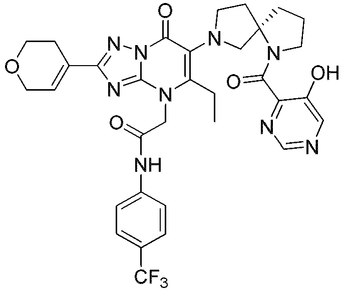 | 693.26 | ¹H NMR (400 MHz, DMSO-*d*6) δ 10.90 (d, *J =* 6.8 Hz, 2H), 8.69 (s, 1H), 8.49 (s, 1H), 7.79 (d, *J* = 8.6Hz, 2H), 7.71 (d, *J* = 8.8 Hz, 2H), 6.81 - 6.76 (m, 1H), 5.20 (s, 2H), 4.24 (t, *J =* 3.0 Hz, 2H), 4.02 (s, 1H),3.80 (t, *J =* 5.4 Hz, 2H), 3.32 - 3.21 (m, 3H), 3.21 - 2.68 (m, 5H), 2.59 - 2.51 (m, 2H), 2.34 - 2.26 (m, 1H), 2.07 (dt, *J =* 13.0, 7.1 Hz, 1H), 1.79 (ddd, *J =* 19.4, 12.0, 6.4 Hz, 3H), 1.20 (t, *J =* 7.4 Hz, 3H).¹⁹F NMR (377 MHz, DMSO-d6) δ -60.39. |
| T058 | 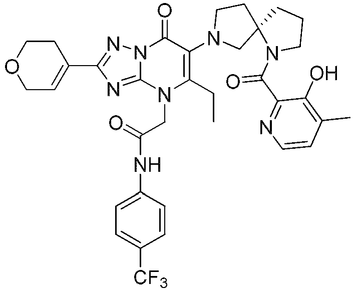 | 706.28 | ¹H NMR (400 MHz, DMSO-*d*6) δ 12.04 (s, 1H), 10.90 (s, 1H), 7.99 (d, *J* = 4.6 Hz, 1H), 7.85 - 7.58 (m, 4H), 7.30 (d, *J* = 4.6 Hz, 1H), 6.79 (s, 1H), 5.20(s, 2H), 4.30 - 4.19 (m, 2H), 4.07 (s, 1H), 3.84 (dt, *J* = 30.8, 6.4 Hz, 4H),2.27 (s, 2H), 2.22 (s, 3H), 2.06 (dt, *J* = 12.7, 6.8 Hz, 1H), 1.91 - 1.72 (m,3H), 1.21 (t, *J* = 7.6 Hz, 3H). ¹⁹F NMR (377 MHz, DMSO-*d*6) δ -60.40 (t, *J* = 5.7 Hz). |
| T059 | 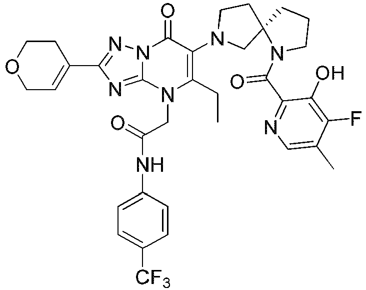 | 724.27 | ¹H NMR (400 MHz, DMSO-*d*6) δ 11.35 (s, 1H), 10.90 (s, 1H), 7.98 (d, *J* = 8.4Hz, 1H), 7.79 (d, *J* = 8.6 Hz, 2H), 7.71 (d, *J* = 8.7 Hz, 2H), 6.81 - 6.76 (m, 1H),5.20 (s, 2H), 4.24 (q, *J* = 2.9 Hz, 2H), 4.06 (s, 1H), 3.79 (q, *J =* 5.5 Hz, 2H), 3.57(t, *J* = 6.7 Hz, 3H), 3.00 (dq, *J* = 30.4, 8.4 Hz, 5H), 2.52 (s, 2H), 2.34 - 2.27 (m, 1H), 2.25 (d, *J =* 1.6 Hz, 3H), 2.05 (dt, *J =* 12.7, 7.0 Hz, 1H), 1.87 - 1.73 (m, 3H). ¹⁹F NMR (377 MHz, DMSO-*d*6) δ - 60.39, -131.13 (d, *J =* 8.5 Hz). |
| T060 | 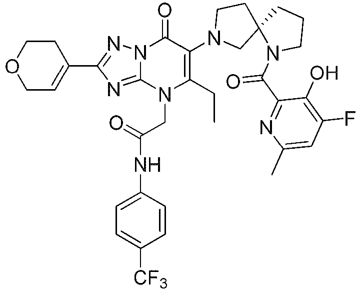 | 724.27 | ¹H NMR (400 MHz, DMSO-*d*6) δ 10.91 (s, 2H), 7.79 (d, *J =* 8.6 Hz, 2H),7.71 (d, *J* = 8.7 Hz, 2H), 7.24 (d, *J =* 11.6 Hz, 1H), 6.81 - 6.77 (m, 1H), 5.20(s, 2H), 4.24 (q, *J =* 2.9 Hz, 2H), 4.04 (s, 1H), 3.80 (t, *J* = 5.4 Hz, 2H), 3.65 -3.40 (m, 3H), 3.13 - 2.64 (m, 5H), 2.52 (d, *J* = 3.4 Hz, 2H), 2.38 (s, 3H), 2.34- 2.26 (m, 1H), 2.06 (dq, *J* = 13.0, 6.9, 6.5 Hz, 1H), 1.79 (dp, *J* = 20.1, 6.3Hz, 3H), 1.21 (q, *J =* 7.6, 6.6 Hz, 3H). ¹⁹F NMR (377 MHz, DMSO-*d*6) δ -60.39, - 125.83 (d, *J* = 11.6 Hz). |
| T061 | 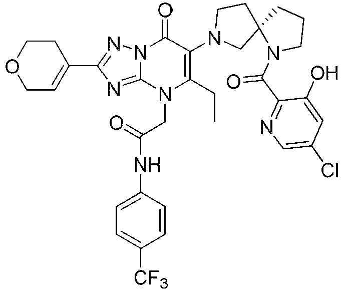 | 726.23 | ¹H NMR (400 MHz, DMSO-*d*6) δ 11.38 (s, 1H), 10.89 (s, 1H), 8.10 (d, *J* = 2.0 Hz, 1H), 7.79 (d,*J* = 8.6Hz, 2H), 7.71 (d, *J* = 8.6 Hz, 2H), 7.43 (d, *J* = 2.1 Hz, 1H), 6.79 (s, 1H), 5.20 (s, 2H), 4.24 (q, *J =* 2.9 Hz,2H), 4.04 (s, 1H), 3.80 (t, *J* = 5.5 Hz, 2H), 3.43 (s, 2H), 3.32 - 2.71 (m, 6H), 2.52 (s, 2H), 2.32 - 2.24 (m,1H), 2.05 (dt, *J* = 13.1, 6.9 Hz, 1H), 1.79 (dp, *J* = 19.6, 6.3 Hz, 3H), 1.20 (t, *J* = 7.4 Hz, 3H). ¹⁹F NMR (377 MHz, DMSO-*d*6) δ -60.39 |
| T062 | 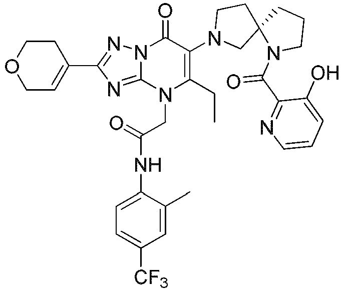 | 706.73 | ¹H NMR (400 MHz, DMSO-*d*6) δ 10.94 (s, 1H), 10.05 (s,1H), 8.05 (dd, *J* = 3.9, 2.0 Hz, 1H), 7.74 (d, *J* = 8.4 Hz, 1H),7.63 (d, *J* = 2.2 Hz, 1H), 7.54 (dd, *J =* 8.6, 2.2 Hz, 1H), 7.31(d, *J =* 3.8 Hz, 2H), 6.84 (t, *J* = 2.1 Hz, 1H), 5.25 (s, 2H),4.27 (q, *J =* 2.9 Hz, 2H), 3.82 (t, *J =* 5.4 Hz, 2H), 3.52 - 3.41(m, 2H), 3.32 (m, 2H), 3.14 - 2.92 (m, 3H), 2.52 (d, *J =* 5.0Hz, 3H), 2.37 (s, 3H), 2.31 (dd, *J =* 11.7, 6.0 Hz, 1H), 2.13 -1.93 (m, 2H), 1.80 (ddq, *J* = 27.8, 13.4, 7.9, 6.9 Hz, 3H), 1.22(d, *J =* 8.0 Hz, 3H). ¹⁹F NMR (377 MHz, DMSO-*d*6) δ - 60.57 |
| T063 | 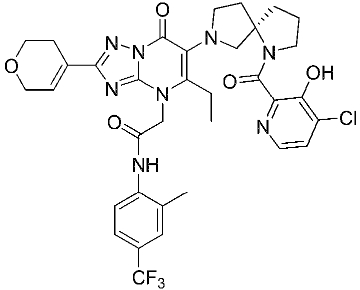 | 741.17 | ¹H NMR (400 MHz, DMSO-*d*6) δ 12.05 (s, 1H), 10.04 (s, 1H), 8.06 (d, *J* = 5.0 Hz, 1H),7.73 (d, *J =* 8.4 Hz, 1H), 7.68 - 7.57 (m, 2H), 7.58 - 7.45 (m, 1H), 6.90 - 6.78 (m, 1H),5.25 (s, 2H), 4.27 (q, *J* = 2.9 Hz, 2H), 4.15 - 3.95 (m, 1H), 3.82 (t, *J =* 5.4 Hz, 2H), 3.66(t, *J* = 6.7 Hz, 2H), 3.13 - 2.90 (m, 3H), 2.52 (d, *J* = 1.7 Hz, 3H), 2.36 (s, 3H), 2.34 - 2.21(m, 1H), 2.14 - 2.01 (m, 1H), 1.83 (ddq, *J* = 30.3, 12.3, 6.7, 5.8 Hz, 3H), 1.23 (t, *J =* 7.4Hz, 3H). ¹⁹F NMR (377 MHz, DMSO-d6) δ -60.57 |
| T064 | 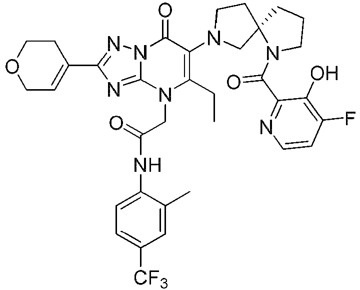 | 724.72 | ¹H NMR (400 MHz, DMSO-*d*6) δ 11.26 (s, 1H), 10.04 (s,1H), 8.06 (dd, *J* = 7.0, 5.3 Hz, 1H), 7.74 (d, *J* = 8.4 Hz, 1H),7.63 (d, *J* = 2.2 Hz, 1H), 7.54 (dd, *J* = 8.6, 2.2 Hz, 1H), 7.38(dd, *J* = 10.9, 5.3 Hz, 1H), 6.83 (s, 1H), 5.25 (s, 2H), *4.27* (q, *J* = 2.9 Hz, 2H), 4.06 (d, *J* = 16.9 Hz, 1H), 3.82 (t, *J* = 5.4 Hz,2H), 3.44 (t, *J =* 6.8 Hz, 2H), 3.32 (m, 2H), 3.05 (p, *J =* 8.7 Hz,3H), 2.51 (s, 3H), 2.36 (s, 3H), 2.33 - 2.21 (m, 1H), 2.06 (dt, *J* = 13.1, 6.6 Hz, 1H), 1.93 - 1.63 (m, 3H), 1.23 (t, *J* = 7.4 Hz,3H). ¹⁹F NMR (377 MHz, DMSO) δ - 60.57, -125.55 - -125.67 (m). |
| T065 | 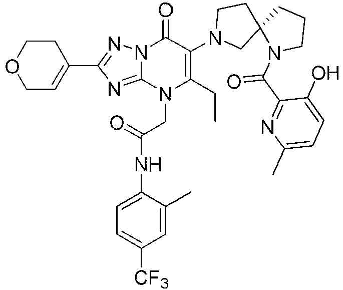 | 720.75 | ¹H NMR (400 MHz, DMSO-*d*6) δ 10.76 (s, 1H), 10.05 (s, 1H), 7.74 (d, *J* = 8.4 1H), 7.63 (d, *J* = 2.2 Hz, 1H), 7.54 (dd, *J* = 8.5, 2.3 Hz, 1H), 7.27 (d, *J* = 8.5 Hz7.19 (d, *J* = 8.5 Hz, 1H), 6.84 (t, *J* = 2.2 Hz, 1H), 5.25 (s, 2H), 4.27 (q, *J =* 2.9 H2H), 3.82 (t, *J =* 5.4 Hz, 2H), 3.59 - 3.46 (m, 4H), 3.15 - 2.90 (m, 4H), 2.54 (d, *J* 7.9 Hz, 2H), 2.38 (d, *J* = 8.4 Hz, 6H), 2.33 - 2.23 (m, 1H), 2.03 (ddd, *J* = 25.6, 14.2,7.0 Hz, 2H), 1.92 - 1.67 (m, 3H), 1.31 - 1.24 (m, 3H)_{.} ¹⁹F NMR (377 MHz, DMSO) δ - 60.57. |
| T066 | 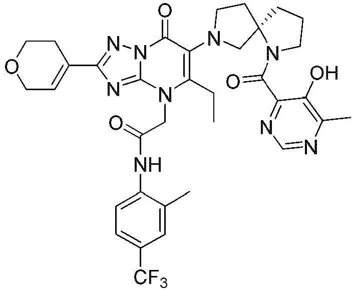 | 721.74 | ¹H NMR (400 MHz, DMSO-*d*6) δ 10.90 (s, 1H), 10.04 (s, 1H), 8.57 (s, 1H), 7.73 (d, *J =* 8.4 Hz, 1H),7.63 (d, *J* = 2.2 Hz, 1H), 7.54 (dd, *J* = 8.5, 2.2 Hz, 1H), 6.87 - 6.76 (m, 1H), 5.25 (s, 2H), 4.27 (q, *J* = 2.9Hz, 2H), 4.05 (s, 1H), 3.82 (t, *J* = 5.4 Hz, 2H), 3.55 (t, *J* = 6.7 Hz, 2H), 3.32 (m, 2H), 3.12 - 2.93 |
| | | | (m,3H),2.52(s,3H),2.44(s,3H),2.32 -2.22(m, 1H), 1.95-1.67(m,3H),1.23(t, *J* =7.4 Hz,3H). ¹⁹F NMR (377 MHz, DMSO) δ - 60.58. |
| T067 | 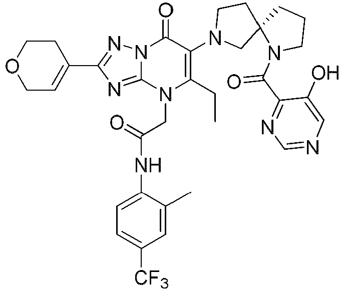 | 707.71 | ¹H NMR (400 MHz, DMSO-*d*6) δ 10.04 (s, 1H), 8.66 (s,1H), 8.46 (s, 1H), 7.74 (d, *J* = 8.5 Hz, 1H), 7.63 (d, *J* = 2.2Hz, 1H), 7.54 (dd,*J* = 8.4, 2.3 Hz, 1H), 6.83 (d, *J* = 2.4 Hz,1H), 5.25 (s, 2H), 4.27 (q, *J* = 2.9 Hz, 2H), 4.15 - 3.92 (m, 1H), 3.82 (t, *J =* 5.4 Hz, 2H), 3.27 (dd, *J* = 6.6, 2.2 Hz, 4H),3.13 - 2.84 (m, 4H), 2.55 - 2.52 (m, 2H), 2.36 (s, 3H), 2.30(d, *J* = 7.7 Hz, 1H), 2.06 (dt, *J* = 13.1, 7.1 Hz, 1H), 1.81(dqt, *J =* 26.5, 13.3, 6.8 Hz, 3H), 1.22 (t, *J* = 7.4 Hz, 3H)_{.} ¹⁹F NMR (377 MHz, DMSO) δ - 60.58. |
| T068 | 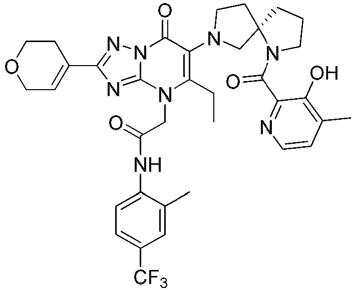 | 720.75 | ¹H NMR (400 MHz, DMSO-*d*6) δ 12.04 (s, 1H), 10.03 (s, 1H), 8.00 (d, *J* = 4.5 Hz, 1H), 7.73 (d,*J* = 8.3 Hz, 1H), 7.63 (d, *J =* 2.2 Hz, 1H), 7.53 (dd, *J* = 8.6, 2.3 Hz, 1H), 7.30 (d, *J* = 4.6 Hz, 1H),6.83 (tt, *J* = 2.9, 1.5 Hz, 1H), 5.24 (s, 2H), 4.26 (q, *J* = 2.9 Hz, 2H), 3.84 (dt, *J* = 19.5, 6.2 Hz,4H), 3.31 (m, 3H), 3.02 (d, *J* = 10.7 Hz, 3H), 2.55 - 2.51 (m, 3H), 2.36 (s, 3H), 2.28 (d, *J =* 17.4Hz, 1H), 2.22 (s, 3H), 2.12 - 1.98 (m, 1H), 1.82 (ddq, *J =* 34.2, 13.9, 7.0, 6.6 Hz, 3H), 1.23 (t, *J* = 7.4 Hz, 3H). ¹⁹F NMR (377 MHz, DMSO) δ - 60.58. |
| T069 | 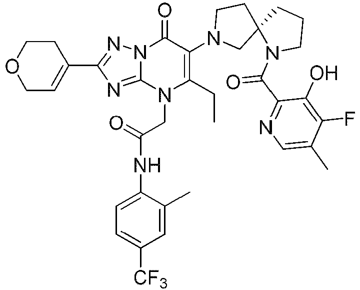 | 738.74 | ¹H NMR (400 MHz, DMSO-*d*6) δ 11.50 (s, 1H), 10.04 (s,1H), 7.98 (d, *J* = 8.4 Hz, 1H), 7.73 (d, *J* = 8.4 Hz, 1H),7.63 (d, *J =* 2.3 Hz, 1H), 7.54 (dd, *J* = 8.6, 2.2 Hz, 1H),6.93 - 6.72 (m, 1H), 5.25 (s, 2H), 4.27 (q, *J =* 2.9 Hz, 2H),4.02 (t, *J* = 24.6 Hz, 1H), 3.82 (t, *J* = 5.5 Hz, 2H), 3.57 (t, *J* = 6.7 Hz, 2H), 3.32 (m, 3H), 3.17 - 2.89 (m, 3H), 2.57 -2.51 (m, 3H), 2.36 (s, 3H), 2.25 (d, *J* = 1.5 Hz, 3H), 2.19 -1.94 (m, 1H), 1.93 - 1.55 (m, 3H), 1.23 (t, *J* = 7.4 Hz, 3H)_{.} ¹⁹F NMR (377 MHz, DMSO) δ - 60.58, -131.11. |
| T070 | 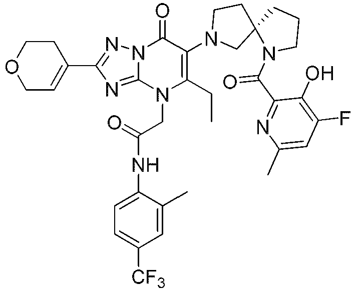 | 738.74 | ¹H NMR (400 MHz, DMSO-*d*6) δ 10.83 (s, 1H), 10.03 (s, 1H), 7.73 (d, *J* = 8.4 Hz, 1H),7.63 (d, *J* = 2.3 Hz, 1H), 7.54 (dd, *J* = 8.5, 2.2 Hz, 1H), 7.24 (d, *J* = 11.6 Hz, 1H), 6.89 -6.76 (m, 1H), 5.25 (s, 2H), 4.26 (t, *J* = 2.9 Hz, 2H), 4.04 (s, 1H), 3.82 (t, *J* = 5.4 Hz, 2H),3.51 - 3.39 (m, 2H), 3.33 (m, 2H), 3.03 (dt, *J* = 22.7, 12.8 Hz, 3H), 2.53 (t, *J =* 2.7 Hz,3H), 2.37 (d, *J =* 8.0 Hz, 6H), 2.31 - 2.22 (m, 1H), 2.05 (dt, *J* = 12.9, 7.0 Hz, 1H), 1.93 - 1.67 (m, 3H), 1.23 (t, *J =* 7.4 Hz, 3H).¹⁹F NMR (377 MHz, DMSO) δ -60.57, -125.83 (d, *J* = 11.5 Hz). |
| T071 | 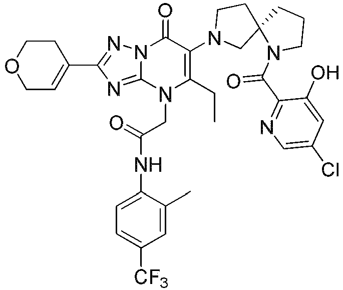 | 741.17 | ¹H NMR (400 MHz, DMSO-*d*6) δ 11.41 (s, 1H), 10.03 (s, 1H), 8.08 (d, *J* = 2.0Hz, 1H), 7.73 (d, *J* = 8.4 Hz, 1H), 7.63 (d, *J =* 2.2 Hz, 1H), 7.53 (dd, *J =* 8.6, 2.3Hz, 1H), 7.42 (d, *J* = 2.1 Hz, 1H), 6.83 (h,*J* = 1.4 Hz, 1H), 5.24 (s, 2H), 4.26 (q,J = 2.9 Hz, 2H), 3.81 (t, *J =* 5.4 Hz, 2H), 3.47 - 3.37 (m, 2H), 3.31 (m, 2H)3.14 -2.90 (m, 3H), 2.52 (d, *J* = 5.7 Hz, 3H), 2.36 (s, 3H), 2.29 (d, *J* = 7.5 Hz, 1H), 2.16- 1.93 (m, 2H), 1.90 - 1.67 (m, 3H), 1.31 - 1.22 (m, 3H). ¹⁹F NMR (377 MHz, DMSO) δ -60.58. |
| T072 | 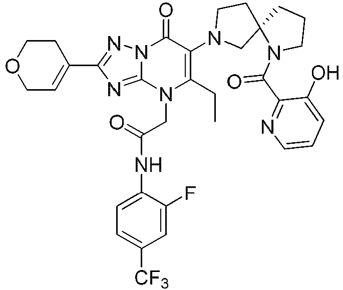 | 710.69 | ¹H NMR (400 MHz, DMSO*-d*6) δ 10.88 (s, 1H), 10.72 (s, 1H), 8.24 (t, *J* = 8.2 Hz, 1H), 8.04 (t, *J* = 2.9Hz, 1H), 7.86 - 7.75 (m, 1H), 7.57 (d, *J* = 8.7 Hz, 1H), 7.35 - 7.25 (m, 2H), 6.80 (s, 1H), 5.30 (s, 2H),4.25 (d, *J* = 2.8 Hz, 2H), 3.80 (t, *J* = 5.5 Hz, 2H), 3.45 (t, *J* = 6.3 Hz, 2H), 3.32 (m, 2H)3.15 - 2.8 5 (m,4H), 2.50 (s, 2H), 2.29 (s, 1H), 2.12 - 1.93 (m, 2H), 1.80 (ddt, *J* = 20.3, 12.9, 6.3 Hz, 3H), 1.19 (d, *J* =7.5 Hz, 3H).¹⁹F NMR (376 MHz, DMSO-d6) δ -60.59, -122.95. |
| T073 | 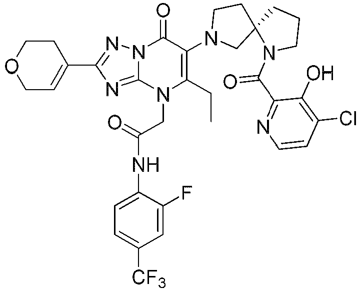 | 745.13 | ¹H NMR (400 MHz, DMSO-*d*6) δ 12.06 (s, 1H), 10.71 (s, 1H), 8.24 (t, *J* = 8.1 Hz, 1H),8.06 (d, *J* = 5.0 Hz, 1H), 7.80 (dd, *J* = 11.1, 2.1 Hz, 1H), 7.67 - 7.50 (m, 2H), 6.80 (dt, *J* =3.5, 1.7 Hz, 1H), 5.30 (s, 2H), 4.25 (q, *J* = 2.9 Hz, 2H), 3.80 (t, *J =* 5.4 Hz, 2H), 3.66 (t, *J* =6.7 Hz, 2H), 3.33 (m, 2H), 3.15 - 2.87 (m, 3H), 2.51 (s, 3H), 2.38 - 2.20 (m, 1H), 2.20 -1.91 (m, 2H), 1.93 - 1.66 (m, 3H), 1.20 (t, *J* = 7.4 Hz, 3H). ¹⁹F NMR (377 MHz, DMSO) δ -60.59, δ -122.94. |
| T074 | 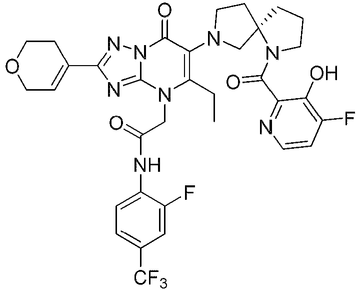 | 728.68 | ¹H NMR (400 MHz, DMSO-*d*6) δ 11.25 (s, 1H), 10.72 (s, 1H), 8.24 (t, *J* = 8.2 Hz, 1H), 8.07 (dd,*J* = 7.0, 5.2 Hz, 1H), 7.80 (dd,*J* = 11.2, 2.1 Hz, 1H), 7.57 (dd, *J*= 8.8, 2.0 Hz, 1H), 7.38 (dd, *J =* 10.9, 5.3 Hz, 1H), 6.81(t, *J* = 2.3 Hz, 1H), 5.30 (s, 2H), 4.25 (q, *J =* 2.9 Hz, 2H), 4.05 (s, 1H),3.81 (t, *J* = 5.5 Hz, 2H), 3.45 (t, *J* = 6.8 Hz, 3H), 3.31 - 2.67 (m, 5H), 2.52(s, 2H), 2.30 (t, *J* = 9.9 Hz, 1H), 2.06 (dt, *J* = 13.0, 6.3 Hz, 1H), 1.81(ddp, *J =* 20.0, 13.0, 6.3 Hz, 3H), 1.21 (t, *J =* 7.4 Hz, 3H)_{.} ¹⁹F NMR (377 MHz, DMSO-d6) δ - 60.60, -122.96 (t, *J* = 9.6Hz), - 125.60 (dd, *J* = 10.7, 7.0 Hz). |
| T075 | 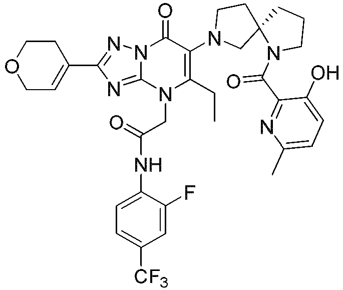 | 724.72 | ¹H NMR (400 MHz, DMSO-*d*6) δ 10.71 (s, 2H), 8.24 (t, *J =* 8.2 Hz, 1H), 7.81 (dd, *J* = 11.0, 2.1 Hz, 1H), 7.58 (d, *J* = 8.6 Hz, 1H), 7.27 - 7.10 (m, 2H), 6.81(s, 1H), 5.30 (s, 2H), 4.25 (d, *J =* 3.0 Hz, 2H), 3.81 (t, *J =* 5.4 Hz, 2H), 3.50 (d,*J* = 6.7 Hz, 2H),3.32 (m, 2H) 2.99 (dd, *J =* 45.9, 9.0 Hz, 4H), 2.53 (s, 2H),2.37 (s, 3H), 2.31 (d, *J* = 15.5 Hz, 1H), 2.02 (ddt, *J* = 21.1, 13.1, 6.7 Hz, 2H),1.80 (ddt, *J* = 23.2, 15.7, 7.0 Hz, 3H), 1.20 (d, *J* = 7.4 Hz, 3H). ¹⁹F NMR (377 MHz, DMSO) δ -60.59, δ -122.94. |
| T076 | 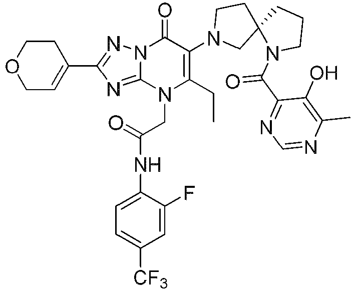 | 724.72 | ¹H NMR (400 MHz, DMSO-*d*6) δ 10.92 (s, 1H), 10.71 (s, 1H), 8.58 (s,1H), 8.24 (t, *J* = 8.2 Hz, 1H), 7.80 (dd, *J* = 11.0, 2.1 Hz, 1H), 7.61 - 7.5 3 (m, 1H), 6.81 (dq, *J* = 3.1, 1.5 Hz, 1H), 5.30 (s, 2H), 4.25 (q, *J* = 2.9 Hz, 2H),4.05 (s, 1H), 3.81 (t, *J* = 5.4 Hz, 2H), 3.56 (t, *J* = 6.7 Hz, 3H), 3.02 (ddt, *J* = 23.3, 16.5, 8.1 Hz, 5H), 2.51 (s, 2H), 2.44 (s, 3H), 2.34 - 2.26 (m, 1H), 2.07(dt, *J =* 12.8, 7.0 Hz, 1H), 1.81 (ddd, *J* = 27.7, 12.8, 6.1 Hz, 3H), 1.20 (t, *J* =7.4 Hz, 3H)_{.} ¹⁹F NMR (377 MHz, DMSO-*d*6) δ - 60.60, -122.75 - -123.11 (m). |
| T077 | 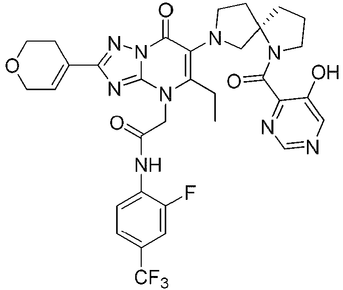 | 711.68 | ¹H NMR (400 MHz, DMSO-*d*6) δ 10.72 (s, 1H), 8.66 (s, 1H), 8.46 (s, 1H), 8.24 (t, *J* = 8.2 Hz, 1H), 7.81 (dd, *J* = 11.1, 2.1 Hz, 1H), 7.57 (dd, *J* = 8.7, 2.1 Hz, 1H), 6.81 (t, *J* = 2.1 Hz, 1H), 5.30 (s, 2H), 4.25 (q, *J* = 3.0 Hz, 2H), 3.80 (t, *J =* 5.4 Hz, 2H), 3.28 (td, *J =* 6.6, 6.1, 2.4 Hz, 5H), 3.09 - 2.87 (m, 3H), 2.51 (d, *J=* 1.9 Hz, 3H), 2.32 (dt, *J* = 14.9, 9.5 Hz, 1H), 2.18 - 1.96 (m, 1H), 1.93 - 1.65 (m, 3H), 1.20 (t, *J =* 7.5Hz, 3H).¹⁹F NMR (377 MHz, DMSO-*d*6) δ -60.60, -122.75 - -123.11 (m). |
| T078 | 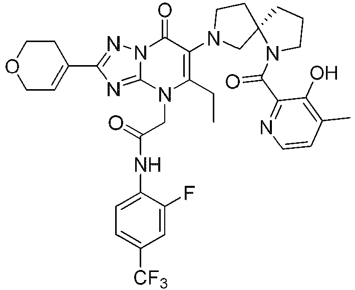 | 724.72 | ¹H NMR (400 MHz, DMSO-*d*6) δ 12.04 (s, 1H), 10.72 (s, 1H), 8.24 (t, *J* = 8.2 Hz, 1H), 8.00 (d, *J* = 4.5 Hz, 1H), 7.80 (dd, *J* = 11.1, 2.2 Hz, 1H), 7.57 (d, *J =* 8.6 Hz, 1H), 7.30 (d, *J* = 4.5 Hz, 1H), 6.89 - 6.73 (m, 1H), 5.30 (s, 2H), 4.25 (q, *J* = 2.9 Hz, 2H), 3.88 (t, *J =* 6.8 Hz, 2H), 3.81 (t, *J* = 5.4 Hz, 2H),3.32 (m, 2H)3.01 (s, 4H), 2.52 (d, *J* = 3.2 Hz, 3H), 2.30 (d, *J =* 27.7 Hz, 1H), 2.22 (s, 3H), 2.03 (ddt, *J*= 22.5, 16.1, 7.3 Hz, 1H), 1.83 (ddt, *J* = 33.1, 12.9, 6.3 Hz, 3H), 1.20 (d, *J* = 7.7 Hz, 3H). ¹⁹F NMR (377 MHz, DMSO-*d*6) δ - 60.60, -122.75 - -123.11 (m). |
| T079 | 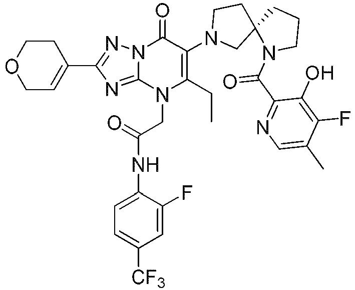 | 742.71 | ¹H NMR (400 MHz, DMSO-*d*6) δ 11.49 (s, 1H), 10.72 (s, 1H), 8.24 (t, *J* = 8.2 Hz, 1H), 7.98 (d, *J* = 8.5 Hz, 1H), 7.80 (dd, *J =* 11.1, 2.1 Hz, 1H), 7.57 (dd, *J* = 8.7, 2.0 Hz, 1H), 6.80 (t, *J* = 2.2 Hz, 1H), 5.29 (s, 2H), 4.25 (q, *J =* 2.9 Hz, 2H), 3.80 (t, *J =* 5.4 Hz, 2H), 3.56 (t, *J* = 6.7 Hz, 2H), 3.32(m, 2H), 3.11 - 2.88 (m, 3H), 2.51 (s, 4H), 2.35 - 2.17 (m, 4H), 2.03 (ddd, *J* = 26.3, 14.3, 7.0 Hz,1H), 1.79 (ddd, *J =* 28.6, 12.7, 6.3 Hz, 3H), 1.20 (t, *J* = 7.5 Hz, 3H)_{.} ¹⁹F NMR (376 MHz, DMSO) δ -60.59, -122.97, - 131.05. |
| T080 | 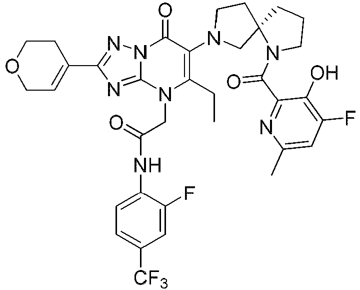 | 742.71 | ¹H NMR (400 MHz, DMSO-*d*6) δ 10.83 (s, 1H), 10.71 (s, 1H), 8.24 (t, *J* = 8.2 Hz, 1H), 7.81 (dd, *J* =11.1, 2.1 Hz, 1H), 7.57 (d,*J* = 8.7 Hz, 1H), 7.25 (d, *J =* 11.6 Hz, 1H), 6.81 (s, 1H), 5.30 (s, 2H), 4.25 (d, *J* = 3.1 Hz, 2H), 4.04 (s, 1H), 3.81 (t, *J =* 5.5 Hz, 2H), 3.55 - 3.40 (m, 2H), 3.33 (m, 2H), 3.10 - 2.87 (m,3H), 2.52 (s, 3H), 2.38 (s, 3H), 2.31 (d, *J* = 12.1 Hz, 1H), 2.05 (dt, *J* = 13.3, 7.0 Hz, 1H), 1.81 (ddt, *J* =21.4*,* 14.5, 7.2 Hz, 3H), 1.20 (t, *J* = 7.4 Hz, 3H)_{.} ¹⁹F NMR (376 MHz, DMSO) δ - 60.59, -122.97, -131.05. |
| T081 | 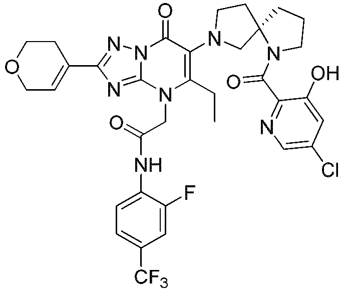 | 745.13 | ¹H NMR (400 MHz, DMSO-*d*6) δ 10.72 (s, 1H), 8.24 (t, *J* = 8.2 Hz, 1H),8.07 (d, *J =* 2.1 Hz, 1H), 7.81 (dd, *J* = 11.1, 2.1 Hz, 1H), 7.57 (dd, *J* = 8.7,2.0 Hz, 1H), 7.41 (d, *J* = 2.1 Hz, 1H), 6.81 (t, *J* = 2.2 Hz, 1H), 5.30 (s, 2H),4.25 (q, *J* = 2.9 Hz, 2H), 3.81 (t, *J* = 5.4 Hz, 2H), 3.51 - 3.38 (m, 4H), 3.11- 2.89 (m, 3H), 2.51 (d, *J =* 1.9 Hz, 4H), 2.40 - 2.21 (m, 1H), 2.02 (ddt, *J* =24.8, 13.8, 6.9 Hz, 1H), 1.80 (ddq, *J* = 26.8, 12.6, 6.8, 6.3 Hz, 3H), 1.20 (t, *J* = 7.4 Hz, 3H).¹⁹F NMR (376 MHz, DMSO) δ - 60.59, -122.96. |
| T082 | 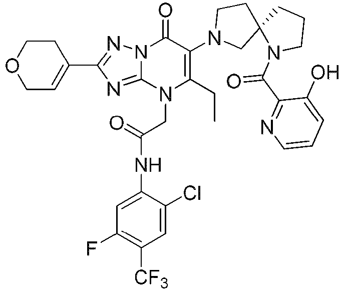 | 745.13 | ¹H NMR (400 MHz, DMSO-d6) δ 10.93 (s, 1H), 10.51 (s, 1H), 8.13 (d, J = 12.8 Hz,1H), 8.10 - 7.92 (m, 2H), 7.38 - 7.25 (m, 2H), 6.86 - 6.75 (m, 1H), 5.35 (s, 2H), 4.26 (q, J = 2.9 Hz, 2H), 4.06 (s, 1H), 3.81 (t, J = 5.5 Hz, 2H), 3.46 (d, J = 6.8 Hz, 4H), 3.13 -2.87 (m, 3H), 2.52 (s, 3H), 2.37 - 2.23 (m, 1H), 2.05 (dt, J = 13.2, 7.1 Hz, 1H), 1.79(ddt, J = 27.8, 13.5, 7.0 Hz, 3H), 1.21 (t, J = 7.6 Hz, 3H). ¹⁹F NMR (377 MHz, DMSO) δ -59.64 (d, *J* = 12.0 Hz), -115.23 - -115.40 (m). |
| T083 | 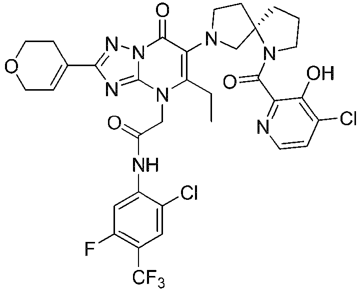 | 779.57 | ¹H NMR (400 MHz, DMSO*-d*6) δ 12.03 (s, 1H), 10.51 (s, 1H), 8.12 (d, *J* = 12.8 Hz, 1H), 8.05 (dd, *J* =13.3, 6.2 Hz, 2H), 7.61 (d,*J* = 5.0 Hz, 1H), 6.82 (s, 1H), 5.36 (s, 2H), 4.26 (q, *J =* 2.9 Hz, 2H), 4.05 (s, 1H), 3.81 (t, *J =* 5.4 Hz, 2H), 3.66 (t, *J* = 6.7 Hz, 2H), 3.12 - 2.90 (m, 3H), 2.53 (d, *J =* 1.8 Hz, 3H), 2.32- 2.20 (m, 1H), 2.07 (d, *J* = 9.2 Hz, 1H), 1.83 (ddq, *J* = 29.8, 12.1, 6.7, 5.7 Hz, 3H), 1.21 (t, *J* = 7.6 Hz,3H).¹⁹F NMR (377 MHz, DMSO) δ -59.64 (d, *J =* 12.0 Hz), -115.23 - -115.40 (m). |
| T084 | 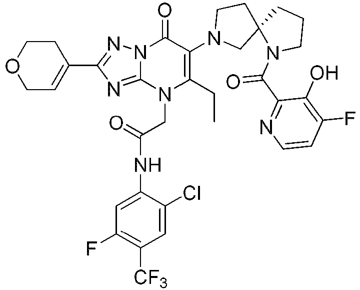 | 763.12 | ¹H NMR (400 MHz, DMSO-*d*6) δ 11.11 (s, 1H), 10.51 (s, 1H), 8.12 (d, *J* = 12.8 Hz, 1H), 8.06 (dd,*J* = 7.0, 5.2 Hz, 1H), 8.03 (d, *J* = 7.3 Hz, 1H), 7.38 (dd, *J* = 10.8, 5.3 Hz, 1H), 6.82 (s, 1H), 5.36 (s, 2H), 4.25 (q, *J =* 3.0 Hz, 2H), 4.17 - 3.90 (m, 1H), 3.81 (t, *J* = 5.4 Hz, 2H), 3.44 (s, 4H), 3.11 - 2.86 (m, 3H), 2.52 (s,3H), 2.39 - 2.21 (m, 1H), 2.06 (dt, *J* = 13.1, 7.0 Hz, 1H), 1.80 (dh, *J* = 20.4, 6.1Hz, 3H), 1.21 (t, *J* = 7.5 Hz, 3H).¹⁹F NMR (377 MHz, DMSO) δ -59.64 (d, *J =* 12.0 Hz), -115.33 (qd, *J =* 12.3, 7.3 Hz), -125.54 (dd, *J =* 10.9, 7.0 Hz). |
| T085 | 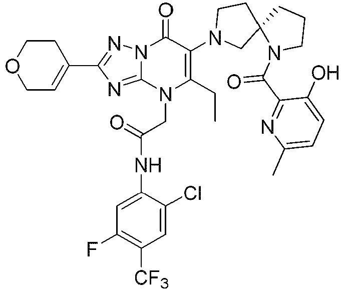 | 759.16 | ¹H NMR (400 MHz, DMSO-d6) δ 10.68 (s, 1H), 10.50 (s, 1H), 8.12 (d, J = 12.8 Hz, 1H), 8.02(d, J = 7.3 Hz, 1H), 7.26 - 7.08 (m, 2H), 6.87 - 6.75 (m, 1H), 5.35 (s, 2H), 4.25 (q, J = 2.9 Hz,2H), 4.04 (s, 1H), 3.81 (t, J *=* 5.4 Hz, 2H), 3.48 (td, J = 6.7, 6.2, 2.6 Hz, 2H), 3.33 (m, 2H), 3.10- 2.91 (m, 3H), 2.53 (d, J = 1.8 Hz, 3H), 2.37 (s, 3H), 2.33 - 2.23 (m, 1H), 2.04 (dd, J = 12.9,6.9 Hz, 1H), 1.80 (ddd, J = 28.1, 12.9, 6.3 Hz, 3H), 1.21 (t, J = 7.5 Hz, 3H). ¹⁹F NMR (377 MHz, DMSO) δ -59.63 (d, *J* = 12.3 Hz), -115.35, -115.40. |
| T086 | 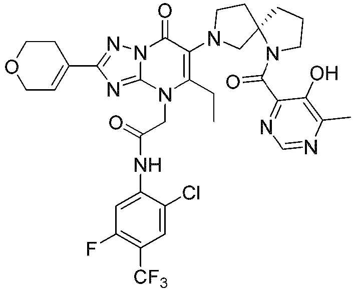 | 760.15 | ¹H NMR (400 MHz, DMSO-*d*6) δ 10.89 (s, 1H), 10.52 (s, 1H), 8.57 (s, 1H), 8.12 (d, *J =* 12.8 Hz, 1H), 8.03 (d, *J* = 7.3 Hz, 1H), 6.82 (tt, *J* = 3.0, 1.5 Hz, 1H), 5.36 (s, 2H), 4.25 (t, *J* =3.0 Hz, 2H), 4.05 (s, 1H), 3.81 (t, *J =* 5.4 Hz, 2H), 3.55 (t, *J* = 6.7 Hz, 2H), 3.23 (m, 2H), 2.99(dq, *J* = 31.7, 7.5, 6.6 Hz, 3H), 2.51 (s, 3H), 2.44 (s, 3H), 2.29 (d, *J* = 5.2 Hz, 1H), 2.15 - 2.02 (m, 1H), 1.81 (ddd, *J =* 26.9, 12.3, 6.0 Hz, 3H), 1.26 - 1.13 (m, 3H)_{.} ¹⁹F NMR (377 MHz, DMSO) δ -59.63 (d, *J* = 12.3 Hz), -115.35, -115.40. |
| T087 | 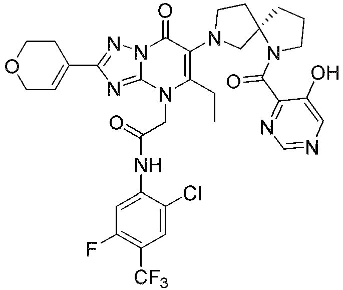 | 746.12 | ¹H NMR (400 MHz, DMSO-d6) δ 10.98 (s, 1H), 10.56 (s, 1H), 8.74 (s, 1H), 8.54 (s, 1H), 8.18 (d,J = 12.7 Hz, 1H), 8.09 (d, J = 7.3 Hz, 1H), 6.88 (h, J = 1.5 Hz, 1H), 5.42 (s, 2H), 4.31 (q, J = 2.9Hz, 2H), 4.07 (s, 1H), 3.87 (t, J = 5.4 Hz, 2H), 3.38 - 3.22 (m, 4H), 3.22 - 2.97 (m, 3H), 2.58 (s,3H), 2.43 - 2.30(m, 1H), 2.13 (dq, J *=* 13.0, 6.6 Hz, 1H), 1.97 - 1.74 (m, 3H), 1.27 (t, J = 7.4 Hz,3H). ¹⁹F NMR (377 MHz, DMSO) δ -59.63 (d, *J* = 12.3 Hz), -115.35, -115.40. |
| T088 | 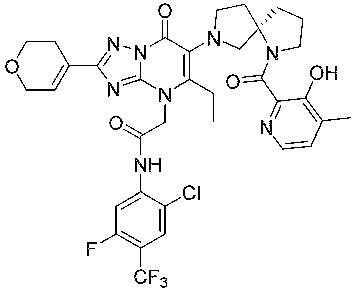 | 759.16 | ¹H NMR (400 MHz, DMSO-*d*6) δ 12.02 (s, 1H), 10.50 (s, 1H), 8.12 (d, *J* = 12.8 Hz, 1H), 8.01 (dd, *J* =10.1, 5.9 Hz, 2H), 7.30 (d, *J =* 4.5 Hz, 1H), 6.89 - 6.73 (m, 1H), 5.35 (s, 2H), 4.25 (d, *J* = 3.2 Hz, 2H),4.05 (s, 1H), 3.84 (dt, *J =* 24.0, 6.2 Hz, 4H), 3.33 (m, 2H), 3.00 (d, *J* = 7.6 Hz, 3H), 2.53 (s, 3H), 2.26 (s, 1H), 2.22 (s, 3H), 2.07 (q, *J =* 6.4 Hz, 1H), 1.83 (ddt, *J* = 34.2, 13.8, 6.8 Hz, 3H), 1.21 (t, *J* = 7.5 Hz, 3H).¹⁹F NMR (377 MHz, DMSO) δ -59.63 (d, *J =* 12.3 Hz), -115.35, -115.40. |
| T089 | 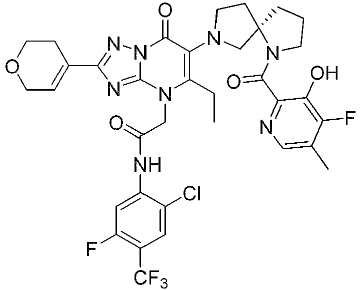 | 777.15 | ¹H NMR (400 MHz, DMSO-*d*6) δ 11.47 (s, 1H), 10.51 (s, 1H), 8.12 (d, *J =* 12.8 Hz, 1H), 8.00 (dd, *J* =15.4, 7.9 Hz, 2H), 6.87 - 6.75 (m, 1H), 5.35 (s, 2H), 4.25 (q, *J =* 2.9 Hz, 2H), 4.04 (m, 1H), 3.81 (t, *J* =5.5 Hz, 2H), 3.57 (t, *J =* 6.8 Hz, 2H), 3.33 (m, 2H), 3.14 - 2.88 (m, 3H), 2.52 (d, *J =* 1.7 Hz, 3H), 2.34 -2.17 (m, 4H), 2.05 (dt, *J =* 12.8, 6.9 Hz, 1H), 1.81 (ddd, *J* = 29.5, 13.2, 6.7 Hz, 3H), 1.21 (t, *J* = 7.5 Hz,3H). ¹⁹F NMR (377 MHz, DMSO) δ -59.62 (d, *J* = 12.1 Hz), -115.38, -131.13 (d, *J* = 8.8 Hz). |
| T090 | 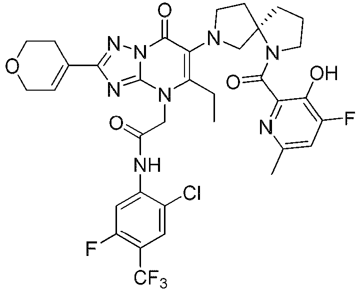 | 777.15 | ¹H NMR (400 MHz, DMSO*-d*6) δ 10.83 (s, 1H), 10.50 (s, 1H), 8.13 (d, *J* = 12.8 Hz, 1H), 8.02 (d, *J* =7.3 Hz, 1H), 7.25 (d, *J* = 11.6 Hz, 1H), 6.82 (dt, *J =* 3.2, 1.6 Hz, 1H), 5.36 (s, 2H), 4.25 (q, *J* = 2.8 Hz,2H), 4.03 (s, 1H), 3.81 (t,*J* = 5.4 Hz, 2H), 3.45 (t, *J* = 6.2 Hz, 2H), 3.33 (m, 2H), 3.14 - 2.90 (m, 3H),2.51 (s, 3H), 2.38 (s, 3H), 2.33 - 2.19 (m, 1H), 2.06 (dt, *J* = 13.0, 6.9 Hz, 1H), 1.81 (ddt, *J =* 19.9, 12.8,6.1 Hz, 3H), 1.21 (t, *J* = 7.4 Hz, 3H).¹⁹F NMR (377 MHz, DMSO) δ -59.62 (d, *J =* 12.1 Hz), -115.38, -125.81 (d, *J* = 8.8 Hz). |
| T091 | 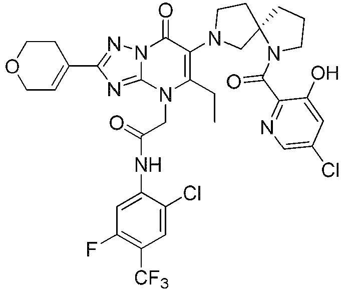 | 779.57 | ¹H NMR (400 MHz, DMSO-*d*6) δ 11.33 (s, 1H), 10.51 (s, 1H), 8.17 - 8.06(m, 2H), 8.03 (d, *J =* 7.3 Hz, 1H), 7.42 (d, *J* = 2.1 Hz, 1H), 6.82 (t, *J* = 2.1Hz, 1H), 5.36 (s, 2H), 4.25 (t, *J* = 3.0 Hz, 2H), 4.02 (s, 1H), 3.81 (t, *J =* 5.5Hz, 2H), 3.39 (d, *J* = 7.6 Hz, 4H), 3.11 - 2.98 (m, 2H), 2.93 (q, *J =* 6.3, 5.1Hz, 1H), 2.51 (s, 3H), 2.35 - 2.24 (m, 1H), 2.09 - 2.00 (m, 1H), 1.79 (ddd,*J* = 20.4, 9.6, 5.4 Hz, 3H), 1.20 (t, *J* = 7.5 Hz, 3H). ¹⁹F NMR (377 MHz, DMSO) δ -59.63 (d, *J* = 12.3 Hz), -115.35, -115.40. |
| T092 | 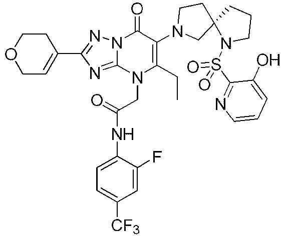 | 746.73 | ¹H NMR (400 MHz, DMSO) δ 10.89 (s, 1H), 10.69 (s, 1H), 8.22 (t, *J* = 8.1 Hz, 1H), 8.02 (t, *J* = 2.9 Hz, 1H), 7.80 (dd, *J* = 11.1, 2.1 Hz, 1H), 7.59 - 7.53 (m, 1H), 7.43 (d, J = 2.9 Hz, 2H), 6.79 (dp, *J =* 2.9, 1.5 Hz, 1H), 5.25 (s, 2H), 4.24 (q, *J =* 2.8 Hz, 2H), 3.79 (t, *J* = 5.4 Hz, 2H), 3.71 - 3.55 (m, 3H), 3.11 (s, 2H), 2.95 - 2.78 (m, 3H), 2.65 - 2.52 (m, 2H), 2.51 (s, 2H), 2.13 (dt, *J* = 12.1, 7.2 Hz, 1H), 2.01 - 1.87 (m, 2H), 1.80 - 1.70 (m, 1H), 1.12 (t, *J =* 7.4 Hz, 3H). |
| | | | ¹⁹F NMR (377 MHz, DMSO) δ - 60.60, -122.94 (t, *J =* 9.6 Hz). |
| T093 | 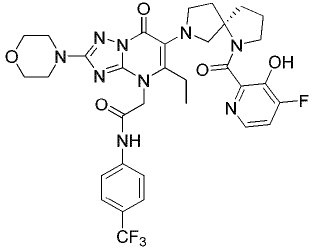 | 713.69 | ¹H NMR (400 MHz, DMSO) δ 11.24 (s, 1H), 10.86 (s, 1H), 8.07 (dd, *J* = 7.0, 5.3 Hz, 1H), 7.78 (d, *J* = 8.6 Hz, 2H), 7.71 (d, *J =* 8.7 Hz, 2H), 7.38 (dd, *J* = 10.9, 5.3 Hz, 1H), 5.10 (s, 2H), 3.69 - 3.62 (m, 4H), 3.45 (d, *J* = 6.5 Hz, 4H), 3.35 (s, 6H), 3.06 (t, *J* = 9.6 Hz, 1H), 3.02 - 2.86 (m, 2H), 2.04 (dt, *J =* 13.0, 7.0 Hz, 1H), 1.83 (dt, *J =* 12.3, 6.2 Hz, 1H), 1.75 (dd, *J =* 13.4, 7.2 Hz, 2H), 1.18 (t, *J =* 7.4 Hz, 3H). |
| T094 | 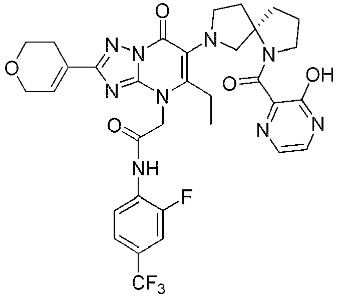 | 711.25 | ¹H NMR (400 MHz, DMSO-d6) δ 12.64 (s, 1H), 10.71 (s, 1H), 8.23 (q, J = 8.5 Hz, 1H), 7.80 (dd, J = 11.1, 2.1 Hz, 1H), 7.63 - 7.42 (m, 2H), 7.32 (s, 1H), 6.84 - 6.76 (m, 1H), 5.29 (s, 2H), 4.25 (q, J = 3.0 Hz, 2H), 3.97 (s, 1H), 3.80 (t, J = 5.5 Hz, 2H), 3.15 - 2.61 (m, 5H), 2.38- 2.12 (m, 2H), 2.04 (dt, J = 13.4, 7.1 Hz, 1H), 1.79 (ddt, J = 26.0, 12.8, 6.5 Hz, 3H), 1.19 (t, J = 7.4 Hz, 3H).¹⁹F NMR (377 MHz, DMSO-d6) δ -60.59, -122.98 (d, J = 10.3 Hz). |
| T095 | 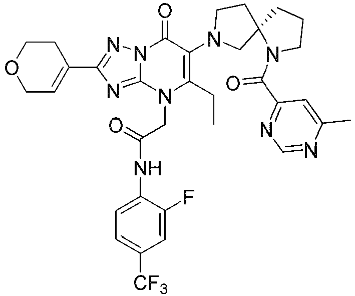 | 709.27 | ¹H NMR (400 MHz, DMSO-d6) δ 10.71 (s, 1H), 9.07 (d, J = 1.3 Hz, 1H), 8.23 (t, J = 8.2 Hz, 1H), 7.80 (dd, J *=* 11.0, 2.1 Hz, 1H), 7.62 7.52 (m, 2H), 6.84 - 6.76 (m, 1H), 5.29 (s, 2H), 4.25 (q, J = 2.9 Hz, 2H), 4.00 (s, 1H), 3.80 (t, J = 5.4 Hz, 2H), 3.68 - 3.37 (m, 3H), 3.30 2.60 (m, 6H), 2.53 (s, 3H), 2.06 (dt, J = 13.0, 7.4 Hz, 1H), 1.80 (ddt, J = 21.2, 14.4, 6.5 Hz, 3H), 1.20 (t, J = 7.4 Hz, 3H). |
| | | | ¹⁹F NMR (377 MHz, DMSO-d6) δ -60.59, -122.09 - -123.76 (m). |
| T096 | 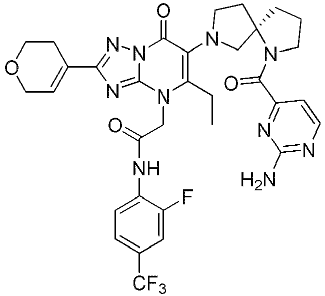 | 710.27 | ¹H NMR (400 MHz, DMSO-d6) δ 10.71 (s, 1H), 8.35 (d, J = 4.9 Hz, 1H), 8.24 (t, J = 8.1 Hz, 1H), 7.80 (dd, J = 11.0, 2.1 Hz, 1H), 7.60 - 7.53 (m, 1H), 6.83 (d, J = 21.1 Hz, 3H), 6.62 (d, J = 4.9 Hz, 1H), 5.30 (s, 2H), 4.25 (q, J = 2.9 Hz, 2H), 4.10 - 3.86 (m, 1H), 3.80 (t, J = 5.5 Hz, 2H), 3.47 - 3.40 (m, 2H), 3.24 2.71 (m, 5H), 2.28 (s, 1H), 2.04 (dt, J = 13.1, 6.9 Hz, 1H), 1.78 (ddt, J = 26.3, 12.7, 6.6 Hz, 3H), 1.19 (t, J = 7.4 Hz, 3H) |
| | | | ¹⁹F NMR (377 MHz, DMSO-d6) δ -60.60, -122.95 (t, J = 9.1 Hz). |
| T097 | 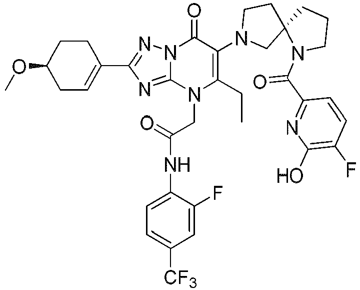 | 738.74 | ¹H NMR (400 MHz, DMSO) δ 10.90 (s, 1H), 8.09 - 8.01 (m, 1H), 7.79 (d, J = 8.6 Hz, 2H), 7.71 (d, J = 8.6 Hz, 2H), 7.37 (dd, J = 10.9, 5.3 Hz, 1H), 6.69 (d, J = 4.4 Hz, 1H), 5.18 (s, 2H), 3.56 - 3.40 (m, 3H), 3.27 (s, 3H), 3.11 - 3.03 (m, 1H), 3.03 - 2.92 (m, 2H), 2.41 (s, 2H), 2.29 (s, 1H), 2.15 (d, J = 6.4 Hz, 1H), 1.96 (dd, J = 14.5, 6.6 Hz, 1H), 1.82 (td, J *=* 12.5, 6.5 Hz, 2H), 1.76 (s, 1H), 1.66 (h, J = 8.0 Hz, 1H), 1.21 (dd, J = 14.8, 7.5 Hz, 4H). ¹⁹F NMR (377 MHz, DMSO) δ -60.38, -125.76. |
| T098 | 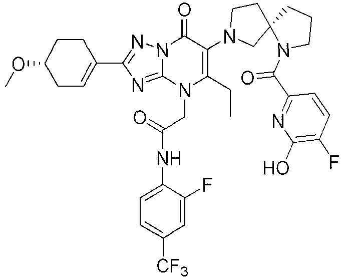 | 738.74 | ¹H NMR (400 MHz, DMSO) δ 10.90 (s, 1H), 8.03 (s, 1H), 7.79 (d, J = 8.5 Hz, 2H), 7.71 (d, J = 8.6 Hz, 2H), 7.36 (dd, J = 10.9, 5.3 Hz, 1H), 6.68 (d, J = 4.2 Hz, 1H), 5.18 (s, 2H), 3.53 - 3.40 (m, 3H), 3.27 (s, 3H), 3.11 - 3.00 (m, 2H), 2.40 (s, 1H), 2.29 (s, 1H), 2.18 - 1.99 (m, 2H), 1.99 - 1.91 (m, 1H), 1.88 - 1.76 (m, 2H), 1.75 (s, 1H), 1.65 (dt, J *=* 14.0, 7.0 Hz, 1H), 1.21 (dd, J = 14.9, 7.6 Hz, 4H). ¹⁹F NMR (377 MHz, DMSO) δ -60.38, - 125.67. |
| T099 | 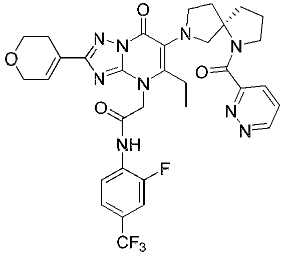 | 695.26 | ¹H NMR (400 MHz, DMSO-d6) δ 10.74 - 10.67 (m, 1H), 9.30 (dd, J = 4.9, 1.8 Hz, 1H), 8.24 (t, J = 8.2 Hz, 1H), 7.91 (dd, J = 8.5, 1.8 Hz, 1H), 7.87 - 7.78 (m, 2H), 7.57 (dd, J = 8.7, 2.1 Hz, 1H), 6.84 - 6.77 (m, 1H), 5.31 (s, 2H), 4.25 (q, J = 2.9 Hz, 2H), 4.08 (s, 1H), 3.81 (t, J = 5.4 Hz, 2H), 3.61 - 3.47 (m, 2H), 3.33 - 2.59 (m, 6H), 2.53 (s, 2H), 2.35 (s, 1H), 2.11 (dt, J = 12.8, 6.8 Hz, 1H), 1.82 (dh, J *=* 19.7, 6.0 Hz, 3H), 1.21 (t, J = 7.4 Hz, 3H). |
| | | | ¹⁹F NMR (377 MHz, DMSO-d6) δ -60.61, -122.95 (t, J = 9.6 Hz). |
| T100 | 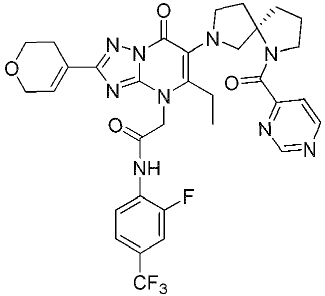 | 695.26 | ¹H NMR (400 MHz, DMSO-d6) δ 10.74 - 10.68 (m, 1H), 9.24 (d, J = 1.4 Hz, 1H), 8.98 (d, J = 5.0 Hz, 1H), 8.24 (t, J = 8.2 Hz, 1H), 7.80 (dd, J = 11.1, 2.1 Hz, 1H), 7.71 (dd, J = 5.1, 1.5 Hz, 1H), 7.61 - 7.54 (m, 1H), 6.84 - 6.78 (m, 1H), 5.30 (s, 2H), 4.25 (q, J = 2.8 Hz, 2H), 4.02 (s, 1H), 3.81 (t, J = 5.5 Hz, 2H), 3.49 (q, J = 6.4, 6.0 Hz, 2H), 3.31 - 2.60 (m, 6H), 2.37 - 2.25 (m, 1H), 2.08 (dt, J *=* 13.1, 7.0 Hz, 1H), 1.82 (pd, J = 12.4, 5.8 Hz, 3H), 1.20 (t, J = 7.4 Hz, 3H). |
| | | | ¹⁹F NMR (377 MHz, DMSO-d6) δ -60.61, -122.87 - -123.04 (m). |
| T101 | 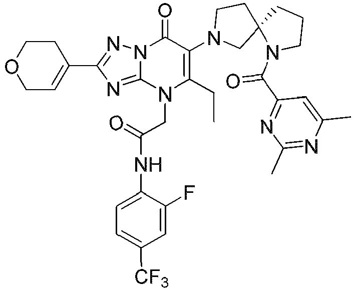 | 723.29 | ¹H NMR (400 MHz, DMSO-d6) δ 10.71 (s, 1H), 8.24 (t, J = 8.2 Hz, 1H), 7.80 (dd, J = 11.1, 2.1 Hz, 1H), 7.57 (dd, J = 8.6, 2.0 Hz, 1H), 7.34 (s, 1H), 6.84 - 6.77 (m, 1H), 5.30 (s, 2H), 4.25 (q, J = 2.9 Hz, 2H), 4.00 (s, 1H), 3.81 (t, J = 5.4 Hz, 2H), 3.50 - 3.41 (m, 3H), 3.27 - 2.73 (m, 5H), 2.60 (s, 3H), 2.47 (s, 3H), 2.31 (s, 1H), 2.06 (dt, J = 12.8, 6.9 Hz, 1H), 1.78 (ddt, J = 20.2, 13.5, 6.9 Hz, 3H), 1.20 (t, J = 7.4 Hz, 3H). |
| | | | ¹⁹F NMR (377 MHz, DMSO-d6) δ -60.61, -122.94 (t, J = 9.3 Hz) |
| T102 | 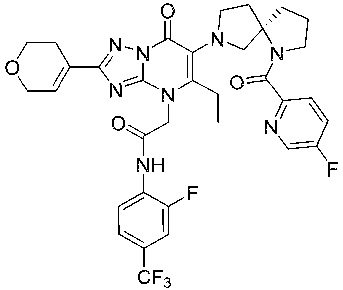 | 712.25 | ¹H NMR (400 MHz, DMSO-d6) δ 10.72 (s, 1H), 8.57 (d, J = 2.8 Hz, 1H), 8.24 (t, J = 8.2 Hz, 1H), 7.91 - 7.72 (m, 3H), 7.57 (dd, J = 8.6, 2.1 Hz, 1H), 6.81 (t, J = 2.2 Hz, 1H), 5.30 (s, 2H), 4.25 (q, J = 2.9 Hz, 2H), 4.05 (s, 1H), 3.81 (t, J = 5.5 Hz, 2H), 3.68 - 3.43 (m, 3H), 3.24-2.64 (m, 5H), 2.38 -2.23 (m, 1H), 2.06 (dt, J *=* 12.8, 6.8 Hz, 1H), 1.88 - 1.66 (m, 3H), 1.21 (t, J = 7.4 Hz, 3H). |
| | | | ¹⁹F NMR (377 MHz, DMSO-d6) δ -60.62, -122.96 (t, J = 9.3 Hz), - 124.95 (dd, J = 8.8, 4.6 Hz). |
| T103 | 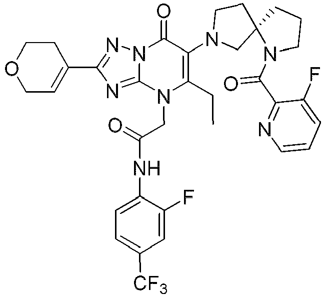 | 712.25 | ¹H NMR (400 MHz, DMSO-d6) δ 10.72 (d, J = 1.6 Hz, 1H), 8.45 (dt, J = 4.6, 1.6 Hz, 1H), 8.25 (t, J = 8.2 Hz, 1H), 7.94 - 7.84 (m, 1H), 7.80 (dd, J = 11.1, 2.1 Hz, 1H), 7.65 - 7.53 (m, 2H), 6.81 (dq, J = 3.0, 1.4 Hz, 1H), 5.31 (s, 2H), 4.25 (q, J = 2.8 Hz, 2H), 4.03 (s, 1H), 3.81 (t, J = 5.4 Hz, 2H), 3.27 (tq, J *=* 6.6, 3.6 Hz, 3H), 3.09 - 2.74 (m, 4H), 2.54 (s, 2H), 2.33 (d, J *=* 11.9 Hz, 1H), 2.09 (dq, J *=* 12.9, 6.5 Hz, 1H), 1.88 - 1.71 (m, 3H), 1.21 (t, J *=* 7.4 Hz, 3H). |
| | | | ¹⁹F NMR (377 MHz, DMSO-d6) δ -60.62, -122.92 - -123.02 (m), - 126.52 - -126.62 (m). |
| T104 | 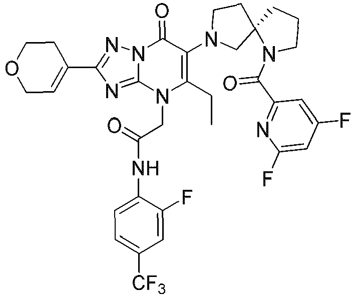 | 730.25 | ¹H NMR (400 MHz, DMSO-d6) δ 10.71 (d, J = 1.6 Hz, 1H), 8.54 (d, J = 2.3 Hz, 1H), 8.24 (t, J = 8.2 Hz, 1H), 8.11 (td, J = 9.5, 2.3 Hz, 1H), 7.80 (dd, J = 11.1, 2.1 Hz, 1H), 7.57 (dd,J = 8.7, 2.1 Hz, 1H), 6.81 (td, J = 3.0, 1.5 Hz, 1H), 5.30 (s, 2H), 4.25 (q, J = 2.9 Hz, 2H), 4.01 (s, 1H), 3.81 (t, J = 5.4 Hz, 2H), 3.29 (td, J = 6.6, 3.1 Hz, 3H), 3.13 - 2.61 (m, 5H), 2.33 (d, J = 12.6 Hz, 1H), 2.08 (dt, J *=* 12.8, 7.1 Hz, 1H), 1.80 (ddd, J = 16.5, 12.9, 6.4 Hz, 3H), 1.20 (t, J = 7.4 Hz, 3H).1.21 (t, J = 7.4 Hz, 3H). ¹⁹F NMR (377 MHz, DMSO-d6) δ -60.62, -122.92 - -123.02 (m), - 126.52 - -126.62 (m). |
| T105 | 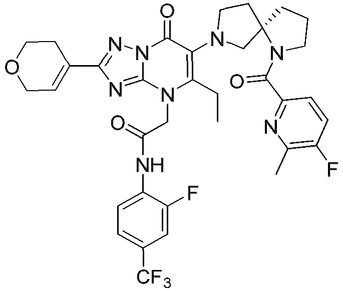 | 726.27 | ¹H NMR (400 MHz, DMSO-d6) δ 10.71 (s, 1H), 8.24 (t, J = 8.2 Hz, 1H), 7.83 - 7.70 (m, 2H), 7.60 - 7.52 (m, 2H), 6.81 (s, 1H), 5.30 (s, 2H), 4.25 (q, J = 2.8 Hz, 2H), 4.04 (s, 1H), 3.81 (t, J = 5.5 Hz, 2H), 3.52 (qd, J = 10.9, 10.5, 4.8 Hz, 2H), 3.16- 2.61 (m, 5H), 2.47 (d, J = 2.9 Hz, 3H), 2.30 (s, 1H), 2.06 (dt, J = 12.9, 7.0 Hz, 1H), 1.78 (ddd, J = 20.7, 9.9, 5.9 Hz, 3H), 1.20 (t, J *=* 7.4 Hz, 3H). |
| | | | ¹⁹F NMR (377 MHz, DMSO-d6) δ -60.60, -122.95 (t, J = 9.3 Hz), - 123.08 (dp, J *=* 9.9, 3.1 Hz). |
| T106 | 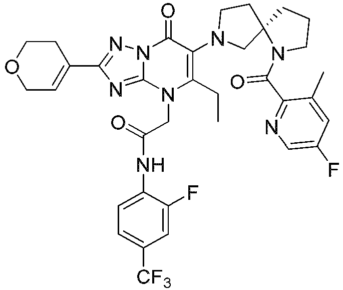 | 726.27 | ¹H NMR (400 MHz, DMSO-d6) δ 10.71 (d, J *=* 1.7 Hz, 1H), 8.38 (d, J = 2.7 Hz, 1H), 8.24 (t, J = 8.2 Hz, 1H), 7.80 (dd, J = 11.1, 2.0 Hz, 1H), 7.73 (dd, J = 9.9, 2.7 Hz, 1H), 7.61 - 7.53 (m, 1H), 6.81 (tt, J = 3.0, 1.6 Hz, 1H), 5.30 (s, 2H), 4.25 (q, J = 2.8 Hz, 2H), 4.06 (s, 1H), 3.81 (t, J *=* 5.4 Hz, 2H), 3.32 - 3.07 (m, 4H), 3.07 - 2.58 (m, 4H), 2.29 (s, 4H), 2.07 (dt, J = 13.0, 7.3 Hz, 1H), 1.78 (tq, J = 10.8, 5.8 Hz, 3H), 1.20 (t, J = 7.4 Hz, 3H). |
| | | | ¹⁹F NMR (377 MHz, DMSO-d6) δ -60.60, -122.80 - -123.12 (m), - 127.31 (d, J = 9.8 Hz) |
| T107 | 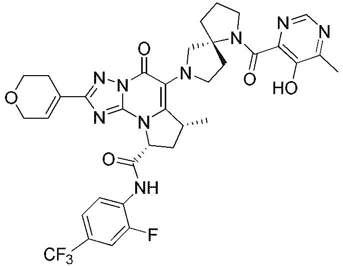 | 737.26 | ¹H NMR (400 MHz, DMSO) δ 10.91 (s, 1H), 10.80 (s, 1H), 8.57 (s, 1H), 8.21 (t, *J* = 8.2 Hz, 1H), 7.81 (d, *J* = 10.8 Hz, 1H), 7.58 (d, *J =* 8.5 Hz, 1H), 6.74 (s, 1H), 5.60 (dd, *J* = 9.1, 4.2 Hz, 1H), 4.22 (d, *J* = 3.5 Hz, 2H), 3.91 (d, *J* = 7.7 Hz, 1H), 3.83 - 3.75 (m, 2H), 3.66 (h, *J* = 7.3 Hz, 1H), 3.56 (t, *J* = 6.7 Hz, 2H), 3.48 - 3.41 (m, 1H), 3.22 - 3.02 (m, 2H), 2.93 (d, *J =* 7.8 Hz, 1H), 2.50(m, 1H), 2.60 (ddd, *J* = 12.7, 8.4, 4.1 Hz, 1H), 2.44 (s, 3H), 2.41 - 2.33 (m, 2H), 2.04 (dt, *J =* 12.8, 7.2 Hz, 1H), 1.92 - 1.68 (m, 3H), 1.52 (d, *J =* 6.9 Hz, 3H), 1.23 (s, 1H). ¹⁹F NMR (377 MHz, DMSO) δ -60.63, -122.65 (d, *J* = 9.3 Hz). |
| T108 | 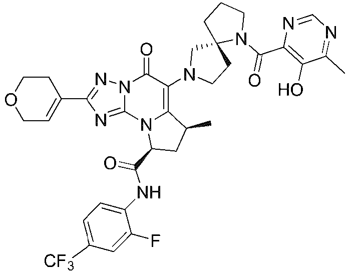 | 737.26 | ¹H NMR (400 MHz, DMSO) δ 10.93 (s, 1H), 10.80 (s, 1H), 8.58 (s, 1H), 8.20 (t, *J* = 8.1 Hz, 1H), 7.86 - 7.78 (m, 1H), 7.60 (d, *J* = 8.5 Hz, 1H), 6.76 (s, 1H), 5.52 (dd, *J =* 10.3, 2.2 Hz, 1H), 4.23 (d, *J =* 3.0 Hz, 2H), 4.17 (d, *J =* 7.9 Hz, 1H), 3.79 (t, *J =* 5.7 Hz, 2H), 3.67 (q, *J* = 7.9 Hz, 1H), 3.56 (t, *J =* 6.7 Hz, 2H),3.34(m,5H), 3.06 - 2.93 (m, 2H), 2.73 (d, *J* = 7.9 Hz, 1H),2.50(m,2H), 2.45 (s, 3H), 2.25 (dt, *J* = 12.1, 6.0 Hz, 1H), 2.13 - 2.00 (m, 2H), 1.90 - 1.75 (m, 1H), 1.41 (d, *J* = 7.2 Hz, 3H), 1.24 (s, 1H). ¹⁹F NMR (377 MHz, DMSO) δ -60.64, -122.26 - -122.35 (m). |
| T109 | 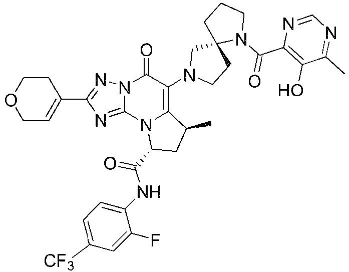 | 737.26 | ¹H NMR (400 MHz, DMSO) δ 10.90 (d, *J =* 1.4 Hz, 1H), 10.78 (s, 1H), 8.58 (s, 1H), 8.20 (t, *J =* 8.2 Hz, 1H), 7.81 (d, *J =* 11.2 Hz, 1H), 7.58 (d, *J* = 8.6 Hz, 1H), 6.74 (s, 1H), 5.60 (dd, *J =* 8.8, 4.4 Hz, 1H), 4.22 (s, 2H), 4.14 (d, *J* = 7.8 Hz, 1H), 3.87 - 3.75 (m, 2H), 3.62 (q, *J* = 7.3 Hz, 1H), 3.55 (t, *J =* 6.7 Hz, 2H), 3.44 (q, *J* = 7.5 Hz, 1H), 3.30 - 3.23 (m, 1H), 3.03 (dd, *J* = 11.6, 8.1 Hz, 1H), 2.74 (d, *J* = 7.9 Hz, 1H), 2.60 (ddd, *J* = 12.8, 8.4, 4.4 Hz, 1H),2.50(m, 1H), 2.44 (s, 3H), 2.43 - 2.34 (m, 1H), 2.27 (dt, *J =* 12.2, 6.1 Hz, 1H), 2.11 - 1.97 (m, 1H), 1.85 (td, *J* = 11.0, 5.0 Hz, 1H), 1.82 - 1.74 (m, 2H), 1.52 (d, *J* = 6.9 Hz, 3H), 1.24 (s, 1H). ¹⁹F NMR (377 MHz, DMSO) δ -60.63, - 122.56 - -122.66 (m). |
| T110 | 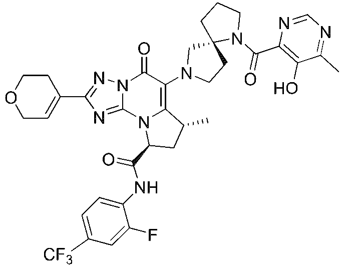 | 737.26 | ¹H NMR (400 MHz, DMSO) δ 10.90 (s, 1H), 10.80 (s, 1H), 8.58 (s, 1H), 8.19 (t, *J =* 8.1 Hz, 1H), 7.82 (dd, *J* = 11.1, 2.1 Hz, 1H), 7.63 - 7.56 (m, 1H), 6.76 (dd, *J =* 3.1, 1.5 Hz, 1H), 5.53 (dd, *J* = 10.3, 2.4 Hz, 1H), 4.23 (d, *J* = 3.0 Hz, 2H), 3.94 (d, *J* = 7.7 Hz, 1H), 3.80 (t, *J =* 5.6 Hz, 2H), 3.69 (t, *J* = 7.9 Hz, 1H), 3.55 (dt, *J =* 10.3, 5.5 Hz, 3H), 3.15 (q, *J* = 7.3 Hz, 1H), 3.10 - 2.94 (m, 2H), 2.89 (d, *J* = 7.7 Hz, 1H),2.50(m.2H), 2.44 (s, 3H), 2.38 - 2.29 (m, 1H), 2.12 - 1.97 (m, 2H), 1.91 - 1.72 (m, 2H), 1.42 (d, *J* = 7.2 Hz, 3H), 1.23 (s, 1H). ¹⁹F NMR (377 MHz, DMSO) δ -60.64, -122.32 (t, *J* = 9.3 Hz). |
| T111 | 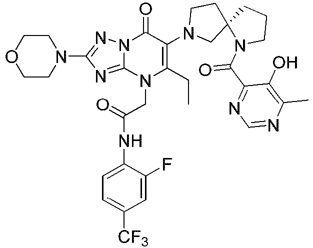 | 728.71 | ¹H NMR (400 MHz, DMSO) δ 10.93 (s, 1H), 10.68 (s, 1H), 8.58 (s, 1H), 8.23 (t, J = 8.2 Hz, 1H), 7.80 (dd, J = 11.1, 2.1 Hz, 1H), 7.58 (dd, J = 8.7, 2.1 Hz, 1H), 5.20 (s, 2H), 3.70 - 3.63 (m, 4H), 3.56 (t, J = 6.6 Hz, 2H), 3.37 (t, J = 4.8 Hz, 7H), 3.09 - 2.84 (m, 3H), 2.44 (s, 3H), 2.32 - 2.27 (m, 1H), 2.05 (dt, J = 13.0, 7.2 Hz, 1H), 1.90 - 1.71 (m, 3H), 1.18 (t, J = 7.4 Hz, 3H). |
| T112 | 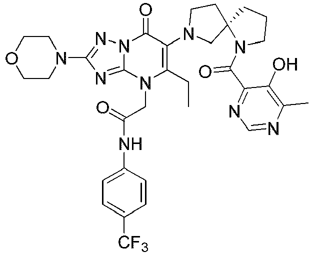 | 710.29 | ¹H NMR (400 MHz, DMSO-d6) δ 10.92 (s, 1H), 10.85 (s, 1H), 8.58 (s, 1H), 7.78 (d, J = 8.5 Hz, 2H), 7.71 (d, J = 8.6 Hz, 2H), 5.10 (s, 2H), 4.03 (s, 1H), 3.65 (t, J = 4.7 Hz, 4H), 3.56 (t, J = 6.6 Hz, 2H), 3.36 (d, J=4.4 Hz, 4H), 3.32 - 2.58 (m, 6H), 2.44 (s, 3H), 2.29 (d, J = 7.2 Hz, 1H), 2.05 (dt, J *=* 13.1, 6.9 Hz, 1H), 1.81 (ddt, J = 27.7, 13.0, 6.5 Hz, 3H), 1.18 (t, J = 7.4 Hz, 3H). |
| | | | ¹⁹F NMR (377 MHz, DMSO-d6) δ -60.39. |
| T113 | 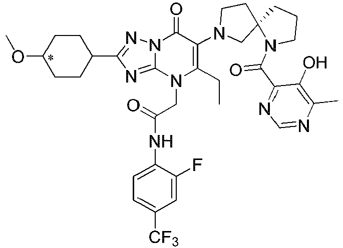 | 753.76 | ¹H NMR (400 MHz, DMSO) δ 10.70 (m, 2H), 8.57 (s, 1H), 8.24 (t, J *=* 8.2 Hz, 1H), 7.81 (dd, J *=* 11.1, 2.1 Hz, 1H), 7.57 (d, J = 8.6 Hz, 1H), 6.70 (d, J = 3.9 Hz, 1H), 5.29 (s, 2H), 3.54 (dt, J = 13.6, 6.4 Hz, 3H), 3.28 (s, 3H), 3.11 - 2.75 (m, 4H), 2.56 (s, 2H), 2.5 (1H), 2.44 (s, 4H), 2.28 (s, 1H), 2.21 - 2.01 (m, 2H), 2.03 - 1.90 (m, 1H), 1.90 - 1.73 (m, 3H), 1.68 (dt, J *=* 13.2, 6.8 Hz, 1H), 1.21 (q, J = 7.5 Hz, 3H). ¹⁹F NMR (377 MHz, DMSO) δ - 60.59, -122.92 - -123.02 (m). |
| T114 | 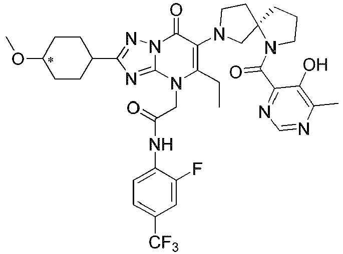 | 753.76 | ¹H NMR (400 MHz, DMSO) δ 10.71 (m, 2H), 8.57 (s, 1H), 8.24 (t, J *=* 8.2 Hz, 1H), 7.81 (dd, J *=* 11.1, 2.1 Hz, 1H), 7.57 (dd, J = 8.7, 2.1 Hz, 1H), 6.70 (d, J = 4.1 Hz, 1H), 5.29 (s, 2H), 3.54 (dt, J *=* 14.2, 6.5 Hz, 3H), 3.28 (s, 3H), 3.10 - 2.73 (m, 4H), 2.58 (d, J = 17.8 Hz, 2H), 2.5(1H),2.44 (s, 4H), 2.29 (s, 1H), 2.20 - 2.01 (m, 2H), 2.00 - 1.91 (m, 1H), 1.91 - 1.73 (m, 3H), 1.66 (ddt, J = 14.0, 8.2, 4.2 Hz, 1H), 1.21 (q, J = 6.7 Hz, 3H). ¹⁹F NMR (377 MHz, DMSO) δ -60.60, -122.91-123.03 (m). |
| T115 | 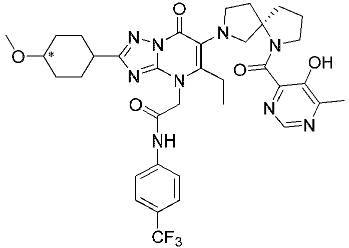 | 735.77 | ¹H NMR (400 MHz, DMSO) δ 10.89 (m, 2H), 8.58 (s, 1H), 7.79 (d, J = 8.5 Hz, 2H), 7.71 (d, J = 8.6 Hz, 2H), 6.69 (d, J = 4.1 Hz, 1H), 5.19 (s, 2H), 4.05 (s, 1H), 3.56 (t, J = 6.7 Hz, 2H), 3.51 (d, J = 6.5 Hz, 1H), 3.27 (s, 3H), 3.09 - 2.91 (m, 3H), 2.81 (s, 1H), 2.63 - 2.51 (m, 4H), 2.44 (s, 3H), 2.29 (s, 1H), 2.18- 2.02 (m, 2H), 1.96 (dd, J = 13.9, 5.9 Hz, 1H), 1.90- 1.72 (m, 3H), 1.66 (dtd, J *=* 13.9, 8.5, 5.8 Hz, 1H), 1.33 - 1.13 (m, 4H). ¹⁹F NMR (377 MHz, DMSO) δ -60.40. |
| T116 | 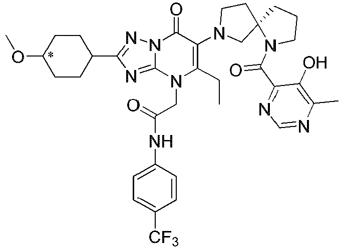 | 735.77 | ¹H NMR (400 MHz, DMSO) δ 10.90 (m, 2H), 8.57 (s, 1H), 7.79 (d, J = 8.6 Hz, 2H), 7.71 (d, J = 8.7 Hz, 2H), 6.70 (d, J = 4.4 Hz, 1H), 5.18 (s, 2H), 4.05 (s, 1H), 3.59 - 3.47 (m, 3H), 3.27 (s, 3H), 3.09 - 2.91 (m, 3H), 2.68- 2.51 (m, 4H), 2.44 (s, 4H), 2.18 - 2.03 (m, 2H), 1.95 (d, J = 11.9 Hz, 1H), 1.89 - 1.73 (m, 3H), 1.65 (dt, J *=* 13.9, 7.0 Hz, 1H), 1.21 (q, J = 7.3 Hz, 3H). ¹⁹F NMR (377 MHz, DMSO) δ - 60.39. |
| T117 | 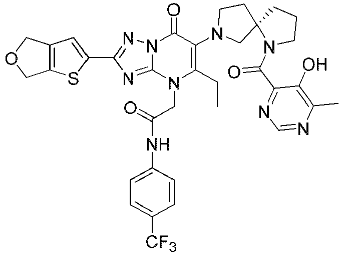 | 749.77 | ¹H NMR (400 MHz, DMSO-*d*6)δ 10.94 (m, 2H), 8.56 (s, 1H), 7.80 (d, J = 8.6Hz, 2H), 7.71 (d, J = 8.6 Hz, 2H), 7.53 (s, 1H), 5.23 (s, 2H), 5.01 (t, J = 3.4 Hz,2H), 4.87 (t, J = 3.3 Hz, 2H), 4.07 (s, 1H), 3.56 (t, J = 6.7 Hz, 3H), 3.24 - 2.73(m, 5H), 2.44 (s, 3H), 2.35 - 2.26 (m, 1H), 2.08 (dt, J = 12.9, 7.0 Hz, 1H), 1.82 (ddd, J = 28.3, 13.3, 7.1 Hz, 3H), 1.21 (t, J = 7.5 Hz, 3H). |
| | | | ¹⁹F NMR (377 MHz, DMSO-d6) δ -60.39. |
| T118 | 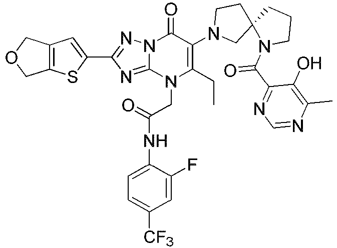 | 767.76 | ¹H NMR (400 MHz, DMSO) δ 10.91 (s, 1H), 10.74 (s, 1H), 8.57 (s, 1H), 8.24 (t, *J* = 8.2 Hz, 1H), 7.81 (dd, *J* = 11.3, 2.0 Hz, 1H), 7.57 (d, *J* = 8.6 Hz, 1H), 7.54 (s, 1H), 5.33 (s, 2H), 5.02 (t, *J* = 3.3 Hz, 2H), 4.88 (t, *J* = 3.5 Hz, 2H), 3.56 (t, *J* = 6.7 Hz, 2H), 3.14 2.87 (m, 4H), 2.44 (s, 3H), 2.29 (s, 2H), 2.22 1.70 (m, 6H), 1.21 (t, *J = 7.5* Hz, 3H). ¹⁹F NMR (377 MHz, DMSO) δ -60.59, -122.83 (d, *J* = 9.5 Hz). |
| T119 | 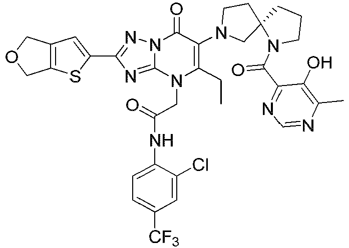 | 784.21 | ¹H NMR (400 MHz, DMSO-d6)δ 10.91 (s, 1H),10.43 (s, 1H), 8.58 (s, 1H), 8.07 (d, J = 8.6 Hz, 1H), 7.98 (d, J = 2.1 Hz, 1H), 7.72 (dd, J = 8.9, 2.1 Hz, 1H), 7.55 (s, 1H), 5.35 (s, 2H), 5.02 (t, J = 3.4 Hz, 2H), 4.89 (t, J = 3.5 Hz, 2H), 4.06 (s, 1H), 3.56 (t, J = 6.6 Hz, 2H), 3.31 (m, 2H)3.02 (dd, J = 18.1, 8.9 Hz, 3H), 2.82 (s, 1H), 2.44 (s, 3H), 2.37 2.23 (m, 1H), 2.08 (dt, J = 12.9, 7.1 Hz, 1H), 1.83 (ddt, J = 21.4, 13.5, 6.6 Hz, 3H), 1.23 (t, J *=* 7.4 Hz, 3H). |
| T120 | 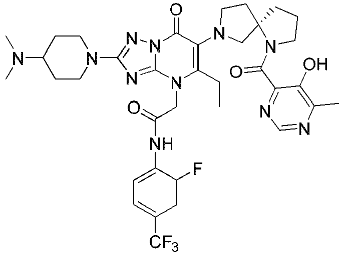 | 769.81 | ¹H NMR (400 MHz, DMSO-d6) δ 10.69 (s,1H), 8.55 (s, 1H), 8.26 - 8.15 (m, 1H), 7.80 (dd,J= 11.0, 2.1 Hz, 1H), 7.60 - 7.52 (m, 1H), 5.19(s, 2H), 4.04 (d, J = 12.9 Hz, 4H), 3.54 (d, J = 13.4 Hz, 2H), 3.14 - 2.69 (m, 6H), 2.43 (s, 3H), 2.39 (s, 1H), 2.28 (s, 1H), 2.21 (d, J = 2.5 Hz,6H), 2.04 (dt, J = 13.0, 7.1 Hz, 1H), 1.91 - 1.61(m, 5H), 1.36 (tt, J *=* 12.6, 6.4 Hz, 2H), 1.17 (t,J = 7.4 Hz, 3H) |
| T121 | 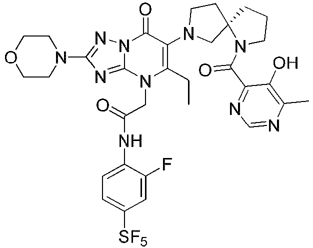 | 786.76 | ¹H NMR (400 MHz, DMSO-d6) δ 10.91 (s, 1H), 10.75 (s, 1H), 8.57 (s, 1H), 8.25 (t, J = 8.5 Hz, 1H),8.04 (dd, J = 11.2, 2.6 Hz, 1H), 7.76 (ddd, J = 9.2, 2.7, 1.2 Hz, 1H), 5.20 (s, 2H), 4.02 (s, 1H), 3.66 (t, J= 4.7 Hz, 4H), 3.55 (t, J = 6.7 Hz, 2H), 3.36 (t, J = 4.9 Hz, 4H), 3.29 - 2.68 (m, 5H), 2.57 - 2.51 (m, 1H),2.43 (s, 3H), 2.36 - 2.22 (m, 1H), 2.05 (dt, J = 13.1, 7.4 Hz, 1H), 1.80 (ddd, J = 27.6, 12.5, 6.5 Hz, 3H),1.17 (t, J *=* 7.4 Hz, 3H). |
| T122 | 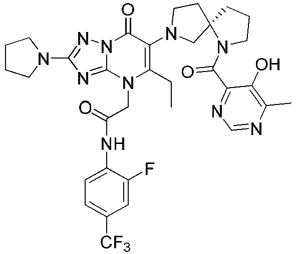 | 712.71 | ¹H NMR (400 MHz, DMSO) δ 10.98 (s, 1H),10.68 (s, 1H), 8.57 (s, 1H), 8.23 (t, J = 8.2 Hz,1H), 7.79 (dd, J = 11.1, 2.1 Hz, 1H), 7.57 (dd, J =8.7, 2.0 Hz, 1H), 5.20 (s, 2H), 4.02 (s, 1H), 3.55 (t,J = 6.7 Hz, 2H), 3.36 (s, 6H), 3.11 - 2.73 (m, 4H),2.43 (s, 3H), 2.36 - 2.23 (m, 1H), 2.10 - 1.97 (m,1H), 1.95 - 1.67 (m, 7H), 1.17 (t, J = 7.4 Hz, 3H) |
| T123 | 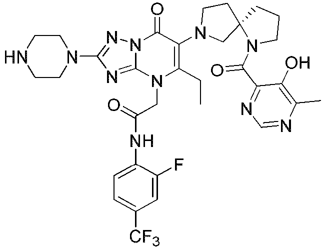 | 727.73 | ¹H NMR (400 MHz, DMSO-d6) δ 10.74 (s,1H), 8.49 (d, J = 1.5 Hz, 1H), 8.30 (d, J = 2.3Hz, 1H), 8.22 (t, J = 8.1 Hz, 1H), 7.79 (dd, J = 11.1, 2.1 Hz, 1H), 7.57 (dd, J = 8.7, 2.1 Hz,1H), 5.20 (s, 2H), 4.02 (s, 1H), 3.50 (td, J =6.6, 6.2, 2.8 Hz, 2H), 3.47 - 3.36 (m, 5H), 3.09-2.95 (m, 2H), 2.94 - 2.82 (m, 6H), 2.70 (s,1H), 2.41 (s, 3H), 2.26 (d, J = 8.3 Hz, 1H),2.03 (dt, J = 12.8, 6.8 Hz, 1H), 1.78 (dtd, J =26.8, 12.6, 6.1 Hz, 3H), 1.17 (t, J *=* 7.4 Hz,3H) |

### Activity Test Examples:

### Example 1: Determination of Growth Inhibition of Tumor Cells by Test Compounds

Taking SW48 cells as an example, the inhibitory activity of the compounds of the present disclosure against tumor growth was tested.

### 1.1 Cell inoculation

Human colon cancer SW48 cells (ZQ0793) were cultured in a DMEM (C3113-0500) medium containing 10% fetal bovine serum (FBS) and 1% penicillin-streptomycin mixture (C3421-0100) at 37 °C with 5% CO₂. The medium in a T25 flask was discarded by pipetting, and the flask was rinsed twice with 1 mL of PBS solution. Subsequently, 1 mL of trypsin (C3530-0500) digestion solution was added, and the flask was incubated in an incubator at 37 °C for 2 min to digest the cells. When the cell edges became round, 1 mL of medium containing 10% FBS was added to terminate the cell digestion, and the cells were pipetted to form a cell suspension and counted. Subsequently, the cell density was diluted to 5 × 10⁴ cells/mL using a complete medium. The cell suspension was added to a white transparent-bottom 96-well plate (Coring, CLS3903) at 100 µL/well (PBS solution was added to the peripheral wells of the plate), and the cell plate was cultured overnight in an incubator at 37 °C with 5% CO₂.

### 1.2 Preparation of compounds

A compound stock solution (100 mM DMSO stock solution) was diluted to a 1 mM or 10 mM secondary stock solution using DMSO. 1 µL of the secondary stock solution was taken and added to 1 mL of DMEM complete medium, followed by shaking on a shaker to achieve dissolution, yielding the highest administration concentration of 1 µM or 10 µM. Dilutions at a 3-fold ratio were performed to generate 8-10 administration concentrations with thorough mixing. 0.1% DMSO vehicle was added to the control group.

### 1.3 Drug treatment

After the cells adhered to the wall, the medium was carefully discarded by pipetting, and a DMEM complete medium containing the drug molecules at different concentrations was added at 100 µL/well to the corresponding wells of the 96-well plate, with 3 replicate wells set for each concentration. After administration, the cell plate was incubated in an incubator at 37 °C with 5% CO₂ for 4 days.

### 1.4 Bioanalytical Method

After 4 days of incubation, 50 µL of CellTiter-Glo^{®} detection reagent (Promega, G7572) was added to each well. The mixture was mixed uniformly by shaking, incubated, and reacted at room temperature for 10 min. The chemiluminescence value, RLU (Relative Luminescence Unit), was measured using a microplate reader. The cell viability at different drug concentrations was calculated according to the following formula, and the IC50 was fitted using Prism. $Cell viability \left(%\right) = \left(\left(As - Ab\right)\right) / \left(\left(Ac - Ab\right)\times 100\right)$
As: RLU of the experimental group (containing cells, medium, and drug molecules)
Ac: RLU of the control group (containing cells, medium, and DMSO vehicle)
Ab: RLU of the blank well (containing medium only)

### 2. Experimental results

The experimental results are shown in Table 2.

### Example 2: Enzyme Activity Assay

### 2.1 Determination of inhibition of WRN helicase activity by test compounds

Reference was made to the assay methods for helicase activity established in the literature to evaluate the effect of the compounds on DNA-dependent WRN helicase activity (PMC6326523 DOI: 10.1371/journal.pone.0210525).

The protein used was WRN (500-1229) (Sino biological, 17475-HNCB), the expression host was baculovirus-insect cells, and the assay was performed in a 384-well black microplate (Labtide, M38-3111). The double-stranded sequences of the fluorescent DNA fork substrate were fork-F (TAMRA-5'-GCACTGGCCGTCGTTTTACGGTCGTGACT-3') and fork-R (5'-TTTTTTCCAAGTAAAACGACGGCCAGTGC-3'-BHQ2). The fluorescent DNA fork substrate was obtained by heating to 96 °C, followed by gradient cooling (annealing buffer: 25 mM Tris, 2 mM MgCl2, 5 mM NaCl, pH 8.0). The reaction system was 50 µL, and the assay buffer was 25 mM Tris, 2 mM MgCl2, 5 mM NaCl, 1 mM DTT, and 2.5 µg/mL calf thymus DNA, pH 8.0.

To test the IC50 of the test compounds for inhibiting the WRN protein helicase activity, the preparation of the reaction system was divided into two parts, with two replicates set for each group. In the first part, the WRN protein was mixed and incubated with the test compounds. A 3-fold serially diluted DMSO compound solution was prepared. 44 µL of WRN protein at a final concentration of 50 nM and 1 µL of the compound at different concentrations (final concentration of 1 µM, 3-fold dilution) were added to a 384-well plate, mixed by shaking, and subsequently incubated for 1 h. In the second part, ATP and the fluorescent DNA substrate were added in a volume of 5 µL, with the final concentration of ATP being 2 mM and the concentration of the fluorescent DNA substrate being 200 nM. After the incubation of the small molecule and the protein was completed, they were added to the reaction system to react for 1 h. The RFU value was measured using a microplate reader with the instrument excitation wavelength set at 544 nm and emission wavelength set at 590 nm. In addition, an experimental group containing only the protein and the corresponding DMSO vehicle was set as a high control, and a group containing no protein and only the buffer was set as a blank control (low control). The calculation formula for the inhibition rate of the compounds against the helicase activity was: Inhibition = (high control - sample)/(high control - low control), and the IC50 values were obtained by four-parameter fitting using GraphPad prism.

### 2.2 Determination of inhibition of WRN ATPase activity by test compounds

The protein used was WRN (500-1229) (Sino biological, 17475-HNCB), and the assay was performed in a 384-well white-bottom white microplate (LABSELECT, 31432). The double-stranded sequences of the DNA fork substrate were fork-F (5'-GCACTGGCCGTCGTTTTACGGTCGTGACT-3') and fork-R (5'-TTTTTTCCAAGTAAAACGACGGCCAGTGC-3'), and the assay buffer was 25 mM Tris, 2 mM MgCl2, 5 mM NaCl, 1 mM DTT, 2.5 µg/mL calf thymus DNA, pH 8.0.

Preparation of compounds: A compound stock solution (10 mM DMSO stock solution) was diluted to a 500 µM secondary stock solution using DMSO. Subsequently, 3-fold dilutions (6 µL + 12 µL) were performed using DMSO to generate 10 gradients of the drug molecules. Each diluted sample was further diluted with the assay buffer (1 µL of secondary stock solution + 11.5 µL of assay buffer), preparing a 3-fold diluted compound solution with a maximum concentration of 40 µM across 10 gradients (dilution and preparation steps were carried out in a skirtless 96-well PCR plate).

Enzyme activity system reaction (3.75 µL pro + 3.75 µL cpds + 7.5 µL DNA/ATP mix): According to the protein concentration determined by the WRN protease activity assay (final: 25 nM), a sufficient amount of protein solution was prepared and added to a skirtless 96-well PCR plate to carry out the enzyme activity reaction. 3.75 µL of the protein solution was dispensed into each well, and subsequently, 3.75 µL of the pre-prepared compound solution was added, followed by centrifugation and incubation for 30 min. After the incubation was completed, 7.5 µL of a mixed solution of DNA (final: 200 nM) and ATP (final: 300 µM) was further added to the plate and incubated for 30 min. After the enzyme activity reaction was completed, the solution was dispensed into a 384-well plate at 5 µL/well, with two replicate wells set. In addition, an experimental group containing only the corresponding DMSO vehicle was set as a high control, and a group containing no protein and only the ATP buffer solution was set as a blank control (low control).

ADP-GloTM system reaction (5 µL + 5 µL + 10 µL): Immediately after the completion of the enzyme activity system reaction and dispensing, 5 µL of ATP depletion reagent was added, followed by reaction for 60 min. After the reagent reaction was completed, 10 µL of kinase detection reagent was added, followed by reaction for 60 min. The chemiluminescence value, RLU (Relative Luminescence Unit), was measured using a microplate reader. Data analysis and the fitting method of IC50 were the same as those in the aforementioned helicase activity assay.

### 3. Experimental results

The experimental results are shown in Table 2.

**Table 2. Biochemical and cellular activities of compounds**

| Compound No. | IC50 for inhibition of WRN helicase activity | IC50 for inhibition of WRN ATPase activity | EC50 for inhibition of SW48 tumor cell growth |
|---|---|---|---|
| T001 | + | + | + |
| T002 | + | + | + |
| T003 | + | + | + |
| T004 | + | + | + |
| T005 | + | + | + |
| T006 | ++++ | +++ | ++++ |
| T007 | ++ | +++ | ++ |
| T008 | + | + | + |
| T009 | ++ | +++ | ++ |
| T010 | + | + | + |
| T011 | ++++ | +++ | ++ |
| T012 | ++ | +++ | ++ |
| T013 | ++++ | +++ | ++++ |
| T014 | ++++ | ++ | ++++ |
| T015 | ++++ | +++ | ++++ |
| T016 | ++++ | +++ | ++++ |
| T017 | ++++ | +++ | ++++ |
| T018 | ++++ | +++ | +++ |
| T019 | ++++ | +++ | ++++ |
| T020 | ++++ | ++++ | ++++ |
| T021 | ++++ | ++ | ++++ |
| T022 | ++++ | +++ | ++++ |
| T023 | ++++ | +++ | ++++ |
| T024 | ++++ | ++++ | ++++ |
| T025 | ++++ | +++ | +++ |
| T026 | ++++ | +++ | ++++ |
| T027 | ++++ | +++ | ++++ |
| T028 | ++++ | +++ | +++ |
| T029 | ++++ | +++ | ++++ |
| T030 | ++++ | +++ | ++++ |
| T031 | ++++ | +++ | ++++ |
| T032 | ++++ | +++ | ++++ |
| T033 | ++++ | +++ | ++++ |
| T034 | ++++ | ++++ | ++++ |
| T035 | ++++ | +++ | +++ |
| T036 | ++++ | ++++ | ++++ |
| T037 | ++++ | ++++ | ++++ |
| T038 | ++++ | +++ | ++++ |
| T039 | ++++ | +++ | ++++ |
| T040 | ++++ | +++ | ++++ |
| T041 | ++++ | +++ | ++++ |
| T042 | ++++ | ++++ | ++++ |
| T043 | ++++ | +++ | ++++ |
| T044 | ++++ | +++ | ++++ |
| T045 | ++++ | +++ | +++ |
| T046 | ++++ | ++++ | ++++ |
| T047 | ++++ | ++++ | ++++ |
| T048 | ++++ | +++ | ++++ |
| T049 | ++++ | +++ | ++++ |
| T050 | ++++ | +++ | ++++ |
| T051 | ++++ | +++ | ++++ |
| T052 | ++++ | +++ | ++++ |
| T053 | ++++ | +++ | ++++ |
| T054 | ++++ | +++ | ++++ |
| T055 | ++++ | +++ | ++++ |
| T056 | ++++ | +++ | ++++ |
| T057 | ++++ | ++++ | ++++ |
| T058 | ++++ | +++ | ++++ |
| T059 | ++++ | +++ | ++++ |
| T060 | ++++ | +++ | ++++ |
| T061 | ++++ | +++ | ++++ |
| T062 | ++++ | ++++ | ++++ |
| T063 | ++++ | +++ | ++++ |
| T064 | ++++ | ++++ | ++++ |
| T065 | ++++ | +++ | +++ |
| T066 | ++++ | +++ | ++++ |
| T067 | ++++ | ++++ | ++++ |
| T068 | ++++ | +++ | ++++ |
| T069 | ++++ | +++ | ++++ |
| T070 | ++++ | +++ | ++++ |
| T071 | ++++ | ++++ | ++++ |
| T072 | ++++ | ++++ | ++++ |
| T073 | ++++ | ++++ | ++++ |
| T074 | ++++ | ++++ | ++++ |
| T075 | ++++ | ++ | +++ |
| T076 | ++++ | ++++ | ++++ |
| T077 | ++++ | +++ | ++++ |
| T078 | ++++ | +++ | ++++ |
| T079 | ++++ | +++ | ++++ |
| T080 | ++++ | +++ | ++++ |
| T081 | ++++ | +++ | ++++ |
| T082 | ++++ | ++ | ++++ |
| T083 | ++++ | +++ | ++++ |
| T084 | ++++ | +++ | ++++ |
| T085 | ++++ | +++ | +++ |
| T086 | ++++ | +++ | ++++ |
| T087 | ++++ | +++ | ++++ |
| T088 | ++++ | +++ | +++ |
| T089 | ++++ | +++ | ++++ |
| T090 | ++++ | +++ | ++++ |
| T091 | ++++ | +++ | ++++ |
| T092 | ++++ | ++++ | +++ |
| T093 | ++++ | ++++ | ++++ |
| T094 | ++++ | ++++ | ++++ |
| T095 | ++ | ++ | ++ |
| T096 | ++ | ++ | ++ |
| T097 | ++++ | ++++ | ++++ |
| T098 | ++++ | ++++ | ++++ |
| T099 | ++ | ++ | ++ |
| T100 | ++ | ++ | ++ |
| T101 | ++ | ++ | ++ |
| T102 | ++ | ++ | ++ |
| T103 | ++ | ++ | ++ |
| T104 | ++ | ++ | ++ |
| T105 | ++ | ++ | ++ |
| T106 | ++ | ++ | ++ |
| T107 | ++ | ++ | ++ |
| T108 | ++ | ++ | ++ |
| T109 | +++ | +++ | ++++ |
| T110 | +++ | +++ | ++++ |
| T111 | +++ | ++++ | ++++ |
| T112 | ++++ | ++++ | ++++ |
| T113 | +++ | ++++ | ++++ |
| T114 | +++ | ++++ | ++++ |
| T115 | +++ | ++++ | ++++ |
| T116 | +++ | ++++ | ++++ |
| T117 | ++++ | ++++ | ++++ |
| T118 | +++ | +++ | ++++ |
| T119 | ++++ | ++++ | ++++ |
| T120 | +++ | +++ | ++++ |
| T121 | ++++ | ++++ | ++++ |
| T122 | ++++ | ++++ | ++++ |
| T123 | ++++ | ++++ | +++ |

| | | | |
|---|---|---|---|
| Note: ++++: IC50 < 100 nM; +++: 100 nM < 1C50 < 1 µM; ++: 1 µM ≤ IC50 < 10 µM; +: 10 µM ≤ IC50. | | | |

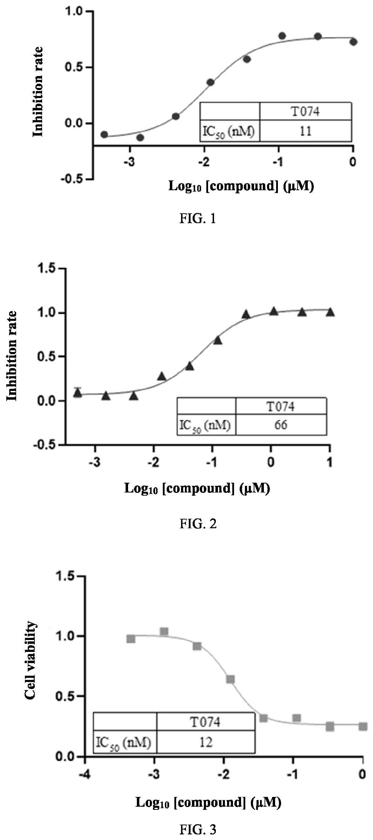

The IC50 curve for inhibition of WRN helicase activity, the IC50 curve for inhibition of ATPase activity, and the EC50 curve for inhibition of tumor cell (SW48) growth of compound T074 are shown in FIGs. 1-3, respectively.

The above description is only for the purpose of illustrating the preferred examples of the present disclosure, and is not intended to limit the scope of the present disclosure. Any modifications, equivalent substitutions, and the like made without departing from the spirit and principle of the present disclosure shall fall within the protection scope of the present disclosure.

The foregoing examples and methods described in the present disclosure may vary based on the abilities, experience, and preferences of those skilled in the art.

The certain order in which the steps of the method are listed in the present disclosure does not constitute any limitation on the order of the steps of the method.

## Claims

1. A compound or a pharmaceutically acceptable salt, stereoisomer, ester, prodrug, solvate, or deuterated compound thereof, wherein the compound has the following structure: 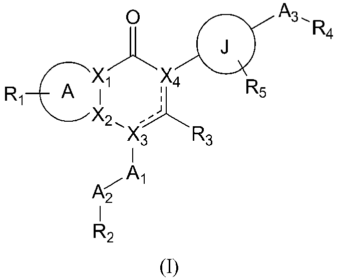 wherein
- -̅ -̅ -̅ -̅ -̅ represents a single bond or a double bond, and two -̅ -̅ -̅ -̅ -̅ -̅ are not both double bonds;
ring A is a 4- to 6-membered heterocyclic ring;
ring J is a 6- to 18-membered spiro ring;
X₁, X₂, X₃, and X₄ are independently selected from: C(R₅) and N; R₅ is selected from: H, D, C₁-C₁₀ alkyl, -(C₀-C₆ alkylene)-(C₃-C₁₀ cycloalkyl), -(C₀-C₆ alkylene)-(C₆-C₁₀ aryl), -(C₀-C₆ alkylene)-(4- to 10-membered heterocyclyl), halogen, cyano, nitro, azido, C₁-C₁₀ haloalkyl, C₁-C₁₀ haloalkoxy, -N(C₀₋₁₀ alkyl)(C₀₋₁₀ alkyl), -N(C₀₋₁₀ alkyl)(C₃-C₁₀ cycloalkyl), -N(C₀₋₁₀ alkyl)CO(C₀₋₁₀ alkyl), -N(C₀₋₁₀ alkyl)CON(C₀₋₁₀ alkyl), -N(C₀₋₁₀ alkyl)SO₂(C₀₋₁₀ alkyl), -O(C₀₋₁₀ alkyl), -O(C₃-C₁₀ cycloalkyl), -S(C₀₋₁₀ alkyl), -S(C₃-C₁₀ cycloalkyl), -SO(C₀₋₁₀ alkyl), -SO₂(C₀₋₁₀ alkyl), -SO₂(C₃-C₁₀ cycloalkyl), -SO₂N(C₀₋₁₀ alkyl)(C₀₋₁₀ alkyl), -SO₂N(C₀₋₁₀ alkyl)(C₃-C₁₀ cycloalkyl), -COO(C₀₋₁₀ alkyl), -OCO(C₀₋₁₀ alkyl), -CON(C₀₋₁₀ alkyl)(C₀₋₁₀ alkyl), -CON(C₀₋₁₀ alkyl)(C₃-C₁₀ cycloalkyl), and -CO(C₀₋₁₀ alkyl);
A₁, A₂, and A₃ are independently selected from: a single bond and C₁-C₁₀ alkylene, wherein 0-6 methylene units in the C₁-C₁₀ alkylene are independently substituted with the following groups: -Cy-, -O-, -S-, -C(O)-, -C(S)-, -C(O)O-, -OC(O)-, -OC(O)O-, -O-S(O)₂-, -OC(O)N(Rₐ)-, -C(O)N(Rₐ)-, -N(Rₐ)C(O)-, -N(Rₐ)C(O)O-, -N(Rₐ)C(O)N(R_{b})-, - N(Rₐ)-, -S(O)₂-, -S(O)₂N(Rₐ)-, -N(Rₐ)S(O)₂-, -S(O)-, -S(O)N(Rₐ)-, -N(Rₐ)S(O)-, -Si-, 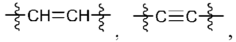 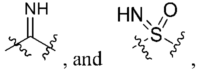 wherein Rₐ and R_{b} are independently selected from: H, C₁-C₁₀ alkyl, C₂-C₁₀ alkenyl, C₂-C₁₀ alkynyl, -(C₀-C₆ alkylene)-(C₃-C₁₀ cycloalkyl), -(C₀-C₆ alkylene)-(C₆-C₁₀ aryl), and -(C₀-C₆ alkylene)-(4- to 10-membered heterocyclyl); each -Cy- is independently an optionally substituted divalent ring selected from: arylene, cycloalkylene, and heterocyclylene; H in the C₁-C₁₀ alkylene is optionally substituted with one or more R₀;
R₁ is one or more independent substituents on ring A and is selected from: H, D, C₁-C₁₀ alkyl, -(C₀-C₆ alkylene)-(C₃-C₁₀ cycloalkyl), -(C₀-C₆ alkylene)-(C₆-C₁₀ aryl), -(C₀-C₆ alkylene)-(4- to 10-membered heterocyclyl), halogen, cyano, nitro, azido, C₁-C₁₀ haloalkyl, C₁-C₁₀ haloalkoxy, -N(C₀₋₁₀ alkyl)(C₀₋₁₀ alkyl), -N(C₀₋₁₀ alkyl)(C₃-C₁₀ cycloalkyl), - N(C₀₋₁₀ alkyl)CO(C₀₋₁₀ alkyl), -N(C₀₋₁₀ alkyl)CON(C₀₋₁₀ alkyl), -N(C₀₋₁₀ alkyl)SO₂(C₀₋₁₀ alkyl), -O(C₀₋₁₀ alkyl), - O(C₃-C₁₀ cycloalkyl), -S(C₀₋₁₀ alkyl), -S(C₃-C₁₀ cycloalkyl), -SO(C₀₋₁₀ alkyl), -SO₂(C₀₋₁₀ alkyl), -SO₂(C₃-C₁₀ cycloalkyl), -SO₂N(C₀₋₁₀ alkyl)(C₀₋₁₀ alkyl), -SO₂N(C₀₋₁₀ alkyl)(C₃-C₁₀ cycloalkyl), -COO(C₀₋₁₀ alkyl), -OCO(C₀₋₁₀ alkyl), -CON(C₀₋₁₀ alkyl)(C₀₋₁₀ alkyl), -CON(C₀₋₁₀ alkyl)(C₃-C₁₀ cycloalkyl), -CO(C₀₋₁₀ alkyl), and -Si(C₀₋₁₀ alkyl)(C₀₋₁₀ alkyl)(C₀₋₁₀ alkyl), wherein H in the C₀-C₆ alkylene, C₁-C₁₀ alkyl, C₂-C₁₀ alkenyl, C₂-C₁₀ alkynyl, C₀₋₁₀ alkyl, C₃-C₁₀ cycloalkyl, C₆-C₁₀ aryl, or 4- to 10-membered heterocyclyl may be optionally substituted with one or more R₀;
R₂, R₃, and R₄ are independently selected from: H, D, C₁-C₁₀ alkyl, -(C₀-C₆ alkylene)-(C₃-C₁₀ cycloalkyl), -(C₀-C₆ alkylene)-(C₆-C₁₀ aryl), -(C₀-C₆ alkylene)-(4- to 10-membered heterocyclyl), halogen, cyano, nitro, azido, C₁-C₁₀ haloalkyl, C₁-C₁₀ haloalkoxy, -N(C₀₋₁₀ alkyl)(C₀₋₁₀ alkyl), -N(C₀₋₁₀ alkyl)(C₃-C₁₀ cycloalkyl), -N(C₀₋₁₀ alkyl)CO(C₀₋₁₀ alkyl), -N(C₀₋₁₀ alkyl)CON(C₀₋₁₀ alkyl), -N(C₀₋₁₀ alkyl)SO₂(C₀₋₁₀ alkyl), -O(C₀₋₁₀ alkyl), -O(C₃-C₁₀ cycloalkyl), -S(C₀₋₁₀ alkyl), -S(C₃-C₁₀ cycloalkyl), -SO(C₀₋₁₀ alkyl), -SO₂(C₀₋₁₀ alkyl), -SO₂(C₃-C₁₀ cycloalkyl), -SO₂N(C₀₋₁₀ alkyl)(C₀₋₁₀ alkyl), -SO₂N(C₀₋₁₀ alkyl)(C₃-C₁₀ cycloalkyl), -COO(C₀₋₁₀ alkyl), -OCO(C₀₋₁₀ alkyl), -CON(C₀₋₁₀ alkyl)(C₀₋₁₀ alkyl), -CON(C₀₋₁₀ alkyl)(C₃-C₁₀ cycloalkyl), -CO(C₀₋₁₀ alkyl), and -Si(C₀₋₁₀ alkyl)(C₀₋₁₀ alkyl)(C₀₋₁₀ alkyl), wherein H in the C₀-C₆ alkylene, C₁-C₁₀ alkyl, C₂-C₁₀ alkenyl, C₂-C₁₀ alkynyl, C₀₋₁₀ alkyl, C₃-C₁₀ cycloalkyl, C₆-C₁₀ aryl, or 4- to 10-membered heterocyclyl may be optionally substituted with one or more R₀; or, R₃ and A₁, together with the atoms to which they are attached, form a carbocyclic or heterocyclic ring, wherein H in the carbocyclic or heterocyclic ring may be optionally substituted with one or more R₀;
R₅ is one or more independent substituents on ring J and is selected from: H, D, C₁-C₁₀ alkyl, -(C₀-C₆ alkylene)-(C₃-C₁₀ cycloalkyl), -(C₀-C₆ alkylene)-(C₆-C₁₀ aryl), -(C₀-C₆ alkylene)-(4- to 10-membered heterocyclyl), halogen, cyano, nitro, azido, C₁-C₁₀ haloalkyl, C₁-C₁₀ haloalkoxy, -N(C₀₋₁₀ alkyl)(C₀₋₁₀ alkyl), -N(C₀₋₁₀ alkyl)(C₃-C₁₀ cycloalkyl), - N(C₀₋₁₀ alkyl)CO(C₀₋₁₀ alkyl), -N(C₀₋₁₀ alkyl)CON(C₀₋₁₀ alkyl), -N(C₀₋₁₀ alkyl)SO₂(C₀₋₁₀ alkyl), -O(C₀₋₁₀ alkyl), - O(C₃-C₁₀ cycloalkyl), -S(C₀₋₁₀ alkyl), -S(C₃-C₁₀ cycloalkyl), -SO(C₀₋₁₀ alkyl), -SO₂(C₀₋₁₀ alkyl), -SO₂(C₃-C₁₀ cycloalkyl), -SO₂N(C₀₋₁₀ alkyl)(C₀₋₁₀ alkyl), -SO₂N(C₀₋₁₀ alkyl)(C₃-C₁₀ cycloalkyl), -COO(C₀₋₁₀ alkyl), -OCO(C₀₋₁₀ alkyl), -CON(C₀₋₁₀ alkyl)(C₀₋₁₀ alkyl), -CON(C₀₋₁₀ alkyl)(C₃-C₁₀ cycloalkyl), -CO(C₀₋₁₀ alkyl), and -Si(C₀₋₁₀ alkyl)(C₀₋₁₀ alkyl)(C₀₋₁₀ alkyl), wherein H in the C₀-C₆ alkylene, C₁-C₁₀ alkyl, C₂-C₁₀ alkenyl, C₂-C₁₀ alkynyl, C₀₋₁₀ alkyl, C₃-C₁₀ cycloalkyl, C₆-C₁₀ aryl, or 4- to 10-membered heterocyclyl may be optionally substituted with one or more R₀;
R₀ is selected from: D, =O, halogen, cyano, nitro, azido, -SF₅, -OR', -C(O)R', -C(S)R', -C(O)OR', -C(S)SR', -OC(O)R', -OC(S)R', -OC(S)SR', -C(O)NR'R", -OC(O)NR'R", -NR'C(O)OR", -NR'SO₂R", -SO₂NR'R", -OSO₂NR'R", - NR'C(O)R", -NR'R", -SR', -SOR', -SO₂R', -OSO₂R', -SO₃H, -SiR'R'R", C₁-C₁₀ alkyl, C₂-C₁₀ alkenyl, C₂-C₁₀ alkynyl, C₁-C₁₀ haloalkyl, C₁-C₁₀ haloalkoxy, -(C₀-C₆ alkylene)-(C₃-C₁₀ cycloalkyl), -(C₀-C₆ alkylene)-(C₆-C₁₀ aryl), and - (C₀-C₆ alkylene)-(4- to 10-membered heterocyclyl);
each R' and R" is independently selected from: H, D, C₁-C₁₀ alkyl, C₂-C₁₀ alkenyl, C₂-C₁₀ alkynyl, -(C₀-C₆ alkylene)-(C₃-C₁₀ cycloalkyl), -(C₀-C₆ alkylene)-(C₆-C₁₀ aryl), and -(C₀-C₆ alkylene)-(4- to 10-membered heterocyclyl), wherein H in the C₀-C₆ alkylene, C₁-C₁₀ alkyl, C₂-C₁₀ alkenyl, C₂-C₁₀ alkynyl, C₃-C₁₀ cycloalkyl, C₆-C₁₀ aryl, or 4-to 10-membered heterocyclyl may be optionally substituted with one or more groups selected from: halogen, cyano, nitro, azido, C₁-C₁₀ alkyl, -(C₀-C₆ alkylene)-(C₃-C₁₀ cycloalkyl), -(C₀-C₆ alkylene)-(C₆-C₁₀ aryl), -(C₀-C₆ alkylene)-(4- to 10-membered heterocyclyl), C₁-C₁₀ haloalkyl, C₁-C₁₀ haloalkoxy, -N(C₀₋₁₀ alkyl)(C₀₋₁₀ alkyl), -N(C₀₋₁₀ alkyl)(C₃-C₁₀ cycloalkyl), -N(C₀₋₁₀ alkyl)CO(C₀₋₁₀ alkyl), -N(C₀₋₁₀ alkyl)CON(C₀₋₁₀ alkyl), -N(C₀₋₁₀ alkyl)SO₂(C₀₋₁₀ alkyl), -O(C₀₋₁₀ alkyl), -O(C₃-C₁₀ cycloalkyl), -S(C₀₋₁₀ alkyl), -S(C₃-C₁₀ cycloalkyl), -SO(C₀₋₁₀ alkyl), -SO₂(C₀₋₁₀ alkyl), -SO₂(C₃-C₁₀ cycloalkyl), -SO₂N(C₀₋₁₀ alkyl)(C₀₋₁₀ alkyl), -SO₂N(C₀₋₁₀ alkyl)(C₃-C₁₀ cycloalkyl), -COO(C₀₋₁₀ alkyl), -OCO(C₀₋₁₀ alkyl), -CON(C₀₋₁₀ alkyl)(C₀₋₁₀ alkyl), -CON(C₀₋₁₀ alkyl)(C₃-C₁₀ cycloalkyl), -CO(C₀₋₁₀ alkyl), and -Si(C₀₋₁₀ alkyl)(C₀₋₁₀ alkyl)(C₀₋₁₀ alkyl);
preferably, ring J is a 6- to 12-membered saturated or partially unsaturated spiro heterocyclic ring, and at least one ring atom thereof is N.

2. The compound according to claim 1, wherein 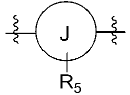 has the following structure: 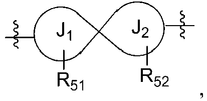 wherein
ring J₁ is a 3- to 8-membered monocyclic carbocyclic or heterocyclic ring;
ring J₂ is a 3- to 8-membered monocyclic carbocyclic or heterocyclic ring;
R₅₁ and R₅₂ are each one or more independent substituents on ring J₁ and ring J₂, and are independently selected from:
H, D, C₁-C₁₀ alkyl, -(C₀-C₆ alkylene)-(C₃-C₁₀ cycloalkyl), -(C₀-C₆ alkylene)-(C₆-C₁₀ aryl), -(C₀-C₆ alkylene)-(4- to 10-membered heterocyclyl), halogen, cyano, nitro, azido, C₁-C₁₀ haloalkyl, C₁-C₁₀ haloalkoxy, -N(C₀₋₁₀ alkyl)(C₀₋₁₀ alkyl), -N(C₀₋₁₀ alkyl)(C₃-C₁₀ cycloalkyl), -N(C₀₋₁₀ alkyl)CO(C₀₋₁₀ alkyl), -N(C₀₋₁₀ alkyl)CON(C₀₋₁₀ alkyl), -N(C₀₋₁₀ alkyl)SO₂(C₀₋₁₀ alkyl), -O(C₀₋₁₀ alkyl), -O(C₃-C₁₀ cycloalkyl), -S(C₀₋₁₀ alkyl), -S(C₃-C₁₀ cycloalkyl), -SO(C₀₋₁₀ alkyl), -SO₂(C₀₋₁₀ alkyl), -SO₂(C₃-C₁₀ cycloalkyl), -SO₂N(C₀₋₁₀ alkyl)(C₀₋₁₀ alkyl), -SO₂N(C₀₋₁₀ alkyl)(C₃-C₁₀ cycloalkyl), - COO(C₀₋₁₀ alkyl), -OCO(C₀₋₁₀ alkyl), -CON(C₀₋₁₀ alkyl)(C₀₋₁₀ alkyl), -CON(C₀₋₁₀ alkyl)(C₃-C₁₀ cycloalkyl), -CO(C₀₋₁₀ alkyl), and -Si(C₀₋₁₀ alkyl)(C₀₋₁₀ alkyl)(C₀₋₁₀ alkyl), wherein H in the C₀-C₆ alkylene, C₁-C₁₀ alkyl, C₂-C₁₀ alkenyl, C₂-C₁₀ alkynyl, C₀₋₁₀ alkyl, C₃-C₁₀ cycloalkyl, C₆-C₁₀ aryl, or 4- to 10-membered heterocyclyl may be optionally substituted with one or more R₀;
preferably, moiety 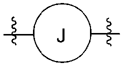 is selected from the following structures: 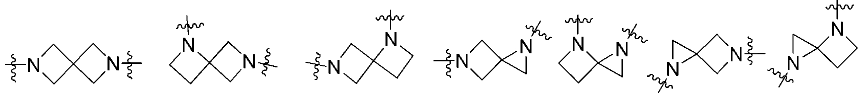 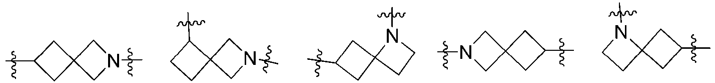 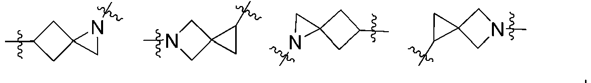 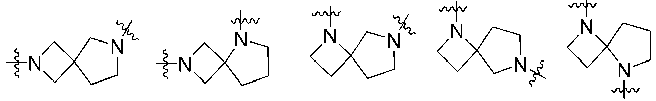 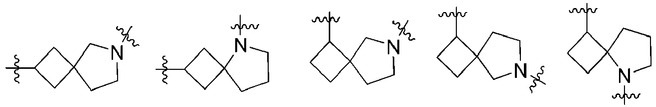 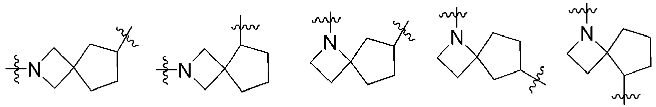 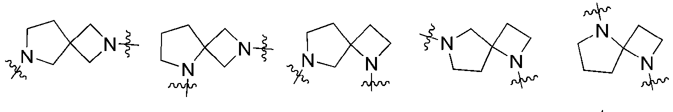 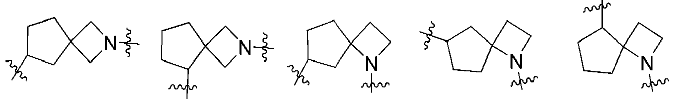 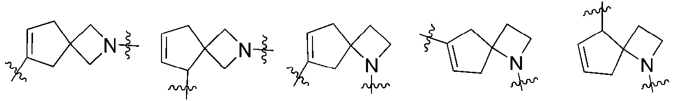 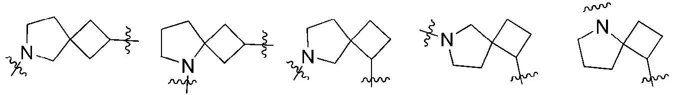 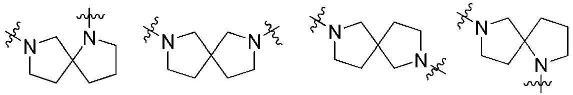 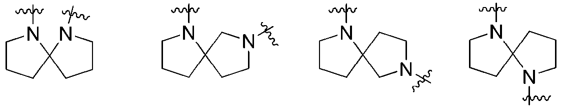 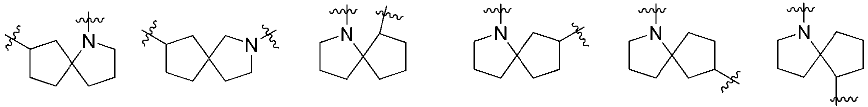 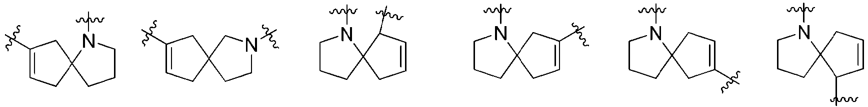 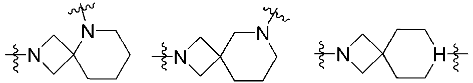 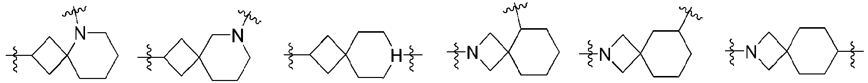 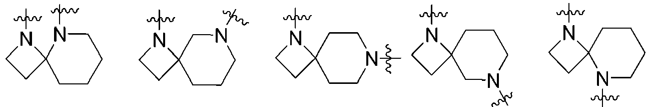 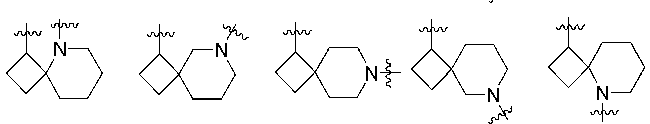 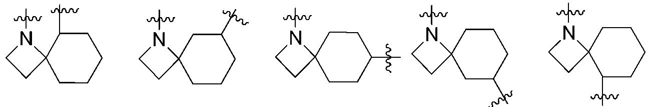 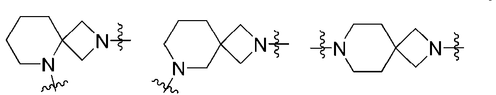 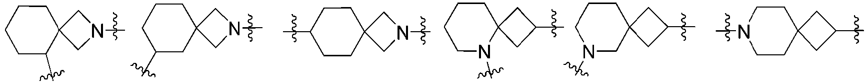 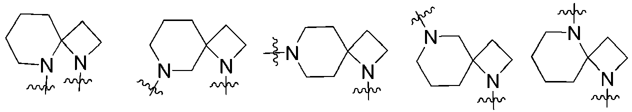 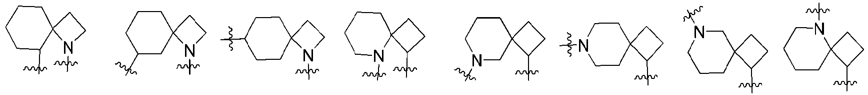 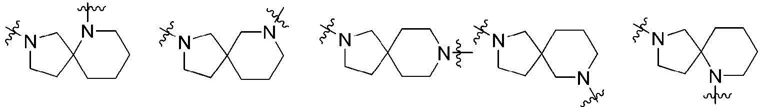 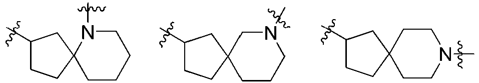 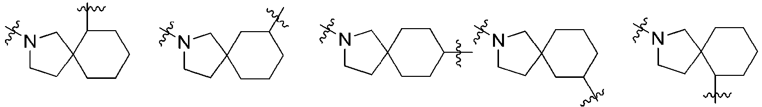 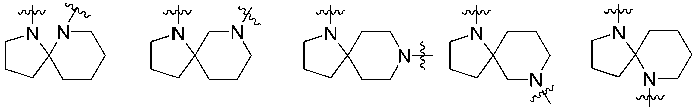 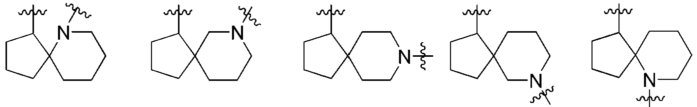 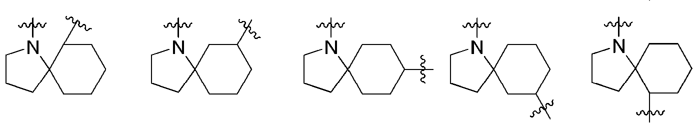 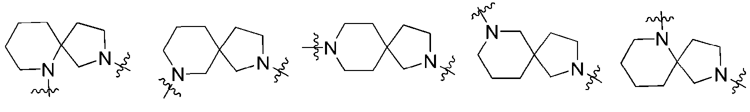 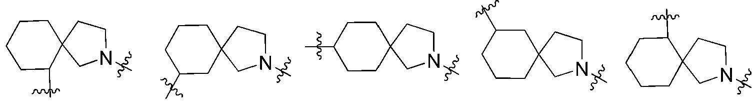 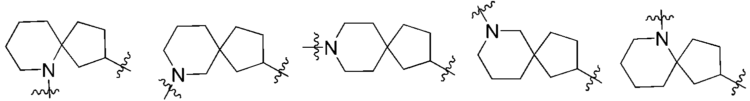 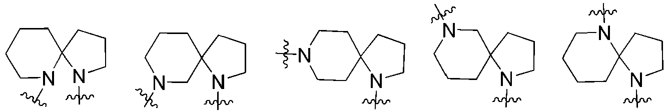 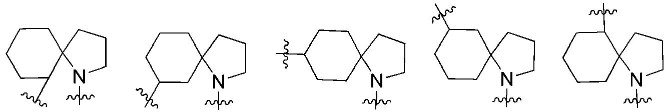 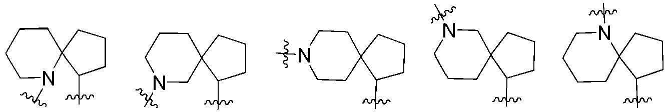 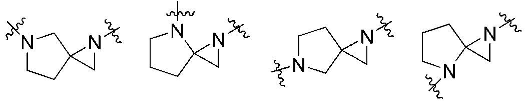 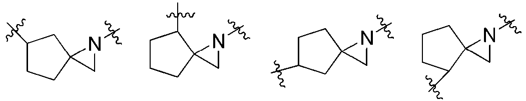 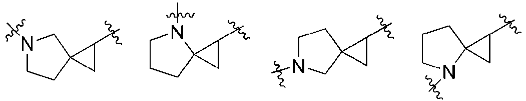 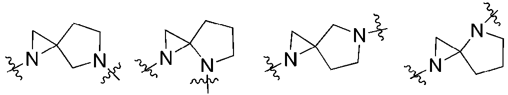 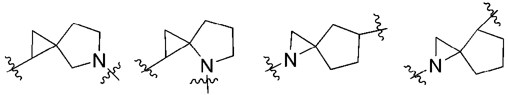 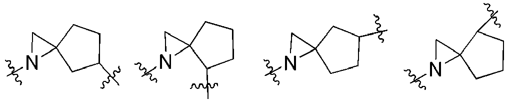 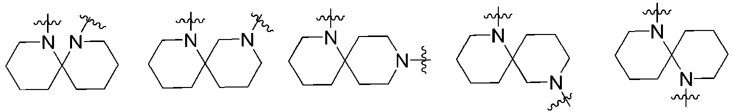 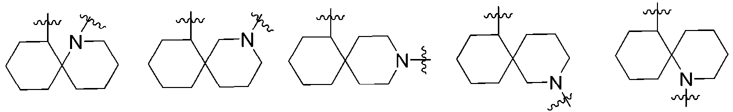 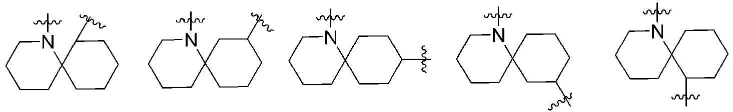 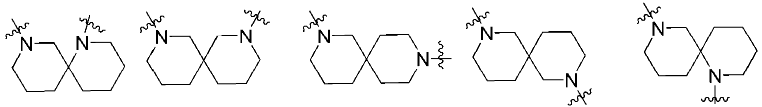 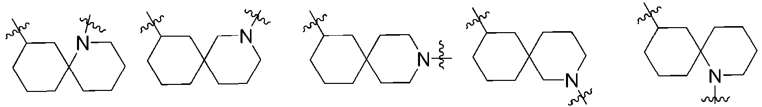 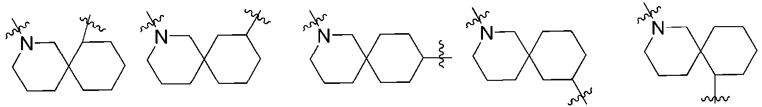 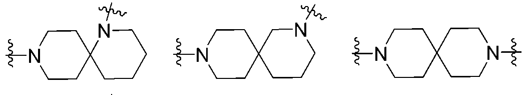 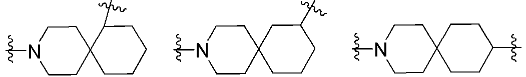 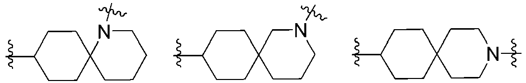 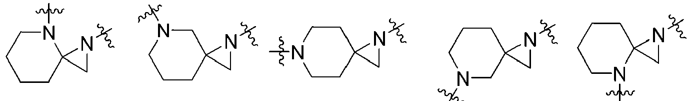 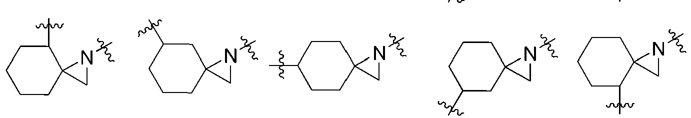 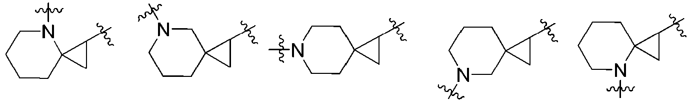 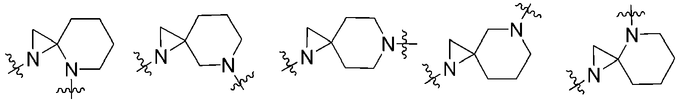 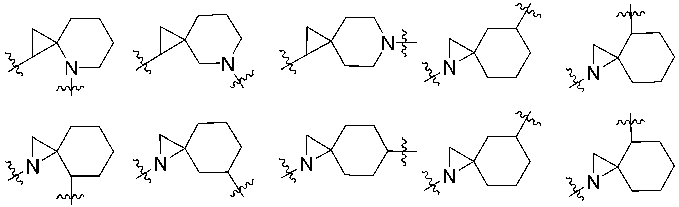
preferably, R₅₁ is selected from: H, halogen, hydroxy, sulfydryl, amino, C₁-C₄ alkyl, C₁-C₄ haloalkyl, C₁-C₄ hydroxy-substituted alkyl, -(C₀-C₃ alkylene)-(C₃-C₅ cycloalkyl), -O(C₁₋₃ alkyl), -S(C₁₋₃ alkyl), -N(H)(C₁₋₃ alkyl), and -N(C₁₋₃ alkyl)(C₁₋₃ alkyl);
preferably, R₅₂ is selected from: H, halogen, hydroxy, sulfydryl, amino, C₁-C₄ alkyl, C₁-C₄ haloalkyl, C₁-C₄ hydroxy-substituted alkyl, -(C₀-C₃ alkylene)-(C₃-C₅ cycloalkyl), -O(C₁₋₃ alkyl), -S(C₁₋₃ alkyl), -N(H)(C₁₋₃ alkyl), and -N(C₁₋₃ alkyl)(C₁₋₃ alkyl);
more preferably, 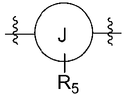 has the following structures:

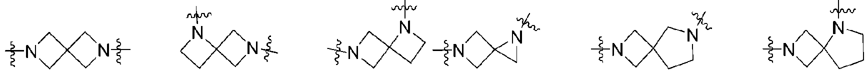

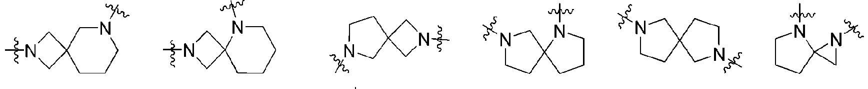

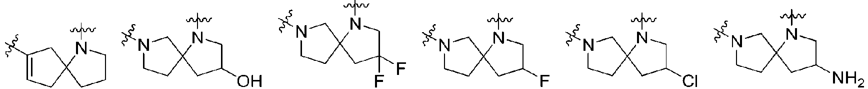

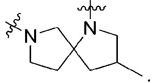

3. The compound according to claim 1 or 2, wherein ring 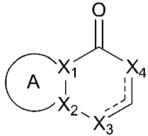 is 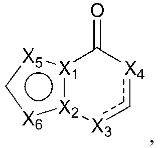 wherein X₅ and X₆ are independently selected from: C(H) and N;
preferably, ring 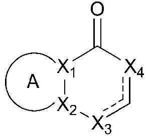 is 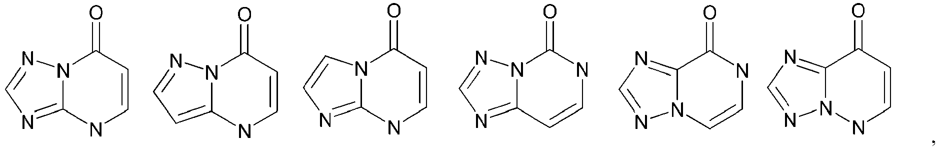 particularly

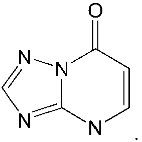

4. The compound according to any one of claims 1-3, wherein A₁ is 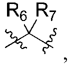 wherein R₆ and R₇ are independently selected from: H, D, C₁-C₁₀ alkyl, C₁-C₁₀ haloalkyl, -O(C₀₋₁₀ alkyl), -N(C₀₋₁₀ alkyl)(C₀₋₁₀ alkyl), and -COO(C₀₋₁₀ alkyl);
preferably, R₆ and R₇ are independently selected from: H, D, and C₁-C₃ alkyl;
preferably, R₃ and R₇, together with the atoms to which they are attached, form a 4- to 8-membered carbocyclic or heterocyclic ring (e.g., a 5- to 7-membered saturated heterocyclic ring), wherein H in the carbocyclic or heterocyclic ring may be optionally substituted with one or more R₀; more preferably, the carbocyclic or heterocyclic ring is optionally substituted with one or more groups selected from: H, D, halogen, cyano, hydroxy, sulfydryl, amino,C₁-C₆ alkyl, -(C₀-C₆ alkylene)-(C₃-C₆ cycloalkyl), -(C₀-C₆ alkylene)-(phenyl), -O(C₁-C₆ alkyl), -S(C₁-C₆ alkyl), - N(H)(C₁-C₆ alkyl), -N(C₁-C₆ alkyl)(C₁-C₆ alkyl), -CON(C₀-C₆ alkyl)(C₀-C₆ alkyl), -N(C₀-C₆ alkyl)CO(C₀-C₆ alkyl), -SO₂N(C₀-C₆ alkyl)(C₀-C₆ alkyl), and -N(C₀-C₆ alkyl)SO₂(C₀-C₆ alkyl), wherein H in the C₀-C₆ alkylene, C₀-C₆ alkyl, C₁-C₆ alkyl, C₃-C₆ cycloalkyl, or phenyl may be optionally substituted with a group selected from: D, halogen, cyano, hydroxy, sulfydryl, amino, C₁-C₃ alkyl, and C₁-C₃ alkoxy.

5. The compound according to any one of claims 1-4, wherein A₂ is -C(O)N(R₈)- or -S(O)₂N(R₈)-, and R₈ is selected from: H, C₁-C₆ alkyl, and -(C₀-C₃ alkylene)-(C₃-C₆ cycloalkyl);
preferably, R₈ is H.

6. The compound according to any one of claims 1-5, wherein R₁ has the following structure: 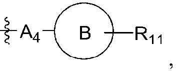 wherein:
A₄ is selected from: a single bond and C₁-C₆ alkylene, wherein 0-3 methylene units in the C₁-C₆ alkylene are independently substituted with the following groups: -O-, -S-, -C(O)-, -C(S)-, -C(O)O-, -OC(O)-, -C(O)N(H)-, - N(H)C(O)-, -N(H)-, and -S(O)₂-;
ring B is a 4- to 10-membered carbocyclic or heterocyclic ring;
R₁₁ is one or more independent substituents on ring B and is selected from: H, D, C₁-C₁₀ alkyl, C₂-C₁₀ alkenyl, C₂-C₁₀ alkynyl, -(C₀-C₆ alkylene)-(C₃-C₁₀ cycloalkyl), -(C₀-C₆ alkylene)-(C₆-C₁₀ aryl), -(C₀-C₆ alkylene)-(4- to 10-membered heterocyclyl), halogen, cyano, nitro, azido, C₁-C₁₀ haloalkyl, C₁-C₁₀ haloalkoxy, -N(C₀₋₁₀ alkyl)(C₀₋₁₀ alkyl), -N(C₀₋₁₀ alkyl)CO(C₀₋₁₀ alkyl), -N(C₀₋₁₀ alkyl)CON(C₀₋₁₀ alkyl), -N(C₀₋₁₀ alkyl)SO₂(C₀₋₁₀ alkyl), -O(C₀₋₁₀ alkyl), -S(C₀₋₁₀ alkyl), -SO(C₀₋₁₀ alkyl), -SO₂(C₀₋₁₀ alkyl), -SO₂N(C₀₋₁₀ alkyl)(C₀₋₁₀ alkyl), -COO(C₀₋₁₀ alkyl), - OCO(C₀₋₁₀ alkyl), -CON(C₀₋₁₀ alkyl)(C₀₋₁₀ alkyl), and -CO(C₀₋₁₀ alkyl), wherein H in the C₀-C₆ alkylene, C₁-C₁₀ alkyl, C₂-C₁₀ alkenyl, C₂-C₁₀ alkynyl, C₀₋₁₀ alkyl, C₃-C₁₀ cycloalkyl, C₆-C₁₀ aryl, or 4- to 10-membered heterocyclyl may be optionally substituted with one or more R₀;
preferably, A₄ is a single bond;
preferably, moiety 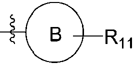 is selected from the following structures: 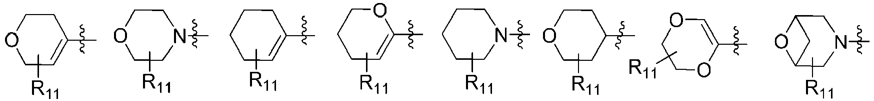 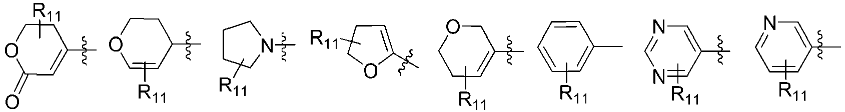 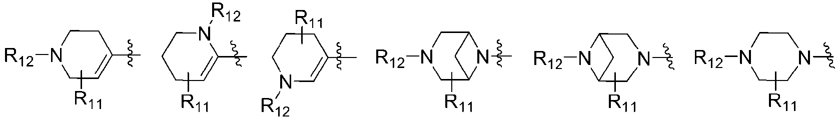 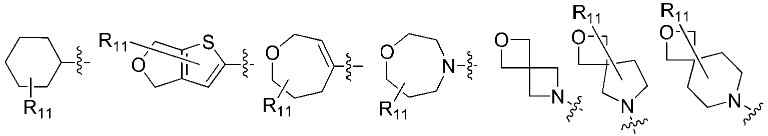 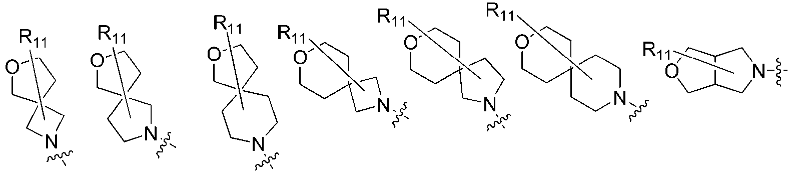 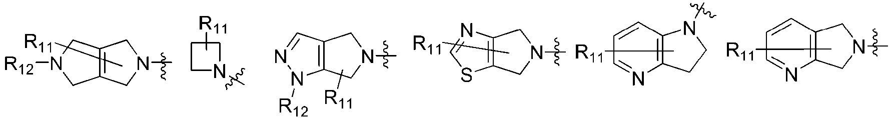 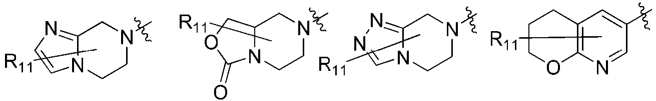
wherein R₁₂ is selected from: H, D, C₁-C₁₀ alkyl, C₂-C₁₀ alkenyl, C₂-C₁₀ alkynyl, -(C₀-C₆ alkylene)-(C₃-C₁₀ cycloalkyl), -(C₀-C₆ alkylene)-(C₆-C₁₀ aryl), -(C₀-C₆ alkylene)-(4- to 10-membered heterocyclyl), C₁-C₁₀ haloalkyl, C₁-C₁₀ haloalkoxy, -SO₂(C₀₋₁₀ alkyl), -SO₂N(C₀₋₁₀ alkyl)(C₀₋₁₀ alkyl), -COO(C₀₋₁₀ alkyl), -CON(C₀₋₁₀ alkyl)(C₀₋₁₀ alkyl), and -CO(C₀₋₁₀ alkyl), wherein H in the C₀-C₆ alkylene, C₁-C₁₀ alkyl, C₂-C₁₀ alkenyl, C₂-C₁₀ alkynyl, C₀₋₁₀ alkyl, C₃-C₁₀ cycloalkyl, C₆-C₁₀ aryl, or 4- to 10-membered heterocyclyl may be optionally substituted with one or more R₀.

7. The compound according to claim 6, wherein R₁₂ is selected from: H, C₁-C₄ alkyl, C₁-C₄ haloalkyl, -(C₁-C₄ alkylene)-C(O)OH, -(C₁-C₄ alkylene)-C(O)-(C₀₋₄ alkyl), -(C₁-C₄ alkylene)-C(O)O-(C₀₋₄ alkyl), and -CO(C₁₋₄ alkyl); preferably, R₁₂ is H,

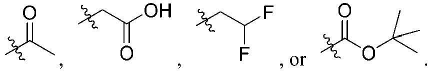

8. The compound according to claim 6, wherein each R₁₁ is independently selected from: H, halogen (e.g., F), C₁-C₄ alkyl, C₁-C₄ haloalkyl, -OH, C₁-C₄ alkoxy, C₁-C₄ haloalkoxy, -NH₂, -N(H)(C₁₋₄ alkyl), -N(H)(C₁₋₄ haloalkyl), -N(C₁₋₄ alkyl)(C₁₋₄ haloalkyl), -(C₁-C₄ alkylene)-C(O)OH, -(C₁-C₄ alkylene)-C(O)-(C₀₋₄ alkyl), -(C₁-C₄ alkylene)-C(O)O-(C₀₋₄ alkyl), and -CO(C₁₋₄ alkyl); preferably, R₁₁ is H, F, methyl, ethyl,

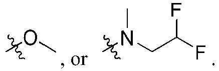

9. The compound according to claim 6, wherein moiety 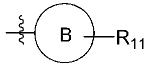 is selected from the following structures: 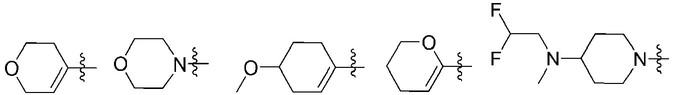 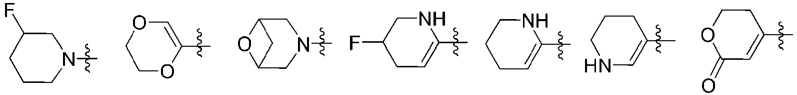 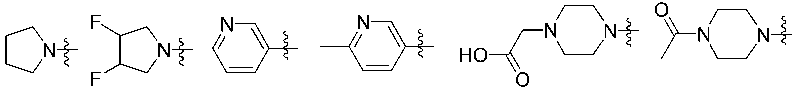 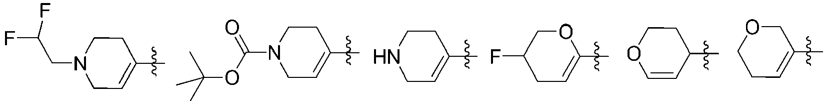 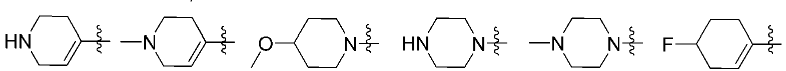 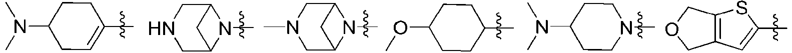 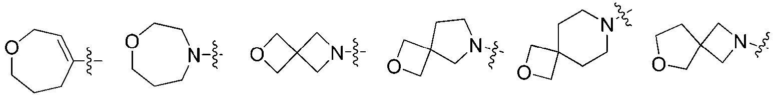 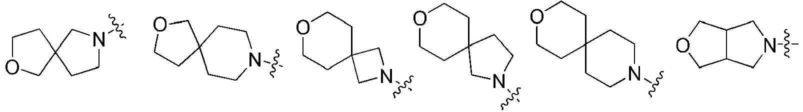 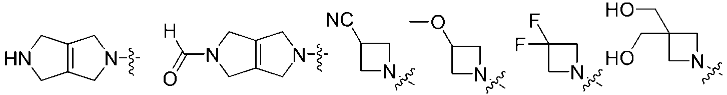 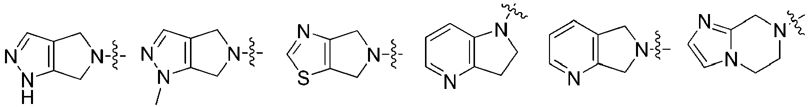 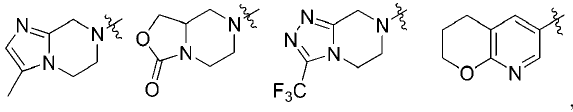 particularly

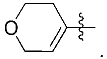

10. The compound according to any one of claims 1-9, wherein R₂ has the following structure: 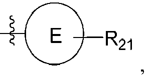 wherein:
ring E is a 4- to 12-membered carbocyclic or heterocyclic ring;
R₂₁ is one or more independent substituents on ring E and is selected from: H, D, C₁-C₁₀ alkyl, C₂-C₁₀ alkenyl, C₂-C₁₀ alkynyl, -(C₀-C₆ alkylene)-(C₃-C₁₀ cycloalkyl), -(C₀-C₆ alkylene)-(C₆-C₁₀ aryl), -(C₀-C₆ alkylene)-(4- to 10-membered heterocyclyl), halogen, cyano, nitro, azido, -SF₅, C₁-C₁₀ haloalkyl, C₁-C₁₀ haloalkoxy, -N(C₀₋₁₀ alkyl)(C₀₋₁₀ alkyl), -N(C₀₋₁₀ alkyl)CO(C₀₋₁₀ alkyl), -N(C₀₋₁₀ alkyl)CON(C₀₋₁₀ alkyl), -N(C₀₋₁₀ alkyl)SO₂(C₀₋₁₀ alkyl), -O(C₀₋₁₀ alkyl), -S(C₀₋₁₀ alkyl), -SO(C₀₋₁₀ alkyl), -SO₂(C₀₋₁₀ alkyl), -SO₂N(C₀₋₁₀ alkyl)(C₀₋₁₀ alkyl), -COO(C₀₋₁₀ alkyl), - OCO(C₀₋₁₀ alkyl), -CON(C₀₋₁₀ alkyl)(C₀₋₁₀ alkyl), and -CO(C₀₋₁₀ alkyl), wherein H in the C₀-C₆ alkylene, C₁-C₁₀ alkyl, C₂-C₁₀ alkenyl, C₂-C₁₀ alkynyl, C₀₋₁₀ alkyl, C₃-C₁₀ cycloalkyl, C₆-C₁₀ aryl, or 4- to 10-membered heterocyclyl may be optionally substituted with one or more R₀; or, two R₂₁, together with the ring atoms to which they are attached, form a carbocyclic or heterocyclic ring, wherein H in the carbocyclic or heterocyclic ring is optionally substituted with one or more R₀;
preferably, 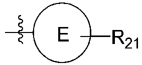 has the following structures: 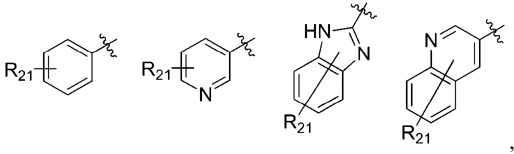 particularly 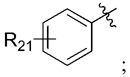
more preferably, R₂ has the following structure: 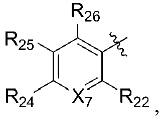 wherein
X₇ is selected from: C(R₂₃) and N;
R₂₂ to R₂₆ are independently selected from: H, halogen, cyano, -SF₅, hydroxy, sulfydryl, -C(O)H, C₁-C₆ alkyl, C₂-C₆ alkenyl, C₂-C₆ alkynyl, -(C₀-C₆ alkylene)-(C₃-C₆ cycloalkyl), -O(C₁₋₆ alkyl), -S(C₁₋₆ alkyl), and -CO(C₁₋₆ alkyl), wherein H in the C₁-C₆ alkyl, C₂-C₆ alkenyl, C₂-C₆ alkynyl, C₀-C₆ alkylene, or C₃-C₆ cycloalkyl may be optionally substituted with a group selected from: halogen, hydroxy, and C₁-C₃ alkoxy; or, two R₂₁, together with the ring atoms to which they are attached, form a carbocyclic or heterocyclic ring, wherein H in the carbocyclic or heterocyclic ring may be optionally substituted with a group selected from: halogen, hydroxy, and C₁-C₃ alkoxy.

11. The compound according to claim 10, wherein R₂₂ is selected from: H, halogen, cyano, hydroxy, C₁-C₆ alkyl, - (C₀-C₆ alkylene)-(C₃-C₆ cycloalkyl), and -O(C₁₋₆ alkyl), wherein H in the C₁-C₆ alkyl or C₃-C₆ cycloalkyl may be optionally substituted with a group selected from: halogen, hydroxy, and C₁-C₃ alkoxy;
preferably, R₂₃ is selected from: H and halogen;
preferably, R₂₄ is selected from: H, halogen, cyano, -SF₅, hydroxy, -C(O)H, C₁-C₆ alkyl, C₂-C₆ alkenyl, C₂-C₆ alkynyl, -(C₀-C₆ alkylene)-(C₃-C₆ cycloalkyl), -CO(C₁₋₆ alkyl), and -O(C₁₋₆ alkyl), wherein H in the C₁-C₆ alkyl or C₃-C₆ cycloalkyl may be optionally substituted with a group selected from: halogen, hydroxy, and C₁-C₃ alkoxy;
preferably, R₂₅ is selected from: H, halogen, C₁-C₆ alkyl, and C₁-C₆ haloalkyl;
preferably, R₂₆ is selected from: H, halogen, cyano, hydroxy, C₁-C₆ alkyl, and -O(C₁₋₆ alkyl).

12. The compound according to claim 10, wherein 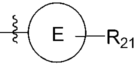 has the following structures: 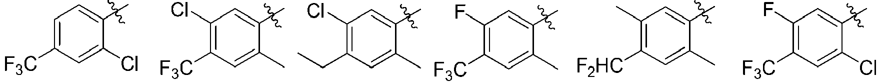 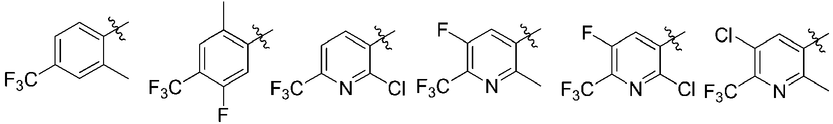 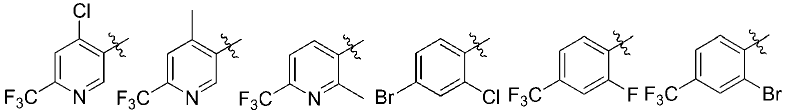 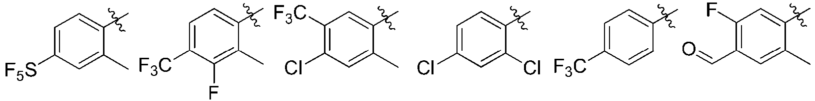 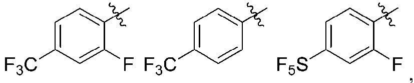 particularly

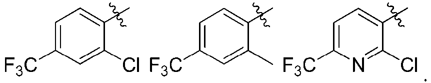

13. The compound according to any one of claims 1-12, wherein A₃ is selected from: -C(O)-, -S(O)-, -S(O)₂-, and 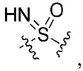 particularly -C(O)-.

14. The compound according to any one of claims 1-13, wherein R₄ has the following structure: 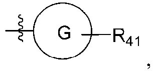 wherein:
ring G is a 4- to 10-membered carbocyclic or heterocyclic ring;
R₄₁ is one or more independent substituents on ring G and is selected from: H, D, C₁-C₁₀ alkyl, C₂-C₁₀ alkenyl, C₂-C₁₀ alkynyl, -(C₀-C₆ alkylene)-(C₃-C₁₀ cycloalkyl), -(C₀-C₆ alkylene)-(C₆-C₁₀ aryl), -(C₀-C₆ alkylene)-(4- to 10-membered heterocyclyl), halogen, cyano, nitro, azido, C₁-C₁₀ haloalkyl, C₁-C₁₀ haloalkoxy, -N(C₀₋₁₀ alkyl)(C₀₋₁₀ alkyl), -N(C₀₋₁₀ alkyl)CO(C₀₋₁₀ alkyl), -N(C₀₋₁₀ alkyl)CON(C₀₋₁₀ alkyl), -N(C₀₋₁₀ alkyl)SO₂(C₀₋₁₀ alkyl), -O(C₀₋₁₀ alkyl), -S(C₀₋₁₀ alkyl), -SO(C₀₋₁₀ alkyl), -SO₂(C₀₋₁₀ alkyl), -SO₂N(C₀₋₁₀ alkyl)(C₀₋₁₀ alkyl), -COO(C₀₋₁₀ alkyl), - OCO(C₀₋₁₀ alkyl), -CON(C₀₋₁₀ alkyl)(C₀₋₁₀ alkyl), and -CO(C₀₋₁₀ alkyl), wherein H in the C₀-C₆ alkylene, C₁-C₁₀ alkyl, C₂-C₁₀ alkenyl, C₂-C₁₀ alkynyl, C₀₋₁₀ alkyl, C₃-C₁₀ cycloalkyl, C₆-C₁₀ aryl, or 4- to 10-membered heterocyclyl may be optionally substituted with one or more R₀;
preferably, moiety 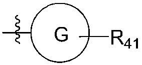 is selected from the following structures: 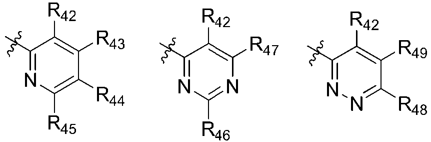
wherein R₄₃ to R₄₇ are independently selected from: H, halogen, cyano, hydroxy, sulfydryl, C₁-C₆ alkyl, C₂-C₆ alkenyl, C₂-C₆ alkynyl, -(C₀-C₆ alkylene)-(C₃-C₆ cycloalkyl), -O(C₁₋₆ alkyl), -O(C₃-C₆ cycloalkyl), -S(C₁₋₆ alkyl), -C(O)H, - CO(C₁₋₆ alkyl), and -NR₄₀₁R₄₀₂, wherein H in the C₀-C₆ alkylene, C₁-C₆ alkyl, or C₃-C₆ cycloalkyl may be optionally substituted with a group selected from: halogen, hydroxy, and C₁-C₃ alkoxy; R₄₀₁ and R₄₀₂ are independently selected from: H, C₁-C₆ alkyl, C₁-C₆ hydroxyalkyl, and C₁-C₆ alkoxyalkyl, or R₄₀₁ and R₄₀₂, together with the nitrogen atom to which they are attached, form a 4- to 8-membered heterocyclic ring, the heterocyclic ring being optionally substituted with a group selected from: halogen, hydroxy, and C₁-C₃ alkoxy;
R₄₂ is selected from: H, hydroxy, and protected hydroxy.

15. The compound according to claim 14, wherein R₄₂ is selected from: H, -OH, -O(C₁-C₆ alkyl), -O(benzyl), -O(p-methoxybenzyl), and -O(C₁-C₆ silyl), preferably -OH and H;
preferably, R₄₃ is selected from: H, halogen (e.g., F, Cl, or Br), C₁-C₄ alkyl (e.g., methyl or ethyl), C₁-C₄ haloalkyl (e.g., -CF₃, -CHF₂, or -CH₂F), C₁-C₄ alkoxy (e.g., methoxy), and C₁-C₄ haloalkoxy (e.g., -OCF₃, -OCHF₂, or -OCH₂F); preferably, R₄₄ is selected from: H, halogen (e.g., F, Cl, or Br), C₁-C₄ alkyl (e.g., methyl or ethyl), and C₁-C₄ haloalkyl (e.g., -CF₃, -CHF₂, or -CH₂F);
preferably, R₄₅ is selected from: H, halogen (e.g., F, Cl, or Br), C₁-C₄ alkyl (e.g., methyl or ethyl), and C₁-C₄ haloalkyl (e.g., -CF₃, -CHF₂, or -CH₂F);
preferably, R₄₆ is selected from: H, halogen (e.g., F, Cl, or Br), C₁-C₄ alkyl (e.g., methyl or ethyl), C₁-C₄ haloalkyl (e.g., -CF₃, -CHF₂, or -CH₂F), -S(C₁-C₄ alkyl) (e.g., -S-CH₃), -NH₂, -N(H)(C₁₋₆ alkyl), and -N(C₁₋₆ alkyl)(C₁₋₆ alkyl); preferably, R₄₇ is selected from: H, halogen (e.g., F, Cl, or Br), C₁-C₄ alkyl (e.g., methyl or ethyl), C₁-C₄ haloalkyl (e.g., -CF₃, -CHF₂, or -CH₂F), C₁-C₄ alkoxy (e.g., methoxy), C₁-C₄ haloalkoxy (e.g., -OCF₃, -OCHF₂, or -OCH₂F), and C₃-C₄ cycloalkyl (e.g., cyclopropyl);
preferably, R₄₈ and R₄₉ are independently selected from: H, halogen (e.g., F, Cl, or Br), C₁-C₄ alkyl (e.g., methyl or ethyl), C₁-C₄ haloalkyl (e.g., -CF₃, -CHF₂, or -CH₂F), C₁-C₄ alkoxy (e.g., methoxy), and C₁-C₄ haloalkoxy (e.g., - OCF₃, -OCHF₂, or -OCH₂F).

16. The compound according to claim 14, wherein 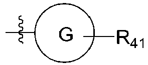 has the following structures: 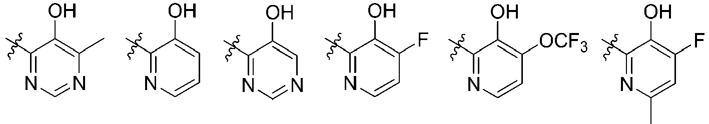 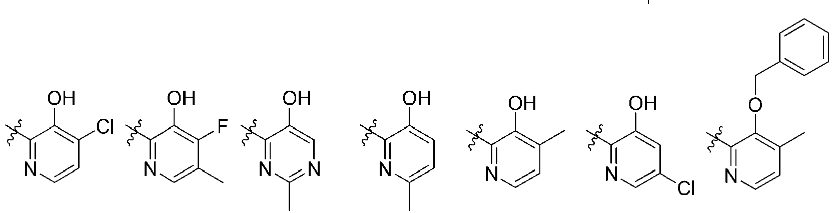 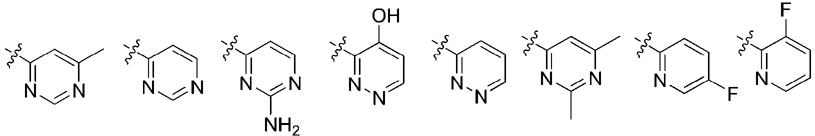 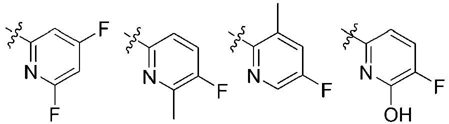 particularly

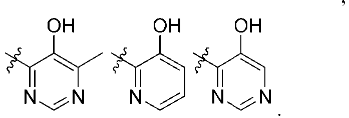

17. The compound according to any one of claims 1-16, wherein R₃ is selected from: H, halogen, hydroxy, sulfydryl, amino, C₁-C₆ alkyl, -(C₀-C₆ alkylene)-(C₃-C₆ cycloalkyl), -O(C₁₋₆ alkyl), -S(C₁₋₆ alkyl), -N(H)(C₁₋₆ alkyl), and -N(C₁₋₆ alkyl)(C₁₋₆ alkyl), wherein H in the C₀-C₆ alkylene, C₁-C₆ alkyl, or C₃-C₆ cycloalkyl may be optionally substituted with a group selected from: D, halogen, hydroxy, and C₁-C₃ alkoxy; 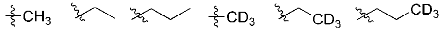 preferably, R₃ is selected from: H and

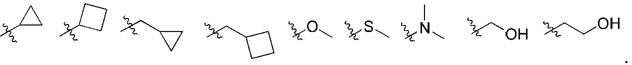

18. The compound according to any one of claims 1-17, wherein the compound has the following structure: 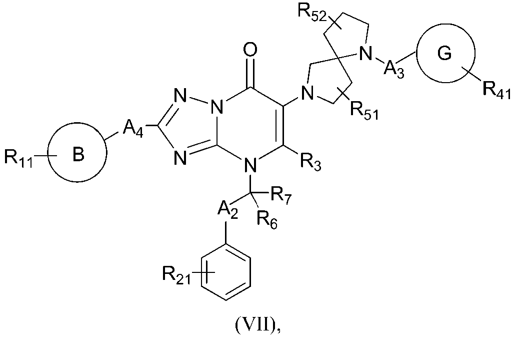 preferably 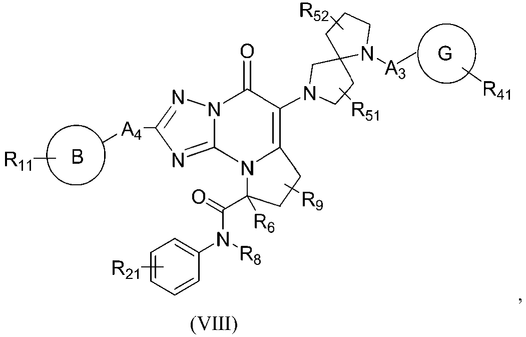
wherein R₉ is one or more independent substituents on ring Y and is selected from: H, D, halogen, cyano, hydroxy, sulfydryl, amino, C₁-C₆ alkyl, -(C₀-C₆ alkylene)-(C₃-C₆ cycloalkyl), -(C₀-C₆ alkylene)-(phenyl), -O(C₁-C₆ alkyl), - S(C₁-C₆ alkyl), -N(H)(C₁-C₆ alkyl), -N(C₁-C₆ alkyl)(C₁-C₆ alkyl), -CON(C₀-C₆ alkyl)(C₀-C₆ alkyl), -N(C₀-C₆ alkyl)CO(C₀-C₆ alkyl), -SO₂N(C₀-C₆ alkyl)(C₀-C₆ alkyl), and -N(C₀-C₆ alkyl)SO₂(C₀-C₆ alkyl), wherein H in the C₀-C₆ alkylene, C₀-C₆ alkyl, C₁-C₆ alkyl, C₃-C₆ cycloalkyl, or phenyl may be optionally substituted with a group selected from: D, halogen, cyano, hydroxy, sulfydryl, amino, C₁-C₃ alkyl, and C₁-C₃ alkoxy;
preferably, each R₉ is independently selected from: H, D, halogen, cyano, hydroxy, sulfydryl, amino, and C₁-C₆ alkyl, wherein the C₁-C₆ alkyl is optionally substituted with one or more groups selected from: D, halogen, cyano, hydroxy, amino, and C₁-C₃ alkoxy.

19. The compound according to any one of claims 1-18, wherein a stereoisomer of the compound has the following structures: 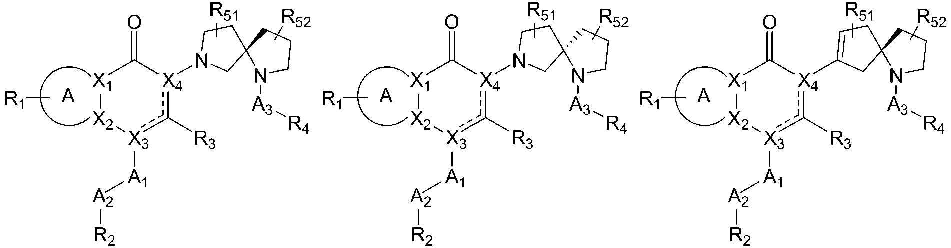 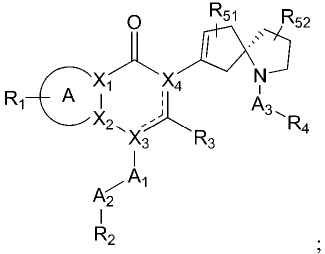 preferably, the stereoisomer of the compound is selected from the following structures: 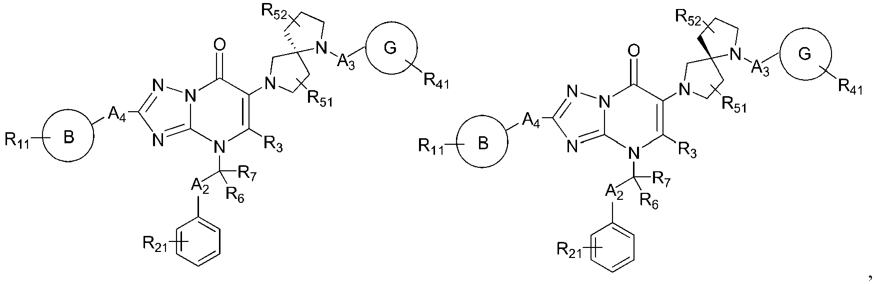 and

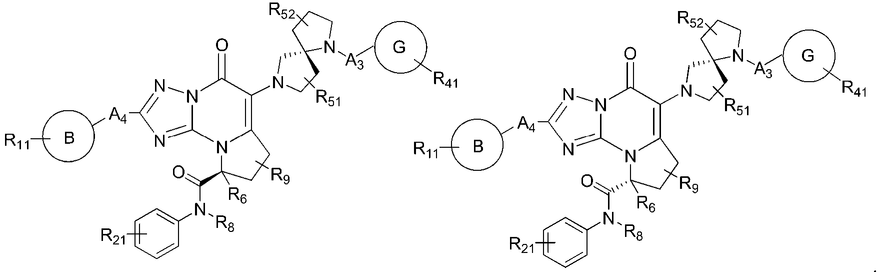

20. The compound according to claim 1, wherein the compound is selected from the following structures:

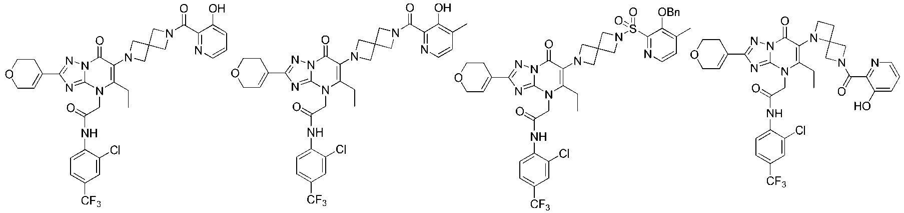

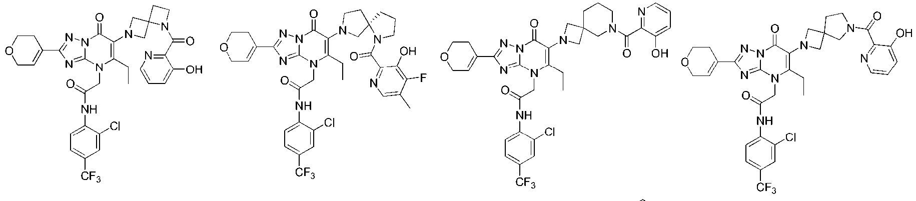

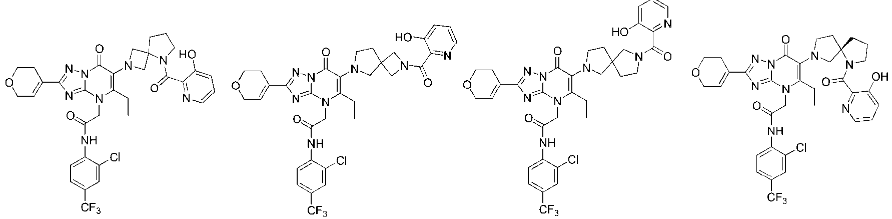

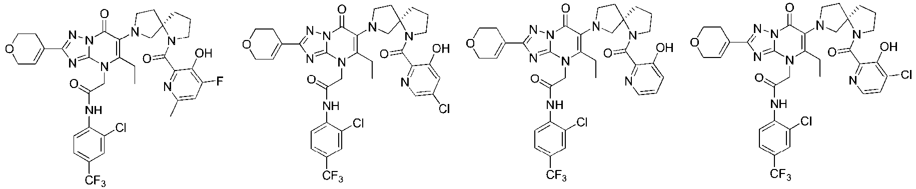

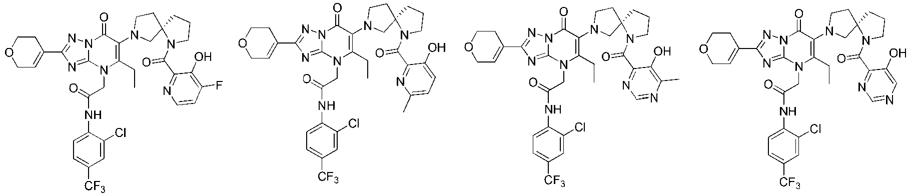

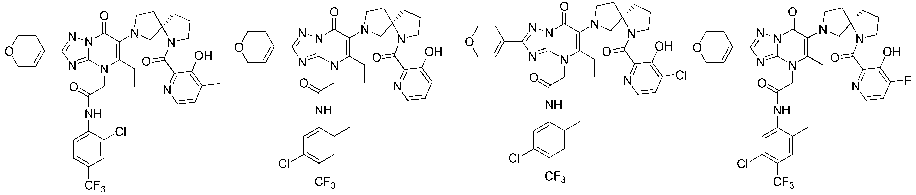

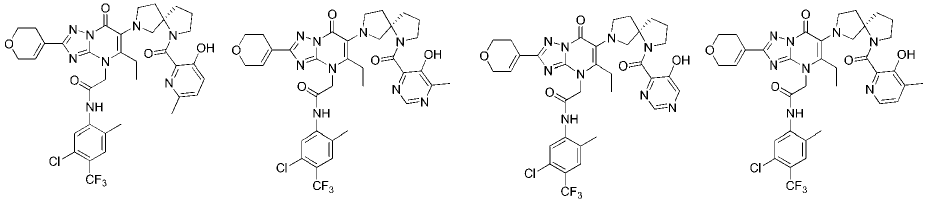

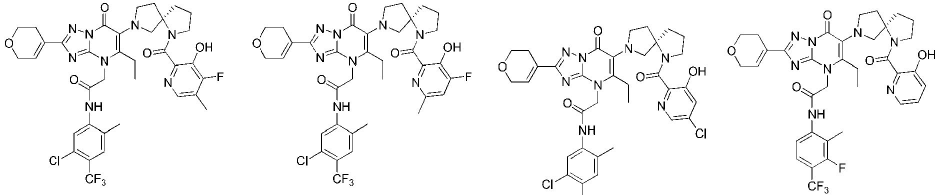

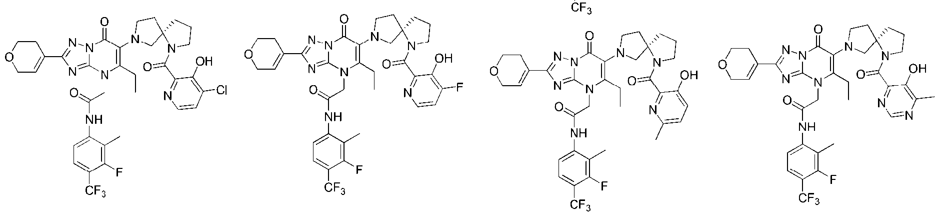

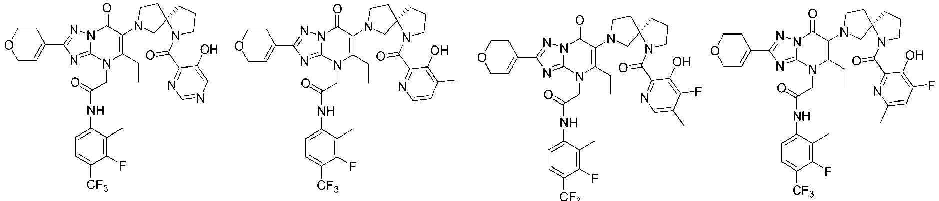

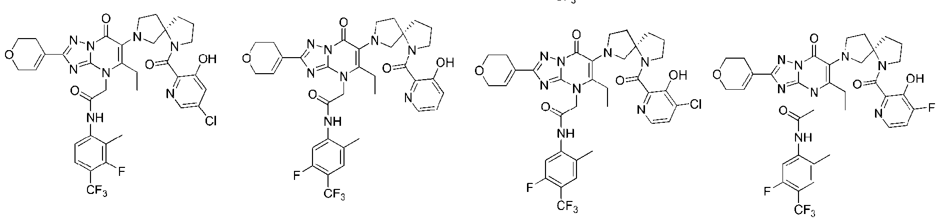

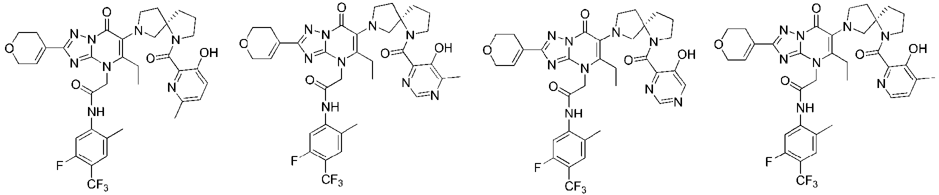

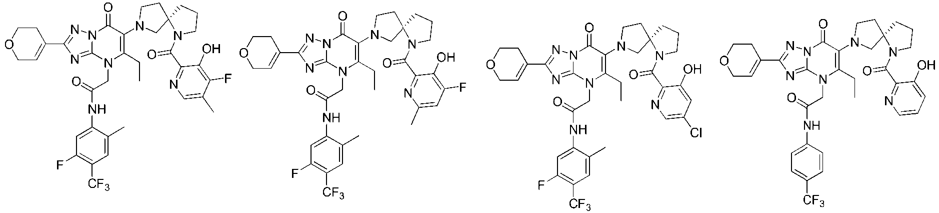

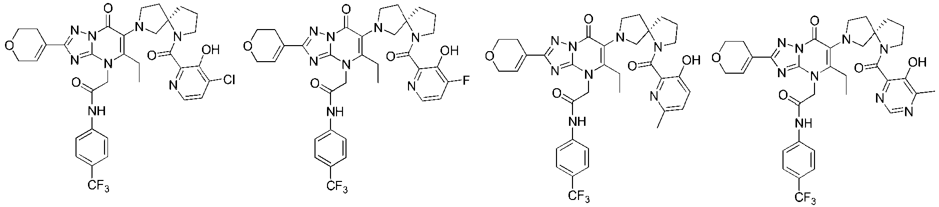

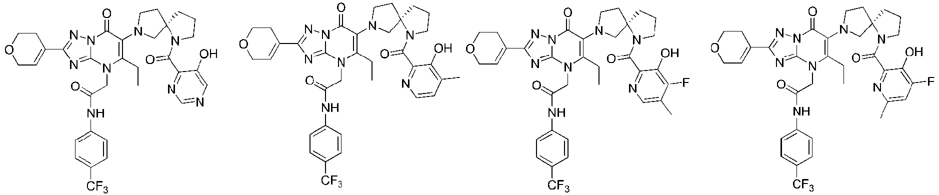

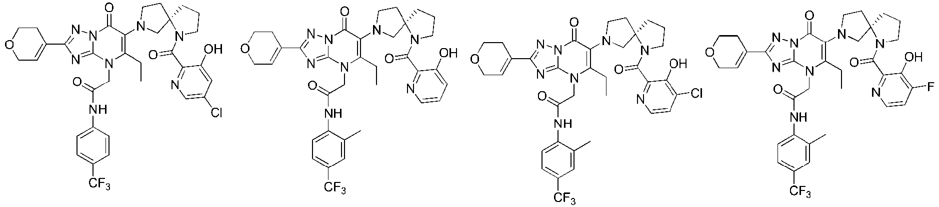

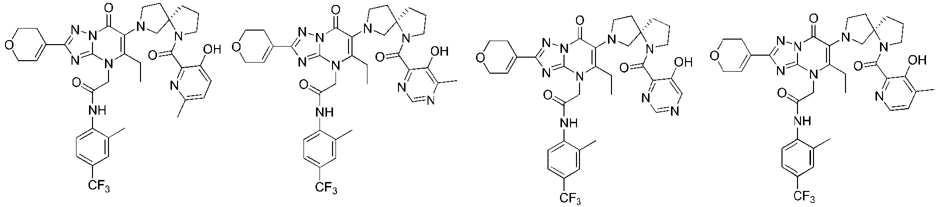

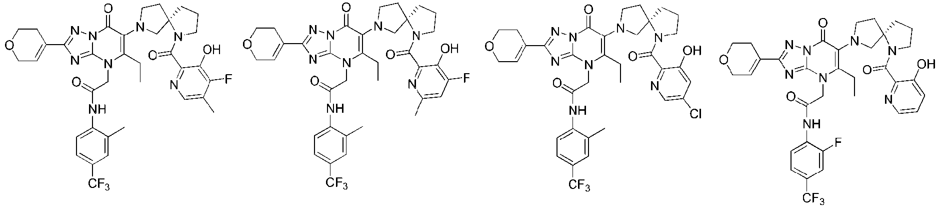

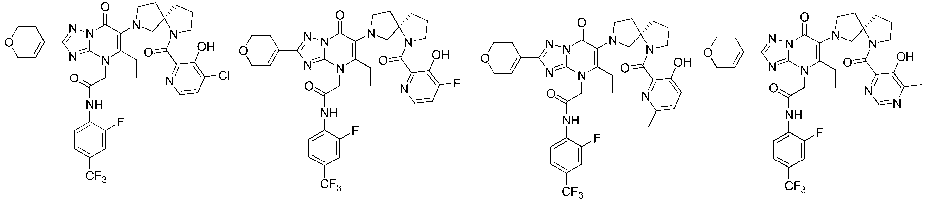

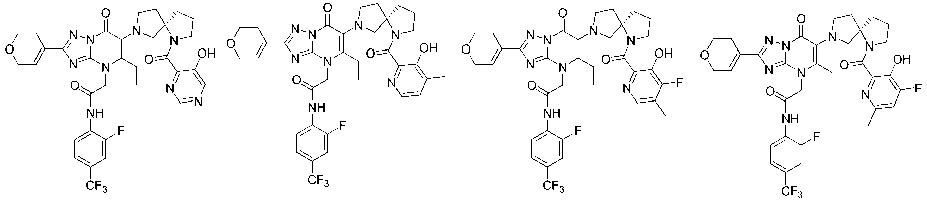

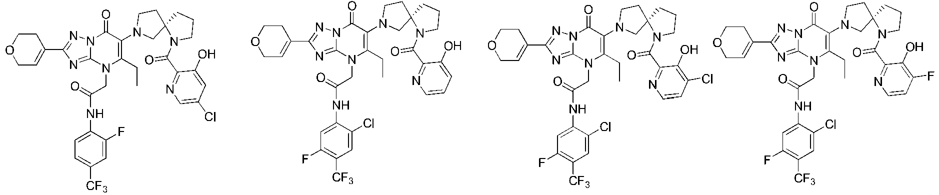

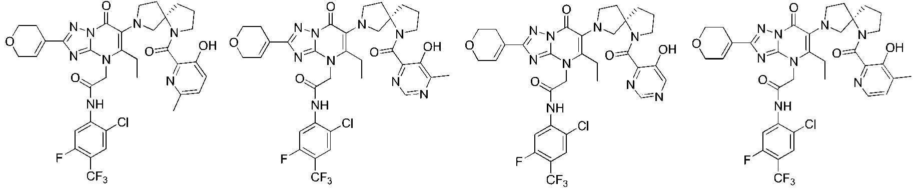

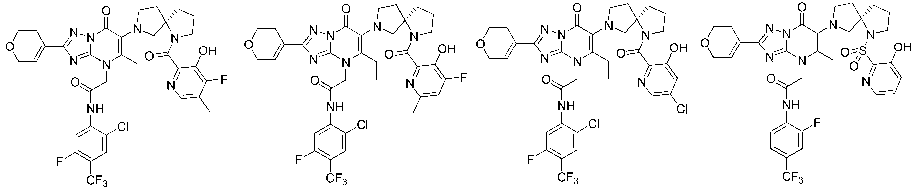

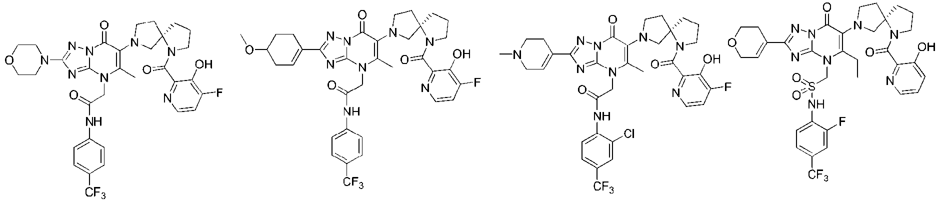

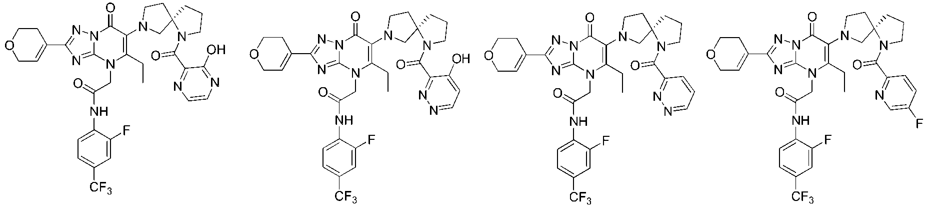

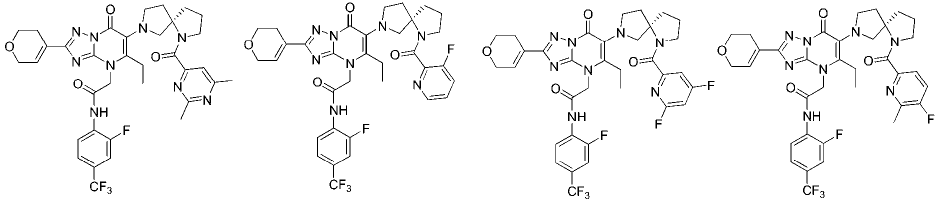

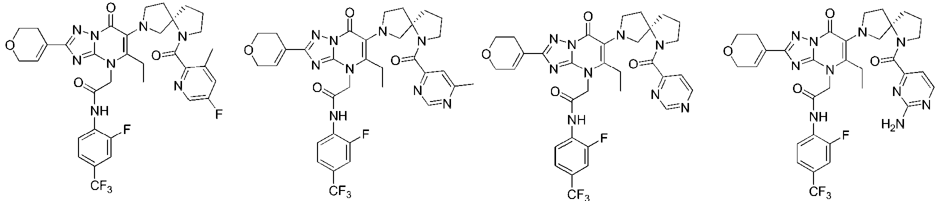

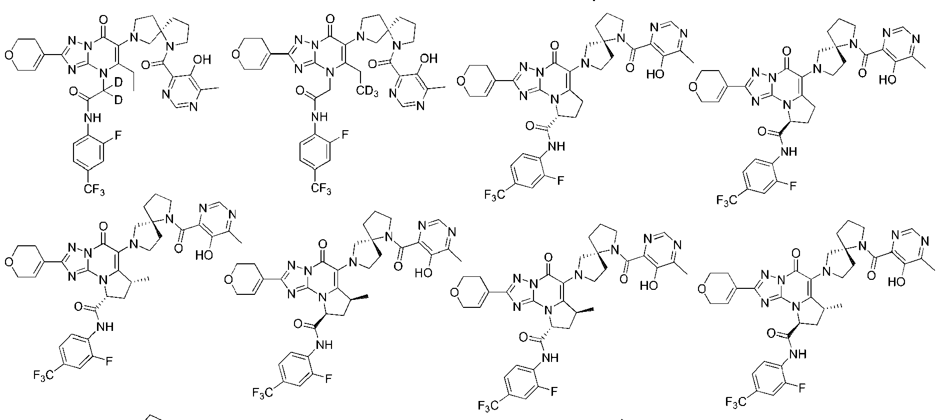

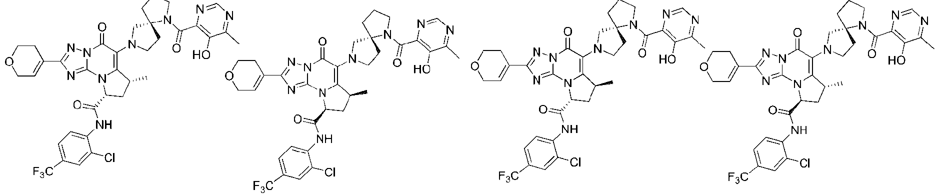

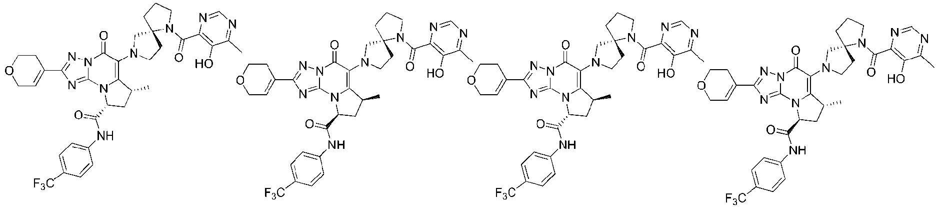

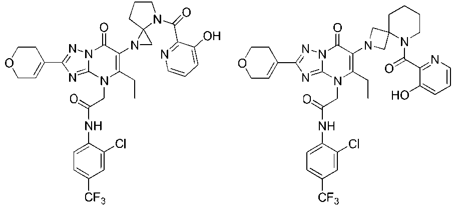

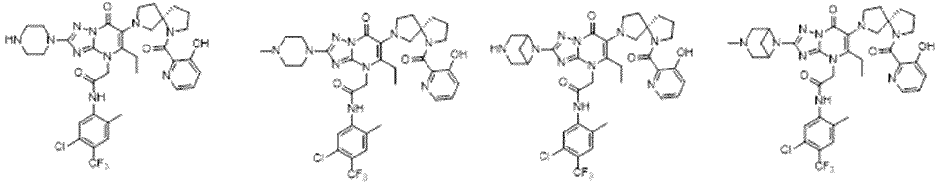

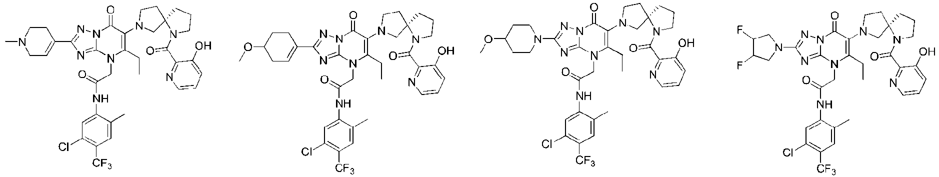

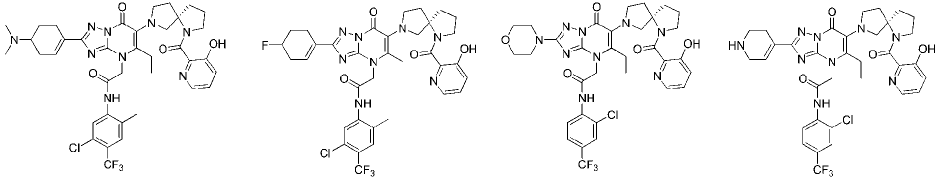

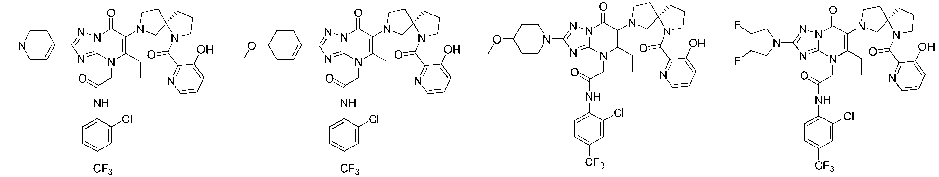

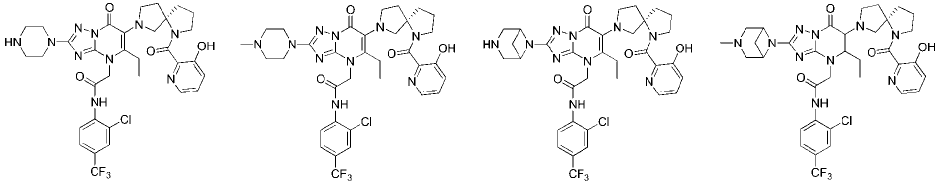

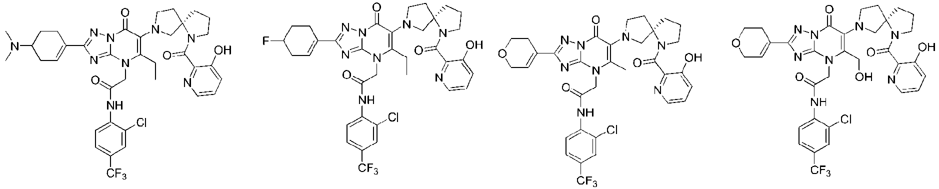

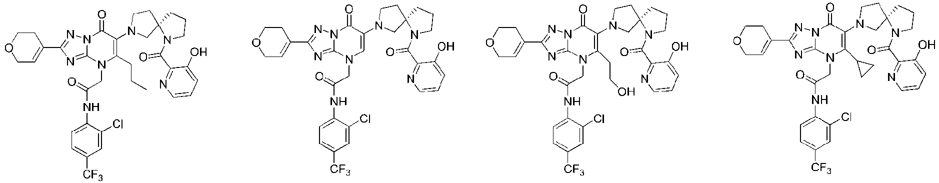

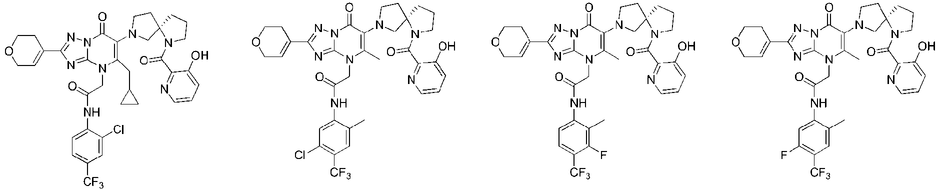

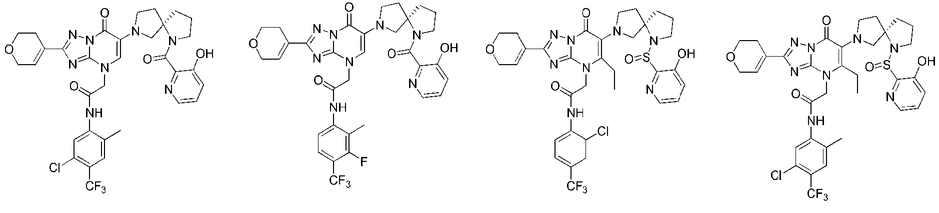

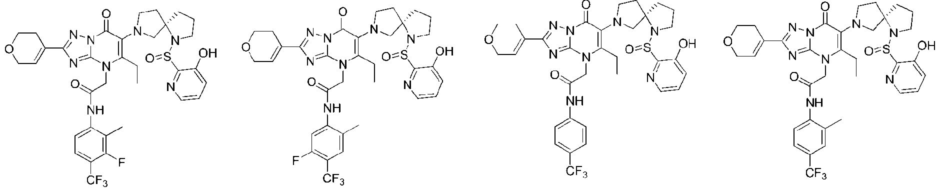

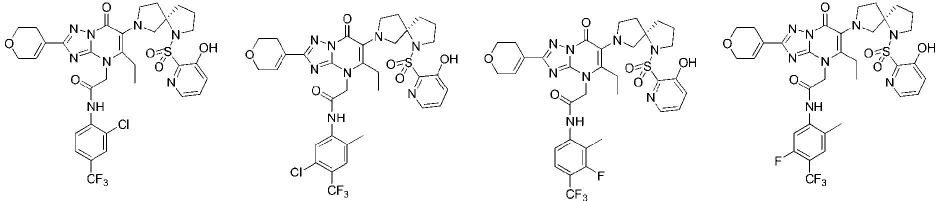

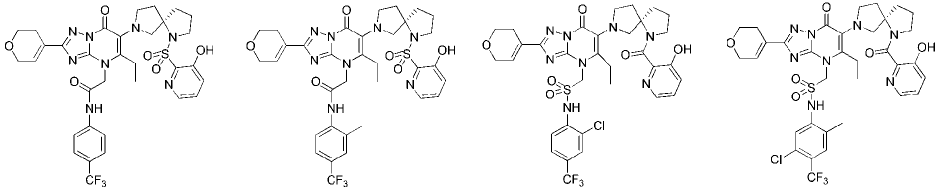

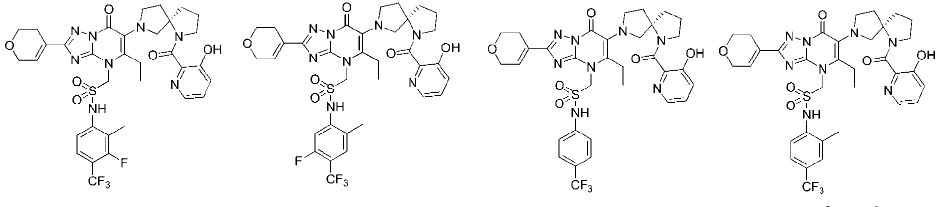

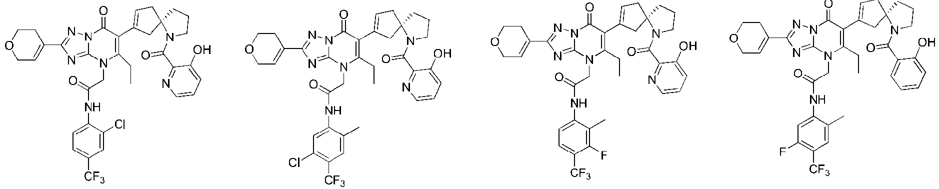

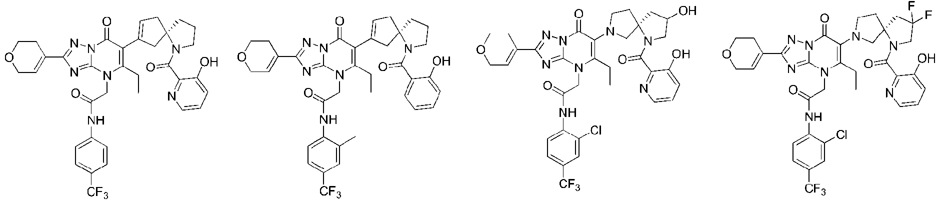

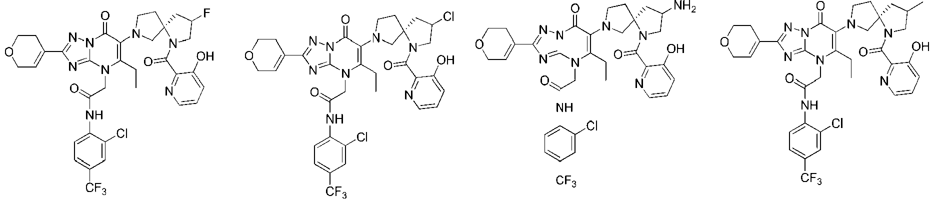

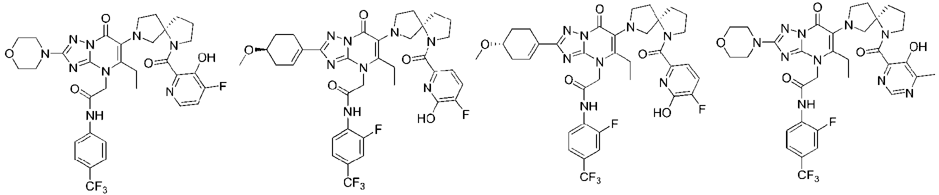

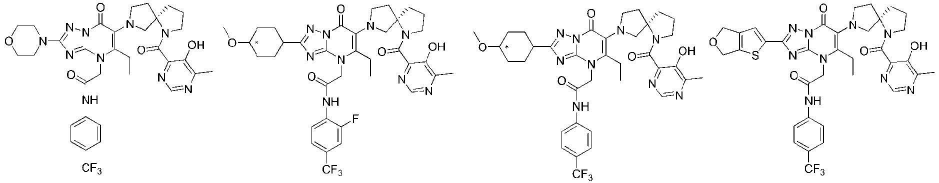

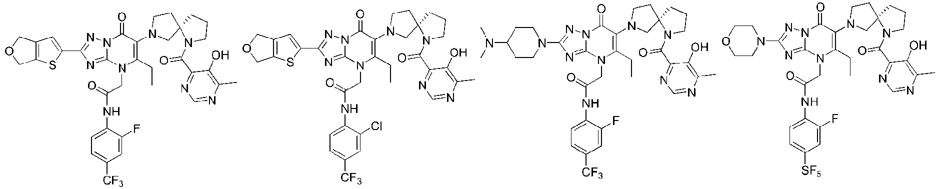

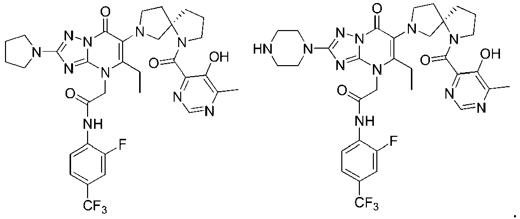

21. An intermediate of the compound according to any one of claims 1-20, wherein the intermediate has the following structure: 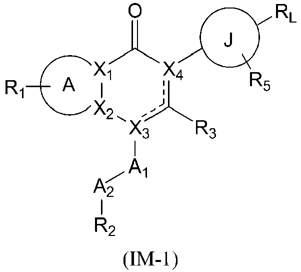
wherein R_{L} is a reactive group;
preferably, the intermediate is selected from the following structures: 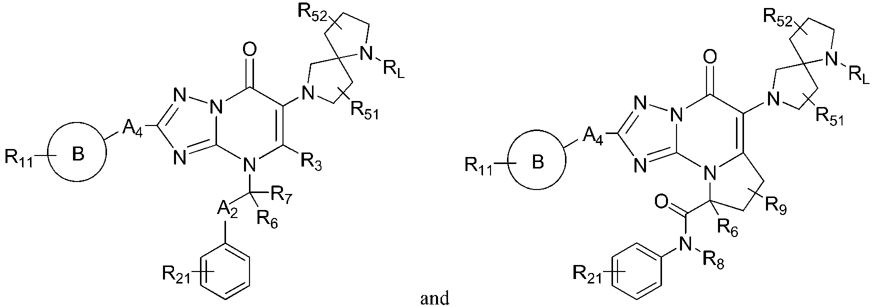
wherein R_{L} is H or an amino-protecting group;
more preferably, the intermediate is selected from the following structures:

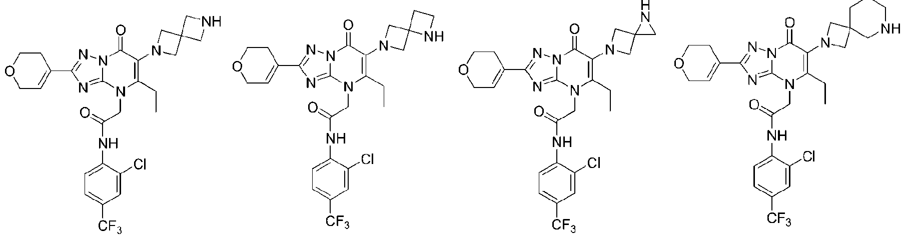

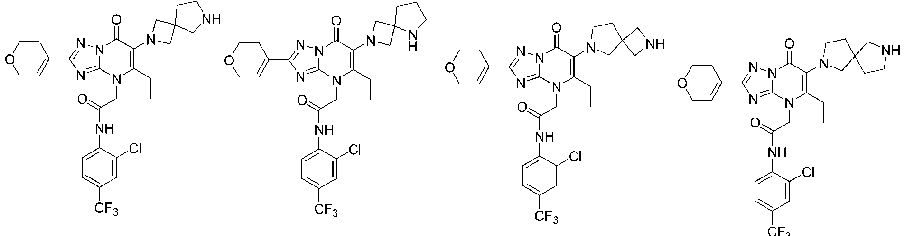

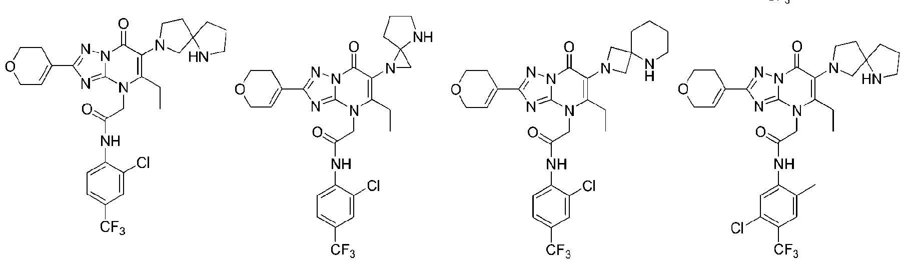

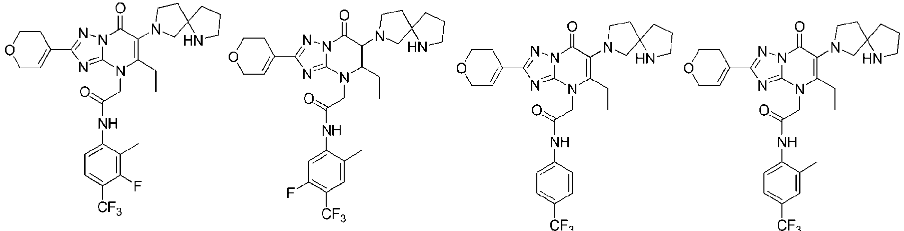

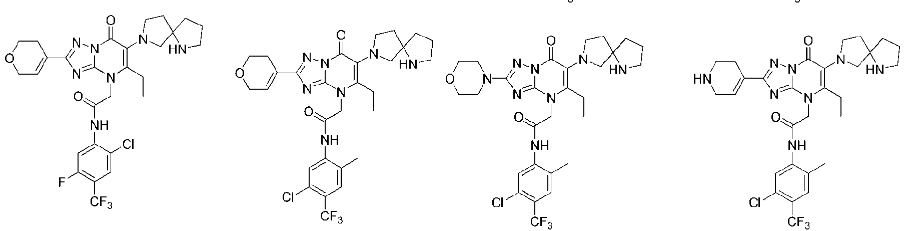

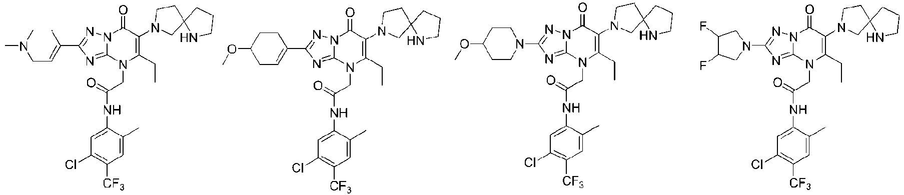

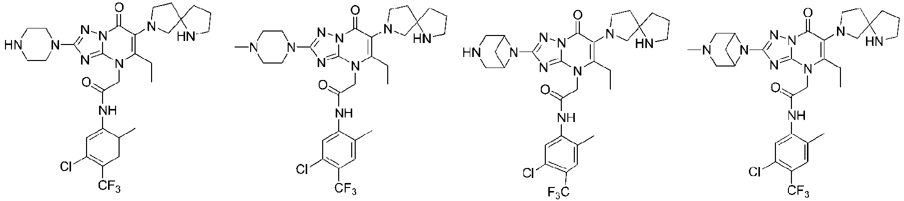

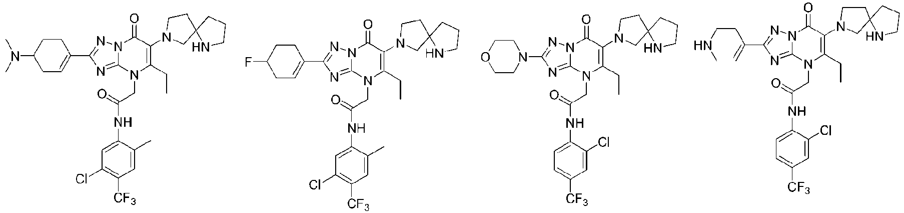

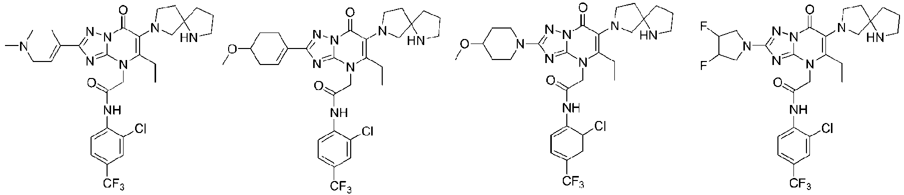

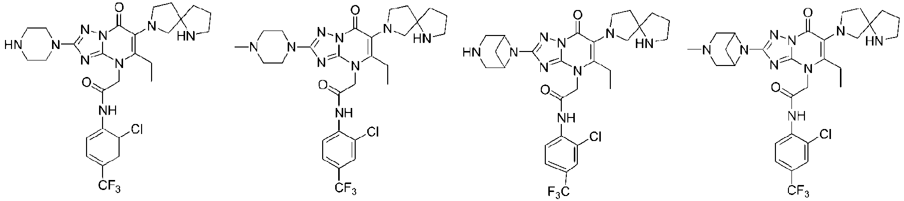

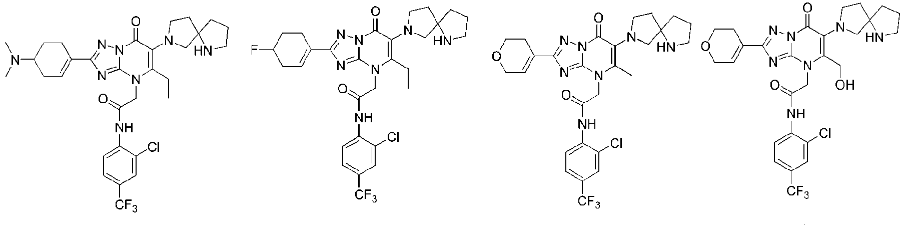

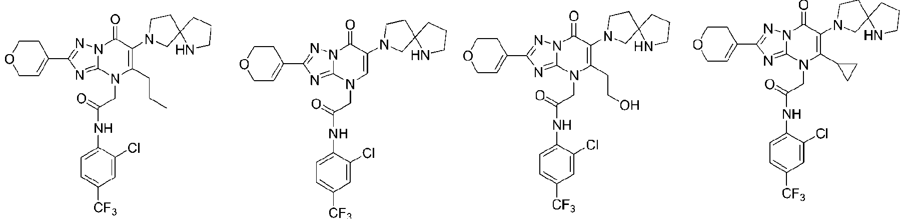

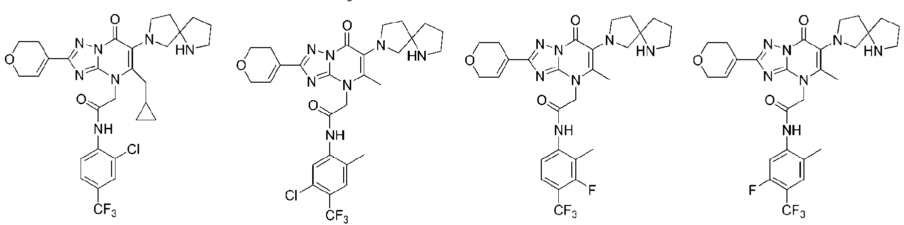

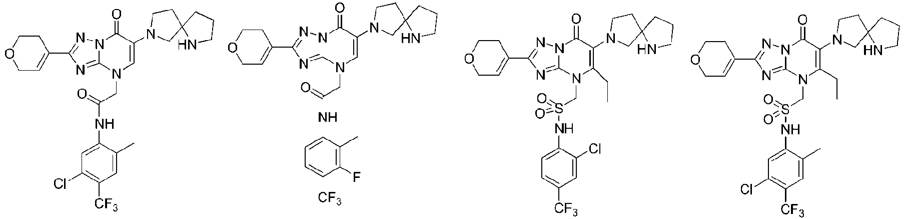

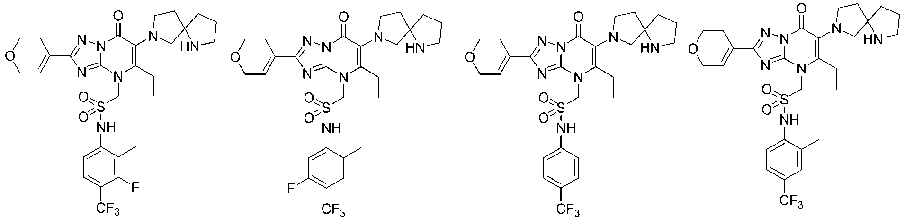

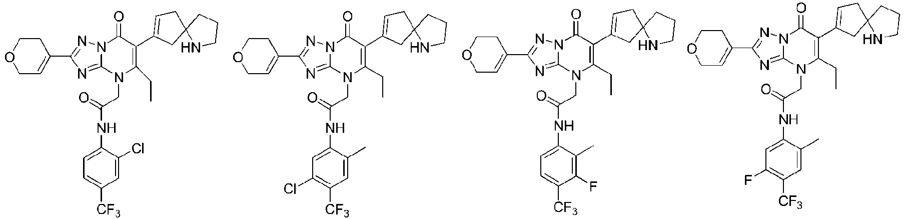

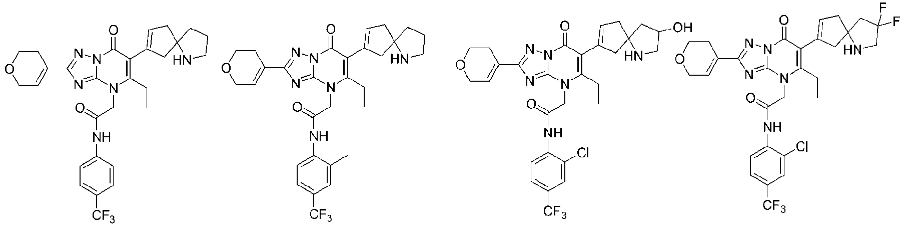

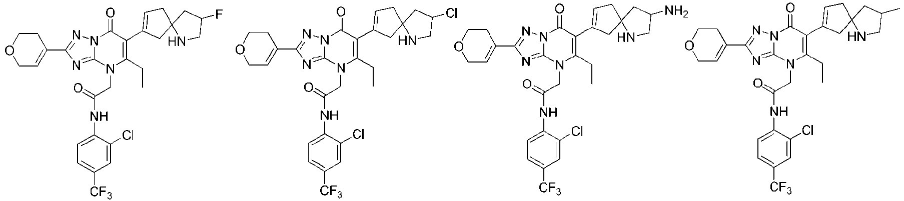

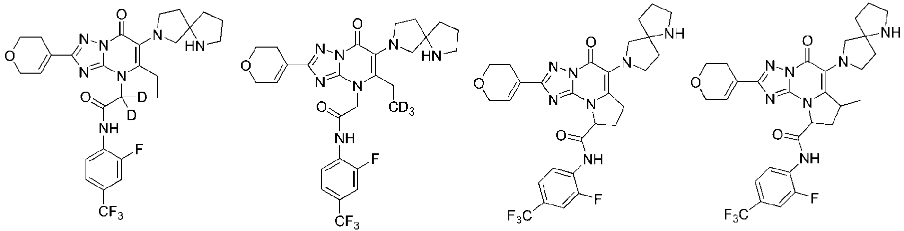

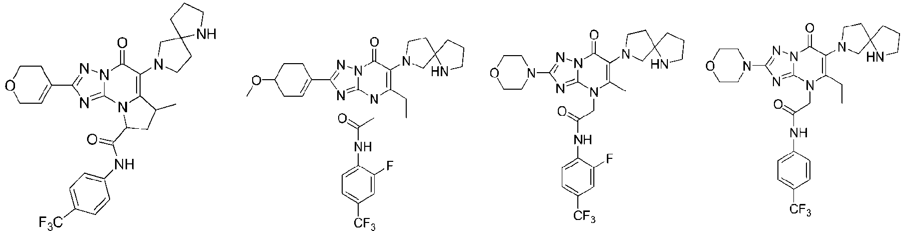

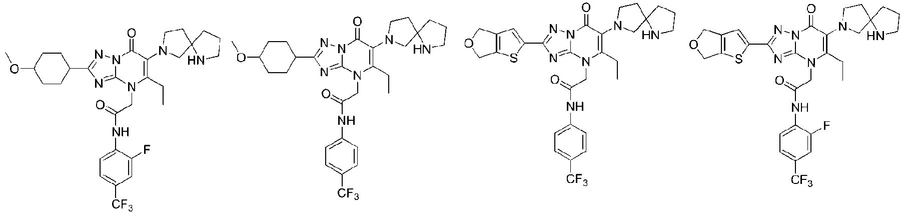

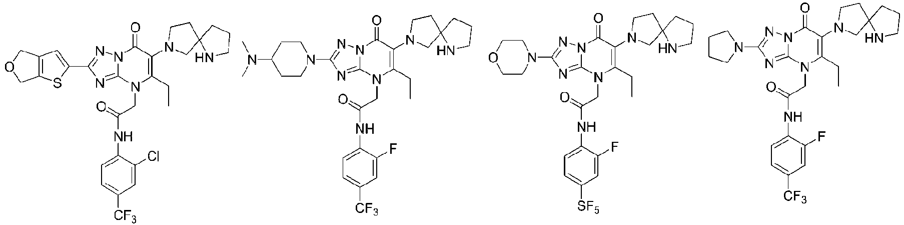

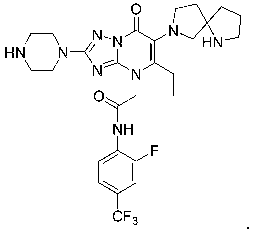

22. An intermediate of the compound according to any one of claims 1-20, wherein the intermediate has the following structures: 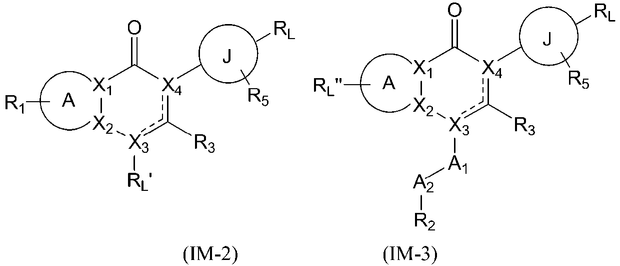
wherein R_{L}, R_{L}', and R_{L}" are independent reactive groups;
preferably, the intermediate has the following structures: 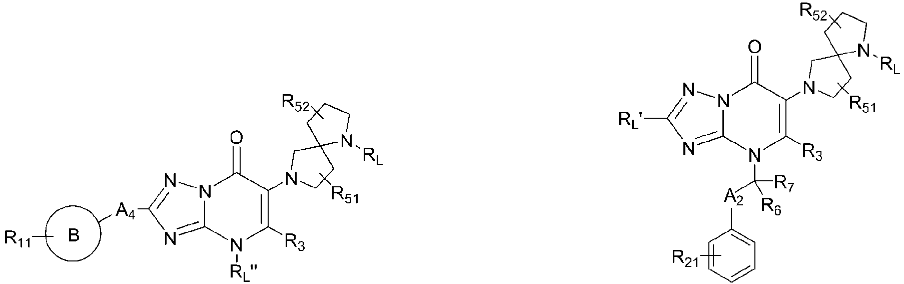 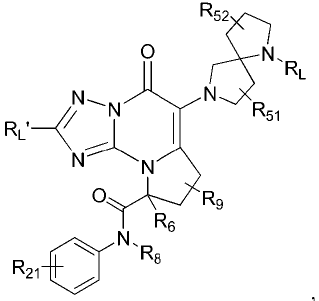
wherein R_{L} is H or an amino-protecting group, R_{L}' is a leaving group, and R_{L}" is H or an amino-protecting group.

23. A preparation method for the compound according to any one of claims 1-20, comprising a step of reacting a compound represented by formula IM-1 with 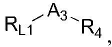 wherein R_{L1} is a leaving group, 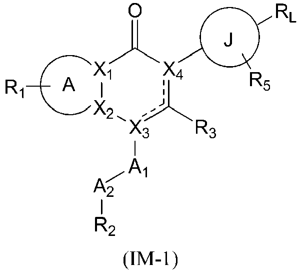 wherein R_{L} is a reactive group.

24. A combination, comprising the compound according to any one of claims 1-20 or a pharmaceutically acceptable salt, stereoisomer, ester, prodrug, solvate, or deuterated compound thereof, and one or more additional therapeutically active agents, wherein
preferably, the additional therapeutically active agent is an anti-cancer agent or a chemotherapeutic agent;
more preferably, the chemotherapeutic agent is selected from: anastrozole, bicalutamide, bleomycin sulfate, busulfan, capecitabine, N4-pentoxycarbonyl-5-deoxy-5-fluorocytidine, carboplatin, carmustine, chlorambucil, cisplatin, cladribine, cyclophosphamide, cytarabine, cytosine arabinoside, cytarabine liposome injection, dacarbazine, dactinomycin, daunorubicin hydrochloride, daunorubicin citrate liposome injection, dexamethasone, docetaxel, doxorubicin hydrochloride, etoposide, fludarabine phosphate 5-fluorouracil, flutamide tezacitibine, gemcitabine, hydroxyurea, idarubicin, ifosfamide, irinotecan, L-asparaginase, calcium folinate, melphalan, 6-mercaptopurine, methotrexate, mitoxantrone, gemtuzumab, paclitaxel, phoenix, pentostatin, polifeprosan 20 with carmustine implant, tamoxifen citrate, teniposide, 6-thioguanine, thiotepa, tirapazamine, topotecan hydrochloride for injection, vinblastine, vincristine, and vinorelbine, particularly irinotecan;
more preferably, the additional therapeutically active agent is a PD-1 inhibitor;
more preferably, the PD-1 inhibitor is selected from: PDR001, nivolumab, pembrolizumab, pidilizumab, MEDI0680, cemiplimab, dostarlimab, PF-06801591, tislelizumab, BGB-108, INCSHR1210, balstilimab, sintilimab, toripalimab, camrelizumab, AMP-224, penpulimab, zimberelimab, and prolgolimab, particularly PDR001, and more particularly tislelizumab.

25. A pharmaceutical composition, comprising the compound according to any one of claims 1-20 or a pharmaceutically acceptable salt, stereoisomer, ester, prodrug, solvate, or deuterated compound thereof, and one or more pharmaceutically acceptable excipients.

26. Use of the compound according to any one of claims 1-20 or a pharmaceutically acceptable salt, stereoisomer, ester, prodrug, solvate, or deuterated compound thereof in preparing a medicament for preventing and/or treating a WRN-mediated disease, wherein
preferably, the disease is a tumor, particularly a cancer with microsatellite instability (MSI);
more preferably, the tumor is selected from: acute myeloid leukemia, adolescent cancer, adrenocortical carcinoma of childhood, AIDS-related cancer, anal cancer, appendix cancer, astrocytoma, atypical teratoid tumor, basal cell carcinoma, bile duct cancer, bladder cancer, bone cancer, brain stem cell glioma, brain tumor, breast cancer, bronchial tumor, Burkitt lymphoma, carcinoid tumor, atypical teratoid tumor, embryonal tumor, blastoma, primary lymphoma, cervical cancer, childhood cancer, chordoma, cardiac tumor, chronic lymphocytic leukemia (CLL), chronic myeloid leukemia (CML), chronic myeloproliferative disorder, colon cancer, colorectal cancer, craniopharyngioma, cutaneous T-cell lymphoma, extrahepatic ductal carcinoma in situ (DCIS), embryonal tumor, CNS cancer, endometrial cancer, ependymoma, esophageal cancer, nasal glioma, Ewing sarcoma, extracranial blastoma, extragonadal blastoma, eye cancer, fibrous histiocytoma of bone, gallbladder cancer, stomach/gastric cancer, gastrointestinal carcinoid tumor, gastrointestinal stromal tumor (GIST), blastoma, gestational trophoblastic tumor, hairy cell leukemia, head and neck cancer, cardiac cancer, liver cancer, Hodgkin lymphoma, hypopharyngeal cancer, intraocular melanoma, islet cell tumor, pancreatic neuroendocrine tumor, renal cancer, laryngeal cancer, lip and oral cancer, liver cancer, lobular carcinoma in situ (LCIS), lung cancer, lymphoma, metastatic squamous neck cancer with occult primary, nuclear protein in testis carcinoma, oral cancer, multiple endocrine neoplasia syndrome, multiple myeloma/plasmacytoma, mycosis fungoides, myelodysplastic syndrome, myelodysplastic/myeloproliferative tumor, multiple myeloma, Merkel cell carcinoma, malignant mesothelioma, malignant fibrous histiocytoma of bone, and osteosarcoma, nasal and paranasal sinus cancer, nasopharyngeal cancer, neuroblastoma, non-Hodgkin lymphoma, non-small cell lung cancer (NSCLC), oral cancer, lip and oral cancer, oropharyngeal cancer, ovarian cancer, pancreatic cancer, papillomatosis, paraganglioma, paranasal sinus and nasal cancer, parathyroid cancer, penile cancer, pharyngeal cancer, pleuropulmonary blastoma, primary central nervous system (CNS) lymphoma, prostate cancer,
rectal cancer, transitional cell carcinoma, retinoblastoma, rhabdomyosarcoma, salivary gland cancer, skin cancer, stomach/gastric cancer, small cell lung cancer, small intestine cancer, soft tissue sarcoma, T-cell lymphoma, testicular cancer, throat cancer, thymoma and thymic carcinoma, thyroid cancer, transitional cell carcinoma of the renal pelvis and ureter, trophoblastic tumor, childhood rare cancer, urethral cancer, uterine sarcoma, vaginal cancer, vulvar cancer, and virus-induced cancer, particularly colorectal cancer, stomach/gastric cancer, ovarian cancer, endometrial tumor, and ovarian cancer;
preferably, the disease is a non-cancerous hyperproliferative disorder, such as benign hyperplasia of skin, restenosis, or prostatic hypertrophy.
